(19) Europäisches Patentamt
European Patent Office
Office européen des brevets

(11) **EP 4 295 760 A1**

(12) **EUROPEAN PATENT APPLICATION**

(43) Date of publication:
**27.12.2023 Bulletin 2023/52**

(21) Application number: **23180767.8**

(22) Date of filing: **21.06.2023**

(51) International Patent Classification (IPC):
*A61B 5/0507* (2021.01)    *A61B 5/11* (2006.01)
*A61B 5/00* (2006.01)      *G01S 7/41* (2006.01)
*G01S 13/00* (2006.01)     *G01S 13/42* (2006.01)
*G01S 13/50* (2006.01)     *G01S 13/66* (2006.01)
*G01S 13/88* (2006.01)     *G01S 13/46* (2006.01)

(52) Cooperative Patent Classification (CPC):
**A61B 5/0507; A61B 5/1113; A61B 5/6889;**
**G01S 7/006; G01S 7/415; G01S 13/003;**
**G01S 13/42; G01S 13/50; G01S 13/66;**
**G01S 13/88;** A61B 5/1117; A61B 5/113;
G01S 2013/462

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(30) Priority: **21.06.2022   US 202263354184 P**
**12.07.2022   US 202263388625 P**
**15.08.2022   US 202217888429**
**18.08.2022   US 202217891037**
**15.09.2022   US 202217945995**
**04.10.2022   US 202217959487**
**04.10.2022   US 202217960080**
**04.10.2022   PCT/US2022/045708**
**10.02.2023   US 202318108563**
**05.05.2023   US 202318144092**
**08.05.2023   US 202318144321**
**19.06.2023   US 202318211567**

(71) Applicants:
• **Origin Wireless, Inc.**
  **Greenbelt, MD 20770 (US)**
• **Ozturk, Muhammed Zahid**
  **Greenbelt, MD 20770 (US)**
• **Regani, Sai Deepika**
  **Greenbelt, MD 20770 (US)**
• **Hu, Yuqian**
  **Greenbelt, MD 20770 (US)**
• **Wang, Beibei**
  **Greenbelt, MD 20770 (US)**
• **Liu, K.J. Ray**
  **Greenbelt, MD 20770 (US)**
• **Han, Yi**
  **Greenbelt, MD 20770 (US)**

• **Claffey, David N.**
  **Greenbelt, MD 20770 (US)**
• **Lai, Hung-Quoc Duc**
  **Greenbelt, MD 20770 (US)**
• **Wang, Linghe**
  **Greenbelt, MD 20770 (US)**
• **Chen, Chun-I**
  **Greenbelt, MD 20770 (US)**
• **Shih, Chun-Chia Jack**
  **Greenbelt, MD 20770 (US)**
• **Chi-Lim Au, Oscar**
  **Greenbelt, MD 20770 (US)**

(72) Inventors:
• **OZTURK, Muhammed Zahid**
  **Greenbelt, MD Maryland 20770 (US)**
• **REGANI, Sai Deepika**
  **Greenbelt, MD Maryland 20770 (US)**
• **HU, Yuqian**
  **Greenbelt, MD Maryland 20770 (US)**
• **WANG, Beibei**
  **Greenbelt, MD Maryland 20770 (US)**
• **LIU, K.J. Ray**
  **Greenbelt, MD Maryland 20770 (US)**
• **HAN, Yi**
  **Greenbelt, MD Maryland 20770 (US)**
• **CLAFFEY, David N.**
  **Greenbelt, MD Maryland 20770 (US)**
• **LAI, Hung-Quoc Duc**
  **Greenbelt, MD Maryland 20770 (US)**
• **WANG, Linghe**
  **Greenbelt, MD Maryland 20770 (US)**
• **CHEN, Chun-I**
  **Greenbelt, MD Maryland 20770 (US)**
• **SHIH, Chun-Chia Jack**
  **Greenbelt, MD Maryland 20770 (US)**
• **CHI-LIM AU, Oscar**
  **Greenbelt, md Maryland 20770 (US)**

(74) Representative: **Boult Wade Tennant LLP**
**Salisbury Square House**

8 Salisbury Square
London EC4Y 8AP (GB)

(54)   **METHOD, APPARATUS, AND SYSTEM FOR WIRELESS MOTION MONITORING BASED ON CLASSIFIED SLIDING TIME WINDOWS**

(57)   Methods, apparatus and systems for wireless monitoring, sensing and localization are described. In one example, a described method comprises: transmitting a wireless signal through a wireless multipath channel of a venue, wherein the wireless multipath channel is impacted by a motion of an object in the venue; receiving the wireless signal through the wireless multipath channel, wherein the received wireless signal differs from the transmitted wireless signal due to the wireless multipath channel and the motion; obtaining a time series of channel information (TSCI) of the wireless multipath channel based on the received wireless signal; performing a classification of a sliding time window by analyzing channel information (CI) of the TSCI in the sliding time window; computing a motion information (MI) for the sliding time window based on the TSCI and the classification of the sliding time window; and monitoring the motion of the object based on the MI.

FIG. 3

**Description**

**TECHNICAL FIELD**

The present teaching generally relates to wireless monitoring, sensing and localization. More specifically, the present teaching relates to: improving accuracy and efficiency of the wireless monitoring based on motion information computation and time window classification; wireless sensing using multiple groups of wireless devices; and wireless monitoring and localization of objects in a venue based on correlation scores.

**BACKGROUND**

With the proliferation of Internet of Things (IoT) applications, billions of household appliances, phones, smart devices, security systems, environment sensors, vehicles and buildings, and other radio-connected devices will transmit data and communicate with each other or people, and everything will be able to be measured and tracked all the time. Among the various approaches to measure what is happening in the surrounding environment, wireless monitoring using wireless channel information (CI) has received a lot of attention in the era of IoT. However, CI, e.g. channel state information (CSI) or channel frequency information (CFI), may be interrupted by many factors, such as hardware imperfection, thermal noise, etc. In addition, there may be many outliers which, if without preprocessing, will affect the performance of wireless monitoring significantly.

In addition, wireless sensing has received an increasing attention in recent years because of the ubiquitous deployment of wireless radio devices. In addition, human activities affect wireless signal propagations, therefore understanding and analyzing the way how wireless signals react to human activities can reveal rich information about the activities. As more bandwidth becomes available in the new generation of wireless systems, wireless sensing will make many smart IoT applications only imagined today possible in the near future. That is because when the bandwidth increases, one can see many more multipaths, in a rich-scattering environment such as in indoors or metropolitan area, which can be treated as hundreds of virtual antennas/sensors. Because there may be many IoT devices available for wireless sensing, an efficient and effective method for making use multiple devices for wireless sensing is desirable.

Further, location services and localization technologies have become indispensable daily, from map navigation to social networking. The global positioning system (GPS) has reshaped human life for several decades and is considered a crucial technological milestone in modem society. However, although GPS meets users' needs for location services in outdoor scenarios, it cannot provide reliable location data in indoor conditions due to signal blockage. Therefore, indoor localization technology is becoming a hot topic in academic research. Indoor localization systems can be realized either actively or passively. Active indoor localization requires specialized devices attached to/carried by the human and localizes the target by continuously monitoring the signals from the devices. On the other hand, the passive indoor localization system usually relies on the perception of the sensors deployed in the environment. Therefore, it does not require the target to carry any devices. Such a design benefits multiple applications, including intruder detection, fall detection, and daily activity monitoring, where it is not possible/inconvenient to ask for the users' continuous cooperation.

Existing passive indoor localization operates in different resolutions serving different applications ranging from centimeter/decimeter level to the room-level or zone-level. While the former approaches aim to provide fine-grained indoor location information enabling applications such as indoor tracking, the latter focus on obtaining coarser location information that can provide behavior analysis and activity logs. As expected, fine-grained localization requires more hardware, complex infrastructure, calibration efforts, and user cooperation. Commercially, room-level localization can be achieved using different approaches, which include camera-based solutions and pyroelectric infra-red (PIR) sensors, amongst others. Although the PIR sensor can responsively detect a person entering the room, it fails to detect the human continuously during low-level activities such as reading and napping, not to mention the blind spots. Cameras are widely deployed to monitor rooms for security purposes which, however, only work in line-of-sight conditions, incur additional hardware costs, and risk privacy invasion. Therefore, a robust, low-cost, and privacy-friendly solution is still highly in need.

Despite many approaches to passive indoor localization, WiFi-based approaches have gained the utmost research focus. The reason is two-fold: excellent sensing ability and negligible cost. A single WiFi access point has more coverage and fewer blind spots than other sensors, thanks to the ubiquitous indoor propagation and ability to penetrate walls. Further, the sensitivity of the WiFi multipath propagation profile to the changes in the physical environment helps to record information linked to human movement. WiFi signals can "see" multiple scales of human movement in the indoor environment, from sizeable bodily movements to chest movements. Moreover, with the channel state information (CSI) availability from commercial WiFi chipsets, these approaches incur negligible additional costs and can reuse the existing WiFi infrastructure. Most WiFi-based localization approaches using CSI rely on dedicated deployment and calibration to infer the geometric relationships, which requires high setup effort. Other works using CSI fingerprinting need laborious training and cannot be generalized well to different environments. As a result, both these approaches are non-scalable to real-life scenarios.

## SUMMARY

The present teaching relates to systems, methods and devices for: improving accuracy and efficiency of the wireless monitoring based on motion information computation and time window classification; wireless sensing using multiple groups of wireless devices; and wireless monitoring and localization of objects in a venue based on correlation scores.

In one embodiment, a system for wireless monitoring is described. The system comprises: a first wireless device, and a processor. The set of heterogeneous wireless devices comprise: a first device, a second device, a second wireless device and a processor. The first wireless device is configured to transmit a wireless signal through a wireless multipath channel of a venue. The wireless multipath channel is impacted by a motion of an object in the venue. The second wireless device is configured to receive the wireless signal through the wireless multipath channel. The received wireless signal differs from the transmitted wireless signal due to the wireless multipath channel and the motion of the object. The processor is configured for: obtaining a time series of channel information (TSCI) of the wireless multipath channel based on the received wireless signal using a processor, a memory communicatively coupled with the processor and a set of instructions stored in the memory; performing a classification of a sliding time window by analyzing channel information (CI) of the TSCI in the sliding time window; computing a motion information (MI) for the sliding time window based on the TSCI and the classification of the sliding time window; and monitoring the motion of the object based on the MI.

In another embodiment, a method performed by a wireless monitoring system, is described. The method comprises: transmitting a wireless signal from a first wireless device through a wireless multipath channel of a venue, wherein the wireless multipath channel is impacted by a motion of an object in the venue; receiving the wireless signal by a second wireless device through the wireless multipath channel, wherein the received wireless signal differs from the transmitted wireless signal due to the wireless multipath channel and the motion of the object; obtaining a time series of channel information (TSCI) of the wireless multipath channel based on the received wireless signal using a processor, a memory communicatively coupled with the processor and a set of instructions stored in the memory; performing a classification of a sliding time window by analyzing channel information (CI) of the TSCI in the sliding time window; computing a motion information (MI) for the sliding time window based on the TSCI and the classification of the sliding time window; and monitoring the motion of the object based on the MI.

In yet another embodiment, a system for wireless sensing is described. The system comprises: a set of heterogeneous wireless devices in a venue, and a processor. The set of heterogeneous wireless devices comprise: a first device, a second device, and a particular device. The particular device comprises a first radio and a second radio. The particular device is configured to communicate with the first device through a first wireless channel based on a first protocol using the first radio, and configured to communicate with the second device through a second wireless channel based on a second protocol using the second radio. The processor is configured for: obtaining a time series of channel information (TSCI) of the second wireless channel based on a wireless signal that is communicated between the particular device and the second device through the second wireless channel using the second radio of the particular device, wherein each channel information (CI) comprises at least one of: channel state information (CSI), channel impulse response (CIR) or channel frequency response (CFR); computing a pairwise sensing analytics based on the TSCI; and computing a combined sensing analytics based on the pairwise sensing analytics. The particular device is configured to transmit the combined sensing analytics to the first device through the first wireless channel using the first radio of the particular device. The set of heterogeneous wireless devices is configured to perform a wireless sensing task based on the combined sensing analytics.

In still another embodiment, a method performed by a set of heterogeneous wireless devices in a venue for wireless sensing, is described. The method comprises: communicatively coupling a particular device of the set with a first device of the set through a first wireless channel based on a first protocol using a first radio of the particular device; communicatively coupling the particular device with a second device of the set through a second wireless channel based on a second protocol using a second radio of the particular device; performing a pairwise sub-task by the particular device and the second device based on a wireless signal communicated between the particular device and the second device through the second wireless channel using the second radio of the particular device; obtaining by the particular device a pairwise sensing analytics computed based on a time series of channel information (TSCI) of the second wireless channel extracted from the wireless signal, wherein each channel information (CI) comprises at least one of: channel state information (CSI), channel impulse response (CIR) or channel frequency response (CFR); computing a combined sensing analytics by the particular device based on the pairwise sensing analytics; transmitting the combined sensing analytics by the particular device to the first device through the first wireless channel using the first radio of the particular device; and performing a wireless sensing task based on the combined sensing analytics.

In a different embodiment, a system for correlation-based wireless monitoring is described. The system comprises: at least two device pairs in a venue, each device pair comprising a first wireless device and a second wireless device, and a processor. The venue includes a number of objects each undergoing a respective motion. For each device pair, the first wireless device of the device pair is configured to transmit a respective wireless signal, and the second wireless device of the device pair is configured to: receive the respective wireless signal through a respective wireless multipath channel of the venue, wherein the received wireless signal differs from the transmitted wireless signal due to the respective wireless multipath channel and motions of a number of objects in the venue, obtain a respective time series of channel information (TSCI) of the respective wireless multipath channel based on the received wireless signal, compute a respective motion information (MI) based on the TSCI, and perform a respective sensing task based on the respective MI and the respective TSCI. The processor is configured for: computing a correlation score based at least partially on: a first TSCI, a second TSCI, a first MI and a second MI, wherein motion of a first object is detected and monitored in a first sensing task based on the first MI computed based on the first TSCI associated with a first device pair, wherein motion of a second object is detected and monitored in a second sensing task based on the second MI computed based on the second TSCI associated with a second device pair; detecting the first object and the second object as a same object when the correlation score is greater than a first threshold; and detecting the first object and the second object as two different objects when the correlation score is less than a second threshold.

In another embodiment, a method for correlation-based wireless monitoring is described. The method comprises: forming, by a plurality of first wireless devices and a plurality of second wireless devices in a venue, at least two device pairs, each device pair comprising a first wireless device and a second wireless device, wherein the venue includes a number of objects each undergoing a respective motion; for each device pair: transmitting, by the first wireless device of the device pair, a respective wireless signal, receiving, by the second wireless device of the device pair, the respective wireless signal through a respective wireless multipath channel of the venue, wherein the received wireless signal differs from the transmitted wireless signal due to the respective wireless multipath channel and the motions of the number of objects in the venue, obtaining a respective time series of channel information (TSCI) of the respective wireless multipath channel based on the received wireless signal, computing a respective motion information (MI) based on the TSCI, and performing a respective sensing task based on the respective MI and the respective TSCI; detecting and monitoring motion of a first object in a first sensing task based on a first MI computed based on a first TSCI associated with a first device pair; detecting and monitoring motion of a second object in a second sensing task based on a second MI computed based on a second TSCI associated with a second device pair; computing a correlation score based at least partially on: the first TSCI, the second TSCI, the first MI and the second MI; detecting the first object and the second object as a same object when the correlation score is greater than a first threshold; and detecting the first object and the second object as two different objects when the correlation score is less than a second threshold.

In yet another embodiment, apparatus for correlation-based wireless monitoring is described. The apparatus comprises: a memory having a set of instructions stored therein; and a processor communicatively coupled with the memory. The processor is configured for: computing a correlation score based at least partially on: a first time series of channel information (TSCI), a second TSCI, a first motion information (MI) and a second MI. Motion of a first object is detected and monitored in a first sensing task associated with a first device pair based on the first MI computed based on the first TSCI obtained from a first wireless signal communicated between the first device pair in a venue, wherein the venue includes a number of objects each undergoing a respective motion. Motion of a second object is detected and monitored in a second sensing task associated with a second device pair based on the second MI computed based on the second TSCI obtained from a second wireless signal communicated between the second device pair in the venue, wherein each device pair comprises a first wireless device and a second wireless device. For each device pair: the first wireless device of the device pair is configured to transmit a respective wireless signal, and the second wireless device of the device pair is configured to: receive the respective wireless signal through a respective wireless multipath channel of the venue, wherein the received wireless signal differs from the transmitted wireless signal due to the respective wireless multipath channel and the motions of the number of objects in the venue, obtain a respective TSCI of the respective wireless multipath channel based on the received wireless signal, compute a respective MI based on the TSCI, and perform a respective sensing task based on the respective MI and the respective TSCI. The processor is further configured for: detecting the first object and the second object as a same object when the correlation score is greater than a first threshold, and detecting the first object and the second object as two different objects when the correlation score is less than a second threshold.

Other concepts relate to software for implementing the present teaching on wireless monitoring, sensing and localization. Additional novel features will be set forth in part in the description which follows, and in part will become apparent to those skilled in the art upon examination of the following and the accompanying drawings or may be learned by production or operation of the examples. The novel features of the present teachings may be realized and attained by practice or use of various aspects of the methodologies, instrumentalities and combinations set forth in the detailed examples discussed below.

## BRIEF DESCRIPTION OF DRAWINGS

The methods, systems, and/or devices described herein are further described in terms of exemplary embodiments. These exemplary embodiments are described in detail with reference to the drawings. These embodiments are non-limiting exemplary embodiments, in which like reference numerals represent similar structures throughout the several views of the drawings.

FIG. 1 illustrates an exemplary block diagram of a first wireless device of a system for wireless sensing or monitoring, according to some embodiments of the present disclosure.

FIG. 2 illustrates an exemplary block diagram of a second wireless device of a system for wireless sensing or monitoring, according to some embodiments of the present disclosure.

FIG. 3 illustrates a flow chart of an exemplary method for an accurate wireless monitoring, according to some embodiments of the present disclosure.

FIGS. 4-8 are flow charts showing detailed operations for an accurate wireless monitoring, according to some embodiments of the present disclosure.

FIGS. 9-17 are flow charts showing detailed operations for a compensated wireless monitoring, according to some embodiments of the present disclosure.

FIG. 18 illustrates an exemplary scenario for wireless sensing in a venue, according to some embodiments of the present disclosure.

FIG. 19 illustrates an exemplary floor plan and placement of wireless devices for wireless sensing, according to some embodiments of the present disclosure.

FIG. 20 illustrates a flow chart of an exemplary method for hybrid radio-plus-aux fall-down detection based on wireless sensing, according to some embodiments of the present disclosure.

FIG. 21 illustrates an exemplary system for performing wireless sensing in a venue with multiple groups of devices with a per-room deployment, according to some embodiments of the present disclosure.

FIG. 22 illustrates an exemplary system for performing wireless sensing in a venue with multiple groups of devices with a whole-home deployment, according to some embodiments of the present disclosure.

FIG. 23 illustrates a flow chart of an exemplary method for performing wireless sensing in a venue with multiple groups of devices, according to some embodiments of the present disclosure.

FIG. 24 illustrates an exemplary floor plan for wireless sensing to display sensing motion statistics and analytics, according to some embodiments of the present disclosure.

FIG. 25 illustrates a flow chart of an exemplary method of a wireless sensing presentation system, according to some embodiments of the present disclosure.

FIG. 26 illustrates a flow chart of an exemplary method for performing a selective sensing-by-proxy procedure, according to some embodiments of the present disclosure.

FIG. 27A shows an exemplary environment for motion monitoring and localization, according to some embodiments of the present disclosure.

FIG. 27B shows different activities extracted by a wireless system, according to some embodiments of the present disclosure.

FIG. 28A shows an exemplary another environment for motion monitoring and localization, according to some embodiments of the present disclosure.

FIG. 28B shows different activities extracted by another wireless system, according to some embodiments of the present disclosure.

FIG. 29 shows an exemplary diagram of a wireless system for motion monitoring and localization, according to some embodiments of the present disclosure.

FIG. 30 shows exemplary features extracted from CSI time series by a wireless system, according to some embodiments of the present disclosure.

FIGS. 31A-31B show exemplary device setup and movement locations in two scenarios for wireless monitoring, according to some embodiments of the present disclosure.

FIGS. 32A-32D show exemplary motion statistics from receivers and a correlation threshold in various scenarios for wireless monitoring, according to some embodiments of the present disclosure.

FIGS. 33A-33B show exemplary locations of transmitters and receivers in different setup environments, according to some embodiments of the present disclosure.

FIG. 34 illustrates a flow chart of an exemplary method for wireless monitoring and localization, according to some embodiments of the present disclosure.

FIG. 35 illustrates a flow chart showing detailed operations for wireless monitoring and localization, according to some embodiments of the present disclosure.

## DETAILED DESCRIPTION

The symbol "j" disclosed herein means "and/or". For example, "A/B" means "A and/or B." In some embodiments, a method/device/system/software of a wireless monitoring system is disclosed. A time series of channel information (CI) of a wireless multipath channel is obtained using a processor, a memory communicatively coupled with processor and a set of instructions stored in memory. The time series of CI (TSCI) may be extracted from a wireless signal transmitted from a Type1 heterogeneous wireless device (e.g. wireless transmitter (TX), "Bot" device) to a Type2 heterogeneous wireless device (e.g. wireless receiver (RX), "Origin" device) in a venue through the channel. The channel is impacted by an expression/motion of an object in venue. A characteristics/spatial-temporal information (STI)/motion information (MI) of object/expression/motion may be computed/monitored based on the TSCI. A task may be performed based on the characteristics/STI/MI. A task-related presentation may be generated in a user-interface (UI) on a device of a user.

Expression may comprise placement, placement of moveable parts, location/speed/ acceleration/position/orientation/direction/ identifiable place/region/presence/spatial coordinate, static expression/presentation/state/size/length/width/height/angle/scale/curve/ surface/area/ volume/pose/posture/manifestation/body language, dynamic expression/motion/sequence/ movement/activity/ behavior/gesture/gait/extension/contraction/distortion/deformation, body expression (e.g. head/face/eye/mouth/tongue/hair/voice/neck/ limbs/arm/hand/leg/foot/ muscle/moveable parts), surface expression/shape/texture/material/color/electromagnetic (EM) characteristics/visual pattern/wetness/reflectance/translucency/flexibility, material property (e.g. living tissue/hair/fabric/metal/ wood/leather/plastic/artificial material/solid/liquid/gas/ temperature), expression change, and/or some combination.

Wireless multipath channel may comprise: communication channel, analog frequency channel (e.g. with carrier frequency near 700/800/900MHz, or 1.8/1.9/2.4/3/5/6/ 27/60/70+ GHz), coded channel (e.g. in CDMA), and/or channel of wireless/cellular network/system (e.g. WLAN, WiFi, mesh, 4G/LTE/5G/6G/7G/8G, Bluetooth, Zigbee, UWB, RFID, microwave). It may comprise multiple channels, which may be consecutive (e.g. adjacent/overlapping bands) or non-consecutive (e.g. non-overlapping bands, 2.4GHz/5GHz). While channel is used to transmit wireless signal and perform sensing measurements, data (e.g. TSCI/feature/component/characteristics/STI/MI/analytics/task outputs, auxiliary/non-sensing data/network traffic) may be communicated/transmitted in channel.

Wireless signal may comprise a series of probe signals. It may be any of: EM radiation, radio frequency (RF)/light/bandlimited/baseband signal, signal in licensed/ unlicensed/ISM band, wireless/mobile/cellular/optical communication/network/mesh/downlink/ uplink/unicast/multicast/broadcast signal. It may be compliant to standard/protocol (e.g. WLAN, WWAN, WPAN, WBAN, international/national/industry/defacto, IEEE/802/802.11/15/16, WiFi, 802.11n/ac/ax/be/bf, 3G/4G/LTE/5G/6G/7G/8G, 3GPP/Bluetooth/BLE/Zigbee/NFC/RFID/ UWB/WiMax). A probe signal may comprise any of: protocol/standard/beacon/pilot/sounding/ excitation/illumination/handshake/synchronization/reference/source/motion probe/detection/ sensing/management/control/data/null-data/beacon/pilot/request/response/association/ reassociation/disassociation/authentication/action/report/poll/announcement/extension/enquiry/acknowledgement frame/packet/signal, and/or null-data-frame (NDP)/RTS/CTS/QoS/CF-Poll/CF-Ack/block acknowledgement/reference/training/synchronization. It may comprise line-of-sight (LOS)/non-LOS components (or paths/links). It may have data embedded. Probe signal may be replaced by (or embedded in) data signal. Each frame/packet/signal may comprise: preamble/header/payload. It may comprise: training sequence, short (STF)/long (LTF) training field, L-STF/L-LTF/L-SIG/HE-STF/HE-LTF/HE-SIG-A/HE-SIG-B, channel estimation field (CEF). It may be used to transfer power wirelessly from Type1 device to Type2 device. Sounding rate of signal may be adjusted to control amount of transferred power. Probe signals may be sent in burst.

TSCI may be extracted/obtained (e.g. by IC/chip) from wireless signal at a layer of Type2 device (e.g. layer of OSI reference model, PHY/MAC/data link/logical link control/ network/transport/session/presentation/application layer, TCP/IP/internet/link layer). It may be extracted from received wireless/derived signal. It may comprise wireless sensing measurements obtained in communication protocol (e.g. wireless/cellular communication standard/network, 4G/LTE/5G/6G/7G/8G, WiFi, IEEE 802.11/11bf/15/16). Each CI may be extracted from a probe/sounding signal, and may be associated with time stamp. TSCI may be associated with starting/stopping time/duration/amount of CI/sampling/sounding frequency/period. A motion detection/sensing signal may be recognized/identified base on probe signal. TSCI may be stored/retrieved/accessed/preprocessed/processed/postprocessed/conditioned/analyzed/ monitored. TSCI/features/components/characteristics/STI/MI/analytics/task outcome may be communicated to edge/cloud server/Type1/Type2/hub/data aggregator/another device/system/network.

Type1/Type2 device may comprise components (hardware/software) such as electronics/chip/integrated circuit (IC)/RF circuitry/antenna/modem/TX/RX/transceiver/RF interface (e.g. 2.4/5/6/27/60/70+ GHz radio/front/back haul radio)/network/interface/processor/ memory/module/circuit/board/software/firmware/connectors/structure/enclosure/housing/ structure. It may comprise access point (AP)/base-station/mesh/router/repeater/hub/wireless station/client/terminal/"Origin Satellite"/"Tracker Bot", and/or internet-of-things (IoT)/appliance/ wearable/accessory/peripheral/furniture/amenity/gadget/vehicle/module/wireless-enabled/ unicast/multicast/broadcasting/node/hub/target/sensor/portable/mobile/cellular/communication/ motion-detection/source/destination/standard-compliant device. It may comprise additional attributes such as auxiliary functionality/network connectivity/purpose/brand/model/ appearance/form/shape/color/material/specification. It may be heterogeneous because the above (e.g. components/device types/additional attributes) may be different for different Type1 (or Type2) devices.

Type1/Type2 devices may/may not be authenticated/associated/collocated. They may be same device. Type1/Type2/portable/nearby/another device, sensing/measurement session/link between them, and/or object/expression/motion/characteristics/STI/MI/task may be associated with an identity/identification/identifier (ID) such as UUID, associated/unassociated STA ID (ASID/USID/AID/UID). Type2 device may passively observe/monitor/receive wireless signal from Type1 device without establishing connection (e.g. association/authentication/ handshake) with, or requesting service from, Type1 device. Type1/Type2 device may move with object/another object to be tracked.

Type1 (TX) device may function as Type2 (RX) device temporarily/sporadically/ continuously/repeatedly/interchangeably/alternately/simultaneously/contemporaneously/ concurrently; and vice versa. Type1 device may be Type2 device. A device may function as Type1/Type2 device temporarily/sporadically/continuously/repeatedly/simultaneously/ concurrently/contemporaneously. There may be multiple wireless nodes each being Type1/Type2 device. TSCI may be obtained between two nodes when they exchange/communicate wireless signals. Characteristics/STI/MI of object may be monitored individually based on a TSCI, or jointly based on multiple TSCI.

Motion/expression of object may be monitored actively with Type1/Type2 device moving with object (e.g. wearable devices/automated guided vehicle/AGV), or passively with Type1/Type2 devices not moving with object (e.g. both fixed devices).

Task may be performed with/without reference to reference/trained/initial database/ profile/baseline that is trained/collected/processed/computed/transmitted/stored in training phase. Database may be re-training/updated/reset.

Presentation may comprise UI/GUI/text/message/form/webpage/visual/image/video/ graphics/animation/graphical/symbol/emoticon/sign/color/shade/sound/music/speech/audio/ mechanical/gesture/vibration/haptics presentation. Time series of characteristic/STI/MI/task outcome/another quantity may be displayed/presented in presentation. Any computation may be performed/shared by processor (or logic unit/chip/IC)/Type1/Type2/user/nearby/another device/local/edge/cloud server/hub/data/signal analysis subsystem/sensing initiator/response/SBP initiator/responder/AP/non-AP. Presentation may comprise any of: monthly/weekly/daily/simplified/detailed/cross-sectional/small/large/form-factor/color-coded/comparative/summary/web view, animation/voice announcement/another presentation related to periodic/repetition characteristics of repeating motion/expression.

Multiple Type1 (or Type 2) devices may interact with a Type2 (or Type1) device. The multiple Type1 (or Type2) devices may be synchronized/asynchronous, and/or may use same/different channels/sensing parameters/settings (e.g. sounding frequency/bandwidth/ antennas). Type2 device may receive another signal from Type1/another Type1 device. Type1 device may transmit another signal to Type2/another Type2 device. Wireless signals sent (or received) by them may be sporadic/temporary/continuous/repeated/synchronous/ simultaneous/concurrent/contemporaneous. They may operate independently/collaboratively. Their data (e.g. TSCI/feature/characteristics/STI/MI/intermediate task outcomes) may be processed/monitored/analyzed independently or jointly/collaboratively.

Any devices may operate based on some state/internal state/system state. Devices may communicate directly, or via another/nearby/portable device/server/hub device/cloud server. Devices/system may be associated with one or more users, with associated settings. Settings may be chosen/selected/pre-programmed/changed/adjusted/modified/varied over time. The method may be performed/executed in shown order/another order. Steps may be performed in parallel/iterated/repeated. Users may comprise human/adult/older adult/man/woman/juvenile/ child/baby/pet/animal/creature/machine/computer module/software. Step/operation/processing may be different for different devices (e.g. based on locations/orientation/direction/roles/user-related characteristics/settings/configurations/available resources/bandwidth/power/network connection/hardware/software/processor/coprocessor/memory/battery life/antennas/directional antenna/power setting/device parameters/characteristics/conditions/status/state). Any/all device may be controlled/coordinated by a processor (e.g. associated with Type1/Type2/nearby/portable/ another device/server/designated source). Some device may be physically in/of/attached to a common device.

Type1 (or Type2) device may be capable of wirelessly coupling with multiple Type2 (or Type1) devices. Type1 (or Type2) device may be caused/controlled to switch/establish wireless coupling (e.g. association/authentication) from Type2 (or Type1) device to another Type2 (or another Type1) device. The switching may be controlled by server/hub device/processor/Type1 device/Type2 device. Radio channel may be different before/after switching. A second wireless signal may be transmitted between Type1 (or Type2) device and second Type2 (or second Type1) device through the second channel. A second TSCI of second channel may be extracted/obtained from second signal. The first/second signals, first/second channels, first/second Type1 device, and/or first/second Type2 device may be same/similar/collocated.

Type1 device may transmit/broadcast wireless signal to multiple Type2 devices, with/without establishing connection (association/authentication) with individual Type2 devices. It may transmit to a particular/common MAC address, which may be MAC address of some device (e.g. dummy receiver). Each Type2 device may adjust to particular MAC address to receive wireless signal. Particular MAC address may be associated with venue, which may be recorded in an association table of an Association Server (e.g. hub device). Venue may be identified by Type1 device/Type2 device based on wireless signal received at particular MAC address.

For example, Type2 device may be moved to a new venue. Type1 device may be newly set up in venue such that Type1 and Type2 devices are not aware of each other. During set up, Type1 device may be instructed/guided/caused/controlled (e.g. by dummy receiver, hardware pin setting/connection, stored setting, local setting, remote setting, downloaded setting, hub device, and/or server) to send wireless signal (e.g. series of probe signals) to particular MAC address. Upon power up, Type2 device may scan for probe signals according to a table of MAC addresses (e.g. stored in designated source, server, hub device, cloud server) that may be used for broadcasting at different locations (e.g. different MAC address used for different venue such as house/office/enclosure/floor/multistorey building/store/airport/mall/stadium/hall/station/ subway/lot/area/zone/region/district/city/country/continent). When Type2 device detects wireless signal sent to particular MAC address, it can use the table to identify venue.

Channel may be selected from a set of candidate/selectable/admissible channels. Candidate channels may be associated with different frequency bands/bandwidth/carrier frequency/modulation/wireless standards/coding/encryption/payload characteristics/network/ ID/SSID/characteristics/settings/parameters. Particular MAC address/selected channel may be changed/adjusted/varied/modified over time (e.g. according to time table/rule/policy/mode/ condition/situation/change). Selection/change may be based on availability/collision/traffic pattern/co-channel/inter-channel interference/effective bandwidth/random selection/pre-selected list/plan. It may be done by a server (e.g. hub device). They may be communicated (e.g. from/to Type1/Type2/hub/another device/local/edge/cloud server).

Wireless connection (e.g. association/authentication) between Type1 device and nearby/portable/another device may be established (e.g. using signal handshake). Type1 device may send first handshake signal (e.g. sounding frame/probe signal/request-to-send RTS) to the nearby/portable/another device. Nearby/portable/another device may reply to first signal by sending second handshake signal (e.g. command/clear-to-send/CTS) to Type1 device, triggering Type1 device to transmit/broadcast wireless signal to multiple Type2 devices without establishing connection with the Type2 devices. Second handshake signals may be response/acknowledge (e.g. ACK) to first handshake signal. Second handshake signal may contain information of venue/Type1 device. Nearby/portable/another device may be a dummy device with purpose (e.g. primary purpose, secondary purpose) to establish wireless connection with Type1 device, to receive first signal, or send second signal. Nearby/portable/another device may be physically attached to Type1 device.

In another example, nearby/portable/another device may send third handshake signal to Type1 device triggering Type1 device to broadcast signal to multiple Type2 devices without establishing connection with them. Type1 device may reply to third signal by transmitting fourth handshake signal to the another device.

Nearby/portable/another device may be used to trigger multiple Type1 devices to broadcast. It may have multiple RF circuitries to trigger multiple transmitters in parallel. Triggering may be sequential/partially sequential/partially/fully parallel. Parallel triggering may be achieved using additional device (s) to perform similar triggering in parallel to nearby/portable/another device. After establishing connection with Type1 device, nearby/portable/another device may suspend/stop communication with Type1 device. It may enter an inactive/hibemation/sleep/stand-by/low-power/OFF/power-down mode. Suspended communication may be resumed. Nearby/portable/another device may have the particular MAC address and Type1 device may send signal to particular MAC address.

The (first) wireless signal may be transmitted by a first antenna of Type1 device to some first Type2 device through a first channel in a first venue. A second wireless signal may be transmitted by a second antenna of Type1 device to some second Type2 device through a second channel in a second venue. First/second signals may be transmitted at first/second (sounding) rates respectively, perhaps to first/second MAC addresses respectively. Some first/second channels/signals/rates/MAC addresses/antennas/Type2 devices may be same/different/synchronous/asynchronous. First/second venues may have same/different sizes/shape/multipath characteristics. First/second venues/immediate areas around first/second antennas may overlap. First/second channels/signals may be WiFi+LTE (one being WiFi, one being LTE), or WiFi+WiFi, or WiFi (2.4GHz)+WiFi (5GHz), or WiFi (5GHz, channe1=a1, BW=a2)+WiFi (5GHz/channel=b1, BW=b2). Some first/second items (e.g. channels/signals/ rates/MAC addresses/antennas/Type1/Type2 devices) may be changed/adjusted/varied/modified over time (e.g. based on time table/rule/policy/mode/condition/situation/another change).

Each Type1 device may be signal source of multiple Type2 devices (i.e. it sends respective probe signal to respective Type2 device). Each respective Type2 device may choose asynchronously the Type1 device from among all Type1 devices as its signal source. TSCI may be obtained by each respective Type2 device from respective series of probe signals from Type1 device. Type2 device may choose Type1 device from among all Type1 devices as its signal source (e.g. initially) based on identity/identification/identifier of Type1/Type2 device, task, past signal sources, history, characteristics, signal strength/quality, threshold for switching signal source, and/or information of user/account/profile/access info/parameters/input/requirement/criteria.

Database of available/candidate Type1 (or Type2) devices may be initialized/ maintained/updated by Type2 (or Type1) device. Type2 device may receive wireless signals from multiple candidate Type1 devices. It may choose its Type1 device (i.e. signal source) based on any of: signal quality/strength/regularity/channel/traffic/characteristics/properties/states/task requirements/training task outcome/MAC addresses/identity/identifier/past signal source/history/user instruction/another consideration.

An undesirable/bad/poor/problematic/unsatisfactory/unacceptable/intolerable/ faulty/demanding/undesirable/inadequate/lacking/inferior/unsuitable condition may occur when (1) timing between adjacent probe signals in received wireless signal becomes irregular, deviating from agreed sounding rate (e.g. time perturbation beyond acceptable range), and/or (2) processed/signal strength of received signal is too weak (e.g. below third threshold, or below fourth threshold for significant percentage of time), wherein processing comprises any lowpass/bandpass/highpass/median/moving/weighted average/linear/nonlinear/smoothing filtering. Any thresholds/percentages/parameters may be time-varying. Such condition may occur when Type1/Type2 device become progressively far away, or when channel becomes congested.

Some settings (e.g. Type1-Type2 device pairing/signal source/network/association/ probe signal/sounding rate/scheme/channel/bandwidth/system state/TSCI/TSMA/task/task parameters) may be changed/varied/adjusted/modified. Change may be according to time table/rule/policy/mode/condition (e.g. undesirable condition)/another change. For example, sounding rate may normally be 100Hz, but changed to 1000Hz in demanding situations, and to 1Hz in low power/standby situation.

Settings may change based on task requirement (e.g. 100 Hz normally and 1000 Hz momentarily for 20 seconds). In task, instantaneous system may be associated adaptively/ dynamically to classes/states/conditions (e.g. low/normal/high priority/emergency/critical/ regular/privileged/non-subscription/subscription/paying/non-paying). Settings (e.g. sounding rate) may be adjusted accordingly. Change may be controlled by: server/hub/Type1/Type2 device. Scheduled changes may be made according to time table. Changes may be immediate when emergency is detected, or gradual when developing condition is detected.

Characteristics/STI/MI may be monitored/analyzed individually based on a TSCI associated with a particular Type1/Type2 device pair, or jointly based on multiple TSCI associated multiple Type1/Type2 pairs, or jointly based on any TSCI associated with the particular Type2 device and any Type1 devices, or jointly based on any TSCI associated with the particular Type1 device and any Type2 devices, or globally based on any TSCI associated with any Type1/Type2 devices.

A classifier/classification/recognition/detection/estimation/projection/feature extraction/processing/filtering may be applied (e.g. to CI/CI-feature/characteristics/STI/MI), and/or trained/re-trained/updated. In a training stage, training may be performed based on multiple training TSCI of some training wireless multipath channel, or characteristic/STI/MI computed from training TSCI, the training TSCI obtained from training wireless signals transmitted from training Type1 devices and received by training Type2 devices. Re-training/updating may be performed in an operating stage based on training TSCI/current TSCI. There may be multiple classes (e.g. groupings/categories/events/motions/expression/activities/ objects/locations) associated with venue/regions/zones/ location/environment/home/office/ building/warehouse/facility object/expression/motion/movement/process/event/manufacturing/ assembly-line/maintenance/repairing/navigation/obj ect/emotional/mental/state/condition/stage/ gesture/gait/action/motion/presence/movement/daily/activity/history/event.

Classifier may comprise linear/nonlinear/binary/multiclass/Bayes classifier/Fisher linear discriminant/logistic regression/Markov chain/Monte Carlo/deep/neural network/ perceptron/self-organization maps/boosting/meta algorithm/decision tree/random forest/genetic programming/kernel learning/KNN/support vector machine (SVM).

Feature extraction/projection may comprise any of: subspace projection/principal component analysis (PCA)/independent component analysis (ICA)/vector quantization/singular value decomposition (SVD)/eigen-decomposition/eigenvalue/time/frequency/orthogonal/non-orthogonal decomposition, processing/preprocessing/postprocessing. Each CI may comprise multiple components (e.g. vector/combination of complex values). Each component may be preprocessed to give magnitude/phase or a function of such.

Feature may comprise: output of feature extraction/projection, amplitude/magnitude/ phase/energy/power/strength/intensity, presence/absence/proximity/likelihood/histogram, time/ period/duration/frequency/component/decomposition/projection/band, local/global/maximum (max)/minimum (min)/zero-crossing, repeating/periodic/typical/habitual/one-time/atypical/ abrupt/mutually-exclusive/evolving/transient/changing/time/related/correlated feature/pattern/ trend/profile/events/tendency/inclination/behavior, cause-and-effect/short-term/long-term/ correlation/statistics/frequency/period/duration, motion/movement/location/map/coordinate/ height/speed/acceleration/angle/rotation/size/volume, suspicious/dangerous/alarming event/ warning/belief/proximity/collision, tracking/breathing/heartbeat/gait/action/event/statistical/ hourly/daily/weekly/monthly/yearly parameters/statistics/analytics, well-being/health/disease/ medical statistics/analytics, an early/instantaneous/contemporaneous/delayed indication/ suggestion/sign/indicator/verifier/detection/symptom of a state/condition/situation/disease/ biometric, baby/patient/machine/ device/temperature/vehicle/parking lot/venue/lift/elevator/ spatial/road/fluid flow/home/room/office/house/building/warehouse/ storage/system/ventilation/ fan/pipe/duct/people/human/car/boat/truck/airplane/drone/downtown/crowd/impulsive event/ cyclo-stationary/environment/vibration/material/surface/3D/2D/local/global, and/or another measurable quantity/variable. Feature may comprise monotonic function of feature, or sliding aggregate of features in sliding window.

Training may comprise AI/machine/deep/supervised/unsupervised/discriminative training/auto-encoder/linear discriminant analysis/regression/clustering/tagging/labeling/Monte Carlo computation.

A current event/motion/expression/object in venue at current time may be classified by applying classifier to current TSCI/characteristics/STI/MI obtained from current wireless signal received by Type2 device in venue from Type1 devices in an operating stage. If there are multiple Type1/Type2 devices, some/all (or their locations/antenna locations) may be a permutation of corresponding training Type1/Type2 devices (or locations/antenna locations). Type1/Type2 device/signal/channel/venue/object/ motion may be same/different from corresponding training entity. Classifier may be applied to sliding windows. Current TSCI/characteristics/STI/MI may be augmented by training TSCI/characteristics/STI/MI (or fragment/extract) to bootstrap classification/classifier.

A first section/segment (with first duration/starting/ending time) of a first TSCI (associated with first Type1-Type2 device pair) may be aligned (e.g. using dynamic time warping/DTW/matched filtering, perhaps based on some mismatch/distance/similarity score/cost, or correlation/autocorrelation/cross-correlation) with a second section/segment (with second duration/starting/ending time) of a second TSCI (associated with second Type1-Type2 device pair), with each CI in first section mapped to a CI in second section. First/second TSCI may be preprocessed. Some similarity score (component/item/link/segment-wise) may be computed. The similarity score may comprise any of: mismatch/distance/similarity score/cost. Component-wise similarity score may be computed between a component of first item (CI/feature/characteristics/STI/MI) of first section and corresponding component of corresponding mapped item (second item) of second section. Item-wise similarity score may be computed between first/second items (e.g. based on aggregate of corresponding component-wise similarity scores). An aggregate may comprise any of: sum/weighted sum, weighted average/robust/trimmed mean/arithmetic/geometric/harmonic mean, median/mode. Link-wise similarity score may be computed between first/second items associated with a link (TX-RX antenna pair) of first/second Type1-Type2 device pairs (e.g. based on aggregate of corresponding item-wise similarity scores). Segment-wise similarity score may be computed between first/second segments (e.g. based on aggregate of corresponding link-wise similarity scores). First/second segment may be sliding.

In DTW, a function of any of: first/second segment, first/second item, another first (or second) item of first (or second) segment, or corresponding timestamp/duration/difference/ differential, may satisfy a constraint. Time difference between first/second items may be constrained (e.g. upper/lower bounded). First (or second) section may be entire first (or second) TSCI. First/second duration/starting/ending time may be same/different.

In one example, first/second Type1-Type2 device pairs may be same and first/second TSCI may be same/different. When different, first/second TSCI may comprise a pair of current/reference, current/current or reference/reference TSCI. For "current/reference", first TSCI may be current TSCI obtained in operating stage and second TSCI may be reference TSCI obtained in training stage. For "reference/reference", first/second TSCI may be two TSCI obtained during training stage (e.g. for two training events/states/classes). For "current/current", first/second TSCI may be two TSCI obtained during operating stage (e.g. associated with two different antennas, or two measurement setups). In another example, first/second Type1-Type2 device pairs may be different, but share a common device (Type1 or Type2).

Aligned first/second segments (or portion of each) may be represented as first/second vectors. Portion may comprise all items (for "segment-wise"), or all items associated with a TX-RX link (for "link-wise"), or an item (for "item-wise"), or a component of an item (for "component-wise"). Similarity score may comprise combination/aggregate/function of any of: inner product/correlation/autocorrelation/correlation indicator/covariance/discriminating score/distance/Euclidean/absolute/$L_k$/weighted distance (between first/second vectors). Similarity score may be normalized by vector length. A parameter derived from similarity score may be modeled with a statistical distribution. A scale/location/another parameter of the statistical distribution may be estimated.

Recall there may be multiple sliding segments. Classifier may be applied to a sliding first/second segment pair to obtain a tentative classification result. It may associate current event with a particular class based on one segment pair/tentative classification result, or multiple segment pairs/tentative classification results (e.g. associate if similarity scores prevail (e.g. being max/min/dominant/matchless/most significant/excel) or significant enough (e.g. higher/lower than some threshold) among all candidate classes for N consecutive times, or for a high/low enough percentage, or most/least often in a time period).

Channel information (CI) may comprise any of: signal strength/amplitude/phase/ timestamp, spectral power measurement, modem parameters, dynamic beamforming information, transfer function components, radio state, measurable variables, sensing data! measurement, coarse/fine-grained layer information (e.g. PHY/MAC/datalink layer), digital gain/RF filter/frontend-switch/DC offset/correction/IQ-compensation settings, environment effect on wireless signal propagation, channel input-to-output transformation, stable behavior of environment, state profile, wireless channel measurements/received signal strength indicator (RSSI)/channel state information (CSI)/channel impulse response (CIR)/channel frequency response (CFR)/characteristics of frequency components (e.g. subcarriers)/channel characteristics/channel filter response, auxiliary information, data/meta/user/account/access/ security/session/status/supervisory/device/network/household/neighborhood/environment/real-time/sensor/stored/encrypted/compressed/protected data, identity/identifier/identification.

Each CI may be associated with timestamp/arrival time/frequency band/signature/ phase/amplitude/trend/characteristics, frequency-like characteristics, time/frequency/time-frequency domain element, orthogonal/non-orthogonal decomposition characteristics of signal through channel. Timestamps of TSCI may be irregular and may be corrected (e.g. by interpolation/resampling) to be regular, at least for a sliding time window.

TSCI may be/comprise a link-wise TSCI associated with an antenna of Type1 device and an antenna of Type2 device. For Type1 device with M antennas and Type2 device with N antennas, there may be MN link-wise TSCI.

CUTSCI may be preprocessed/processed/postprocessed/stored/retrieved/ transmitted/received. Some modem/radio state parameter may be held constant. Modem parameters may be applied to radio subsystem and may represent radio state. Motion detection signal (e.g. baseband signal, packet decoded/demodulated from it) may be obtained by processing (e.g. down-converting) wireless signal (e.g. RF/WiFi/LTE/5G/6G signal) by radio subsystem using radio state represented by stored modem parameters. Modem parameters/radio state may be updated (e.g. using previous modem parameters/radio state). Both previous/updated modem parameters/radio states may be applied in radio subsystem (e.g. to process signal/decode data). In the disclosed system, both may be obtained/compared/analyzed/processed/monitored.

Each CI may comprise N1 CI components (CIC) (e.g. time/frequency domain component, decomposition components), each with corresponding CIC index. Each CIC may comprise a real/imaginary/complex quantity, magnitude/phase/Boolean/flag, and/or some combination/subset. Each CI may comprise a vector/matrix/set/collection of CIC. CIC of TSCI associated with a particular CIC index may form a CIC time series. TSCI may be divided into N1 time series of CIC (TSCIC), each associated with respective CIC index. Characteristics/STI/MI may be monitored based on TSCIC. Some TSCIC may be selected based on some criteria/cost function/signal quality metric (e.g. SNR, interference level) for further processing.

Multi-component characteristics/STI/MI of multiple TSCIC (e.g. two components with indices 6 and 7, or three components indexed at 6, 7, 10) may be computed. In particular, k-component characteristics may be a function of k TSCIC with k corresponding CIC indices. With k=1, it is single-component characteristics which may constitute/form a one-dimensional (1D) function as CIC index spans all possible values. For k=2, two-component characteristics may constitute/form a 2D function. In special case, it may depend only on difference between the two indices. In such case, it may constitute 1D function. A total characteristics may be computed based on one or more multi-component characteristics (e.g. weighted average/aggregate). Characteristics/STI/MI of object/motion/expression may be monitored based on any multi-component characteristics/total characteristics.

Characteristics/STI/MI may comprise: instantaneous/short-/long-term/historical/ repetitive/repeated/repeatable/recurring/ periodic/pseudoperiodic/regular/habitual/incremental/ average/initial/final/current/past/future/predicted/ changing/deviational/ change/time/frequency/ orthogonal/non-orthogonal/transform/decomposition/deterministic /stochastic/probabilistic/ dominant/key/prominent/representative/characteristic/significant/insignificant/indicative/ common/averaged/shared /typical/prototypical/persistent/abnormal/abrupt/impulsive/sudden/ unusual/unrepresentative/atypical/suspicious/ dangerous/alarming/evolving/transient/one-time quantity/characteristics/analytics/feature/information, cause-and-effect, correlation indicator/ score, auto/cross correlation/covariance, autocorrelation function (ACF), spectrum/spectrogram/ power spectral density, time/frequency function/transform/projection, initial/final/temporal/ changeltrend/pattern/tendency/inclination/behavior/activity /history/profile/event, location/ position/localization/spatial coordinate/change on map/path/navigation/tracking, linear/ rotational/horizontal/vertical/location/distance/displacement/height/speed/velocity/acceleration/ change/angular speed, direction/orientation, size/length/width/height/azimuth/area/volume/ capacity, deformation/transformation, object/motion direction/angle/shape/form/shrinking/ expanding, behavior/activity/movement, occurrence, fall-down/accident/security/event, period/frequency/rate/cycle/rhythm/count/quantity, timing/duration/interval, starting/initiating/ ending/current/past/next time/quantity/information, type/grouping/classification/composition, presence/absence/proximity/approaching/receding/entrance/exit, identity/identifier, head/mouth/ eye/breathing/heart/hand/handwriting/arm/body/gesture/leg/gait/organ characteristics, tidal volume/depth of breath/airflow rate/inhale/exhale time/ratio, gait/walking/tool/machine/complex motion, signal/motion characteristic/information/feature/statistics/parameter/magnitude/phase/ degree/dynamics/anomaly/variability/detection/estimation/ recognition/identification/indication, slope/derivative/higher order derivative of function/feature/mapping/transformation of another characteristics, mismatch/distance/similarity score/cost/metric, Euclidean/statistical/weighted distance, L1/L2/Lk norm, inner/outer product, tag, test quantity, consumed/unconsumed quantity, state/physical/health/well-being/emotional/mental state, output responses, any composition/combination, and/or any related characteristics/information/combination.

Test quantities may be computed. Characteristics/STI/MI may be computed/ monitored based on CI/TSCI/features/similarity scores/test quantities. Static (or dynamic) segment/profile may be identified/computed/analyzed/monitored/extracted/obtained/marked/ presented/indicated/highlighted/stored/communicated by analyzing CI/TSCI/features/functions of features/test quantities/characteristics/STI/MI (e.g. target motion/movement presence/ detection/estimation/recognition/identification). Test quantities may be based on CI/TSCI/features/functions of features/characteristics/STI/MI. Test quantities may be processed/tested/analyzed/compared.

Test quantity may comprise any/any function of: data/vector/matrix/structure, characteristics/STI/MI, CI information (CII, e.g. CI/CIC/feature/magnitude/phase), directional information (DI, e.g. directional CII), dominant/representative/characteristic/ indicative/key/ archetypal/exemplary/paradigmatic/prominent/common/shared/typical/prototypical/averaged/ regular/persistent/usual/normal/atypical/unusual/abnormal/unrepresentative data/vector/matrix/ structure, similarity/mismatch/distance score/cost/metric, auto/cross correlation/covariance, sum/mean/average/weighted/trimmed/arithmetic/geometric/harmonic mean, variance/deviation/ absolute/square deviation/averaged/median/total/standard deviation/derivative/slope/variation/ total/absolute/square variation/spread/dispersion/variability, divergence/skewness/kurtosis/range/ interquartile range/coefficient of variation/dispersion/L-moment/quartile coefficient of dispersion/mean absolute/square difference/Gini coefficient/relative mean difference/entropy/ maximum (max)/minimum (min)/median/percentile/quartile, variance-to-mean ratio, max-to-min ratio, variation/regularity/similarity measure, transient event/behavior, statistics/mode/likelihood/histogram/probability distribution function (pdf)/moment generating function/expected function/value, behavior, repeatedness/periodicity/pseudo-periodicity, impulsiveness/suddenness/occurrence/recurrence, temporal profile/characteristics, time/timing/duration/period/frequency/trend/ history, starting/initiating/ending time/quantity/count, motion classification/type, change, temporal/frequency/cycle change, etc.

Identification/identity/identifier/ID may comprise: MAC address/ASID/USID/AID/ UID/UUID, label/tag/index, web link/address, numeral/alphanumeric ID, name/password/ account/account ID, and/or another ID. ID may be assigned (e.g. by software/firmware/user/ hardware, hardwired, via dongle). ID may be stored/retrieved (e.g. in database/memory/cloud/ edge/local/hub server, stored locally/remotely/permanently/temporarily). ID may be associated with any of: user/customer/household/information/data/address/phone number/social security number, user/customer number/record/account, timestamp/duration/timing. ID may be made available to Type1/Type2 device/sensing/SBP initiator/responder. ID may be for registration/ initialization/communication/identification/verification/detection/recognition/authentication/ access control/cloud access/networking/social networking/logging/recording/cataloging/ classification/tagging/association/pairing/transaction/electronic transaction/intellectual property control (e.g. by local/cloud/server/hub, Type1/Type2/nearby/user/another device, user).

Object may be person/pet/animal/plant/machine/user, baby/child/adult/older person, expert/specialist/leader/commander/ manager/personnel/staff/officer/doctor/nurse/worker/teacher/ technician/serviceman/repairman/passenger/patient/customer/ student/traveler/inmate/high-value person/, object to be tracked, vehicle/car/AGV/drone/robot/wagon/transport/remote-controlled machinery/cart/moveable objects/goods/items/material/parts/components/machine/lift/elevator, merchandise/goods/cargo/ people/items/food/package/luggage/equipment/cleaning tool in/on workflow/assembly-line/warehouse/factory/store/ supermarket/distribution/logistic/transport/ manufacturing/retail/wholesale/business center/facility/hub, phone/computer/laptop/tablet/ dongle/plugin/companion/tool/peripheral/accessory/wearable/furniture/appliance/amenity/gadget, IoT/networked/smart/portable devices, watch/glasses/speaker/toys/stroller/keys/wallet/purse/ handbag/backpack, goods/cargo/luggage/equipment/motor/ machine/utensil/table/chair/air-conditioner/door/window/heater/fan, light/fixture/stationary object/television/camera/audio/video/surveillance equipment/parts, ticket/parking/toll/airplane ticket, credit/plastic/access card, object with fixed/changing/no form, mass/solid/liquid/gas/fluid/smoke/fire/flame, signage, electromagnetic (EM) source/medium, and/or another object.

Object may have multiple parts, each with different movement (e.g. position/ location/direction change). Object may be a person walking forward. While walking, his left/right hands may move in different directions, with different instantaneous motion/speed/ acceleration.

Object may/may not be communicatively coupled with some network, such as WiFi, MiFi, 4G/LTE/5G/6G/7G/8G, Bluetooth/NFC/BLE/WiMax/Zigbee/mesh/adhoc network. Object may be bulky machinery with AC power supply that is moved during installation/cleaning/ maintenance/renovation. It may be placed on/in moveable platforms such as elevator/conveyor/ lift/pad/belt/robot/drone/forklift/car/boat/vehicle. Type1/Type2 device may attach to/move with object. Type1/Type2 device may be part of/embedded in portable/another device (e.g. module/ device with module, which may be large/sizeable/small/heavy/bulky/light, e.g. coin-sized/ cigarette-box-sized). Type1/Type2/portable/another device may/may not be attached to/move with object, and may have wireless (e.g. via Bluetooth/BLE/Zigbee/NFC/WiFi) or wired (e.g. USB/micro-USB/Firewire/HDMI) connection with a nearby device for network access (e.g. via WiFi/cellular network). Nearby device may be object/phone/AP/IoT/device/appliance/peripheral/ amenity/furniture/vehicle/gadget/wearable/networked/computing device. Nearby device may be connected to some server (e.g. cloud server via network/internet). It may/may not be portable/moveable, and may/may not move with object. Type1/Type2/portable/nearby/ another device may be powered by battery/solar/DC/AC/other power source, which may be replaceable/non-replaceable, and rechargeable/non-rechargeable. It may be wirelessly charged.

Type1/Type2/portable/nearby/another device may comprise any of: computer/laptop/tablet/pad/phone/printer/monitor/battery /antenna, peripheral/accessory/socket/ plug/charger/switch/adapter/dongle, internet-of-thing (IoT), TV/sound bar/HiFi/speaker/set-top box/remote control/panel/gaming device, AP/cable/broadband/router/repeater/extender, appliance/utility/fan/refrigerator/ washer/ dryer/ microwave/oven/stove/range/light/lamp/tube/pipe/tap/lighting/air-conditioner/heater/smoke detector, wearable/watch/ glasses/goggle/button/bracelet/chain/jewelry/ring/belt/clothing/garment/fabric/shirt/pant/dress/glove/handwear/shoe/footwear/hat/head wear/bag/purse/wallet/makeup/cosmetic/ornament/book/magazine/paper/stationary/signage/poster/display/printed matter, furniture/fix ture/table/ desk/ chair/so fa/bed/ cabinet/ shelf/ rack/storage/box/bucket/basket/packaging/ carriage/tile/shingle/brick /block/mat/panel/curtain/cushion/pad/carpet/material/building material/glass, amenity/sensor/clock/pot/pan/ware/container/ bottle/can/utensil/plate/cup/bowl/toy/ball/tool/pen/racket/lock/bell/camera/microphone/painting/frame/mirror/coffee-maker/door/window, food/pill/medicine, embeddable/implantable/gadget/ instrument/equipment/ device/apparatus/machine/ controller/mechanical tool, garage-opener, key/plastic/payment/credit card/ticket, solar panel, key tracker, fire-extinguisher, garbage can/bin, WiFi-enabled device, smart device/machine/machinery/system/house/office/building/ warehouse/facility/vehicle/ car/bicycle/motorcycle/boat/vessel/airplane/cart/wagon, home/vehicle/office/factory/building/ manufacturing/production/computing/ security/ another device.

One/two/more of Type1/Type2/portable/nearby/another device/server may determine an initial characteristics/STI/MI of object, and/or may share intermediate information. One of Type1/Type2 device may move with object (e.g. "Tracker Bot"). The other one of Type1/Type2 device may not move with object (e.g. "Origin Satellite", "Origin Register"). Either may have known characteristics/STI/MI. Initial STI/MI may be computed based on known STI/MI.

Venue may be any space such as sensing area, room/house/home/office/workplace/ building/facility/warehouse/factory/store/ vehicle/property, indoor/outdoor/enclosed/semi-enclosed/open/semi-open/closed/over-air/floating/underground space/area/structure/enclosure, space/area with wood/glass/metal/material/structure/frame/beam/panel/column/wall/floor/door/ ceiling/window/cavity/gap/opening/reflection/refraction medium/fluid/construction material fixed/adjustable layout/shape, human/animal/plant body/cavity/organibone/blood/vessel/air-duct/windpipe/teeth/soft/hard/rigid/non-rigid tissue, manufacturing/repair/maintenance/mining/ parking/storage/transportation/shipping/logistic/sports/entertainment/amusement/ public/recreational/government/community/seniors/elderly care/geriatric/space facility/terminal/hub, distribution center/store, machine/engine/device/assembly line/workflow, urban/rural/suburban/metropolitan area, staircase/escalator/elevator/hallway/ walkway/tunnel/cave/cavern/channel/duct/pipe/tube/ lift/well/pathway/roof/basement/den/alley/road/path/highway/sewage/ventilation system/ network, car/truck/bus/van/container/ship/boat/submersible/train/tram/airplane/mobile home, stadium/city/playground/ park/field/track/court/gymnasium/hall/mart/market/supermarket/plaza/ square/construction site/hotel/museum/school/hospital/ university/garage/mall/airport/train/bus station/terminal/hub/platform, valley/forest/wood/terrain/landscape/garden/park/patio/land, and/or gas/oil/water pipe/line. Venue may comprise inside/outside of building/facility. Building/facility may have one/multiple floors, with a portion underground.

An event may be monitored based on TSCI. Event may be object/motion/gesture/gait related, such as fall-down, rotation/hesitation/pause, impact (e.g. person hitting sandbag/door/bed/ window/chair/table/desk/cabinet/box/another person/animal/bird/fly/ball/bowling/tennis/ soccer/volley ball/football/baseball/basketball), two-body action (e.g. person releasing balloon/catching fish/molding clay/writing paper/typing on computer), car moving in garage, person carrying smart phone/walking around venue, autonomous/moveable object/machine moving around (e.g. vacuum cleaner/utility/self-driving vehicle/car/drone).

Task may comprise: (a) sensing task, any of: monitoring/sensing/detection/ recognition/estimation/ verification/identification/authentication/classification/locationing/ guidance/navigation/tracking/counting of/in any of: object/objects/vehicle/machine/tool/human/ baby/elderly/patient/intruder/pet presence/proximity/activity/daily-activity/well-being/breathing/ vital sign/heartbeat/health condition/sleep/sleep stage/walking/location/distance/speed/ acceleration/navigation/tracking/exercise/safety/danger/fall-down/intrusion/security/life-threat/emotion/movement/motion/degree/ pattern/periodic/repeated/cyclo-stationary/stationary/ regular/transient/sudden/suspicious motion/irregularity/trend/change/ breathing/human biometrics/environment informatics/gait/gesture/room/region/zone/venue, (b) computation task, any of: signal processing/preprocess/postprocessing/conditioning/denoising/calibration/analysis/ feature extraction/transformation/mapping/ supervised/ unsupervised/semi-supervised/ discriminative/machine/deep learning/training/clustering/training/PCA/eigen-decomposition/ frequency/time/functional decomposition/neural network/map-based/model-based processing/ correction/geometry estimation/analytics computation, (c) IoT task, any of: smart task for venue/user/object/human/pet/house/home/office/workplace /building/facility/warehouse/factory/ store/vehicle/property/structure/assembly-line/IoT/device/system, energy/power management/ transfer, wireless power transfer, interacting/engage with user/object/intruder/human/animal (e.g. presence/motion/gesture/ gait/activity/ behavior/voice/command/instruction/query/music/sound/image/video/location/movement/danger/threat detection/recognition/monitoring/analysis/ response/execution/synthesis, generate/retrieve/play/display/render/synthesize dialog/exchange/ response/presentation/experience/media/multimedia/expression/sound/speech/music/image/ imaging/video/animation/webpage/text/message/notification/reminder/enquiry/warning, detect/recognize/monitor /interpret/analyze/record/store user/intruder/object input/motion/gesture/ location/activity), activating/controlling/configuring (e.g. turn on/off/control/lock/unlock/open/ close/adjust/configure) a device/system (e.g. vehicle/drone/electrical/mechanical/air-conditioning/heating/lighting/ventilation/clearning/entertainment/IoT/security/siren/access system/device/door/window/ garage/lift/elevator/escalator/speaker/television/light/peripheral/ accessory/wearable/furniture/appliance/amenity/gadget/alarm/ camera/ gaming/coffee/cooking/heater/fan/housekeeping/home/office machine/device/robot/vacuum cleaner/assembly line), (d) miscellaneous task, any of: transmission/coding/encryption/storage/analysis of data/parameters/ analytics/derived data, upgrading/administration/configuration/coordination/broadcasting/ synchronization/networking/encryption/ communication/protection/compression/storage/ database/archiving/query/cloud computing/presentation/augmented/virtual reality/other processing/task. Task may be performed by some of: Type1/Type2/nearby/portable/another device, and/or hub/local/edge/cloud server.

Task may also comprise: detect/recognize/monitor/locate/interpret/analyze/record/ store user/visitor/intruder/object/pet, interact/engage/converseldialog/exchange with user/object/ visitor/intruder/human/baby/pet, detect/locate/localize/recognize/monitor/ analyze/ interpret/ learn/ train/respond/execute/synthesize/generate/record/store/summarize health/well-being/daily-life/activity/behavior/pattern/exercise/food-intake/restroom visit/work/play/rest/sleep/relaxation/ danger/routine/timing/ habit/trend/normality/normalcy/anomaly/regularity/irregularity/change/ presence/motion/gesture/ gait/expression/ emotion/state/stage/voice/command/instruction/question/query /music/sound/location/movement/fall-down/threat/ discomfort/sickness/environment/, generate/retrieve/play/display/render/synthesize dialog/exchange/response/presentation/report/        experience/media/multimedia/expression/sound/speech/music/image/imaging/video/animation/webpage/text/message/notification/rem inder/enquiry/warning, detect/recognize/monitor/interpret/ analyze/record/store user/intruder/object input/motion/gesture/location/ activity), detect/check/ monitor/locate/manage/control/adjust/configure/lock/unlock/arm/disarm/open/close/fully/partially/activate/tum on/off some system/device/object (e.g. vehicle/robot/drone/electrical/mechanical/ air-conditioning/heating/ventilation/HVAC /lighting/cleaning/entertainment/IoT/security/siren/ access systems/devices/items/components, door/window/garage/lift/elevator/ escalator/speaker/ television/light/peripheral/accessory/wearable/furniture/appliance/amenity/gadget/alarm/camera/gaming/ coffee/cooking/heater/fan/housekeeping/home/office machine/device/vacuum cleaner/ assembly line/window/garage/door/ blind/curtain/panel/solar panel/sun shade), detect/monitor/ locate user/pet do something (e.g. sitting/sleeping on sofa/in bedroom/running on treadmill/ cooking/watching TV/eating in kitchen/dining room/going upstairs/downstairs/outside/inside/ using rest room), do something (e.g. generate message/response/warning/clarification/ notification/report) automatically upon detection, do something for user automatically upon detecting user presence, turn on/off/wake/control/adjust/dim light/music/radio/TV/HiFi/STB/ computer/speaker/smart device/air-conditioning/ventilation/heating system/curtains/light shades, turn on/off/pre-heat/control coffee-machine/hot-water-pot/cooker/oven/microwave oven/another cooking device, check/manage temperature/setting/weather forecast/telephone/message/mail/ system check, present/interact/engage/dialog/converse (e.g. through smart speaker/display/screen; via webpage/email/messaging system/notification system).

When user arrives home by car, task may be to, automatically, detect user/car approaching, open garage/door upon detection, turn on driveway/garage light as user approaches garage, and/or turn on air conditioner/heater/fan. As user enters house, task may be to, automatically, turn on entrance light/off driveway/garage light, play greeting message to welcome user, turn on user's favorite music/radio/news/channel, open curtain/blind, monitor user's mood, adjust lighting/sound environment according to mood/current/imminent event (e.g. do romantic lighting/music because user is scheduled to eat dinner with girlfriend soon) on user's calendar, warm food in microwave that user prepared in morning, do diagnostic check of all systems in house, check weather forecast for tomorrow/news of interest to user, check calendar/to-do list, play reminder, check telephone answering/messaging system/email, give verbal report using dialog system/speech synthesis, and/or remind (e.g. using audible tool such as speakers/HiFi/speech synthesis/sound/field/voice/music/song/dialog system, using visual tool such as TV/entertainment system/computer/notebook/tablet/display/light/color/brightness/ patterns symbols, using haptic/virtual reality/gesture/tool, using smart device/appliance/material/ furniture/fixture, using server/hub device/cloud/fog/edge server/home/mesh network, using messaging/notification/communication/scheduling/email tool, using UI/GUI, using scent/smell/ fragrance/taste, using neural/nervous system/tool, or any combination) user of someone's birthday/call him, prepare/give report. Task may turn on air conditioner/heater/ventilation system in advance, and/or adjust temperature setting of smart thermostat in advance. As user moves from entrance to living room, task may be to turn on living room light, open living room curtain, open window, turn off entrance light behind user, turn on TV/set-top box, set TV to user's favorite channel, and/or adjust an appliance according to user's preference/conditions/states (e.g. adjust lighting, choose/play music to build romantic atmosphere).

When user wakes up in morning, task may be to detect user moving around in bedroom, open blind/curtain/window, turn off alarm clock, adjust temperature from night-time to day-time profile, turn on bedroom light, turn on restroom light as user approaches restroom, check radio/streaming channel and play morning news, turn on coffee machine, preheat water, and/or turn off security system. When user walks from bedroom to kitchen, task may be to turn on kitchen/hallway lights, turn off bedroom/restroom lights, move music/message/reminder from bedroom to kitchen, turn on kitchen TV, change TV to morning news channel, lower kitchen blind, open kitchen window, unlock backdoor for user to check backyard, and/or adjust temperature setting for kitchen.

When user leaves home for work, task may be to detect user leaving, play farewell/have-a-good-day message, open/close garage door, turn on/off garage/driveway light, close/lock all windows/doors (if user forgets), turn off appliance (e.g. stove/microwave/oven), turn on/arm security system, adjust light/air-conditioning/heating/ventilation systems to "away" profile to save energy, and/or send alerts/reports/updates to user's smart phone.

Motion may comprise any of: no-motion, motion sequence, resting/non-moving motion, movement/change in position/location, daily/weekly/monthly/yearly/repeating/activity/ behavior/action/routine, transient/time-varying/fall-down/repeating/repetitive/periodic/pseudo-periodic motion/breathing/heartbeat, deterministic/non-deterministic/probabilistic/chaotic/ random motion, complex/combination motion, non-/pseudo-/cyclo-/stationary random motion, change in electro-magnetic characteristics, human/animal/plant/body/machine/mechanical/ vehicle/drone motion, air-/wind-/weather-/water-/fluid-/ground/subsurface/seismic motion, man-machine interaction, normal/abnormal/dangerous/warning/suspicious motion, imminent/ rain/fire/flood/tsunami/explosion/collision, head/facial/eye/mouth/tongue/neck/finger/hand/arm/ shoulder/upper/lower/body/chest/abdominal/hip/leg/foot/joint/knee/elbow/skin/below-skin/ subcutaneous tissue/blood vessel/intravenous/organ/heart/lung/stomach/intestine/bowel/eating/ breathing/talking/singing/dancing/coordinated motion, facial/eye/mouth expression, and/or hand/arm/gesture/gait/UI/keystroke/typing stroke.

Type1/Type2 device may comprise heterogeneous IC, low-noise amplifier (LNA), power amplifier, transmit-receive switch, media access controller, baseband radio, and/or 2.4/3.65/4.9/5/6/sub-7/over-7/28/60/76 GHz/another radio. Heterogeneous IC may comprise processor/memory/software/firmware/instructions. It may support broadband/wireless/ mobile/mesh/cellular network, WLAN/WAN/MAN, standard/IEEE/3GPP/WiFi/4G/LTE/5G/ 6G/7G/8G, IEEE 802.11/a/b/g/n/ac/ad/af/ah/ax/ay/az/be/bf/15/16, and/or Bluetooth/BLE/NFC/Zigbee/WiMax.

Processor may comprise any of: general-/special-/purpose/embedded/multi-core processor, microprocessor/microcontroller, multi-/parallel/CISC/RISC processor, CPU/GPU/ DSP/ASIC/FPGA, and/or logic circuit. Memory may comprise non-/volatile, RAM/ROM/ EPROM/EEPROM, hard disk/SSD, flash memory, CD-/DVD-ROM, magnetic/optical/organic/ storage system/network, network/cloud/edgellocal/external/internal storage, and/or any non-transitory storage medium. Set of instructions may comprise machine executable codes in hardware/IC/software/firmware, and may be embedded/pre-loaded/loaded upon-boot-up/on-the-fly/on-demand/pre-installed/installed/downloaded.

Processing/preprocessing/postprocessing may be applied to data (e.g. TSCI/feature/ characteristics/STI/MI/test quantity/intermediate/data/analytics) and may have multiple steps. Step/pre-/post-/processing may comprise any of: computing function of operands/LOS/non-LOS/single-link/multi-link/component/item/quantity, magnitude/norm/phase/feature/ energy/timebase/similarity/distance/characterization score/measure computation/extraction/ correction/cleaning, linear/nonlinear/FIR/IIR/MA/AR/ARMA/Kalman/particle filtering, lowpass/bandpass/highpass/median/rank/ quartile/percentile/mode/selective/adaptive filtering, interpolation/intrapolation/extrapolation/decimation/subsampling/ upsampling/resampling, matched filtering/enhancement/restoration/denoising/smoothing/conditioning/spectral analysis/mean subtraction/removal, linear/nonlinear/inverse/frequency/time transform, Fourier transform (FT)/DTFT/DFT/FFT/wavelet/Laplace/ Hilbert/Hadamard/trigonometric/sine/cosine/ DCT/power-of-2/sparse/fast/frequency transform, zero/cyclic/padding, graph-based transform/processing, decomposition/orthogonal/non-orthogonal/over-complete projection/ eigen-decomposition/SVD/PCA/ICA/ compressive sensing, grouping/folding/sorting/ comparison/soft/hard/thresholding/ clipping, first/second/high order derivative/ integration/ convolution/multiplication/division/ addition/subtraction, local/global/maximization/ minimization, recursive/iterative/constrained/batch processing, least mean square/absolute error/deviation, cost function optimization, neural network/detection/recognition/classification/ identification/estimation/labeling/ association/tagging/mapping/remapping/training /clustering/ machine/supervised/unsupervised/semi-supervised learning/network, vector/quantization/ encryption/compression/ matching pursuit/scrambling/coding/storing/retrieving/transmitting/ receiving/time-domain/frequency-domain/normalization/ scaling/expansion/representing/ merging/combining/splitting/tracking/ monitoring/shape/silhouette/ motion/activity/analysis, pdf/histogram estimation/importance/Monte Carlo sampling, error detection/protection/ correction, doing nothing, time-varying/adaptive processing, conditioning/weighted/averaging/ over selected components/links, arithmetic/geometric/ harmonic/trimmed mean/centroid/medoid computation, morphological/logical operation/permutation/ combination/sorting/ AND/OR/XOR/ union/intersection, vector operation/addition/subtraction/multiplication/ division, and/or another operation. Processing may be applied individually/jointly. Acceleration using GPU/DSP/coprocessor/multicore/multiprocessing may be applied.

Function may comprise: characteristics/feature/magnitude/phase/energy, scalar/vector/discrete/continuous/ polynomial/exponential/ logarithmic/trigonometric/transcendental/logical/piecewise/linear/algebraic/nonlinear/circular/piecewise linear/real/complex/vector-valued/inverse/absolute/indicator/limiting/floor/rounding/sign/composite/sliding/moving function, derivative/integration, function of function, one-to-one/one-to-many/many-to-one/many-to-many function, mean/mode/median/percentile/max/min/range/statistics/histogram, local/global max/min/zero-crossing, variance/variation/spread/ dispersion/deviation/standard deviation/divergence/range/interquartile range/total variation/absolute/total deviation, arithmetic/geometric/harmonic/trimmed mean/square/cube/root/power, thresholding/clipping/ rounding/truncation/ quantization/approximation, time function processed with an operation (e.g. filtering), sine/cosine/tangent/ cotangent/secant/cosecant/elliptical/parabolic/hyperbolic/game/ zeta function, probabilistic/stochastic/ random/ergodic/ stationary/deterministic/periodic/repeated function, inverse/transformation/frequency/discrete time/Laplace/Hilbert/sine/ cosine/triangular/ wavelet/integer/power-of-2/sparse transform, orthogonal/non-orthogonal/eigen projection/ decomposition/ eigenvalue/ singular value/PCA/ICA/SVD/compressive sensing, neural network, feature extraction, function of moving window of neighboring items of time series, filtering function/convolution, short-time/discrete transform/Fourier/cosine/sine/ Hadamard/wavelet/ sparse transform, matching pursuit, approximation, graph-based processing/transform/graph signal processing, classification/identification/class/group/category/labeling, processing/ preprocessing/postprocessing, machine/learning/ detection/estimation/feature extraction/learning network/feature extraction/denoising/signal enhancement/coding /encryption/mapping/vector quantization/remapping/lowpass/highpass/bandpass/matched/Kalman/particle/FIR/IIR/MA/AR/ARMA/ median/mode/adaptive filtering, first/second/high order derivative/integration/zero crossing/smoothing, up/down/random/ importance/Monte Carlo sampling/resampling/converting, interpolation/extrapolation, short/long term statistics/auto/ cross correlation/moment generating function/time averaging/weighted averaging, special/Bessel/Beta/Gamma/Gaussian/Poisson/ integral complementary error function.

Sliding time window may have time-varying width/size. It may be small/large at beginning to enable fast/accurate acquisition and increase/decrease over time to steady-state size comparable to motion frequency/period/transient motion duration/characteristics/STI/MI to be monitored. Window size/time shift between adjacent windows may be constant/adaptively/ dynamically/automatically changed/adjusted/varied/modified (e.g. based on battery life/power consumption/available computing power/change in amount of targets/nature of motion to be monitored/user request/choice/instruction/command).

Characteristics/STI/MI may be determined based on characteristic value/point of function and/or associated argument of function (e.g. time/frequency). Function may be outcome of a regression. Characteristic value/point may comprise local/global/constrained/significant/ first/second/i^th maximum/minimum/extremum/zero-crossing (e.g. with positive/negative time/ frequency/argument) of function. Local signal-to-noise-ratio (SNR) or SNR-like parameter may be computed for each pair of adjacent local max (peak)/local min (valley) of function, which may be some function (e.g. linear/log/exponential/monotonic/power/polynomial) of fraction or difference of a quantity (e.g. power/magnitude) of local max over the quantity of local min. Local max (or min) may be significant if its SNR is greater than threshold and/or if its amplitude is greater (or smaller) than another threshold. Local max/min may be selected/identified/computed using persistence-based approach. Some significant local max/min may be selected based on selection criterion (e.g. quality criterion/condition, strongest/consistent significant peak in a range). Unselected significant peaks may be stored/monitored as "reserved" peaks for use in future selection in future sliding time windows. E.g. a particular peak (e.g. at particular argument/time/frequency) may appear consistently over time. Initially, it may be significant but not selected (as other peaks may be stronger). Later, it may become stronger/dominant consistently. When selected, it may be back-traced in time and selected in earlier time to replace previously selected peaks (momentarily strong/dominant but not persistent/consistent). Consistency of peak may be measured by trace, or duration of being significant. Alternatively, local max/min may be selected based on finite state machine (FSM). Decision thresholds may be time-varying, adjusted adaptively/dynamically (e.g. based on back-tracing timing/FSM, or data distribution/statistics).

A similarity score (SS)/component SS may be computed based on two temporally adjacent CI/CIC, of one TSCI or of two different TSCI. The pair may come from same/different sliding window (s). SS or component SS may comprise: time reversal resonating strength (TRRS), auto/cross correlation/covariance, inner product of two vectors, L1/L2/Lk/Euclidean/statistical/ weighted/distance score/norm/metric/quality metric, signal quality condition, statistical characteristics, discrimination score, neural network/deep learning network/machine learning/training/discrimination/weighted averaging/preprocessing/denoising/signal conditioning/filtering/time correction/timing compensation/phase offset compensation/ transformation/component-wise operation/feature extraction/FSM, and/or another score.

Any threshold may be fixed (e.g. 0, 0.5, 1, 1.5, 2), pre-determined and/or adaptively/dynamically determined (e.g. by FSM, or based on time/space/location/antenna/ path/link/state/battery life/remaining battery life/available resource/power/computation power/network bandwidth). Threshold may be applied to test quantity to differentiate two events/conditions/situations/states, A and B. Data (e.g. CI/TSCI/feature/similarity score/test quantity/characteristics/STI/MI) may be collected under A/B in training situation. Test quantity (e.g. its distribution) computed based on data may be compared under A/B to choose threshold based on some criteria (e.g. maximum likelihood (ML), maximum aposterior probability (MAP), discriminative training, minimum Type 1 (or 2) error for given Type 2 (or 1) error, quality criterion, signal quality condition). Threshold may be adjusted (e.g. to achieve different sensitivity), automatically/semi-automatically/manually/adaptively/dynamically, once/ sometimes/often/periodically/repeatedly/occasionally/ sporadically/ on-demand (e.g. based on object/movement/location direction/action/characteristics/STI/MI/size/property/trait/habit/ behavior/venue/feature/fixture/furniture/barrier/material/machine/living thing/thing/boundary/ surface/medium/map/constraint/ model/event/state/situation/condition/time/timing/duration/state/history/user/preference). An iterative algorithm may stop after N iterations, after time-out period, or after test quantity satisfies a condition (e.g. updated quantity greater than threshold) which may be fixed/adaptively/dynamically adjusted.

Searching for local extremum may comprise constrained/minimization/ maximization, statistical/dual/constraint /convex/global/local/combinatorial/infinite-dimensional/multi-objective/multi-modal/non-differentiable/particle-swarm/simulation-based optimization, linear/nonlinear/quadratic/higher-order regression, linear/nonlinear/stochastic/ constraint/dynamic /mathematical/disjunctive/convex/semidefinite/conic/cone/interior/fractional/ integer/sequential/quadratic programming, conjugate/gradient/subgradient/coordinate/reduced descent, Newton's/simplex/iterative/point/ellipsoid/quasi-Newton/interpolation/memetic/ genetic/evolutionary/pattern-/gravitational-search method/algorithm, constraint satisfaction, calculus of variations, optimal control, space mapping, heuristics/metaheuristics, numerical analysis, simultaneous perturbation stochastic approximation, stochastic tunneling, dynamic relaxation, hill climbing, simulated annealing, differential evolution, robust/line/Tabu/reactive search/optimization, curve fitting, least square, variational calculus, and/or variant. It may be associated with an objective/loss/cost/utility/fitness/energy function.

Regression may be performed using regression function to fit data, or function (e.g. ACF/transform/mapped) of data, in regression window. During iterations, length/location of regression window may be changed. Regression function may be linear/quadratic/cubic/ polynomial/another function. Regression may minimize any of: mean/weighted/absolute/square deviation, error, aggregate/component/weighted/mean/sum/absolute/square/high-order/another error/cost (e.g. in projection domain/selected axes/orthogonal axes), robust error (e.g. first error (e.g. square) for smaller error magnitude, second error (e.g. absolute) for larger error magnitude), and/or weighted sum/mean of multiple errors (e.g. absolute/square error). Error associated with different links/path may have different weights (e.g. link with less noise may have higher weight). Regression parameter (e.g. time-offset associated with max/min regression error of regression function in regression window, location/width of window) may be initialized and/or updated during iterations (e.g. based on target value/range/profile, characteristics/STI/MI/test quantity, object motion/quantity/ count/location/state, past/current trend, location/amount/ distribution of local extremum in previous windows, carrier/subcarrier frequency/bandwidth of signal, amount of antennas associated with the channel, noise characteristics, histogram/distribution/central/F-distribution, and/or threshold). When converged, current time offset may be at center/left/right (or fixed relative location) of regression window.

In presentation, information may be displayed/presented (e.g. with venue map/environmental model). Information may comprise: current/past/corrected/approximate/ map/location/speed/acceleration/zone/region/area/segmentation/coverage-area, direction/path/trace/history/traffic/summary, frequently-visited areas, customer/crowd event/distribution/behavior, crowd-control information, acceleration/speed/vital-sign/breathing/heart-rate/activity/emotion/sleep/state/rest information, motion-statistics/MI/STI, presence/absence of motion/people/pets/object/vital sign, gesture (e.g. hand/arm/foot/leg/body/head/face/mouth/eye)/meaning/control (control of devices using gesture), location-based gesture-control/motion-interpretation, identity/identifier (ID) (e.g. of object/person/user/pet/zone/region, device/machine/vehicle/drone/car/boat/bicycle/TV/air-con/fan/, self-guided machine/device/vehicle), environment/weather information, gesture/gesture control/motion trace, earthquake/ explosion/storm rain/fire/temperature, collision/impact/ vibration, event/door/window/open/close/fall-down/accident/buming/freezing/water-/wind-/air-movement event, repeated/pseudo-periodic event (e.g. running on treadmill, jumping up/down, skipping rope, somersault), and/or vehicle event. Location may be one/two/three dimensional (e.g. expressed/represented as 1D/2D/3D rectangular/polar coordinates), relative (e.g. w.r.t. map/environmental model) or relational (e.g. at/near/distance-from a point, halfway between two points, around corner, upstairs, on table top, at ceiling, on floor, on sofa).

Information (e.g. location) may be marked/displayed with some symbol. Symbol may be time-varying/flashing/pulsating with changing color/intensity/size/orientation. Symbol may be a number reflecting instantaneous quantity (e.g. analytics/gesture/ state/status/action/ motion/breathing/heart rate, temperature/network traffic/connectivity/remaining power). Symbol/size/orientation/ color/intensity/rate/characteristics of change may reflect respective motion. Information may be in text or presented visually/verbally (e.g. using pre-recorded voice/voice synthesis)/mechanically (e.g. animated gadget, movement of movable part).

User device may comprise smart phone/tablet/speaker/camera/display/TV/gadget/ vehicle/appliance/device/IoT, device with UI/GUI/voice/audio/record/capture/sensor/playback/ display/animation/VR/AR (augmented reality)/voice (assistance/recognition/synthesis) capability, and/or tablet/laptop/PC.

Map/floor plan/environmental model (e.g. of home/office/building/store/warehouse/ facility) may be 2-/3-/higher-dimensional. It may change/evolve over time (e.g. rotate/zoom/ move/jump on screen).

Walls/windows/doors/entrances/exits/forbidden areas may be marked. It may comprise multiple layers (overlays). It may comprise maintenance map/model comprising water pipes/gas pipes/wiring/cabling/air ducts/crawl-space/ceiling/underground layout.

Venue may be segmented/subdivided/zoned/grouped into multiple zones/regions/ sectors/sections/territories/districts/precincts/localities/neighborhoods/areas/stretches/expance such as bedroom/living/dining/rest/ storage/utility/warehouse/conference/work/walkway/ kitchen/foyer/garage/first/second floor/offices/reception room/area/regions. Segments/regions/ areas may be presented in map/floor plan/model with presentation characteristic (e.g. brightness/intensity/luminance/color/ chrominance/ texture/animation/flashing/rate).

An example of disclosed system/apparatus/method. Stephen and family want to install disclosed wireless motion detection system to detect motion in their 2000 sqft two-storey town house in Seattle, Washington. Because his house has two storeys, Stephen decides to use one Type2 device (named A) and two Type1 devices (named B and C) in ground floor. His ground floor has three rooms: kitchen, dining and living rooms arranged in straight line, with dining room in middle. He put A in dining room, and B in kitchen and C in living room, partitioning ground floor into 3 zones (dining room, living room, kitchen). When motion is detected by AB pair and/or AC pair, system would analyze TSCI/feature/characteristics/STI/MI and associate motion with one of 3 zones.

When Stephen and family go camping in holiday, he uses mobile phone app (e.g. Android phone app or iPhone app) to turn on motion detection system. If system detects motion, warning signal is sent to Stephen (e.g. SMS, email, push message to mobile phone app). If Stephen pays monthly fee (e.g. $10/month), a service company (e.g. security company) will receive warning signal through wired (e.g. broadband)/wireless (e.g. WiFi/LTE/5G) network and perform security procedure (e.g. call Stephen to verify any problem, send someone to check on house, contact police on behalf of Stephen).

Stephen loves his aging mother and cares about her well-being when she is alone in house. When mother is alone in house while rest of family is out (e.g. work/shopping/vacation), Stephen turns on motion detection system using his mobile app to ensure mother is ok. He uses mobile app to monitor mother's movement in house. When Stephen uses mobile app to see that mother is moving around house among the three regions, according to her daily routine, Stephen knows that mother is ok. Stephen is thankful that motion detection system can help him monitor mother's well-being while he is away from house.

On typical day, mother would wake up at 7am, cook her breakfast in kitchen for 20 minutes, eat breakfast in dining room for 30 minutes. Then she would do her daily exercise in living room, before sitting down on sofa in living room to watch favorite TV show. Motion detection system enables Stephen to see timing of movement in 3 regions of house. When motion agrees with daily routine, Stephen knows roughly that mother should be doing fine. But when motion pattern appears abnormal (e.g. no motion until 10am, or in kitchen/motionless for too long), Stephen suspects something is wrong and would call mother to check on her. Stephen may even get someone (e.g. family member/neighbor/paid personnel/friend/social worker/service provider) to check on mother.

One day Stephen feels like repositioning a device. He simply unplugs it from original AC power plug and plugs it into another AC power plug. He is happy that motion detection system is plug-and-play and the repositioning does not affect operation of system. Upon powering up, it works right away.

Sometime later, Stephen decides to install a similar setup (i.e. one Type2 and two Type1 devices) in second floor to monitor bedrooms in second floor. Once again, he finds that system set up is extremely easy as he simply needs to plug Type2 device and Type1 devices into AC power plug in second floor. No special installation is needed. He can use same mobile app to monitor motion in both ground/second floors. Each Type2 device in ground/second floors can interact with all Type1 devices in both ground/second floors. Stephen has more than double capability with combined systems.

Disclosed system can be applied in many applications. Type1/Type2 devices may be any WiFi-enabled devices (e.g. smart IoT/appliance/TV/STB/speaker/refrigerator/stove/ oven/microwave/fan/heater/air-con/router/phone/computer/tablet/accessory/plug/ pipe/lamp/ smoke detector/furniture/fixture/shelf/cabinet/door/window/lock/sofa/tablelchair/piano/utensil/ wearable/watch/tag/key/ ticket/belt/wallet/pen/hat/necklace/implantable/phone/eyeglasses/glass panel/gaming device) at home/office/facility, on table, at ceiling, on floor, or at wall. They may be placed in conference room to count people. They may form a well-being monitoring system to monitor daily activities of older adults and detect any sign of symptoms (e.g. dementia, Alzheimer's disease). They may be used in baby monitors to monitor vital signs (breathing) of babies. They may be placed in bedrooms to monitor sleep quality and detect any sleep apnea. They may be placed in cars to monitor well-being of passengers and drivers, detect sleepy drivers or babies left in hot cars. They may be used in logistics to prevent human trafficking by monitoring any human hidden in trucks/containers. They may be deployed by emergency service at disaster area to search for trapped victims in debris. They may be deployed in security systems to detect intruders.

In some embodiments, to perform a wireless sensing task, a motion statistics (MS) or motion information (MI) may be computed in a sliding time window based on all the CI (e.g. CSI/CIR/CFR) in the sliding time window. The task may be to monitor a motion of an object in a venue based on the MS/MI. The MS/MI may be a STI or characteristics of the object or the motion of the object. Sometimes some CI may be abnormal (e.g. due to interference, noise) causing abnormal behavior of MS/MI and disturbing/disrupting the wireless sensing task. Such abnormal CI (and abnormal MS/MI) may be considered outliers. In some embodiments, a scheme is disclosed to detect/suppress/remove/exclude the outliers or effects of the outliers in the sliding time window. The system may classify the sliding time window into either "normal", "moderately abnormal" and "severely abnormal".

In some embodiments, for a "normal" sliding time window (or trustworthy/regular time window, e.g. with no abnormal/outlier CI), the MS/MI may be computed in a normal way and the task may be performed based on the MS/MI. For a "severely abnormal" sliding time window (or severely untrustworthy/irregular time window, e.g. with lots of abnormal/outlier CI), the MS/MI of the current window may not be computed and the task may be performed in an alternative way without the MS/MI of the current time window (e.g. with a replacement MS/MI computed from neighboring MS/MI). For a "moderately abnormal" sliding time window (or moderately untrustworthy/irregular time window, e.g. with acceptable amount of good/normal/trustworthy CI in certain arrangement (e.g. at least N1 consecutive ones) at certain location (e.g. at beginning/end/middle) in the sliding time window), an alternate MS/MI (e.g. simplified/reduced/alternative MS/MI) may be computed based on some good/normal/trustworthy CI in the sliding time window such that the task may be performed based on the alternate MS/MI (which may replace the MS/MI). The alternate MS/MI may be the MS/MI computed without the abnormal CI (i.e. with the abnormal CI removed or excluded). Not all the good/normal/trustworthy CI may be included in the computation of alternate MS/MI.

In some embodiments, a test score (TS) may be computed. For a current sliding time window with N time stamps, a characteristic value may be computed. The system may compute at least one test score (TS) each based on M temporal adjacent CI in the sliding time window of a TSCI, wherein M may be 2, 3, or more. Some or all of the M temporal adjacent CI may be consecutive. Each TS may be a scalar. Each TS may be associated with a respective time. The test score may comprise/may be any of, or a measure or score of any of: similarity, dis-similarity, difference, distance, norm, discrimination, ratio, fraction, variance, variation, divergence, spread, deviation, TRRS, correlation, covariance, autocorrelation, cross-correlation, inner product, etc. In some embodiments, each TSCI is associated with a "link", which is a pairing of a Tx antenna of a Type1 device and a Rx antenna of a Type2 device. There may be one or more links for a pair of Type1/Type2 devices, with more than one associated TSCI.

In some embodiments, a component-wise test score (CTS) may be computed. Each CI may have L components (e.g. L subcarriers for CI=CFR, L tabs for CI=CIR). A CTS may be computed for each component. The CTS may be a scalar. Each CTS may be associated with a respective time. All CTS(t) for any time t may be computed based on M temporal adjacent CI (e.g. similar/identical to those used for TS) in the sliding time window of a test, where M may be 2, 3, or more. The M temporal adjacent CI used for computing all of the L CTS(t) at same time t may be same/different for all components. The CTS may comprise/may be any of, or a measure or score of any of: similarity, dissimilarity, difference, distance, norm, discrimination, ratio, fraction, variance, variation, divergence, spread, deviation, component-wise-TRRS, correlation, covariance, autocorrelation, cross-correlation, inner-product, etc. In some embodiments, TS(t) may be an aggregate (e.g. sum, mean, weighted mean, median, mode, maximum, minimum, percentile) of the L CTS(t), or an aggregate of a weighted quantity of each CTS(t), or an aggregate of a function (e.g. magnitude, phase, magnitude square) of each CTS(t).

In some embodiments, some components/associated CTS may be "selected" (e.g. K largest (magnitude) CTS; or CTS magnitude>threshold; or selected based on a function of respective CTS in relation to the function of the other CTS), and TS(t) may be an aggregate of only the selected CTS/quantity (e.g. function, magnitude, phase, magnitude square) of selected CTS. If TS is a weighted quantity (e.g. weighted sum, weighted mean, weighted product) of CTS, the CTS of selected components may have larger weight than CTS of non-selected components. A weight may be computed for each component (e.g. based on CTS of the component in relation to CTS of the other components). The TS may be a weighted quantity of CTS with each CTS weighted by the respective weight.

In some embodiments, a link-wise test score (LTS) may be computed based on the CI in the sliding time window of a TSCI. For example, the LTS may be a first aggregate of more than one TS. The LTS may also be an "overall" test score based on all CI of the TSCI in the sliding time window (i.e. M=N). For the link, the sliding time window may be classified as "normal", "moderately abnormal" or "severely abnormal" based on the LTS or the more than one TS associated with the TSCI. A link-wise MS/MI (or an alternate linkwise MS/MI) may/may not be computed based on the CI in the sliding time window of the TSCI. Any aggregate may comprise any of: average/mean, weighted average/mean, trimmed mean, sum, weighted sum, product, weighted product, arithmetic mean, geometric mean, harmonic mean, median, weighted median, mode, histogram, statistics, autocorrelation function, spectrum, spectrogram, variance, variation, divergence, spread, range, deviation, minimum, maximum, percentile, characteristic value, etc.

In some embodiments, a device-pair-wise test score (TTS) may be computed for the sliding time window based on more than one TSCI associated with a pair of Type1 device and Type2 device (each TSCI associated with a "link" associated with a TX antenna of Type1 device and a RX antenna of Type2 device). For example, the TTS may be a second aggregate of more than one associated LTS, each LTS associated with a respective link. The TTS may also be an "overall" test score based on all CI in all of the more than one TSCI in the sliding time window. For the Type1-Type2 device pair, the sliding time window may be classified as "normal" or "severely abnormal" based on the TTS, the more than one LTS or the more than one TS (each TS associated with a respective link). A device-pair-wise MS/MI (or alternate device-pair-wise) may/may not be computed based on the CI in the sliding time window of the TSCI.

In some examples, outlier detection/suppression/removal/ is performed for a TSCI. The Test Score (TS) associated with a time t, TS(t), may be a difference score such as CI-difference (CID) between two or more temporally adjacent CI may be computed. In the time window with N CI in each TSCI, the system may compute N-1 CID, with each CID associated with time t being CID1(t)=CI(t)-CI(t-1). For each CID, a feature of CID or f(CID(t)) (CID-feature or CIDF, with the feature f(.) being/comprising magnitude/magnitude square/norm/absolute value/power or a monotonic function of it) may be computed.

In some embodiments, CID may be CID2(t)=f(CI(t))-f(CI(t-1)). In such case, CIDF may be CIDF2(t)= f2(CID2(t)), where f and f2 may be different. In some embodiments, CID may be CID3(t)=CI(t)-CI(t-k), for some k= 1, -1, 2, -2, 3, -3, ... In some embodiments, CID may also be CID=(CID1+CID3)/2=CI(t)- (CI(t-1) +CI(t-k))/2. For k=-1, CID= CI(t)-(CI(t-1) +CI(t+1))/2. For k=2, CID= CI(t)-(CI(t-1) +CI(t-2))/2. In some embodiments, CID may also be a linear combination of CID3 each with a respective k, or a linear combination of any CID1, CID2, CID3. For example, CID may be CID=(CID1+CID1+CID3)/3=CI(t)- (2*CI(t-1) +CI(t-k))/3. In some embodiments, CID may also be CI(t) minus a weighted average of some neighboring CI, such as CID=CI(t)-(CI(t-1)+CI(t-2)+CI(t-3))/3, or even CID=CI(t)-(4*CI(t-1)+2*CI(t-2)+CI(t-3))/7.

In some embodiments, the TS may be any CID or any CIDF or any combination. Large TS may suggest/indicate abrupt change, abnormality, or a high probability/likelihood of abnormality. The LTS may be an aggregate (e.g. arithmetic/geometric/harmonic/trimmed/ weighted mean or sum, median/mode/max/percentile/min, variance/variation/divergence/ spread/deviation, etc.) of the N-1 CID. Arithmetic mean, geometric mean, median or mode may reflect a "typical" behavior. Max or min or percentile (e.g. 95% or 5%) may reflect worst/best case behavior. Variance/variation/divergence may reflect fluctuation behavior.

To perform CI-level abnormality classification, for a CI at a time t, it may be classified/ computed/ determined as "abnormal"/outlier/atypical/uncommon/unusual/unrepresentative/ erroneous/anomalous/strange/irregular/deviant/deviating/divergent/eccentric/exceptional/ singular/perverse/perverted/corrupted) if the associated TS(t) satisfies a first condition (e.g. greater than or smaller than a threshold). Otherwise, it may be classified as "normal". In some embodiments, the TS(t) may be a score/measure of local dis-similarity, difference (e.g. CID/CIDF), distance, discrimination, ratio, fraction, variance, variation, divergence, spread, or deviation in a window around time t and the first condition may be that TS(t) is greater than a threshold. The TS(t) may be a score/measure of local similarity, TRRS, correlation, covariance, autocorrelation, cross-correlation, or inner product in a window around time t, and the first condition may be that TS(t) is less than a threshold. For an "abnormal" CI, an "abnormality score"/AS (e.g. a real number between 0 and 1, or between -1 and +1) may be computed (e.g. based on TS, or all/selected associated CTS; based on a number of neighboring/ temporally neighboring TS or associated CTS). For a run of consecutive abnormal CI, a run-wise abnormality score/RAS may be computed as an aggregate (sum, weighted sum, product, weighted product, mean, weighted mean/median/mode, (weighted) arithmetic/geometric/ harmonic mean) of the AS of each abnormal CI in the run.

To perform link-level abnormality classification, for a sliding time window of CI of a link, e.g. k^{th} link (or link k), the sliding time window may be determined/classified/computed as "abnormal" (e.g. "moderately abnormal" or "severely abnormal") if a second condition is satisfied (e.g. LTS(k) being greater or smaller than a threshold, or a maximum/minimum/ majority/minority/percentage/sufficient amount/minimum amount/maximum amount of TS(t) in the sliding time window of link k being greater or smaller than another threshold or satisfying the first condition, or a distribution characteristic of abnormal CI in link k satisfying a certain condition). Otherwise, the sliding time window of link k may be classified as "normal". If it is "abnormal", it may be further classified as "moderately abnormal" or "severely abnormal" (or more abnormal levels based on AS) if some additional condition (e.g. T1<AS<T2 for some T1, T2) is satisfied. There may be more than one class or sub-class of "moderately abnormal".

In some embodiments, the sliding time window of link k may be classified as "moderately abnormal" if a percentage/an amount of normal CI in the time window (or in an initial portion of the time window, or in an ending portion of the time window) exceeds a threshold, or if an initial (e.g. beginning) run of normal CI has a run-length exceeding a threshold, or an ending (e.g. trailing) run of normal CI has a run-length exceeding a threshold, or some other condition. Otherwise, it may be "severely abnormal". Any threshold may depend on abnormality score/AS/run-wise AS/RAS. Any threshold may be predefined or computed adaptively based on associated AS/RAS. In some embodiments, the sliding time window of link k may be classified as "abnormal" if maximum of TS(t) in the sliding time window of link k is larger than a threshold.

In some embodiments, runs of consecutive "normal" CI and their respective "run-length" may be determined/computed/identified. For example, an isolated normal CI is a run of one and has a run-length of 1. Two consecutive normal CI is a run of two and has a run-length of 2, so on and so forth. The sliding time window of link k may be further classified as "moderately abnormal" if run-length of any run of normal CI satisfies a condition (e.g. greater than ½, or 1/3, or 1/4 of length of sliding time window). Otherwise, it may be further classified as "severely abnormal".

In some embodiments, runs of consecutive "normal CI" may further include (consecutive/connecting) "abnormal" CI with AS less than a threshold. In some embodiments, runs of consecutive abnormal CI with AS less than a threshold may be re-classified as "normal CI" if their respective run-length is greater than another threshold. Alternatively, runs of consecutive abnormal CI with all AS less than a T1 and a percentage of AS less than a T2<T1 may be re-classified as "normal CI" if their respective run-length is greater than a T3. In some embodiments, amount of TS(t) larger than a first threshold may be computed. The sliding time window of link k may be further classified as "moderately abnormal" if a percentage of TS(t) being larger than the first threshold (e.g. a percentage of the max of TS(t) in sliding time window of link k) is less than a second threshold, or alternatively, if a percentile (e.g. 90-percentile) of TS(t) in the sliding time window of link k is less than some threshold.

In some embodiments, any threshold may be predefined or computed adaptively based on associated abnormality score/AS/run-wise AS/RAS. In some embodiments, MS/MI may be computed differently in accordance with "normal", "abnormal", "moderately abnormal" and/or "severely abnormal" classification. In some embodiments, a second condition is satisfied (e.g. LTS(k) being greater or smaller than a threshold, or a majority/minority/percentage/sufficient amount/minimum amount/maximum amount of TS(t) in the sliding time window of link k being greater or smaller than another threshold or satisfying the first condition, or a distribution characteristic of abnormal CI in link k satisfying a certain condition). In some embodiments, The LTS(k) may be a score/measure of window-wide aggregate, max, min, percentile (e.g. 95% or 5%), dis-similarity, difference, distance, discrimination, ratio, fraction, variance, variation, divergence, spread, or deviation of TS(t) in the sliding time window in link k.

To perform device-pair-level abnormality classification, for a pair of Type1 device and Type2 device (i.e. a device pair) comprising M antenna pairings (Type1 device with M1 antennas and Type2 device with M2 antennas such that M=M1*M2) and thus M links and M associated TSCI, the sliding time window of the device-pair may be determined/classified/computed as "abnormal" if a third condition is satisfied (e.g. TTS being greater or smaller than a threshold, or a majority/minority/percentage/sufficient amount/minimum amount/maximum amount of LTS being greater/less than another threshold or satisfying the second condition, or a majority/minority/percentage/sufficient amount/minimum amount/maximum amount of TS in the sliding time window being greater/smaller than yet another threshold or satisfying the first condition, or a distribution characteristic of abnormal links satisfying a certain condition, or a distribution characteristic of abnormal CI satisfying another condition). Otherwise, the sliding time window of the device-pair may be classified as "normal".

In some embodiments, if current time window is "normal", MS/MI may be computed for current sliding time window based on all the CI in link k (or two or more links) in the time window. This may be the normal way of computing MS/MI. If current time window is "abnormal", especially if "severely abnormal", MS/MI may/may not be computed based on CI in current time window. If already computed, the MS/MI may be discarded (or may not be used). An alternative/replacement/substitute/another/auxiliary/fallback/standby/alternate/fill-in/stand-in/proxy MS/MI may be used. The alternative MS/MI may be a predicted/estimated/substitute MS/MI value computed based on some neighboring MS/MI (e.g. temporally neighboring, contemporaneous or past or future; or spatially neighboring such as other TSCI of the same TX/RX device pair, or a TSCI of a "neighboring" TX/RX device pair). The alternative MS/MI may be an aggregate such as mean/median/mode/weighted mean/trimmed mean, or zero/first/second/higher order predictor/estimator, or another aggregate of multiple neighboring MS/MI. If current time window is "moderately abnormal", a reduced/partial/ simplified/limited/slanted/biased/partisan/skewed/colored/one-sided MS/MI may be computed based on some (or all) remaining/available "normal" CI in the current time window. For example, the reduced MS/MI may be computed based on one or more long/longest run(s) of normal CI (each with long enough run-length, e.g. run-length>T1), or a run of beginning or trailing normal CI (with sufficient run-length, e.g. run-length>T2) in the time window.

In some embodiments, if there are two or more runs of normal CI with sufficient length in the current time window, multiple tentative reduced MS/MI may be computed each based on a respective run of normal CI and the reduced MS/MI may be computed as an aggregate of the multiple tentative reduced MS/MI. A beginning or trailing run of normal CI of current time window may be combined with a connected run of normal CI of a temporally adjacent time window in order to compute the reduced MS/MI. In particular, a beginning (or trailing) run of normal CI may be combined with trailing (or beginning) run of normal CI in the previous (or next) adjacent time window and the reduced MS/MI may be computed based on the combined run of normal CI.

In some embodiments, the MS/MI may be computed as the reduced MS/MI. Alternatively, the MS/MI may be computed as an aggregate (e.g. weighted average) of the reduced MS/MI, and one or more neighboring MS/MI (e.g. if longest run-length is less than T1 but greater than T3). Any threshold may be pre-determined or adjusted or adaptively adjusted.

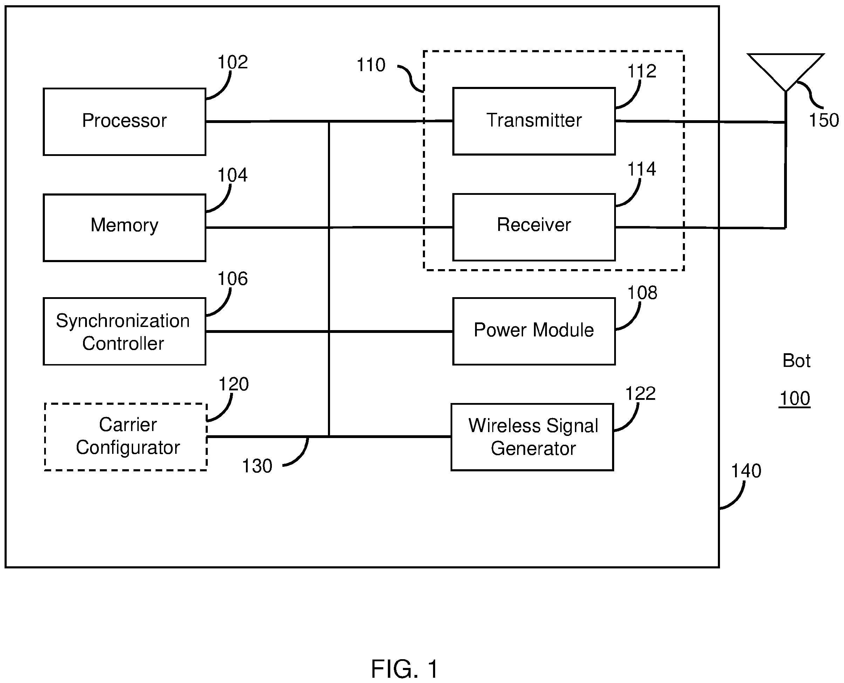

FIG. 1 illustrates an exemplary block diagram of a first wireless device, e.g. a Bot 100, of a wireless sensing or monitoring system, according to one embodiment of the present teaching. The Bot 100 is an example of a device that can be configured to implement the various methods described herein. As shown in FIG. 1, the Bot 100 includes a housing 140 containing a processor 102, a memory 104, a transceiver 110 comprising a transmitter 112 and receiver 114, a synchronization controller 106, a power module 108, an optional carrier configurator 120 and a wireless signal generator 122.

In this embodiment, the processor 102 controls the general operation of the Bot 100 and can include one or more processing circuits or modules such as a central processing unit (CPU) and/or any combination of general-purpose microprocessors, microcontrollers, digital signal processors (DSPs), field programmable gate array (FPGAs), programmable logic devices (PLDs), controllers, state machines, gated logic, discrete hardware components, dedicated hardware finite state machines, or any other suitable circuits, devices and/or structures that can perform calculations or other manipulations of data.

The memory 104, which can include both read-only memory (ROM) and random access memory (RAM), can provide instructions and data to the processor 102. A portion of the memory 104 can also include non-volatile random access memory (NVRAM). The processor 102 typically performs logical and arithmetic operations based on program instructions stored within the memory 104. The instructions (a.k.a., software) stored in the memory 104 can be executed by the processor 102 to perform the methods described herein. The processor 102 and the memory 104 together form a processing system that stores and executes software. As used herein, "software" means any type of instructions, whether referred to as software, firmware, middleware, microcode, etc. which can configure a machine or device to perform one or more desired functions or processes. Instructions can include code (e.g., in source code format, binary code format, executable code format, or any other suitable format of code). The instructions, when executed by the one or more processors, cause the processing system to perform the various functions described herein.

The transceiver 110, which includes the transmitter 112 and receiver 114, allows the Bot 100 to transmit and receive data to and from a remote device (e.g., an Origin or another Bot). An antenna 150 is typically attached to the housing 140 and electrically coupled to the transceiver 110. In various embodiments, the Bot 100 includes (not shown) multiple transmitters, multiple receivers, and multiple transceivers. In one embodiment, the antenna 150 is replaced with a multi-antenna array 150 that can form a plurality of beams each of which points in a distinct direction. The transmitter 112 can be configured to wirelessly transmit signals having different types or functions, such signals being generated by the processor 102. Similarly, the receiver 114 is configured to receive wireless signals having different types or functions, and the processor 102 is configured to process signals of a plurality of different types.

The Bot 100 in this example may serve as a Bot, Type 1 device, transmitter, or STA in the systems disclosed herein. For example, the wireless signal generator 122 may generate and transmit, via the transmitter 112, a wireless signal through a wireless multipath channel impacted by a motion of an object in the venue. The wireless signal carries information of the channel. Because the channel was impacted by the motion, the channel information includes motion information that can represent the motion of the object. As such, the motion can be indicated and detected based on the wireless signal. The generation of the wireless signal at the wireless signal generator 122 may be based on a request for motion detection from another device, e.g. an Origin, or based on a system pre-configuration. That is, the Bot 100 may or may not know that the wireless signal transmitted will be used to detect motion.

The synchronization controller 106 in this example may be configured to control the operations of the Bot 100 to be synchronized or un-synchronized with another device, e.g. an Origin or another Bot. In one embodiment, the synchronization controller 106 may control the Bot 100 to be synchronized with an Origin that receives the wireless signal transmitted by the Bot 100. In another embodiment, the synchronization controller 106 may control the Bot 100 to transmit the wireless signal asynchronously with other Bots. In another embodiment, each of the Bot 100 and other Bots may transmit the wireless signals individually and asynchronously.

The carrier configurator 120 is an optional component in Bot 100 to configure transmission resources, e.g. time and carrier, for transmitting the wireless signal generated by the wireless signal generator 122. In one embodiment, each CI of the time series of CI has one or more components each corresponding to a carrier or sub-carrier of the transmission of the wireless signal. The detection of the motion may be based on motion detections on any one or any combination of the components.

The power module 108 can include a power source such as one or more batteries, and a power regulator, to provide regulated power to each of the above-described modules in FIG. 1. In some embodiments, if the Bot 100 is coupled to a dedicated external power source (e.g., a wall electrical outlet), the power module 108 can include a transformer and a power regulator.

The various modules discussed above are coupled together by a bus system 130. The bus system 130 can include a data bus and, for example, a power bus, a control signal bus, and/or a status signal bus in addition to the data bus. It is understood that the modules of the Bot 100 can be operatively coupled to one another using any suitable techniques and mediums.

Although a number of separate modules or components are illustrated in FIG. 1, persons of ordinary skill in the art will understand that one or more of the modules can be combined or commonly implemented. For example, the processor 102 can implement not only the functionality described above with respect to the processor 102, but also implement the functionality described above with respect to the wireless signal generator 122. Conversely, each of the modules illustrated in FIG. 1 can be implemented using a plurality of separate components or elements.

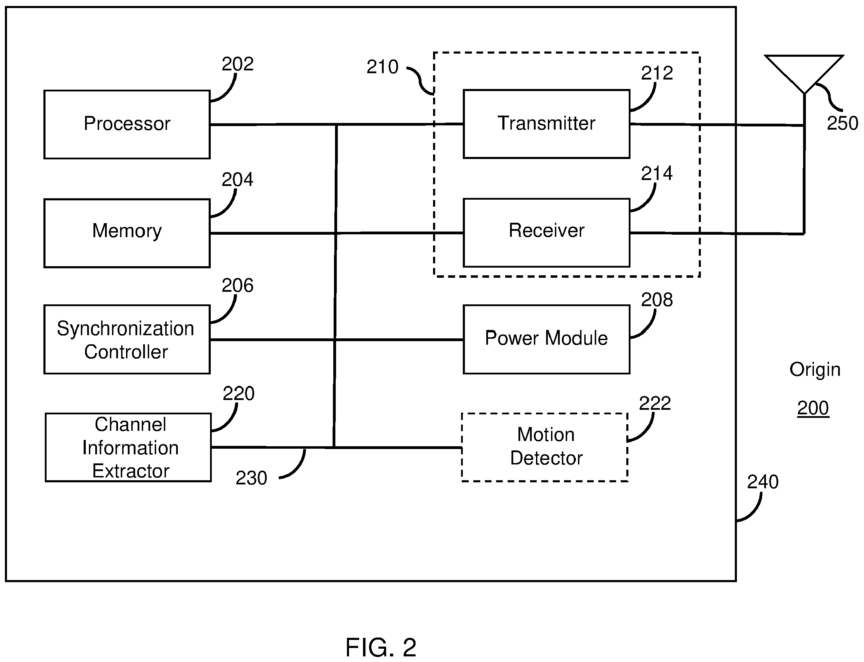

FIG. 2 illustrates an exemplary block diagram of a second wireless device, e.g. an Origin 200, of a wireless sensing or monitoring system, according to one embodiment of the present teaching. The Origin 200 is an example of a device that can be configured to implement the various methods described herein. The Origin 200 in this example may serve as an Origin, receiver, Type 2 device or AP in systems disclosed herein. As shown in FIG. 2, the Origin 200 includes a housing 240 containing a processor 202, a memory 204, a transceiver 210 comprising a transmitter 212 and a receiver 214, a power module 208, a synchronization controller 206, a channel information extractor 220, and an optional motion detector 222.

In this embodiment, the processor 202, the memory 204, the transceiver 210 and the power module 208 work similarly to the processor 102, the memory 104, the transceiver 110 and the power module 108 in the Bot 100. An antenna 250 or a multi-antenna array 250 is typically attached to the housing 240 and electrically coupled to the transceiver 210.

The Origin 200 may be a second wireless device that has a different type from that of the first wireless device (e.g. the Bot 100). In particular, the channel information extractor 220 in the Origin 200 is configured for receiving the wireless signal through the wireless multipath channel impacted by the motion of the object in the venue, and obtaining a time series of channel information (CI) of the wireless multipath channel based on the wireless signal. The channel information extractor 220 may send the extracted CI to the optional motion detector 222 or to a motion detector outside the Origin 200 for detecting object motion in the venue.

The motion detector 222 is an optional component in the Origin 200. In one embodiment, it is within the Origin 200 as shown in FIG. 2. In another embodiment, it is outside the Origin 200 and in another device, which may be a Bot, another Origin, a cloud server, a fog server, a local server, and an edge server. The optional motion detector 222 may be configured for detecting the motion of the object in the venue based on motion information related to the motion of the object. The motion information associated with the first and second wireless devices is computed based on the time series of CI by the motion detector 222 or another motion detector outside the Origin 200.

The synchronization controller 206 in this example may be configured to control the operations of the Origin 200 to be synchronized or un-synchronized with another device, e.g. a Bot, another Origin, or an independent motion detector. In one embodiment, the synchronization controller 206 may control the Origin 200 to be synchronized with a Bot that transmits a wireless signal. In another embodiment, the synchronization controller 206 may control the Origin 200 to receive the wireless signal asynchronously with other Origins. In another embodiment, each of the Origin 200 and other Origins may receive the wireless signals individually and asynchronously. In one embodiment, the optional motion detector 222 or a motion detector outside the Origin 200 is configured for asynchronously computing respective heterogeneous motion information related to the motion of the object based on the respective time series of CI.

The various modules discussed above are coupled together by a bus system 230. The bus system 230 can include a data bus and, for example, a power bus, a control signal bus, and/or a status signal bus in addition to the data bus. It is understood that the modules of the Origin 200 can be operatively coupled to one another using any suitable techniques and mediums.

Although a number of separate modules or components are illustrated in FIG. 2, persons of ordinary skill in the art will understand that one or more of the modules can be combined or commonly implemented. For example, the processor 202 can implement not only the functionality described above with respect to the processor 202, but also implement the functionality described above with respect to the channel information extractor 220. Conversely, each of the modules illustrated in FIG. 2 can be implemented using a plurality of separate components or elements.

In one embodiment, in addition to the Bot 100 and the Origin 200, the system may also comprise: an assistance device, a third wireless device, e.g. another Bot, configured for transmitting an additional heterogeneous wireless signal through an additional wireless multipath channel impacted by the motion of the object in the venue, or a fourth wireless device, e.g. another Origin, that has a different type from that of the third wireless device. The fourth wireless device may be configured for: receiving the additional heterogeneous wireless signal through the additional wireless multipath channel impacted by the motion of the object in the venue, and obtaining a time series of additional channel information (CI) of the additional wireless multipath channel based on the additional heterogeneous wireless signal. The additional CI of the additional wireless multipath channel is associated with a different protocol or configuration from that associated with the CI of the wireless multipath channel. For example, the wireless multipath channel is associated with LTE, while the additional wireless multipath channel is associated with Wi-Fi. In this case, the optional motion detector 222 or a motion detector outside the Origin 200 is configured for detecting the motion of the object in the venue based on both the motion information associated with the first and second wireless devices and additional motion information associated with the third and fourth wireless devices computed by at least one of: an additional motion detector and the fourth wireless device based on the time series of additional CI.

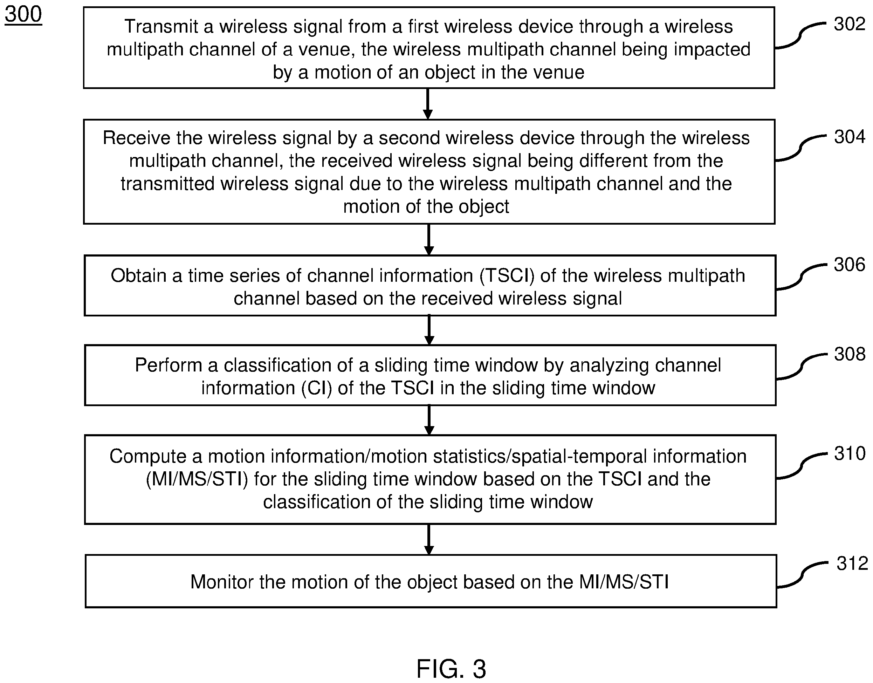

FIG. 3 illustrates a flow chart of an exemplary method 300 for an accurate wireless monitoring, according to some embodiments of the present disclosure. In various embodiments, the method 300 can be performed by the systems disclosed above. At operation 302, a wireless signal is transmitted from a first wireless device through a wireless multipath channel of a venue, the wireless multipath channel being impacted by a motion of an object in the venue. At operation 304, the wireless signal is received by a second wireless device through the wireless multipath channel, the received wireless signal being different from the transmitted wireless signal due to the wireless multipath channel and the motion of the object. At operation 306, a time series of channel information (TSCI) of the wireless multipath channel is obtained based on the received wireless signal. At operation 308, a classification of a sliding time window is performed by analyzing channel information (CI) of the TSCI in the sliding time window. At operation 310, a motion information (MI) for the sliding time window is computed based on the TSCI and the classification of the sliding time window. At operation 312, the motion of the object is monitored based on the MI.

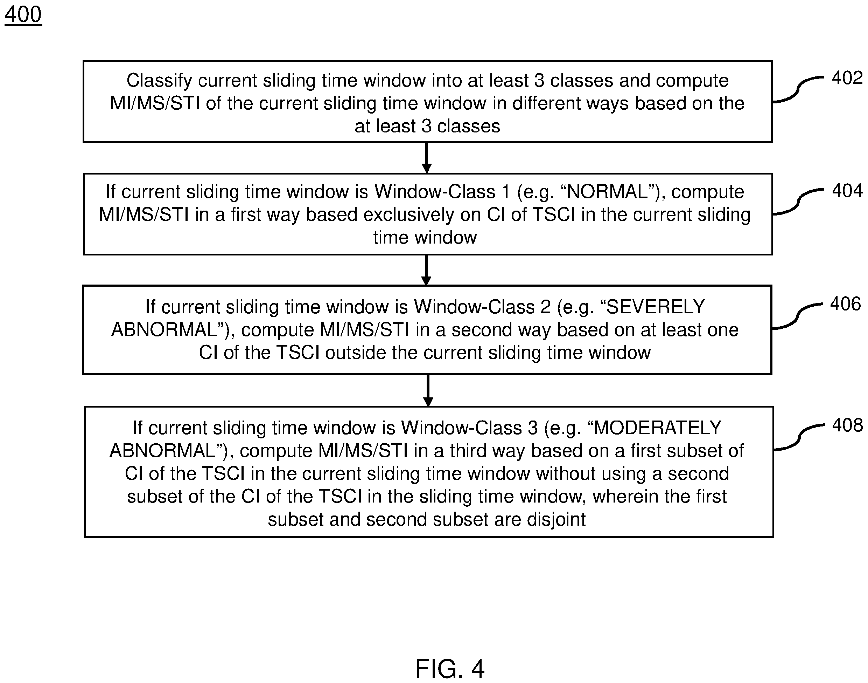

FIG. 4 illustrates a flow chart of an exemplary method 400 for performing a classification of a sliding time window, according to some embodiments of the present disclosure. In various embodiments, the method 400 can be performed by a system as disclosed above. At operation 402, the system can classify current sliding time window into at least 3 classes and compute MI/MS/STI of the current sliding time window in different ways based on the at least 3 classes. At operation 404, if current sliding time window is Window-Class 1 (e.g. "NORMAL"), the system can compute MI/MS/STI in a first way based exclusively on CI of TSCI in the current sliding time window. At operation 406, if current sliding time window is Window-Class 2 (e.g. "SEVERELY ABNORMAL"), the system compute MI/MS/STI in a second way based on at least one CI of the TSCI outside the current sliding time window. At operation 408, if current sliding time window is Window-Class 3 (e.g. "MODERATELY ABNORMAL"), the system can compute MI/MS/STI in a third way based on a first subset of CI of the TSCI in the current sliding time window without using a second subset of the CI of the TSCI in the sliding time window, wherein the first subset and second subset are disjoint.

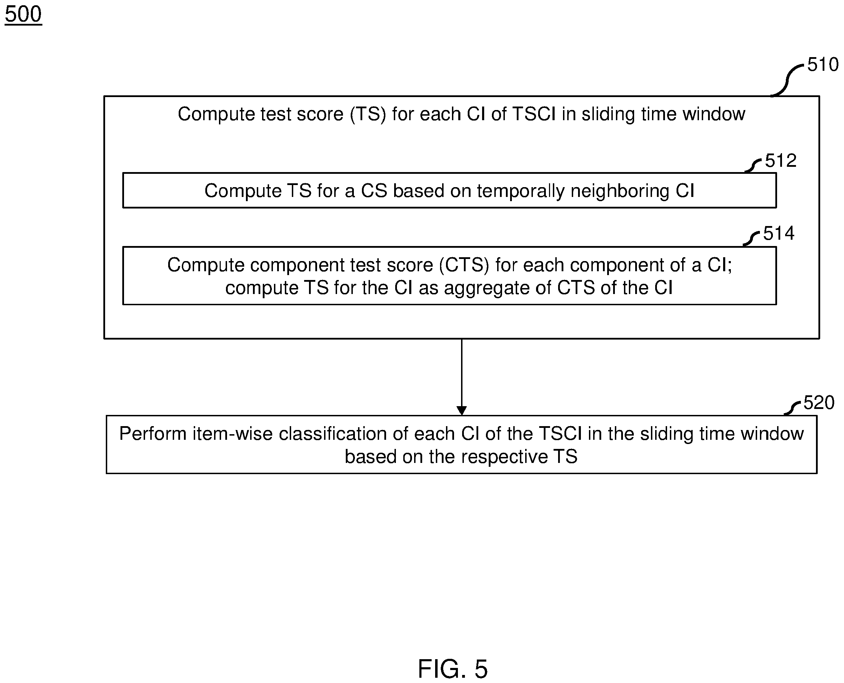

FIG. 5 illustrates a flow chart of an exemplary method 500 for computing test score (TS) and performing item-wise classification of each CI, according to some embodiments of the present disclosure. In various embodiments, the method 500 can be performed by a system as disclosed above. At operation 510, the system can compute test score (TS) for each CI of TSCI in sliding time window, based on one of sub-operations 512, 514. At sub-operation 512, the system can compute TS for a CS based on temporally neighboring CI. At sub-operation 514, the system can compute component test score (CTS) for each component of a CI; and then compute TS for the CI as aggregate of CTS of the CI. In some embodiments, TS comprises at least one of: test quantity, similarity/dis-similarity/matching/mismatch score, distance score, TRRS, correlation/covariance/ autocorrelation/cross-correlation, inner product, norm, aggregate, difference, absolute/square difference, variance/variation/variability, deviation/standard deviation, spread, dispersion, divergence, skewness, range, kurtosis, interquartile range, Gini coefficient, entropy, mean, median, mode, maximum, minimum, percentile, quartile, max-to-min ratio, variance-to-mean ratio, regularity, irregularity, statistics, histogram, probability, impulsiveness, suddenness, occurrence, recurrence, change, a difference of CI(t) and a linear combination of CI(t+k) for k=+-1/2/3/..., a difference of F(CI(t)) and a linear combination of F(CI(t+k)), an inner product of CI(t) and a linear combination of CI(t+k) for k=+-1/2/3/..., an inner product of F(CI(t)) and a linear combination of F(CI(t+k)), a dis-similarity score of CI(t) and a linear combination of CI(t+k) for k=+-1/2/3/..., or a dis-similarity of F(CI(t)) and a linear combination of F(CI(t+k)). At operation 520, the system can perform item-wise classification of each CI of the TSCI in the sliding time window based on the respective TS.

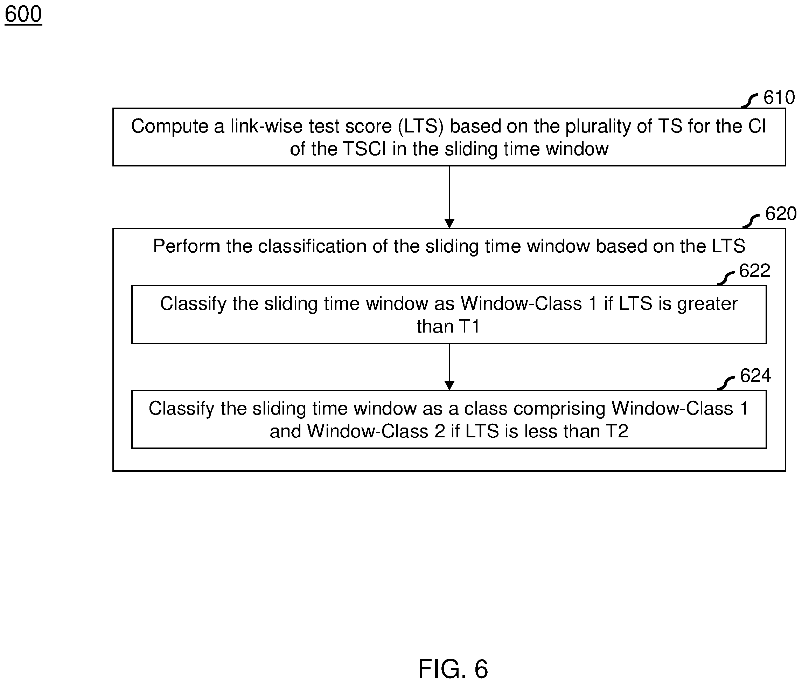

FIG. 6 illustrates a flow chart of an exemplary method 600 for performing a classification of a sliding time window, according to some embodiments of the present disclosure. In various embodiments, the method 600 can be performed by a system as disclosed above. At operation 610, the system can compute a link-wise test score (LTS) based on the plurality of TS for the CI of the TSCI in the sliding time window. In some embodiments, LTS is an aggregate of the plurality of TS, wherein the aggregate may comprise at least one of: sum, weighted sum, average, weighted average, geometric mean, weighted geometric mean, harmonic mean, weighted harmonic mean, arithmetic mean, weighted mean, trimmed mean, median, weighted median, mode, histogram, statistics, percentile, maximum, minimum, variance, variation, divergence, spread, range, deviation, or characteristic value. At operation 620, the system can perform the classification of the sliding time window based on the LTS, which comprises sub-operations 622, 624. At sub-operation 622, the system can classify the sliding time window as Window-Class 1 if LTS is greater than T1. At sub-operation 624, the system can classify the sliding time window as a class comprising Window-Class 1 and Window-Class 2 if LTS is less than T2.

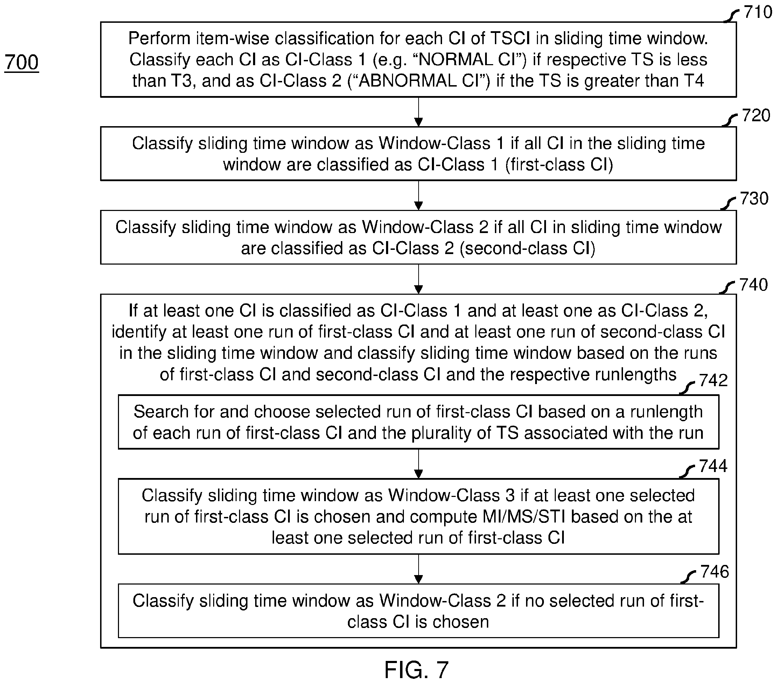

FIG. 7 illustrates a flow chart of an exemplary method 700 for performing a classification of a sliding time window, according to some embodiments of the present disclosure. In various embodiments, the method 700 can be performed by a system as disclosed above. At operation 710, the system can perform item-wise classification for each CI of TSCI in sliding time window. Classify each CI as CI-Class 1 (e.g. "NORMAL CI") if respective TS is less than T3, and as CI-Class 2 ("ABNORMAL CI") if the TS is greater than T4. At operation 720, the system can classify sliding time window as Window-Class 1 if all CI in the sliding time window are classified as CI-Class 1 (first-class CI). At operation 730, the system can classify sliding time window as Window-Class 2 if all CI in sliding time window are classified as CI-Class 2 (second-class CI). At operation 740, if at least one CI is classified as CI-Class 1 and at least one as CI-Class 2, the system may identify at least one run of first-class CI and at least one run of second-class CI in the sliding time window and classify sliding time window based on the runs of first-class CI and second-class CI and the respective runlengths, which comprises sub-operations 742, 744, 746. At sub-operation 742, the system may search for and choose selected run of first-class CI based on a runlength of each run of first-class CI and the plurality of TS associated with the run. In some embodiments, N1 runs of first-class CI with longest runlengths among all runs are chosen. For example, N1=1, such that the run of first-class CI with longest runlength among all runs are chosen. In some embodiments, any run of first-class CI with runlength greater than T5 is chosen. In some embodiments, a beginning run of first-class CI with runlength greater than T6 is chosen. In some embodiments, a beginning run of first-class CI with runlength greater than T6 is chosen. In some embodiments, a count of selected run of first-class CI is less than or equal to N1. In some embodiments, a run of first-class CI is chosen provided all associated TS satisfy a condition. In some embodiments, a run of first-class CI is chosen provided all associated TS is less than a threshold. At sub-operation 744, the system can classify sliding time window as Window-Class 3 if at least one selected run of first-class CI is chosen and compute MI/MS/STI based on the at least one selected run of first-class CI. At sub-operation 746, the system can classify sliding time window as Window-Class 2 if no selected run of first-class CI is chosen.

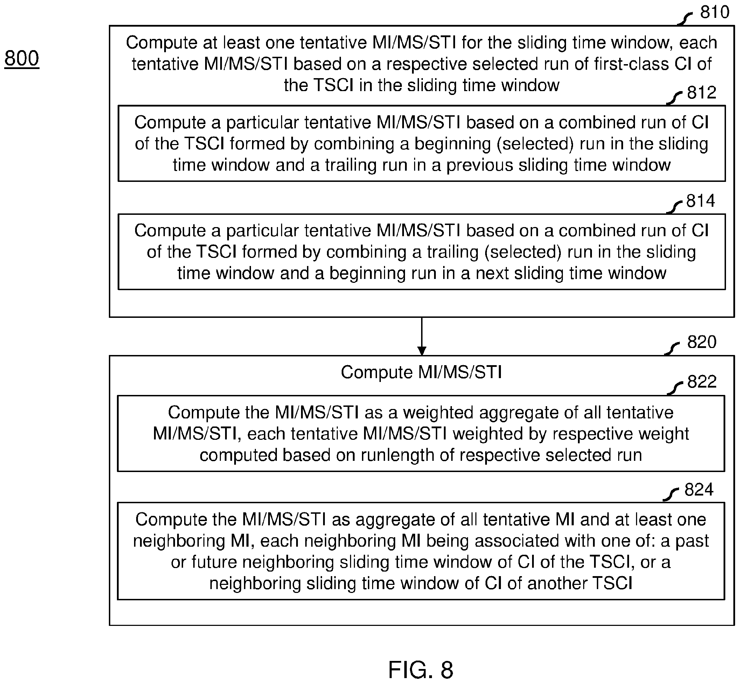

FIG. 8 illustrates a flow chart of an exemplary method 800 for computing MI/MS/STI, according to some embodiments of the present disclosure. In various embodiments, the method 800 can be performed by a system as disclosed above. At operation 810, the system can compute at least one tentative MI/MS/STI for the sliding time window, each tentative MI/MS/STI based on a respective selected run of first-class CI of the TSCI in the sliding time window, which may comprises sub-operations 812, 814. At sub-operation 812, the system may compute a particular tentative MI/MS/STI based on a combined run of CI of the TSCI formed by combining a beginning (selected) run in the sliding time window and a trailing run in a previous sliding time window. At sub-operation 814, the system may compute a particular tentative MI/MS/STI based on a combined run of CI of the TSCI formed by combining a trailing (selected) run in the sliding time window and a beginning run in a next sliding time window. At operation 820, the system can compute MI/MS/STI, by at least one of the sub-operations 822, 824. At sub-operation 822, the system may compute the MI/MS/STI as a weighted aggregate of all tentative MI/MS/STI, each tentative MI/MS/STI weighted by respective weight computed based on runlength of respective selected run. At sub-operation 824, the system may compute the MI/MS/STI as aggregate of all tentative MI and at least one neighboring MI, each neighboring MI being associated with one of: a past or future neighboring sliding time window of CI of the TSCI, or a neighboring sliding time window of CI of another TSCI.

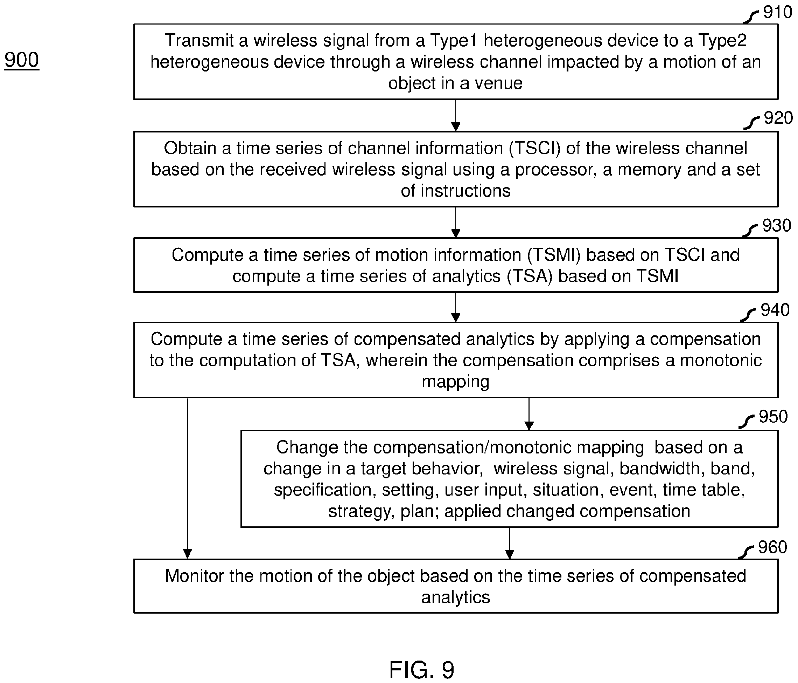

FIG. 9 illustrates a flow chart of an exemplary method 900 for performing a compensated wireless monitoring, according to some embodiments of the present disclosure. In various embodiments, the method 900 can be performed by one or more systems as disclosed above. At operation 910, a wireless signal is transmitted from a Type1 heterogeneous device to a Type2 heterogeneous device through a wireless channel impacted by a motion of an object in a venue. At operation 920, a time series of channel information (TSCI) of the wireless channel is obtained based on the received wireless signal using a processor, a memory and a set of instructions. At operation 930, the system can compute a time series of motion information (TSMI) based on TSCI and compute a time series of analytics (TSA) based on TSMI. At operation 940, the system may compute a time series of compensated analytics by applying a compensation to the computation of TSA, wherein the compensation comprises a monotonic mapping. In one embodiment, the system may change the compensation/monotonic mapping based on a change in a target behavior, wireless signal, bandwidth, band, specification, setting, user input, situation, event, time table, strategy, plan; applied changed compensation, at operation 950, and then monitor at operation 960 the motion of the object based on the time series of compensated analytics. In another embodiment, the system may directly monitor the motion of the object based on the time series of compensated analytics at operation 960, after performing operation 940.

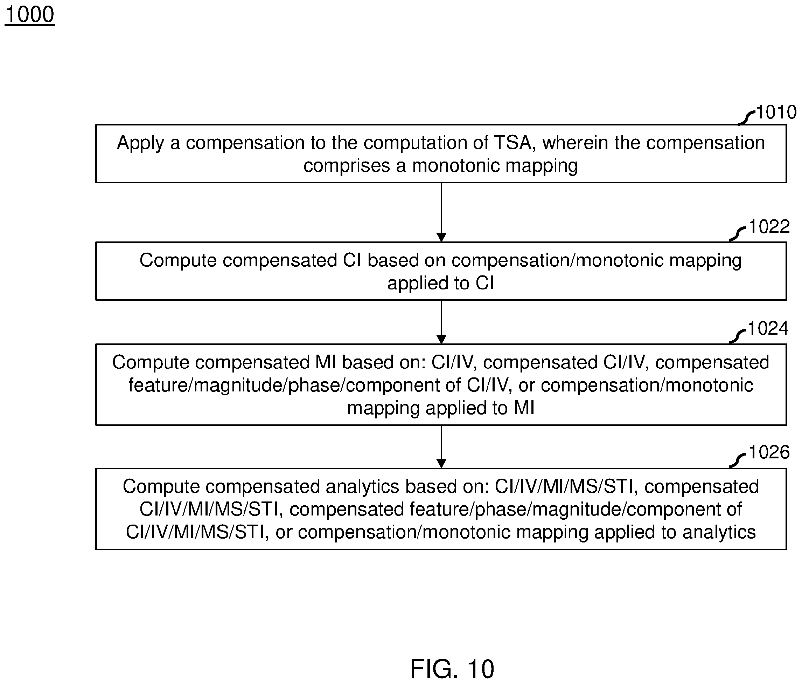

FIG. 10 illustrates a flow chart of an exemplary method 1000 for computing a time series of compensated analytics, according to some embodiments of the present disclosure. In various embodiments, the method 1000 can be performed by a system as disclosed above. At operation 1010, the system can apply a compensation to the computation of TSA, wherein the compensation comprises a monotonic mapping. In some embodiments, the monotonic mapping comprises at least one of: convex/concave/ univariate/ bivariate/multi-variate mapping, linear/nonlinear/ affine/piecewise-linear mapping, monotonic non-decreasing/ non-increasing/increasing/decreasing mapping, quadratic/ cubic/polynomial/exponential/logarithmic mapping, fitted/non-parametric/parametric/regression/spline mapping, function/ inverse function/ function of function, mapping of mapping/ composite mapping, time-varying/time-invariant mapping. In some embodiments, the compensation/monotonic mapping may comprise at least one compensation tailor-made for: (1) a pair of generic Type1 device and Type2 device, (2) the Type1 device, (3) the Type2 device or (4) the pair of the Type1 device and the Type2 device. In some embodiments, the compensation/monotonic mapping may be applied to at least one of: CI/IV/MI/MS/STI/analytics, feature/magnitude/phase/ component of CI/IV/MI/ MS/STI/analytics, TSCI/TSMI/TSA. At operation 1022, the system can compute compensated CI based on compensation/monotonic mapping applied to CI. At operation 1024, the system may compute compensated MI based on: CI/IV, compensated CI/IV, compensated feature/magnitude/phase/component of CI/IV, or compensation/monotonic mapping applied to MI. At operation 1026, the system may compute compensated analytics based on: CI/IV/MI/MS/STI, compensated CI/IV/MI/MS/STI, compensated feature/phase/magnitude/ component of CI/IV/MI/MS/STI, or compensation/monotonic mapping applied to analytics.

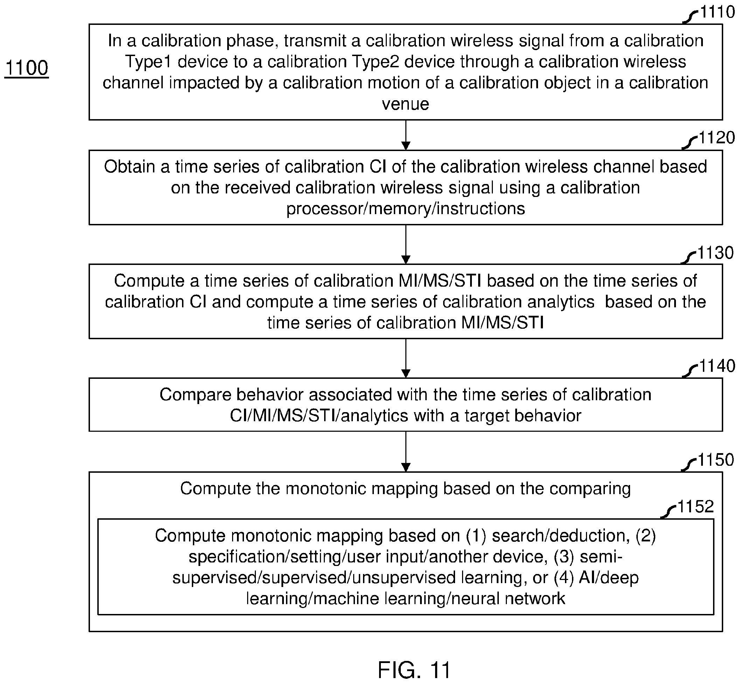

FIG. 11 illustrates a flow chart of an exemplary method 1100 for computing a monotonic mapping, according to some embodiments of the present disclosure. In various embodiments, the method 1100 can be performed by a system as disclosed above. At operation 1110, in a calibration phase, a calibration wireless signal is transmitted from a calibration Type1 device to a calibration Type2 device through a calibration wireless channel impacted by a calibration motion of a calibration object in a calibration venue. At operation 1120, a time series of calibration CI of the calibration wireless channel is obtained based on the received calibration wireless signal using a calibration processor/memory/instructions. At operation 1130, the system can compute a time series of calibration MI/MS/STI based on the time series of calibration CI, and compute a time series of calibration analytics based on the time series of calibration MI/MS/STI. At operation 1140, the system may compute behavior associated with the time series of calibration CI/MI/MS/STI/analytics with a target behavior. In some embodiments, the behavior comprises: feature/magnitude/phase/component behavior, statistical/time/frequency/ projection/transform behavior, uni-/bi-/multi-variate/conditional/cumulative/restricted distribution/histogram, behavior of CI/IV/MI/MS/ STI/analytics or associated observables, behavior restricted to time period, statistics, mean, median, percentile, max, min, range, variance, dispersion, variability, kurtosis, information, entropy, moment, correlation, covariance, ACF, etc. At operation 1150, the system may compute the monotonic mapping based on the comparing, which may comprise a sub-operation 1152, where the system may compute the monotonic mapping based on (1) search/deduction, (2) specification/setting/user input/another device, (3) semi-supervised/supervised/unsupervised learning, or (4) AI/deep learning/machine learning/neural network.

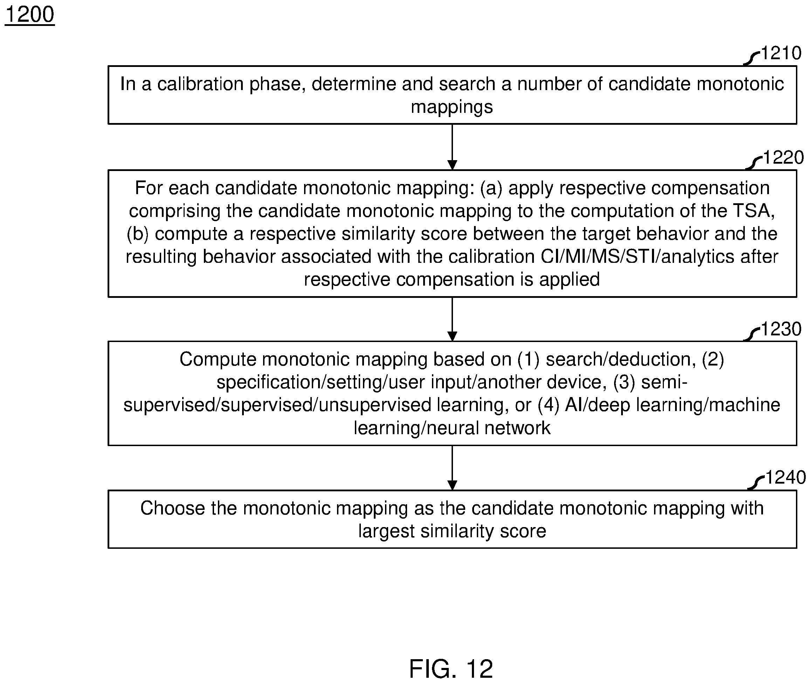

FIG. 12 illustrates a flow chart of an exemplary method 1200 for determining a monotonic mapping, according to some embodiments of the present disclosure. In various embodiments, the method 1200 can be performed by a system as disclosed above. At operation 1210, in a calibration phase, the system can determine and search a number of candidate monotonic mappings. At operation 1220, for each candidate monotonic mapping, the system can: (a) apply respective compensation comprising the candidate monotonic mapping to the computation of the TSA, (b) compute a respective similarity score between the target behavior and the resulting behavior associated with the calibration CI/MI/MS/STI/analytics after respective compensation is applied. At operation 1230, the system can compute monotonic mapping based on (1) search/deduction, (2) specification/setting/user input/another device, (3) semi-supervised/supervised/ unsupervised learning, or (4) AI/deep learning/machine learning/neural network. At operation 1240, the system may choose the monotonic mapping as the candidate monotonic mapping with largest similarity score.

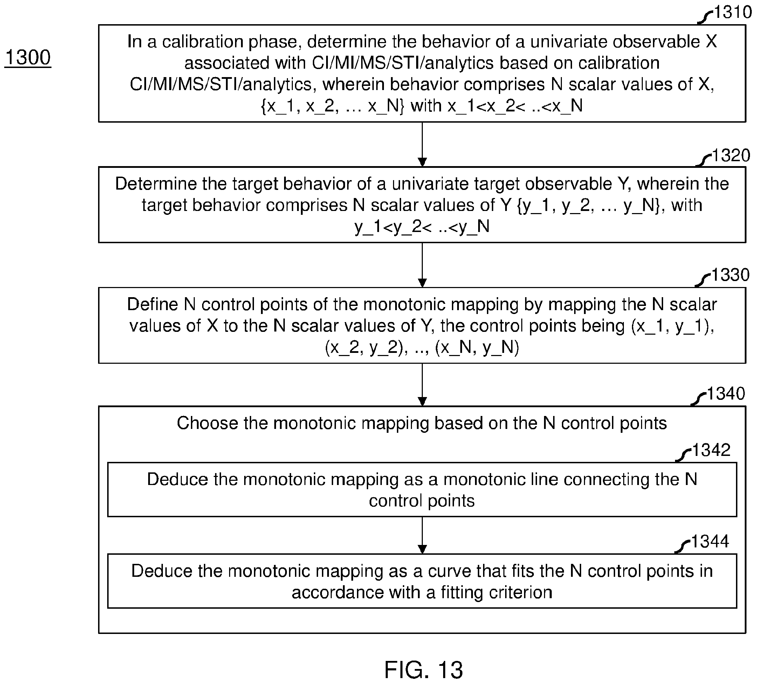

FIG. 13 illustrates a flow chart of an exemplary method 1300 for choosing a monotonic mapping, according to some embodiments of the present disclosure. In various embodiments, the method 1300 can be performed by a system as disclosed above. At operation 1310, in a calibration phase, the system can determine the behavior of a univariate observable X associated with CI/MI/MS/STI/analytics based on calibration CI/MI/MS/STI/ analytics, wherein behavior comprises N scalar values of X, $\{x\_1, x\_2, ... x\_N\}$ with $x\_1<x\_2< ..<x\_N$. At operation 1320, the system can determine the target behavior of a univariate target observable Y, wherein the target behavior comprises N scalar values of Y $\{y\_1, y\_2, ... y\_N\}$, with $y\_1<y\_2< ..<y\_N$. At operation 1330, the system may define N control points of the monotonic mapping by mapping the N scalar values of X to the N scalar values of Y, the control points being $(x\_1, y\_1), (x\_2, y\_2), .., (x\_N, y\_N)$. At operation 1340, the system may choose the monotonic mapping based on the N control points, which may comprise sub-operations 1342, 1344. At sub-operation 1342, the system may deduce the monotonic mapping as a monotonic line connecting the N control points. In some embodiments, the monotonic line comprises: linear/affine/quadratic/ cubic/polynomial/ exponential/logarithmic/convex/concave/spline map, piecewise linear/quadratic/cubic /polynomial map, or monotonic increase/non-decreasing map. At sub-operation 1344, the system may deduce the monotonic mapping as a curve that fits the N control points in accordance with a fitting criterion, e.g. using: linear/robust/orthogonal/Deming/ major axis/segmented/polynomial regression of N control points.

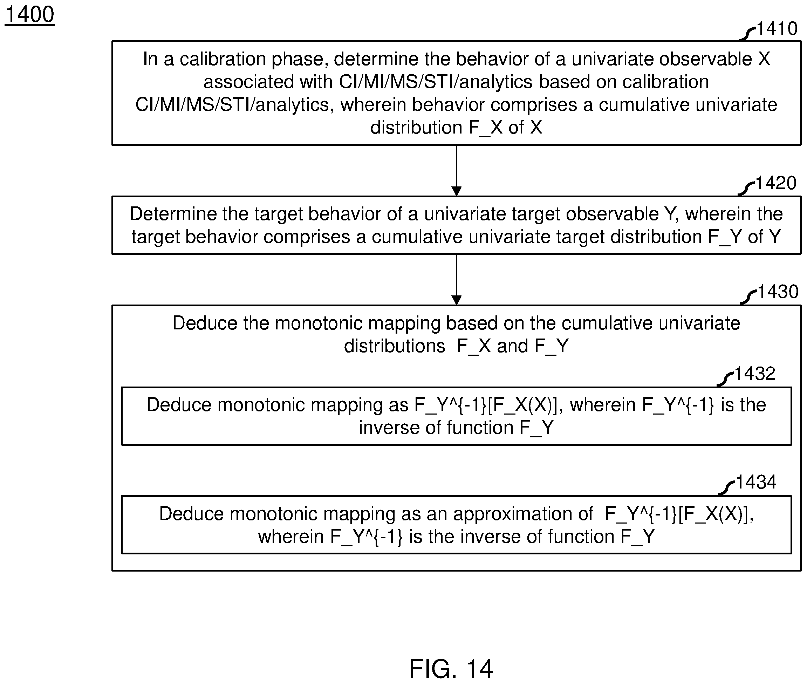

FIG. 14 illustrates a flow chart of an exemplary method 1400 for deducing a monotonic mapping, according to some embodiments of the present disclosure. In various embodiments, the method 1400 can be performed by a system as disclosed above. At operation 1410, in a calibration phase, the system can determine the behavior of a univariate observable X associated with CI/MI/MS/STI/analytics based on calibration CI/MI/MS/STI/analytics, wherein behavior comprises a cumulative univariate distribution $F\_X$ of X. At operation 1420, the system can determine the target behavior of a univariate target observable Y, wherein the target behavior comprises a cumulative univariate target distribution $F\_Y$ of Y. At operation 1430, the system may deduce the monotonic mapping based on the cumulative univariate distributions $F\_X$ and $F\_Y$, e.g. based on performing one of sub-operations 1432, 1414. At sub-operation 1432, the system may deduce monotonic mapping as $F\_Y^{-1}[F\_X(X)]$, wherein $F\_Y^{-1}$ is the inverse of function $F\_Y$. At sub-operation 1434, the system may deduce monotonic mapping as an approximation of $F\_Y^{-1}[F\_X(X)]$, wherein $F\_Y^{-1}$ is the inverse of function $F\_Y$. In some embodiments, the approximation comprises: linear/affine/quadratic/ cubic/polynomial/ exponential/logarithmic/convex/concave/spline map, piecewise linear/quadratic/cubic /polynomial map, or monotonic increase/non-decreasing map.

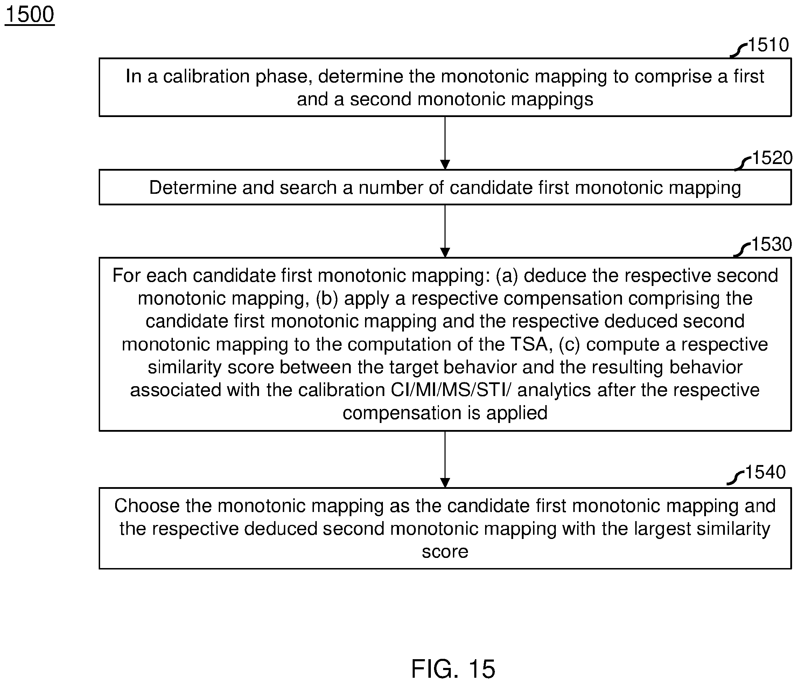

FIG. 15 illustrates a flow chart of an exemplary method 1500 for choosing a monotonic mapping, according to some embodiments of the present disclosure. In various embodiments, the method 1500 can be performed by a system as disclosed above. At operation 1510, in a calibration phase, the system can determine the monotonic mapping to comprise a first and a second monotonic mappings. At operation 1520, the system can determine and search a number of candidate first monotonic mapping. At operation 1530, for each candidate first monotonic mapping, the system may: (a) deduce the respective second monotonic mapping, (b) apply a respective compensation comprising the candidate first monotonic mapping and the respective deduced second monotonic mapping to the computation of the TSA, and (c) compute a respective similarity score between the target behavior and the resulting behavior associated with the calibration CI/MI/MS/STI/ analytics after the respective compensation is applied. At operation 1540, the system may choose the monotonic mapping as the candidate first monotonic mapping and the respective deduced second monotonic mapping with the largest similarity score.

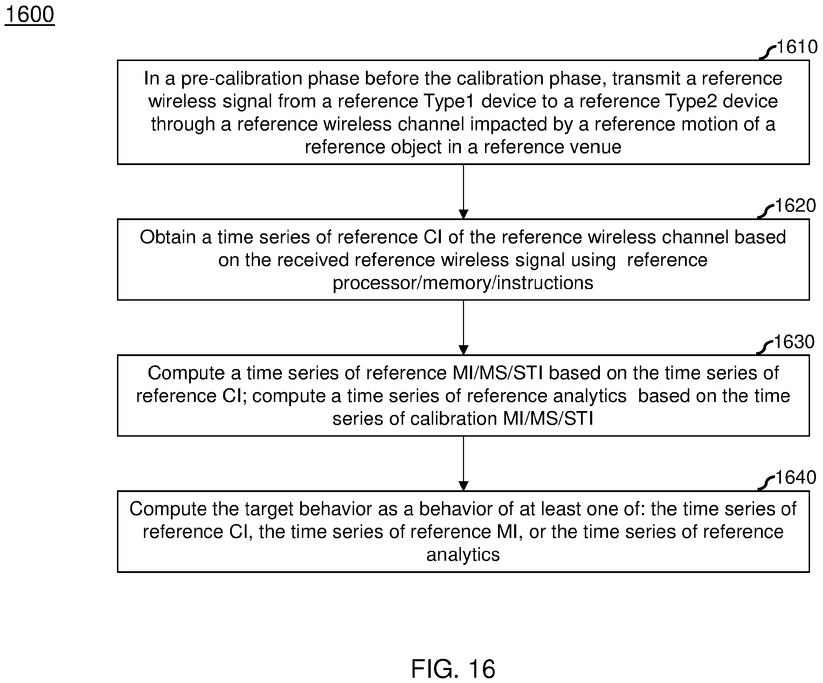

FIG. 16 illustrates a flow chart of an exemplary method 1600 for computing the target behavior, according to some embodiments of the present disclosure. In various embodiments, the method 1600 can be performed by a system as disclosed above. At operation 1610, in a pre-calibration phase before the calibration phase, a reference wireless signal is transmitted from a reference Type1 device to a reference Type2 device through a reference wireless channel impacted by a reference motion of a reference object in a reference venue. At operation 1620, a time series of reference CI of the reference wireless channel is obtained based on the received reference wireless signal using reference processor/memory/instructions. At operation 1630, the system can compute a time series of reference MI/MS/STI based on the time series of reference CI; compute a time series of reference analytics based on the time series of calibration MI/MS/STI. At operation 1640, the system may compute the target behavior as a behavior of at least one of: the time series of reference CI, the time series of reference MI, or the time series of reference analytics.

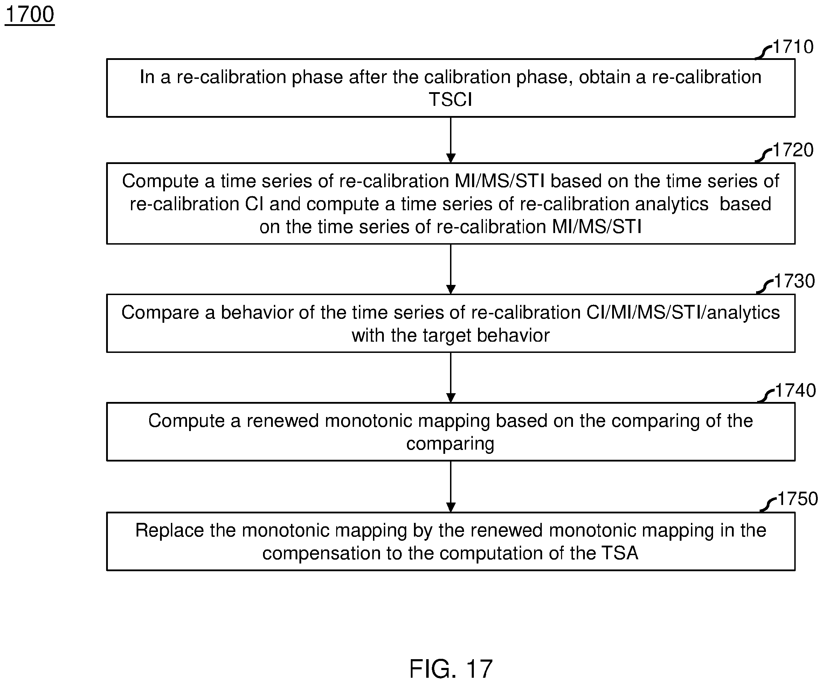

FIG. 17 illustrates a flow chart of an exemplary method 1700 for updating a monotonic mapping, according to some embodiments of the present disclosure. In various embodiments, the method 1700 can be performed by a system as disclosed above. At operation 1710, in a re-calibration phase after the calibration phase, the system can obtain a re-calibration TSCI. At operation 1720, the system can compute a time series of re-calibration MI/MS/STI based on the time series of re-calibration CI, and compute a time series of re-calibration analytics based on the time series of re-calibration MI/MS/STI. At operation 1730, the system can compare a behavior of the time series of re-calibration CI/MI/MS/STI/analytics with the target behavior. At operation 1740, the system may compute a renewed monotonic mapping based on the comparing of the comparing. At operation 1750, the system may replace the monotonic mapping by the renewed monotonic mapping in the compensation to the computation of the TSA.

The order of the operations in any one of the drawings herein may be changed according to various embodiments of the present teaching.

While a motion statistics (MS) or motion information (MI) may be computed in a sliding time window to perform wireless sensing and monitoring, one goal of the present teaching is to provide an accurate wireless monitoring by detecting, suppressing, removing and/or excluding outliers or effects of the outliers in a sliding time window. In some embodiments, a sliding time window may be classified to be "normal", "moderately abnormal" or "severely abnormal". Depending on the classification, the MS/MI for the sliding time window may be computed differently. When "normal", the MS/MI may be computed in a normal way. When "severely abnormal", the MS/MI may be computed, not based on CI in the sliding time window, but based on neighboring CI or MS/MI. When "moderately abnormal", the MS/MI may be computed based on the good/reliable (or "normal") CI in the sliding time window. Bad/unreliable (or "abnormal", or "severely abnormal") CI in the sliding time window will not be used to compute the MS/MI.

The following numbered clauses provide implementation examples for wireless monitoring.

Clause 1. A method for wireless monitoring, comprising: transmitting a wireless signal from a first wireless device through a wireless multipath channel of a venue, wherein the wireless multipath channel is impacted by a motion of an object in the venue; receiving the wireless signal by a second wireless device through the wireless multipath channel, wherein the received wireless signal differs from the transmitted wireless signal due to the wireless multipath channel and the motion of the object; obtaining a time series of channel information (TSCI) of the wireless multipath channel based on the received wireless signal using a processor, a memory communicatively coupled with the processor and a set of instructions stored in the memory; performing a classification of a sliding time window by analyzing channel information (CI) of the TSCI in the sliding time window; computing a motion information (MI) for the sliding time window based on the TSCI and the classification of the sliding time window; and monitoring the motion of the object based on the MI.

Clause 2. The method of clause 1, wherein the MI is computed based on at least one of: a similarity score of two temporally adjacent CI of the TSCI; an autocorrelation function (ACF) of the TSCI; or a characteristic point of the ACF.

Clause 3. The method of clause 1 or clause 2, further comprising: computing the MI in a first manner based exclusively on CI of the TSCI in the sliding time window when the sliding time window is classified as a first sliding-window-class based on the classification; computing the MI in a second manner based on at least one CI of the TSCI outside the sliding time window when the sliding time window is classified as a second sliding-window-class based on the classification; and computing the MI in a third manner based on a first subset of the CI of the TSCI in the sliding time window without using a second subset of the CI of the TSCI in the sliding time window, when the sliding time window is classified as a third sliding-window-class based on the classification, wherein the first subset and the second subset are disjoint.

Clause 4. The method of clause 3, further comprising: computing a test score (TS) for each CI of the TSCI in the sliding time window based on a number of respective temporally neighboring CI, wherein the TS comprises at least one of: a difference, a magnitude, a vector similarity, a vector dissimilarity, an inner product, or an outer product; and classifying each CI of the TSCI in the sliding time window based on the respective TS.

Clause 5. The method of clause 4, further comprising: computing a link-wise test score (LTS) based on an aggregate of all TS for the CI of the TSCI in the sliding time window; and performing the classification of the sliding time window based on the LTS.

Clause 6. The method of clause 5, wherein: the sliding time window is classified as the first sliding-window-class when the LTS is greater than a first threshold; and the sliding time window is classified as the second sliding-window-class or the third sliding window-class when the LTS is less than a second threshold.

Clause 7. The method of clause 6, wherein: each CI of the TSCI in the sliding time window is classified as a first CI-class when the respective TS is less than a third threshold, and classified as a second CI-class when the respective TS is greater than a fourth threshold.

Clause 8. The method of clause 7, wherein: the sliding time window is classified as the first sliding-window-class when all CI of the TSCI in the sliding time window are first-class CI classified as first CI-class; and the sliding time window is classified as the second sliding-window-class when all CI of the TSCI in the sliding time window are second-class CI classified as second CI-class, wherein the TSCI in the sliding time window comprises at least one first-class CI and at least one second-class CI.

Clause 9. The method of clause 8, further comprising: identifying at least one run of first-class CI and at least one run of second-class CI in the sliding time window, each run comprising a respective runlength of consecutive CI of a respective same CI-class in the sliding time window, wherein any runlength is one of: a number, a quantity or a count, that is greater than zero; and classifying the sliding time window based on the runs of first-class CI and second-class CI and the respective run-lengths.

Clause 10. The method of clause 9, further comprising: the sliding time window is classified as the third sliding-window-class when at least one selected run of first-class CI is chosen; and the sliding time window is classified as the second sliding-window-class when no selected run of first-class CI is chosen.

Clause 11. The method of clause 10, further comprising: choosing at least one selected run of first-class CI based on the runlength of each run of first-class CI and the TS associated with the run; and computing the MI based on the at least one selected run of first-class CI, wherein the at least one selected run is the at least one run of first-class CI with longest run-lengths among all runs of first-class CI in the sliding time window.

Clause 12. The method of clause 11, wherein: a first selected run is chosen as a beginning run of consecutive first-class CI comprising a CI being the first in the sliding time window when the respective run-length of the beginning run is greater than a first respective threshold; a second selected run is chosen as a trailing run of consecutive first-class CI comprising a CI being the last in the sliding time window when the respective run-length of the trailing run is greater than a second respective threshold; and any selected run that is not a beginning run or a trailing run is chosen when the respective run-length of the run is greater than a third respective threshold.

Clause 13. The method of clause 12, wherein: the at least one selected run is chosen such that a quantity of the at least one selected run is less than or equal to a predetermined number; and the at least one selected run is chosen such that, for each selected run, all the associated TS is less than a threshold.

Clause 14. The method of clause 13, further comprising: when the sliding time window is classified as the third sliding-window-class: constructing the first subset by including all of the at least one selected run of first-class CI of the TSCI in the sliding time window, and constructing the second subset by including all second-class CI of the TSCI in the sliding time window; and when the sliding time window is classified as the second sliding-window-class: computing the MI as an aggregate of at least one neighboring MI, wherein each neighboring MI is associated with one of: a past neighboring sliding time window of CI of the TSCI, a future neighboring sliding time window of CI of the TSCI, or a neighboring sliding time window of CI of another TSCI, wherein a neighboring MI is computed based on at least one CI of the TSCI outside the sliding time window.

Clause 15. The method of clause 14, further comprising: computing at least one tentative MI for the sliding time window, wherein each tentative MI is computed based on a respective selected run of first-class CI of the TSCI in the sliding time window; and computing the MI as an aggregate of the at least one tentative MI.

Clause 16. The method of clause 15, further comprising: determining a selected run as a beginning run of CI of the TSCI in the sliding time window; combining the beginning run of CI with a trailing run of CI of the TSCI in a previous sliding time window to form a combined run of CI of the TSCI; and computing a first tentative MI based on the combined run of CI.

Clause 17. The method of clause 15 or 16, further comprising: determining a selected run as a trailing run of CI of the TSCI in the sliding time window; combining the trailing run of CI with a beginning run of CI of the TSCI in a next sliding time window to form a combined run of CI of the TSCI; and computing a second tentative MI based on the combined run of CI.

Clause 18. The method of clause 15-17, further comprising: for each tentative MI computed based on a respective selected run, computing a respective computed weight based on the run-length of the respective selected run; and computing the MI as a weighted aggregate of the at least one tentative MI, wherein each tentative MI is weighted by the respective computed weight.

Clause 19. The method of clause 15-18, further comprising: computing the MI as an aggregate of the at least one tentative MI and at least one neighboring MI, wherein each neighboring MI is associated with one of: a past neighboring sliding time window of CI of the TSCI, a future neighboring sliding time window of CI of the TSCI, or a neighboring sliding time window of CI of another TSCI.

Clause 20. A wireless monitoring system, comprising: a first wireless device configured to transmit a wireless signal through a wireless multipath channel of a venue, wherein the wireless multipath channel is impacted by a motion of an object in the venue; a second wireless device configured to receive the wireless signal through the wireless multipath channel, wherein the received wireless signal differs from the transmitted wireless signal due to the wireless multipath channel and the motion of the object; and a processor configured for: obtaining a time series of channel information (TSCI) of the wireless multipath channel based on the received wireless signal using a processor, a memory communicatively coupled with the processor and a set of instructions stored in the memory, performing a classification of a sliding time window by analyzing channel information (CI) of the TSCI in the sliding time window, computing a motion information (MI) for the sliding time window based on the TSCI and the classification of the sliding time window, and monitoring the motion of the object based on the MI.

The following numbered clauses provide examples for an accurate wireless monitoring.

Clause A1. A method/device/system/software of a wireless monitoring system, comprising: transmitting a wireless signal from a Type1 heterogeneous wireless device of the wireless sensing system through a wireless multipath channel of a venue, wherein the wireless multipath channel is impacted by a motion of an object in the venue; receiving the wireless signal by a Type2 heterogeneous wireless device of the system through the wireless multipath channel, wherein the received wireless signal differs from the transmitted wireless signal due to the wireless multipath channel of the venue and the periodic vital sign motion of the object; obtaining a time series of channel information (CI) of the wireless multipath channel based on the received wireless signal using a processor, a memory and a set of instructions; performing a classification of a sliding time window by analyzing the CI of the TSCI in the sliding time window; computing a motion information (MI) for the sliding time window based on the TSCI and the classification of the sliding time window; monitoring the motion based on the MI.

In some embodiments, some characteristic steps used to compute the MS/MI in the wireless sensing, whether "normal", "abnormal", "moderately abnormal", or "severely abnormal".

Clause A2. The method/device/system/software of the wireless monitoring system of clause A1, comprising: computing the MI based on a similarity score of two temporally adjacent CI of the TSCI.

Clause A3. The method/device/system/software of the wireless monitoring system of clause A1 or A2, comprising: computing the MI based on an autocorrelation function (ACF) of the TSCI.

Clause A4. The method/device/system/software of the wireless monitoring system of clause A3, comprising: computing the MI based on a characteristic point of the ACF.

In some embodiments, each sliding time window may be classified as one of: "moderately abnormal", "severely abnormal" or "normal" sliding-window-classes. In some embodiments, first sliding-window-class ="normal" In some embodiments, second sliding-window-class ="severely abnormal" In some embodiments, third sliding-window-class= "moderately abnormal" In some embodiments, Third sliding-window-class= "moderately abnormal": In some embodiments, When the (current) sliding time window is classified as a "moderately abnormal" sliding-window class (third class), In some embodiments, Each CI in the current sliding time window may be classified as one of: "abnormal" or "normal" CI-classes. CI-level test score (TS) may be computed for each CI based on some CI in a respective temporal neighborhood of the CI. Each CI may be classified based on the respective TS (e.g. "normal" if TS<threshold T1).

In some embodiments, the second subset in clause A5 may be the collection of all the abnormal CI. The first subset may be the collection of all the normal CI (which may comprise one or more runs of normal CI, each run comprising a run-length of consecutive normal CI, with run-length>=1). The first subset may also be a subset of the collection of normal CI (e.g. only the normal CI in the longest run or the selected runs, or only the beginning run of normal CI, or only the trailing run of normal CI).

In some embodiments, a reduced MI may be computed based on the first subset (e.g. only the longest run of normal CI, or only the beginning run, or only the trailing run, or the selected runs). Alternatively, there may be multiple disjoint runs of normal CI. Some runs may be "satisfactory" (e.g. with sufficient run-length, or with all TS <T2<T1). Some runs may be not satisfactory. Multiple tentative reduced MI may be computed, each based on a respective disjoint run of (satisfactory) normal CI. A reduced MI may be computed as an aggregate of the multiple tentative reduced MI. The aggregate may be a weighted quantity (e.g. weighted sum, weighted average, weighted product, weighted median, etc.). The weight for each tentative reduced MI may be adaptively computed based on the run-length of the run of normal CI used to compute the tentative reduced MI. In some embodiments, the (current) MS/MI of the (current) sliding time window may be computed as the reduced MS/MI. Alternatively the current MS/MI may be computed as an aggregate (e.g. weighted average) of the reduced MS/MI and some neighboring MS/MI (e.g. past MI, future MI). Any MS/MI (e.g. current MI, past MI, future MI, reduced MI, tentative reduced MI) may be normalized (e.g. by the amount of CI used to compute the MS/MI). In some embodiments, each MS/MI may be computed based on any of: feature (e.g. magnitude, magnitude square, phase), decomposition of CI into multiple CI-components (CIC), decomposition of TSCI into multiple time series of CIC (TSCIC), similarity score (e.g. TRRS) between two temporally adjacent CI/CIC, ACF of CI/CIC, frequency transform/eigen-decomposition of CI/CIC, characteristic point/value (e.g. local max/local min/zero-crossing) of ACF/transform/decomposition, component processing, etc.

In some embodiments, second sliding-window class--"severely abnormal": when the (current) sliding time window is severely abnormal, the MS/MI may not be computed using the sliding time window. Instead, the MS/MI may be replaced by (i.e. computed as) some neighboring MS/MI (e.g. past MI, immediate past MI, future MI, next MI, or MI computed based on another TSCI) or an aggregate of them. In some embodiments, Any past MI may be associated with a respective past sliding time window and may be computed based on some past CI in the respective past sliding time window outside the current sliding time window. Similarly any future MI may be associated with a respective future sliding time window and may be computed based on some future CI in the respective future sliding time window outside the current sliding time window. In some embodiments, first sliding-window-class ="normal": In some embodiments, When everything is normal, the MI of each sliding time window may be computed based exclusively on the CI of TSCI in the sliding time window, without using any CI of TSCI outside the sliding time window.

Clause A5. The method/device/system/software of the wireless monitoring system of any previous A clause, comprising: when the sliding time window is classified as a first sliding-window-class: computing the MI in a first way based exclusively on the CI of the TSCI in the sliding time window; when the sliding time window is classified as a second sliding-window-class: computing the MI in a second way based on at least one CI of the TSCI outside the sliding time window; and when the sliding time window is classified as a third sliding-window-class: excluding a second subset of the CI of the TSCI in the sliding time window in the computation of the MI, and computing the MI in a third way based on a first subset of the CI of the TSCI in the sliding time window without using the second subset, wherein the first subset and the second subset are disjoint.

In some embodiments, to classify the sliding time window, each of the plurality of CI in the sliding time window may be classified, based on corresponding test score (TS). In some embodiments, for second (item-wise) classification, each CI may be classified into "normal" or "abnormal" CI-class.

Clause A6. The method/device/system/software of the wireless monitoring system of clause A5, comprising: computing a test score (TS) for each CI of the TSCI in the sliding time window; performing an item-wise classification of each CI of the TSCI in the sliding time window based on the respective TS.

Clause A6b. The method/device/system/software of the wireless monitoring system of clause A6, comprising: wherein each CI has N1 components; computing a component-wise test score (CTS) for each component of each CI of the TSCI in the sliding time window; computing the TS of each CI as an aggregate of the N1 CTS associated with the CI.

Clause A6c. The method/device/system/software of the wireless monitoring system of clause A6b, comprising: computing the CTS for a component of a CI based on the respective components of a number of respective temporally neighboring CI.

Clause A6d. The method/device/system/software of the wireless monitoring system of clause A6b or A6c, comprising: computing the TS of each CI as an aggregate of a selected subset of the N1 CTS associated with the CI, wherein the selected subset is selected based on a criterion.

In some embodiments, TS at time $t$, i.e. $TS(t)$, may be a function of temporally neighboring $CI(t-i)$, where $i=0,+-1, +-2, +-3, ...$ etc. In some embodiments, $TS(t)$ may be $TS(t)= CI(t)-pastCI(t)$, or $TS(t)=mag[CI(t)-pastCI(t)]^a$, where mag=magnitude, wherein $pastCI(t)$ may be $CI(t-1)$, or $[CI(t-1)+CI(t-2)]/2$, or $[2*CI(t-1)+CI(t-2)]/3$, or $[CI(t-1)+CI(t-2)+CI(t-3)]/3$, or $[2*CI(t-1)+CI(t-2)+CI(t-3)]/4$, or $[3*CI(t-1)+2*CI(t-2)+CI(t-3)]/6$, etc. In some embodiments, $TS(t)$ may also be $TS(t)= CI(t) - futureCI(t)$, or $TS(t)=mag[CI(t)-futureCI(t)]^b$, where mag=magnitude, wherein $futureCI(t)$ may be $CI(t+1)$, or $[CI(t+1)+CI(t+2)]/2$, or $[2*CI(t+1)+CI(t+2)]/3$, or $[CI(t+1)+CI(t+2)+CI(t+3)]/3$, or $[2*CI(t+1)+CI(t+2)+CI(t+3)]/4$, or $[3*CI(t+1)+2*CI(t+2)+CI(t+3)]/6$, etc. In some embodiments, $TS(t)$ may also be $TS(t)= CI(t) - d*pastCI(t) - (1-d)*future(t)$, where $0<d<1$.

Clause A7. The method/device/system/software of the wireless monitoring system of clause A6, A6b, or A6c, comprising: computing the TS for the CI based on a number of respective temporally neighboring CI, wherein the TS comprises at least one of: a test quantity, a similarity score, a dis-similarity score, a matching score, a mismatch score, a distance score, a TRRS, a correlation, a covariance, an autocorrelation, a cross-correlation, an inner product, a norm, an aggregate, a difference, an absolute difference, a square difference, a variance, a variation, a deviation, a standard deviation, a spread, a dispersion, a variability, a divergence, a skewness, a kurtosis, a range, an inter-quartile range, a coefficient, a Gini coefficient, an entropy, a maximum, a minimum, a mean, a median, a mode, a percentile, a quartile, a variance-to-mean ratio, a max-to-min ratio, a regularity, an irregularity, a statistics, a histogram, a probability, an impulsiveness, a suddenness, an occurrence, a recurrence, or a change.

In some embodiments, TS may comprise a difference (i.e. subtraction, or vector subtraction). In some embodiments, For a vector X (N-tuple), $F(X)$ may be a vector of same size (also N-tuple) such that $i^{th}$ component of $F(X)$ is a function of the $i^{th}$ component of X. The function may comprise any of: magnitude, magnitude square, phase, another function of magnitude, another function of phase.

Clause A8. The method/device/system/software of the wireless monitoring system of clause A6, A6b, A6c, or A7, comprising: wherein the TS comprises at least one of, or a magnitude of at least one of: $CI(t)-CI(t-1)$, $CI(t)-CI(t+1)$, $CI(t)-[CI(t-1)+CI(t+1)]/2$, $CI(t)-CI(t-2)$, $CI(t)-CI(t+2)$, $CI(t)-[CI(t-2)+CI(t+2)]/2$, $CI(t)-CI(t-k)$, $CI(t)-CI(t+1)$, $CI(t)-[CI(t-k)+CI(t+1)]/2$, $CI(t) -[a\_1*CI(t-1)+a 2*CI(t-2)+ ... +a\_k*CI(t-k)]/(a\_1+a\_2+...+a\_k)$, $CI(t)-[b\_1*CI(t+1)+b\_2*CI(t+2)+ ... +b\_1*CI(t+1)]/(b\_1+b\_2+...+b\_1)$, $CI(t)-[a\_1*CI(t-1)+a 2*CI(t-2)+ ... +a\_k*CI(t-k)+b\_1*CI(t+1)+b\_2*CI(t+2)+ ... +b\_1*CI(t+1)]/( a\_1+a\_2+...+a\_k+b-1+b\_2+...+b\_1)$, $F(CI(t))-F(CI(t-1))$, $F(CI(t))-F(CI(t+1))$, $F(CI(t))-[F(CI(t-1))+F(CI(t+1))]/2$, $F(CI(t))-F(CI(t-2))$, $F(CI(t))-F(CI(t+2))$, $F(CI(t))-[F(CI(t-2))+F(CI(t+2))]/2$, $F(CI(t))-F(CI(t-k))$, $F(CI(t))-F(CI(t+1))$, $F(CI(t))-[F(CI(t-k))+F(CI(t+1))]/2$, $F(CI(t)) -[a\_1*F(CI(t-1))+a\_2*F(CI(t-2))+ ... +a\_k*F(CI(t-k))]/(a\_1+a\_2+...+a\_k)$, $F(CI(t)) - [b-1*F(CI(t+1))+b-2*F(CI(t+2))+ ... +b\_1*F(CI(t+1))]/(b\_1+b\_2+...+b\_1)$, or $F(CI(t))-[a\_1*F(CI(t-1))+a\_2*F(CI(t-2))+ ... +a\_k*F(CI(t-k))+b\_1*F(CI(t+1))+b\_2*F(CI(t+2))+ ... +b\_1*F(CI(t+1))]/( a\_1+a\_2+...+a\_k+b\_1+b\_2+...+b\_1)$; wherein, for any vector X, $F(X)$ is a vector of same size of X such that the $i^{th}$ component of $F(X)$ is a function of the $i^{th}$ component of X; wherein each of $a\_1, a\_2, ..., a\_k, b\_1$,

b_2, ..., and b_l is a scalar; wherein CI(t) is a CI at time t.

In some embodiments, TS may comprise a vector dissimilarity/similarity, inner product, outer product.

Clause A9. The method/device/system/software of the wireless monitoring system of clause A6, A6b, A6c, A7 or A8, comprising: wherein the TS comprises at least one of: CI(t) x CI(t-1), CI(t) x CI(t+1), CI(t) x [CI(t-1)+CI(t+1)]/2, CI(t) x CI(t-2), CI(t) x CI(t+2), CI(t) x [CI(t-2)+CI(t+2)]/2, CI(t) x CI(t-k), CI(t) x CI(t+1), CI(t) x [CI(t-k)+CI(t+l)]/2, CI(t) x [a_1*CI(t-1)+a_2*CI(t-2)+ ... +a_k*CI(t-k)]/(a_1+a_2+...+a_k), CI(t) x [b_1*CI(t+1)+b_2*CI(t+2)+ ... +b_1*CI(t+1)]/(b_1+b_2+...+b_1), CI(t) x [a_1*CI(t-1)+a 2*CI(t-2)+ ... +a_k*CI(t-k)+b_1*CI(t+1)+b_2*CI(t+2)+ ... +b_1*CI(t+l)]/( a_1+a_2+...+a_k+b_1+b_2+...+b_1), F(CI(t)) x F(CI(t-1)), F(CI(t)) x F(CI(t+1)), F(CI(t)) x [F(CI(t-1))+F(CI(t+1))]/2, F(CI(t)) x F(CI(t-2)), F(CI(t)) x F(CI(t+2)), F(CI(t)) x [F(CI(t-2))+F(CI(t+2))]/2, F(CI(t)) x F(CI(t-k)), F(CI(t)) x F(CI(t+1)), F(CI(t)) x [F(CI(t-k))+F(CI(t+l))]/2, F(CI(t)) x [a_1*F(CI(t-1))+a_2*F(CI(t-2))+ ... +a_k*F(CI(t-k))]/(a_1+a_2+...+a_k), F(CI(t)) x [b_1*F(CI(t+1))+b_2*F(CI(t+2))+ ... +b_1*F(CI(t+1))]/(b_1+b_2+...+b_1), F(CI(t)) x [a_1*F(CI(t-1))+a_2*F(CI(t-2))+ ... +a_k*F(CI(t-k))+b_1*F(CI(t+1))+b_2*F(CI(t+2))+ ... +b_1*F(CI(t+1))]/( a_1+a_2+...+a_k+b_1+b_2+...+b_1); D[CI(t), CI(t-1)], D[CI(t), CI(t+1)], D[CI(t), [CI(t-1)+CI(t+1)]/2], D[CI(t), CI(t-2)], D[CI(t), CI(t+2)], D[CI(t), [CI(t-2)+CI(t+2)]/2], D[CI(t), CI(t-k)], D[CI(t), CI(t+1)], D[CI(t), [CI(t-k)+CI(t+l)]/2], D[CI(t), [a_1*CI(t-1)+a 2*CI(t-2)+ ... +a_k*CI(t-k)]/(a_1+a_2+...+a_k)], D[CI(t), [b_1*CI(t+1)+b_2*CI(t+2)+ ... +b_1*CI(t+1)]/(b_1+b_2+...+b_1)], D[CI(t), [a_1*CI(t-1)+a_2*CI(t-2)+ ... +a_k*CI(t-k)+b_1*CI(t+1)+b_2*CI(t+2)+ ... +b_1*CI(t+1)]/( a_1+a_2+...+a_k+b_1+b_2+...+b_1)], D[F(CI(t)), F(CI(t-1))], D[F(CI(t)), F(CI(t+1))], D[F(CI(t)), [F(CI(t-1))+F(CI(t+1))]/2], D[F(CI(t)), F(CI(t-2))], D[F(CI(t)), F(CI(t+2))], D[F(CI(t)), [F(CI(t-2))+F(CI(t+2))]/2], D[F(CI(t)), F(CI(t-k))], D[F(CI(t)), F(CI(t+1))], D[F(CI(t)), [F(CI(t-k))+F(CI(t+l))]/2], D[F(CI(t)), [a_1*F(CI(t-1))+a_2*F(CI(t-2))+ ... +a_k*F(CI(t-k))]/(a_1+a_2+...+a_k)], D[F(CI(t)), [b_1*F(CI(t+1))+b_2*F(CI(t+2))+ ... +b_1*F(CI(t+1))]/(b_1+b_2+...+b_1)], or D[F(CI(t)), [a_1*F(CI(t-1))+a_2*F(CI(t-2))+ ... +a_k*F(CI(t-k))+b_1*F(CI(t+1))+ b_2*F(CI(t+2))+ ... +b_1*F(CI(t+1))]/( a_1+a_2+...+a_k+b_1+b_2+...+b_1)]; wherein, for any vector A, F(A) is a vector of same size of A such that the $i^{th}$ component of F(A) is a function of the $i^{th}$ component of A; wherein each of a_1, a_2, ..., a_k, b_1, b_2, ..., and b_l is a scalar; wherein A x B is an inner product of a vector A and a vector B; wherein D[A, B] is a dissimilarity score between a vector A and a vector B; wherein CI(t) is a CI at time t.

In some embodiments, linkwise TS (LTS) for the sliding time window may be computed based on the all TS in the sliding time window. Classification of sliding time window may be based on LTS.

Clause A10. The method/device/system/software of the wireless monitoring system of clause A6, A6b, A6c, or A7-A9, comprising: computing a link-wise test score (LTS) based on the plurality of TS for the CI of TSCI in the sliding time window; performing the classification of the sliding time window based on the LTS.

In some embodiments, LTS may be aggregate (e.g. average, weighted average, median, max, min, percentile) of all TS in the sliding time window.

Clause A11. The method/device/system/software of the wireless monitoring system of clause A10, comprising: computing the LTS as an aggregate of the plurality of TS, wherein the aggregate comprises at least one of: a sum, a weighted sum, an average, a weighted average, a geometric mean, a weighted geometric mean, a harmonic mean, a weighted harmonic mean, an arithmetic mean, a weighted mean, a trimmed mean, a median, a weighted median, a mode, a histogram, a statistics, a percentile, a maximum, a minimum, a variance, a variation, a divergence, a spread, a range, a deviation, or a characteristic value.

In some embodiments, sliding time window may be classified as "normal" (first sliding-window-class) when LTS is greater (or smaller) than a threshold. E.g. "normal" when TS=dissimilarity score and LTS=max TS (or 95% percentile) and LTS > threshold; or "normal" when TS=similarity score and LTS=min TS (or 5% percentile), and LTS < threshold.

Clause A12. The method/device/system/software of the wireless monitoring system of clause A10 or A11, comprising: classifying the sliding time window as the first sliding-window-class when the LTS is greater than a first threshold; classifying the sliding time window as a class comprising the second sliding-window-class and the third sliding window-class when the LTS is less than a second threshold.

In some embodiments, LTS may be computed directly based on CI in sliding time window (without computing TS).

Clause A13. The method/device/system/software of the wireless monitoring system of any previous A clause, comprising: computing a link-wise test score (LTS) based on the CI of TSCI in the sliding time window, wherein the LTS comprises at least one of: a test quantity, a similarity score, a dis-similarity score, a matching score, a mismatch score, a distance score, a TRRS, a correlation, a covariance, an autocorrelation, a cross-correlation, an inner product, a norm, an aggregate, a difference, an absolute difference, a square difference, a variance, a variation, a deviation, a standard deviation, a spread, a dispersion, a variability, a divergence, a skewness, a kurtosis, a range, an inter-quartile range, a coefficient, a Gini coefficient, an entropy, a maximum, a minimum, a mean, a median, a mode, a percentile, a quartile, a variance-to-mean ratio, a max-to-min ratio, a regularity, an irregularity, a statistics, a histogram, a probability, an impulsiveness, a suddenness, an occurrence, a recurrence, or a change; performing the classification of the sliding time window based on the LTS.

In some embodiments, to classify each CI into multiple CI-classes, there may be a "normal" CI-class and/or an "abnormal" CI-class. There may be a "moderately abnormal" CI-class, a "severely abnormal" CI-class, and/or a "mildly abnormal" CI-class. In some embodiments, the first CI-class="normal", second CI-class="abnormal". In some embodiments, a CI may be classified as "abnormal" (or "severely abnormal") if TS>T4, and as "normal" if TS<T3<=T4. T4 may be equal to T3. If T3<T4, CI may be classified as "moderately abnormal" if T3<TS<T4. Alternatively, CI may be classified as "mildly abnormal" if T3<TS<T5, and "moderately abnormal" if T5<TS<T4. In some embodiments, second subset may comprise all "abnormal" CI. Second subset may be excluded from computation of MI. In some embodiments, first subset may comprise all "normal" CI (or all selected runs of normal CI). First subset, or a portion/subset of first subset, may be included in the computation of MI. In some embodiments, the first subset or the second subset may comprise "moderately abnormal" CI or "mildly abnormal" CI. In particular, first subset may comprise some/all "moderately abnormal" CI or "mildly abnormal" CI. In some embodiments, one or more reduced MI (or a tentative reduced MI) may be computed based on the first subset. Basically, "normal" CI may be more trustworthy than "mildly abnormal" CI, which in turn may be more trustworthy than "moderately abnormal" CI. A first reduced MI computed based purely on "normal" CI may be more "trustworthy" than a second reduced MI computed based on a combination of "normal" CI and "moderately abnormal" CI (or "mildly abnormal" CI). A third reduced MI computed based on (N-N1) "normal" CI and N1 "moderately abnormal" CI may be more "trustworthy" than a fourth reduced MI computed based on (N-N2) "normal" CI and N2 "moderately abnormal" CI, with N2>N1. In some embodiments, if MI comprises a weighted quantity (e.g. weighted average or weighted sum or weighted geometric mean, etc.) of the multiple reduced MI, a more trustworthy reduced MI (e.g. the first reduced MI, or the third reduced MI) may have a larger weight than a less trustworthy reduced MI (e.g. the second reduced MI or the fourth reduced MI). The weight for a reduced MI in the weighted quantity may depend on a count of "normal" CI, a count of "mildly abnormal" CI and a count of "moderately abnormal" CI used in the computation of the reduced MI. In some embodiments, if MI comprises a weighted quantity of a reduced MI and some "neighboring MI" from "neighboring sliding time windows" and/or "neighboring TSCI". The weight for the reduced MI in the weighted quantity may depend on a count of "normal" CI, a count of "mildly abnormal" CI and a count of "moderately abnormal" CI used in the computation of the reduced MI.

Clause A14. The method/device/system/software of the wireless monitoring system of clause A6, A6b, A6c, or A7-A13, comprising: in the second (item-wise) classification, classifying each CI as a first CI-class when the respective TS is less than a third threshold, and as a second CI-class when the respective TS is greater than a fourth threshold.

In some embodiments, the first CI-class="normal", second CI-class="abnormal". In some embodiments, not all "normal" CI may be used in the computation of MI. First subset may comprise only some (e.g. all, or not all) of the "normal" CI. E.g. MI may be computed based a longest run of "normal" CI. In other words, the second, third, fourth ... longest runs of "normal" CI may/may not be used in the computation of MI. In some embodiments, first subset may comprise all of the "normal" CI. In some embodiments, first sliding-window-class ="normal" In some embodiments, second sliding-window-class ="severely abnormal." In some embodiments, third sliding-window-class= "moderately abnormal." In some embodiments, If all (or a great majority) CI are "normal" (first CI-class), the sliding time window may be classified as "normal" (first sliding-window-class). In some embodiments, if all (or a great majority) CI are "abnormal" (second CI-class), the sliding time window may be classified as "severely abnormal" (second sliding-window-class). In some embodiments, great majority may be 80% or 90% or 95% or 99% or 99.9% or another percentage.

Clause A15. The method/device/system/software of the wireless monitoring system of clause A14, comprising: classifying the sliding time window as the first sliding-window-class when all CI of the TSCI in the sliding time window are first-class CI; classifying the sliding time window as the second sliding-window-class when all CI of the TSCI in the sliding time window are classified as second CI-class; wherein any first-class CI is a CI being classified as first CI-class, wherein any second-class CI is a CI being classified as second CI-class.

In some embodiments, if there is at least one first-class CI (i.e. not all CI are second-class) and at least one second-class CI (i.e. not all CI are first-class), there will be at least one run of first-class CI and at least one run of second-class CI, each run with a run-length of consecutive CI of the same-class. In some embodiments, the classification of the sliding time window may be based on the run-length and the plurality of TS associated with each run (especially the runs of first-class CI).

Clause A16. The method/device/system/software of the wireless monitoring system of clause A15, comprising: wherein there are at least one first-class CI and at least one second-class CI of the TSCI in the sliding time window; identifying at least one run of first-class CI and at least one run of second-class CI in the sliding time window, each run comprising a respective run-length of consecutive CI of a respective same class in the sliding time window, wherein any run-length is one of: a number, a quantity or a count, greater than zero; classifying the sliding time window based on the runs of first-class CI and second-class CI and the respective run-lengths.

In some embodiments, some "selected" run(s) of "normal" CI may be chosen (or selected or identified). The "selected" run(s) must satisfy certain criteria/requirements/conditions. If at least one selected run can be found (and used to compute reduced MS/MI), the sliding time window may be classified as "moderately abnormal" (third class). Otherwise, the sliding time window may be classified as "severely abnormal" (second class).

Clause A17. The method/device/system/software of the wireless monitoring system of clause A16, comprising: classifying the sliding time window as the third sliding-window-class when at least one selected run of first-class CI is chosen; classifying the sliding time window as the second sliding-window-class when no selected run of first-class CI is chosen.

In some embodiments, some "selected" run(s) of "normal" CI (first-class CI) may be chosen (or selected or identified).

Clause A18. The method/device/system/software of the wireless monitoring system of clause A17, comprising: choosing at least one selected run of first-class CI based on the run-length of each run of first-class CI and the plurality of TS associated with the run; computing the MI based on the at least one selected run of first-class CI.

In some embodiments, one "selected" run may be the run with longest run-length in the sliding time window.

Clause A19. The method/device/system/software of the wireless monitoring system of clause A18, comprising: wherein a particular selected run is the run of first-class CI with longest run-length among all runs of first-class CI in the sliding time window.

In some embodiments, the at least one selected run may be those with longest run-length in the sliding time window.

Clause A20. The method/device/system/software of the wireless monitoring system of clause A19, comprising: wherein the at least one selected run is the at least one run of first-class CI with longest run-lengths among all runs of first-class CI in the sliding time window.

In some embodiments, one "selected" run may be the beginning run with sufficient run-length. A run with very short run-length may not be sufficient to compute MI with good accuracy. In some embodiments, run-length requirement of beginning run or trailing run may be less stringent than the other runs. In other words, the run-length threshold (e.g. in clause A21 or clause A22) for beginning run or trailing run may be smaller (i.e. less stringent) than the run-length threshold for the other runs (e.g. in clause A23).

Clause A21. The method/device/system/software of the wireless monitoring system of clause A18-A20, comprising: wherein a particular selected run is chosen as a beginning run of consecutive first-class CI comprising the very first CI in the sliding time window when the respective run-length of the beginning run is greater than a respective threshold.

In some embodiments, one "selected" run may be the trailing run with sufficient run-length.

Clause A22. The method/device/system/software of the wireless monitoring system of clause A18-A21, comprising: wherein a particular selected run is chosen as a trailing run of consecutive first-class CI comprising the very last CI in the sliding time window when the respective run-length of the trailing run is greater than a respective threshold.

In some embodiments, all "selected" run (except the beginning run or trailing run) may have sufficient run-length.

Clause A23. The method/device/system/software of the wireless monitoring system of clause A18-A22, comprising: wherein any selected run that is not a beginning run or a trailing run is chosen when the respective run-length of the run is greater than a respective threshold.

In some embodiments, the amount of selected run may be upper-bounded by a predetermined number.

Clause A24. The method/device/system/software of the wireless monitoring system of clause A18-A23, comprising: wherein the at least one selected run is chosen such that a count of the at least one selected run is less than or equal to a predetermined number.

In some embodiments, the amount of selected run may be a predetermined number.

Clause A25. The method/device/system/software of the wireless monitoring system of clause A24, comprising: wherein the at least one selected run is chosen such that a count of the at least one selected run is a predetermined number.

In some embodiments, a selected run may have all associated TS satisfy a condition.

Clause A26. The method/device/system/software of the wireless monitoring system of clause A18-A25, comprising: wherein the at least one selected run is chosen such that, for each selected run, all the associated TS satisfy a condition.

In some embodiments, a selected run may have associated TS less than a threshold.

Clause A27. The method/device/system/software of the wireless monitoring system of clause A18-A26, comprising: wherein the at least one selected run is chosen such that, for each selected run, all the associated TS is less than a threshold.

In some embodiments, there may be more than one selected runs. A tentative reduced MS/MI may be computed based on each selected run. The MS/MI (or reduced MS/MI) may be computed as an aggregate of more than one tentative reduced MS/MI.

Clause A28. The method/device/system/software of the wireless monitoring system of clause A18-A27, comprising: computing at least one tentative MI for the sliding time window, each tentative MI based on a respective selected run of first-class CI of the TSCI in the sliding time window; computing the MI as an aggregate of the at least one tentative MI.

In some embodiments, beginning run of (current) sliding time window may be combined with trailing run of CI of previous sliding time window to form a combined run of CI. In some embodiments, a tentative reduced MS/MI may be computed based on the combined run of CI.

Clause A29. The method/device/system/software of the wireless monitoring system of clause A28 comprising: computing a particular tentative MI based on a selected run which is a beginning run of CI of the TSCI in the sliding time window; combining the beginning run of CI with a trailing run of CI of the TSCI in a previous sliding time window to form a combined run of CI of the TSCI; computing the particular tentative MI based on the combined run of CI.

In some embodiments, trailing run of (current) sliding time window may be combined with beginning run of CI of next sliding time window to form a combined run of CI. In some embodiments, a tentative reduced MS/MI may be computed based on the combined run of CI.

Clause A30. The method/device/system/software of the wireless monitoring system of clause A28 or A29, comprising: computing a particular tentative MI based on a selected run which is a trailing run of CI of the TSCI in the sliding time window; combining the trailing run of CI with a beginning run of CI of the TSCI in a next sliding time window to form a combined run of CI of the TSCI; computing the particular tentative MI based on the combined run of CI.

In some embodiments, the MS/MI (or reduced MS/MI) may be a weighted aggregate (e.g. weighted sum/average/mean/ median/product) of more than one tentative reduced MS/MI. In some embodiments, the weight of each tentative reduced MS/MI may be computed adaptively based on the run-length of the respective selected run of first-class CI used to compute the tentative reduced MS/MI. The weight of each tentative reduced MS/MI may also be computed adaptively based on the plurality of TS associated with the respective selected run.

Clause A31. The method/device/system/software of the wireless monitoring system of clause A28-A30, comprising: for each tentative MI computed based on a respective selected run, computing a respective computed weight based on the run-length of the respective selected run, computing the MI as a weighted aggregate of the at least one tentative MI, each tentative MI weighted by the respective computed weight.

In some embodiments, the MS/MI (or reduced MS/MI) may be a weighted aggregate (e.g. weighted sum/average/mean/ median/product) of one or more tentative reduced MS/MI and at least one neighboring MS/MI. Each neighboring MI may be associated with a past neighboring sliding time window, a future neighboring sliding time window, or a neighboring (past, current or future) time window of CI of another TSCI, which may be obtained based on another wireless signal communicated between the Type1 device and the Type2 device. For example, the another wireless signal may be the wireless signal in clause A1.

Clause A32. The method/device/system/software of the wireless monitoring system of clause A28-A31, comprising: computing the MI as an aggregate of the at least one tentative MI and at least one neighboring MI, wherein each neighboring MI is associated with one of: a past neighboring sliding time window of CI of the TSCI, a future neighboring sliding time window of CI of the TSCI, or a neighboring sliding time window of CI of another TSCI.

In some embodiments, when sliding time window is classified as "moderately abnormal" (third sliding-window-class), MS/MI may be computed using the first subset of CI of TSCI in the sliding time window without using the second subset. First subset may comprise all "normal" (first-class) CI of the TSCI in the sliding time window. Second subset may comprise all "abnormal" (second-class) CI of the TSCI in the sliding time window.

Clause A33. The method/device/system/software of the wireless monitoring system of clause A14-A32, comprising: when the sliding time window is classified as the third sliding-window-class: constructing the first subset by including all first-class CI of the TSCI in the sliding time window, and constructing the second subset by including all second-class CI of the TSCI in the sliding time window.

In some embodiments, first subset may comprise only the at least one selected run of "normal" (first-class) CI of the TSCI in the sliding time window. In some embodiments, second subset may comprise all "abnormal" (second-class) CI of the TSCI in the sliding time window.

Clause A34. The method/device/system/software of the wireless monitoring system of clause A18-A33, comprising: when the sliding time window is classified as the third sliding-window-class: constructing the first subset by including all of the at least one selected run of first-class CI of the TSCI in the sliding time window, and constructing the second subset by including all second-class CI of the TSCI in the sliding time window.

In some embodiments, second sliding-window class--"severely abnormal" In some embodiments, The MI may not be computed based on the CI of the TSCI in the (current) sliding time window, because the CI are too abnormal/unreliable (i.e. severely abnormal). In some embodiments, a replacement MI may be computed based on an aggregate of a number of neighboring MI. Each neighboring MI may be associated with a past/future/neighboring sliding time window that is classified as "normal".

Clause A35. The method/device/system/software of the wireless monitoring system of clause A17-A34, comprising: when the sliding time window is classified as the second sliding-window-class: computing the MI as an aggregate of at least one neighboring MI, wherein each neighboring MI is associated with one of: a past neighboring sliding time window of CI of the TSCI, a future neighboring sliding time window of CI of the TSCI, or a neighboring sliding time window of CI of another TSCI, wherein a neighboring MI is compute based on at least one CI of the TSCI outside the sliding time window.

In some embodiments, a degree of similarity between current channel information (CI) and a previous CI may be computed for wireless sensing. CI may comprise any of channel response (CR), channel impulse response (CIR), channel frequency response (CFR), channel state information (CSI), received signal strength (RSS), or RSS indicator (RSSI).

The following numbered clauses provide examples for wireless sensing and positioning.

Clause B1. A wireless sensing system comprising: a receiver configured to receive a wireless signal transmitted from a transmitter through a wireless channel, and one or more data processors configured to: determine a channel information (CI) based on the received wireless signal, wherein the CI comprises at least one of: channel response, channel impulse response (CIR), channel frequency response (CFR), channel state information (CSI), received signal strength (RSS), or RSS indicator (RSSI), comparing the CI with a previous CI obtained based on a previously received wireless signal, compute a degree of similarity between the CI and the previous CI, wherein the degree of similarity comprise at least one of: a time-reversal resonating strength (TRRS), a pattern recognition, a matching, a distance, a Euclidean distance, a correlation, an autocorrelation function, or a cosine function, and compute at least one of the following items based on the degree of similarity: a location of a target, an arrangement of the target, a configuration of the target, a distance from a wireless access point device, a distance between the transmitter and the receiver, an identification of the target, a motion of the target, a proximity of the target, a position of the target, an orientation of the target, a pose of the target, a presence of the target, a state of the target, an information of an arrangement of the target, a change, an indication of a change, a detection of the target, a characteristics of the target, a gesture, a fall of the target to the ground, a navigation, a guidance, a targeted advertising, a state in a venue, or a condition of the venue, wherein the target is one of: the receiver, the transmitter, an object, a person or an event.

In some embodiments, there are more than one wireless signals. More than one CI may be obtained. Composite CI may be computed based on the more than one CI.

Clause B2. The system of Clause B1, further comprising: at least one receiver each configured to receive a respective wireless signal transmitted from a respective transmitter through a respective wireless channel, and the one or more data processors configured to: determine at least one CI based on the at least one received wireless signal, comparing one of the at least one CI with a respective previous CI obtained based on a respective previously received wireless signal, compute the degree of similarity between the CI and the respective previous CI, determine a composite CI based on the at least one wireless signal and the at least one CI, comparing the composite CI with a previous composite CI, wherein the previous composite CI is determined based on at least one previously received wireless signal and at least one previous CI, wherein the at least one previous CI is determined based on the at least one previously received wireless signal, compute a second degree of similarity between the composite CI and the previous composite CI, wherein the second degree of similarity is computed based on at least one of: the TRRS between two CI, a second TRRS between two composite CI, the pattern recognition based on CI, a second pattern recognition based on composite CI, the matching based on CI, a second matching based on composite CI, the distance, a second distance based on composite CI, the Euclidean distance, a second Euclidean distance based on composite CI, the correlation based on CI, a second correlation based on composite CI, the autocorrelation function based on CI, a second autocorrelation function based on composite CI, a cosine function based on CI, or a second cosine function based on composite CI, and compute the at least one of the computed items based on at least one of: the degree of similarity or the second degree of similarity.

In some embodiments, wireless signal may comprise more than one probe signals. A sequence of CI may be obtained.

Clause B3. The system of Clause B1 or B2, wherein: wherein the wireless signal comprises a sequence of probe signals transmitted periodically; wherein a sequence of CI is determined based on the received wireless signal, one CI for each probe signal.

In some embodiments, if the current CI is an outlier, it may be discarded.

Clause B4. The system of any previous B Clause, wherein the one or more data processors is further configured to: compare the CI with a plurality of other previous CI; determine the CI to be an outlier CI when a degree of similarity of the CI and the plurality of other previous CI is less than a threshold; and discard the outlier CI.

In some embodiments, discarded outlier CI may be replaced by another CI (a good CI).

Clause B5. The system of any previous B Clause, wherein the one or more data processors is further configured to: replace the CI by another CI obtained based on another received wireless signal transmitted from a respective transmitter to a respective receiver, compute the degree of similarity based on the another CI instead of the CI, the degree of similarity being between the another CI and the previous CI.

In some embodiments, if a particular CI is an outlier, it may be discarded.

Clause B6. The system of any previous B Clause, wherein the one or more data processors is further configured to: compare a particular CI of the at least one CI with a plurality of other previous CI; determine the particular CI to be an outlier CI when a degree of similarity of the particular CI and the plurality of other previous CI is less than a threshold; and discard the particular CI.

In some embodiments, discarded outlier CI may be replaced by another CI (a good CI).

Clause B7. The system of Clause B6, wherein the one or more data processors is further configured to: replace the particular CI by another CI obtained based on another received wireless signal transmitted from a respective transmitter to a respective receiver, compute the composite CI based on the another CI instead of the particular CI.

In some embodiments, if the current CI is innovative/new compared with some reference CI, it may be used to replace one of the reference CI.

Clause B8. The system of any previous B Clause, wherein the one or more data processors is further configured to: compare the CI with a plurality of reference CI in a database; determine the CI to be new when a degree of similarity of the CI and the plurality of reference CI is less than a threshold; and update the database by replacing a particular reference CI in the database by the CI.

In some embodiments, if current CI is "similar" to past CI, the channel/venue may be considered stationary/static.

Clause B9. The system of Clause B1, wherein the one or more data processors is further configured to: compare the CI with a plurality of other previous CI; determine the wireless channel is stationary when a degree of similarity of the CI and the plurality of other previous CI is greater than a threshold; compute the at least one of the computed items based on the stationarity of the wireless channel.

In some embodiments, the transmitter and receiver and CI may need to be qualified (for some task). In some embodiments, if current CI is "similar" to reference CI, the transmitter/receiver/CI may be considered good enough/qualified.

Clause B10. The system of any previous B Clause, wherein the one or more data processors is further configured to: determine the CI determined based on the received wireless signal communicated between the transmitter and the receiver is accurate enough when a degree of similarity of the CI and the plurality of other previous CI is greater than a threshold.

In some embodiments, CI may have multiple components (e.g. due to subcarrier).

Clause B11. The system of Clause B2-B10: wherein each respective wireless signal comprises a respective amount of subcarriers, a respective frequency band, a respective bandwidth; wherein each CI comprises more than one CI-components, each CI-component associated with a subcarrier.

In some embodiments, transmitter, receiver, wireless signal and CI may be standard/protocol compliant.

Clause B12. The system of Clause B2-B10: wherein each respective wireless signal is communicated between the respective transmitter and the respective receiver in compliance with a wireless signaling protocol or standard; wherein the wireless signaling protocol or standard comprises at least one of the following wireless signaling protocols or standards: IEEE 802.11, IEEE 802.11xx, IEEE 802.15, IEEE 802.15.3, IEEE 802.15.4, WiFi, 3G/LTE/4G/5G/6G/7G/8G, Bluetooth, BLE, Zigbee, UWB, NFC, a wireless data transmission standard or protocol, a wireless data communication standard or protocol, a wireless network standard or protocol, a cellular network standard or protocol, a wireless local area network (WLAN) standard, OFDM, OFDMA, or CDMA.

In some embodiments, wireless signal may comprise standard/protocol compliant frames/packets.

Clause B13. The system of Clause B12: wherein each wireless signal comprises one or more frames having a format that complies with the wireless signaling standard or protocol.

In some embodiments, CI may be obtained based on standardized mechanism.

Clause B14. The system of Clause B12 or B13: wherein the CI is determined based on a standardized mechanism according to the wireless signaling standard or protocol.

In some embodiments, CI may be obtained based on preamble in standard compliant frame.

Clause B15. The system of Clause B13 or B14: wherein the CI is determined based on a long preamble of one of the frames.

In some embodiments, CI may be obtained based on standard/protocol-compliant signaling (control signals, parameters being negotiated).

Clause B16. The system of Clause B14 or B15: wherein the CI is determined based on parameters and control signals passed between the transmitter and the receiver, in accordance with the wireless signaling standard or protocol.

Clause B17. The system of any previous B Clause, wherein the one or more data processors is further configured to: the CI is determined based on at least one of: a discretization, a derivation, an "accurate-enough" determination and a desired accuracy of system performance.

In some embodiments, CI may be obtained based on preamble in wireless signal.

Clause B18. The system of any previous B Clause: wherein the CI is determined from a received wireless signal based on a preamble of the wireless signal.

Clause B19. The system of any previous B Clause: wherein the CI is processed to mitigate variations in the CI owing to at least one of: imperfect transmission by the respective transmitter, imperfect reception by the respective receiver, imperfect timing, imperfect frequency synchronization, channel frequency offset (CFO), or sampling frequency offset (SFO).

In some embodiments, CSI is reported locally.

Clause B20. The system of any previous B Clause: wherein a CI determined based on a respective received wireless signal is obtained and processed locally in a device receiving the respective received wireless signal.

In some embodiments, CI may be processed remotely by another device.

Clause B21. The system of any previous B Clause: wherein a CI determined based on a respective received wireless signal is processed remotely in another device different from a device receiving the respective received wireless signal.

In some embodiments, CSI is reported to another device (sensing initiator in 802.11bf).

Clause B22. The system of Clause B21: wherein the CI is reported (or transmitted) from the device to the another device.

Clause B23. The system of any previous B Clause, wherein one or more data processors is further configured to: detect a change based on the comparing and the degree of similarity.

In some embodiments, transmitter and receiver may be in same device.

Clause B24. The system of any previous B Clause further comprises: a same device comprising one of the transmitter and one of the receiver.

In some embodiments, user is allowed to enter sensing related info.

Clause B25. The system of any previous B Clause, wherein one or more data processors is further configured to: enable a user to provide a user-provided information, computing the at least one of the computed items based on the user-provided information and the degree of similarity.

Clause B26. The system of Clause B25, wherein: the user-provided information comprises at least one of: a first identifier associated with the transmitter, a second identifier associated with the receiver, a service set identifier (SSID) associated with a wireless network access point in the venue.

Clause B27. The system of any previous B Clause, wherein the one or more data processors is further configured to: determine an identifier of the transmitter or the receiver.

Clause B28. The system of Clause B25-B27: wherein the user-provided information comprise an identifier of a location or arrangement.

In some embodiments, there are multiple transmitters with same receiver (many-to-one), same transmitter with multiple receivers (one-to-many), multiple transmitters with multiple receivers (many-to-many).

Clause B29. The system of any previous B Clause, wherein one or more data processors is further configured to: wherein at least one of the following is true: the at least one transmitter is a same transmitter, the at least one receiver is a same receiver, or the at least one wireless channel is a same wireless channel associated with a same wireless network.

In some embodiments, for multicast, broadcast, unicast, tracker bot may be carried by and moves with each mobile object.

Clause B30. The system of Clause B29, wherein one or more data processors is further configured to: wherein the at least one transmitter is a same transmitter which transmits the at least one wireless signal in a uni-cast, multi-cast or broadcast manner; wherein each of at least one receiver is a respective terminal device to be carried by a respective user for locating the location of the respective user.

In some embodiments, there are many Tx and one Rx.

Clause B31. The system of Clause B29 or B30, wherein one or more data processors is further configured to: wherein the at least one receiver is a same receiver; wherein each of the at least one transmitter is a respective terminal device to be carried by a respective user for locating the location of the respective user.

Clause B32. The system of any previous B Clause, wherein one or more data processors is configured with at least one of a software or a firmware of the system.

In some embodiments, degree of confidence may be computed (e.g. for qualifying the Tx/Rx/CI/system).

Clause B33. The system of any previous B Clause, wherein one or more data processors is further configured to: compute a degree of confidence associated with the at least one of the computed items.

In some embodiments, degree of confidence may be computed in the form of an "accuracy" (e.g. for a sensing task).

Clause B34. The system of any previous B Clause, wherein one or more data processors is further configured to: compute an accuracy based on the CI.

In some embodiments, magnitude of CI may be used to compute similarity.

Clause B35. The system of any previous B Clause, wherein one or more data processors is further configured to: wherein the degree of similarity is computed based on an amplitude of the CI.

In some embodiments, phase of CI may be used to compute similarity.

Clause B36. The system of any previous B Clause, wherein one or more data processors is further configured to: wherein the degree of similarity is computed based on a phase of the CI.

In some embodiments, amount of CI may be determined to be combined to form composite CI.

Clause B37. The system of Clause B2-B36, wherein one or more data processors is further configured to: determine a number of individual CI to be included in the composite channel response to achieve a desired accuracy.

In some embodiments, a "preferred" (e.g. default) amount of CI is to be combined to form composite CI.

Clause B38. The system of Clause B2-B37: wherein a number of individual CI are preferable to be included in the composite channel response.

Clause B38b. The system of Clause B2-B37: wherein a number of individual CI associated with respective transmitters and respective receivers are preferred.

Clause B38c. The system of Clause B38b: wherein the number of individual CI and the respective transmitters are preferred.

Clause B38d. The system of Clause B38b or B38c: wherein the number of individual CI and the respective receivers are preferred.

In some embodiments, each CI is associated with a Tx antenna and a Rx antenna.

Clause B39. The system of any previous B Clause: wherein each CI is determined for a pairing of a transmit antenna of the transmitter and a receive antenna of the receiver.

In some embodiments, feature value (e.g. magnitude, phase, spatial-temporal info(STI), motion info(MI)) may be computed based on CI.

Clause B40. The system of any previous B Clause, wherein the one or more data processors are further configured to: determine a feature value based on a mathematical function performed on the CI and the previous CI; compute the at least one of the computed items based on the feature value.

In some embodiments, dimension reduction of CI may be performed before feature value is computed.

Clause B41. The system of Clause B40, wherein the one or more data processors are further configured to: determine the feature value of the CI based on a dimension reduction of the CI.

In some embodiments, feature value may be computed based on the similarity score.

Clause B42. The system of Clause B40 or B41, wherein the one or more data processors are further configured to: determine the feature value based on the degree of similarity between the CI and the previous CI.

In some embodiments, there may be multiple feature values. They may be normalized.

Clause B43. The system of any previous B Clause, wherein the one or more data processors are further configured to: determine a set of feature values based on a mathematical function performed on the CI and a set of previous CI; normalize the set of feature values; perform the at least one computation based on the set of normalized feature values.

In some embodiments, Tx and Rx are housed in respective devices.

Clause B44. The system of any previous B Clause, wherein: the transmitter is housed in a first device; the receiver is housed in a second device.

In some embodiments, Tx (e.g. sensing transmitter) may be AP, or non-AP.

Clause B45. The system of any previous B Clause, wherein: the transmitter is one of: a wireless network router, a wireless network access point (AP), a wireless terminal device, or a wireless non-AP terminal device.

In some embodiments, Rx (e.g. sensing receiver) may be AP, or non-AP.

Clause B46. The system of any previous B Clause, wherein: the receiver is one of: a wireless network router, a wireless network access point (AP), a wireless terminal device, or a wireless non-AP terminal device.

In some embodiments, Rx (e.g. sensing receiver) may not be associated (e.g. in 802.11bf).

Clause B47. The system of any previous B Clause, wherein: the receiver is not associated with the network router and the wireless network access point.

In some embodiments, CSI may be sent to the cloud.

Clause B48. The system of any previous B Clause, wherein the one or more data processors are further configured to: provide the CI to another device.

In some embodiments, computation is non-local (e.g. CSI reported from sensing receiver to sensing initiator in 802.11bf, and sensing computation is NOT done in sensing receiver).

Clause B49. The system of Clause B48, wherein: the receiver is part of a first device; one of the one or more data processors is part of a second device; the CI is reported from the first device (or the receiver) to the second device (or the one or more data processors).

In some embodiments, CSI is reported in a report frame in 802.11bf.

Clause B50. The system of Clause B49, wherein: the CI is reported in a report generated by the system.

In some embodiments, CSI is reported in a report frame in 802.11bf, with an initialization (sensing measurement initiation phase in 802.11bf).

Clause B51. The system of any previous B Clause, wherein: the system or a service associated with the system is initiated.

In some embodiments, probe signal transmission is based on a trigger signal (NDP transmission triggered by trigger frame (TF) or NDP announcement frame (NDPA) in trigger-based (TB) sensing in 802.11bf).

Clause B52. The system of Clause B51, wherein: the transmission of the wireless signal from the transmitter to the receiver of the system is initiated based on a trigger signal.

Clause B53. The system of any previous B Clause in which a data processor of a user device of a user is configured to provide a user interface (UI) to present to the user a second information about the at least one of the computed items.

Clause B54. The system of Clause B53 in which the user interface is configured to display a map and enable the user to indicate a location associated with the CI on the map.

Clause B55. The system of any previous B Clause in which the one or more data processors are configured to determine a first identifier of the target or the object, associate the CI with the first identifier, and store the first identifier along with the CI.

Clause B56. The system of any previous B Clause in which the one or more data processors are configured to obtain a prediction, and compute the at least one of the computed items based on the CI and the prediction.

Clause B57. The system of Clause B56 in which the one or more data processors are configured to compare the CI with the prediction and compute the at least one of the computed items based on the comparison.

Clause B58. The system of Clause B57 wherein the prediction comprises a predicted CI.

Clause B59. The system of Clause B57 or B58 wherein the prediction comprises a coarse position data received by the one or more data processors.

Clause B60. The system of Clause B57-B59 wherein the prediction comprises a coarse location data computed by the one or more data processors based on a mapping, wherein the receiver is previously mapped to a particular location based on the mapping, wherein a more-precious location of the transmitter is computed based on the mapping.

Clause B61. The system of Clause B60 in which the one or more data processors are configured to: determine the coarse position based on at least one of Wi-Fi, Bluetooth, or cellular signals, and use a time-reversal positioning system to determine a fine position of the transmitter based on the coarse position.

Clause B62. The system of any previous B Clause, comprising: a storage device storing a three-dimensional rendering of a venue, and a 3D electromagnetic simulator configured to generate the multiple channel paths based on data derived from the three-dimensional rendering of the venue.

Clause B63. The system of any previous B Clause, comprising: a storage device storing a three dimensional model of a venue that is constructed using at least one of photos, images, videos, ranging techniques such as laser ranging, optical coherence tomography, or echo location, wherein the 3D electromagnetic simulator is configured to generate a database of location-specific estimated Cis in the venue using the three dimensional model.

Clause B64. A system comprising: a receiver configured to receive a wireless signal transmitted from a transmitter through a wireless channel, and one or more data processors configured to: determine a channel information (CI) based on the received wireless signal, wherein the CI comprises at least one of: channel response, channel impulse response (CIR), channel frequency response (CFR), channel state information (CSI), received signal strength (RSS), or RSS indicator (RSSI), execute a 3D electromagnetic simulator to perform a 3D electromagnetic simulation to predict multiple channel paths between the transmitter and the receiver, determine a predicted CI based on predicted multiple channel paths between the transmitter and the receiver, at least one of (i) train the 3D electromagnetic simulator based on a comparison of the CI and the predicted CI to generate a trained 3D electromagnetic simulator, or (ii) calibrate the 3D electromagnetic simulator based on a comparison of the CI and the predicted CI to generate a calibrated 3D electromagnetic simulator, use the trained or calibrated 3D electromagnetic simulator to generate a database of location-specific estimated Cis in a venue, and establish a mapping between locations in the venue and corresponding Cis.

Clause B65. The system of Clause B64, comprising: a storage device storing a three-dimensional rendering of a venue, and the 3D electromagnetic simulator configured to predict the multiple channel paths based on data derived from the three-dimensional rendering of the venue.

Clause B66. The system of Clause B64 or B65, comprising: a storage device storing a three dimensional model of a venue that is constructed using at least one of photos, images, videos, ranging techniques such as laser ranging, optical coherence tomography, or echo location, wherein the 3D electromagnetic simulator is configured to generate the database of location-specific estimated Cis in the venue using the three dimensional model.

Clause B67. The system of Clause B64-B66, comprising: a storage device storing parameters comprising reflection coefficients of different objects in a venue, wherein the parameters have multiple values to account for one or more environmental factors comprising at least one of temperature, humidity, or smog, and wherein the 3D electromagnetic simulator is configured to predict the multiple channel paths based on data derived from the parameters.

Clause B68. The system of Clause B64-B67 in which the one or more data processors are configured to use the database of location-specific estimated CIs in a venue and the mapping to determine a position of a terminal device within the venue.

Clause B69. The system of Clause B64-B68 in which the one or more data processors are configured to: receive a probe signal from a terminal device, determine a CI based on the received probe signal, and compare the CI with location-specific estimated CIs in the database to identify a closest match.

A motion of an object in a venue may be monitored based on a TSCI obtained based on a wireless signal transmitted from a Type1 device to a Type2 device. A motion statistics (MS)/motion information (MI)/spatial-temporal-information (STI) may be computed based on the TSCI. An analytics may be computed based on the MS/MI/STI. Often there may be many possible different choices of Type1 device(s). And there may be many possible different choices of Type2 devices.

Different choices of Type1 device (or Type2 device or both) may cause device-dependent behavior (e.g. shifting/mapping/distortion/systematic variation which may be linear/affine/nonlinear/monotonic/time-invariant/time varying/device-dependent/stochastic) of CI/TSCI/ MS/MI/STI/analytics (e.g. large motion may cause a first MS/MI/STI to be around 0.9 and a second MS to be around 0.75; or NIL/no motion may cause the first MS/MI/STI to be around 0.1 and the second MS to be around 0.3). The device-dependent nature of the behavior is undesirable. In some embodiments, a device-dependent compensation is disclosed to "undo" or compensate the device-dependent behavior to make it independent, being similar to some reference/desirable/standard behavior. The undesirable behavior/mapping may resemble/comprise/be modeled as any of: shifting, linear/affine/piecewise-linear/nonlinear mapping, monotonic mapping, monotonic increasing (or non-decreasing) mapping, monotonic decreasing (or non-increasing) mapping, etc.

For example, choices of Type1 or Type2 device may comprise: smart speaker, thermostat, TV, sound bar, DVD player, streaming device, lamp, bulb, plug, door bell, camera, router, etc. Choices of Type1 or Type2 device may also comprise items (e.g. speaker) from different brands/models/packaging/parts/ components (e.g. Amazon Echo, Echo Dot, Echo Dot generation 1, Echo Dot gen2, Echo Dot gen3, Echo Show, Google Nest, Nest Mini, Nest Hub, or other brands). Even for a particular model of a particular brand (e.g. Echo Dot), different devices may still be different (e.g. due to variation/defects/wear-and-tear/degradation/depreciation in/of circuit components/parts/amplifiers/antennas/chips/wirings/fabrication/assembling/ manufacturing/firmware/software). In particular, there may be different antenna count and/or antenna type (e.g. with different gain, type, size, directional characteristics, radiation pattern). All these may cause undesirable behavior of CI/TSCI/MS/MI/STI/analytics. Undesirable behavior may also arise due to different bands (e.g. 2.4GHz, 5GHz, 6GHz) or different bandwidth (e.g. 20/40/80/160/320/640/1280 MHz) used for wireless signal. Undesirable behavior may arise from different amount of antennas (e.g. Echo may have 3 antennas while Echo Dot may have 2 antennas) or different choice of antennas (e.g. dipole, micro-strip, slot antenna, waveguide, spiral antenna, omni-directional antenna, directional antenna, etc.). Undesirable behavior may arise from different placement of devices/device antennas, different transmission power of wireless signal, different amplifier setting, different digital/analog gain, or different placement of objects, or environment elements such as mechanical/electrical/ electromagnetic interference.

In some embodiments, compensation may be applied to compensate for undesirable behavior. In some embodiments, the MS/MI/STI/analytics may be compensated based on a compensation to get/give/obtain compensated MS/MI/STI/analytics. One or more separate/respective compensation(s) may be applied. After the compensation, all compensated MS/MI/STI/analytics for any choice of Type1/Type2 devices may have similar "target behavior" - taking on similar values in response to same motion of object regardless of the choices of Type1/Type2 devices. The similar target behavior may be a user-preferred/pre-defined/user-defined/user-designed/ user-specified behavior (e.g. MS/MI/STI being around 0.8 for large motion and being around 0.2 for NIL/no motion), or a "reference" (or "representative" or "generic") behavior associated with a reference/ representative/generic Type1/Type2 device.

In some embodiments, the user-defined/designed/specified/preferred behavior may be programmable/adjustable. It may be specified by user in a setting of the system. It may be specified once and remained unchanged. Or, it may be changed over time. In particular, it may change adaptively based on a strategy/time table/user-intervention/user-real-time-adjustment, or in reaction to events/situations, perhaps as specified in a setting of the system.

In some embodiments, each Type1 device (or Type2 device) may be tested/calibrated/trained in a controlled manner (e.g. in a testing/controlled environment, with a testing setting and procedure, for a testing task) in order to compute/obtain/train a respective compensation, or a respective sequence of compensation. Any compensation may be a pre-processing/processing/ post-processing to be applied in/before/after the computation of the MS/MI/STI/analytics. For CI-compensation, a compensation may be applied to a CI, a feature of the CI, a group/sliding window of CI, a TSCI, a number of TSCI, a number of TSCI associated with a particular antenna of Type1 device, a number of TSCI associated with a particular antenna of Type2 device. For intermediate value compensation, a compensation may be applied to intermediate value (or a number of them), which may be computed based on CI/CI feature/sliding window of CI/TSCI, compensated CI/CI feature/sliding window of CI/TSCI, MS/MI/STI and/or compensated MS/MI/STI. For MS/MI/STI compensation, a compensation may be applied to the MS/MI/STI/analytics (or a number of them), which may be computed based on CI/CI feature/sliding window of CI/TSCI, compensated CI/CI feature/sliding window of CI/TSCI, intermediate values, and/or compensated intermediate values. For analytic compensation, a compensation may be applied to the analytic, which may be computed based on TSCI, compensated CI/CI feature/sliding window of CIITSCI, MS/MI/STI, compensated MS/MI/STI, intermediate values, compensated intermediate values. In some embodiments, the respective sequence of compensation may comprise any of the above compensation. Compensation may be adaptive, time-varying or time invariant. Compensation may be different for different frequency band (e.g. 2.4GHz, 5GHz, 6GHz, 24GHz, 60GHz, 77GHz) or bandwidth (e.g. 20MHz, 40MHz, 80 MHz, 160MHz, 320MHz, 640MHz, 1280MHz) of the wireless signal.

In some embodiments, the compensation may comprise a mapping: univariate/bivariate/ multivariate mapping, mapping of real/imaginary component, mapping of magnitude/phase, mapping of feature, monotonic mapping, piecewise monotonic mapping, monotonic increasing/decreasing mapping, monotonic non-decreasing/non-increasing mapping, piecewise linear mapping, linear mapping, DC adjustment, affine mapping, composite mapping, mapping of mapping. The mapping may be time-varying (e.g. adaptively modified) or time-invariant. The mapping may be spatially varying or spatially independent. The mapping may/may not be varying with respect to frequency band/bandwidth. The mappings may be the same or different for different sensing tasks (e.g. motion detection, breathing detection/estimation, fall-down detection, presence detection, gait/gesture detection/estimation, etc.).

In some embodiments, in a calibration/tuning/customization/factory-testing/ pre-shipping-testing/training phase of a pair of Type1 device and Type2 device for a wireless sensing task, a compensation may be computed for the pair of Type1 device and Type2 device for the wireless sensing task. (In some embodiment, the Type1 device may be a reference/typical/representative Type1 device and the Type2 device may need to be calibrated/tuned/ customized/tested/trained such that the computed compensation is for the Type2 device for the wireless sensing task. In another embodiment, the Type2 device may be a reference/typical/ representative Type2 device and the Type1 device is to be calibrated/tuned/customized/tested/trained such that the computed compensation is for the Type1 device for the wireless sensing task.) In some embodiments, in the calibration/tuning/ customization/testing/training phase, a time series of training/calibration wireless signals (e.g. training sounding signal) may be transmitted from a calibration Type1 device (e.g. the Type1 device or a reference/representative Type1 device) to a calibration Type2 device (e.g. the Type2 device or a reference/representative Type2 device) through a training/calibration wireless multipath channel of a training/calibration venue, wherein the training/calibration wireless multipath channel is impacted by a training/calibration motion of a training/calibration object for a training/calibration period of time. The training/calibration wireless signal may span some or all possible bands of the wireless (sounding) signal. Training/calibration TSCI may be obtained/extracted from the received time series of training/ calibration wireless signals. Training/calibration MI/MS/STI for the wireless sensing task may be computed based on the training/calibration TSCI.

In some embodiments, the compensation may be computed based on a behavior of the training CI/TSCI/MI/MS/STI/analytics/ intermediate values. The behavior may comprise any of: statistical behavior, temporal/frequency/ transform/projection domain behavior, CI/TSCI/MS/MI/STI/analytics domain behavior, magnitude/ phase/component behavior, histogram, univariate/bivariate/multivariate histogram, restricted histogram, conditional histogram, histogram between two time stamps, histogram restricted to be between the two time/frequency/transform/projection domain stamps (i.e. a range in time/frequency/transform/projection domain), histogram between a local maximum and a local minimum, histogram between a local maximum and a local minimum conditioned on a range (e.g. magnitude range or time range or frequency range) between the local max and local min exceeding a threshold, histogram between a local maximum and a local minimum after a filtering/processing (e.g. lowpass/bandpass/highpass/median filtering), statistics, conditional statistics, trimmed statistics, first/second/higher order statistics, mean, weighted mean, arithmetic mean, trimmed mean, geometric mean, harmonic mean, median, mode, maximum, minimum, zero crossing, percentile, range, variance, magnitude, phase, etc. The statistical behavior may be compared with a target behavior (e.g. target statistical behavior, target temporal/frequency/transform/projection domain behavior, target CI/TSCI/MS/MI/STI/analytics behavior). The target behavior may be associated with a target MS. The target behavior may be the behavior of the target MS. The target behavior may be a user-preferred/pre-defined/ user-defined/user-designed/user-specified/user-selected/user-chosen behavior (e.g. specified/provided/designed/defined/inputted/ selected/chosen by user using some user-interface of a user device). The target behavior may be learned (e.g. in a "pre-calibration"/reference behavior generation/ learning procedure/ phase) from one (or more) "reference" Type1 device and/or one (or more) "reference" Type2 device performing one (or more) reference sensing task (e.g. the sensing task) by transmitting one (or more) time series of reference wireless (sounding) signals from the Type1 device through one (or more) reference wireless channel in one (or more) reference venue to the Type2 device, the reference wireless channel being impacted by a reference motion of a reference object. One or more "reference" TSCI may be obtained/extracted from the received time series of reference wireless sounding signals. Reference MS/MI/STI may be computed based on the reference TSCI. Reference analytics may be computed based on the reference MS/MI/STI. The resulting behavior (or aggregate/average/characteristic/ clustered behavior) may be used as the target behavior. In some embodiments, the time series of wireless sounding signal may use a 40MHz band. The time series of reference wireless sounding signal may cover/scan/use some or all possible 40MHz bands.

In some embodiments, a compensation may be a mapping that may be deduced, or obtained by searching. For deduced mapping, a compensation may be a mapping that may be deduced from the comparison of the behavior and the target behavior such that, when the mapping is applied, the behavior (the "compensated behavior") matches/approaches/resembles the target behavior. In some embodiments, the behavior (and the target behavior) may be a univariate histogram (e.g. marginal distribution or cumulative distribution) of a magnitude of a MS. The mapping may map a MS value to a compensated MS value such that the behavior (univariate histogram) of the compensated MS value matches (or approximate, or resemble) the target behavior (a target univariate histogram).

In some embodiments, a number of "control points" may be determined for the univariate histogram of MS and for the target univariate histogram. The control points may comprise any of: mean (the mean value of MS, or the mean value of the target MS), mode, median (median value of MS), percentile point (percentile value of MS), 0% point, 1% point, 5% point, 10% point, 20% point, 30% point, 40% point, 50% point, 60% point, 70% point, 80% point, 90% point, 95% point, 99% point, 100% point, other percentile point. Each pair of corresponding control points of the MS histogram and the target histogram may together form/become an ordered pair (x, y) or a point on an x-y plot (or a function, y=f(x)), where x is the MS value and y is the target MS value. For example, (10% point of MS, 10% point of target MS) is an ordered pair that constitutes a "control point" in the x-y plot. Straight lines (or polynomial lines or piecewise spline) may be used to connect the control points such that a monotonic non-decreasing (or monotonic increasing) mapping y=f(x) is obtained. The compensation may be the mapping y=f(x) obtained by connecting the control points using some kind of monotonic line (e.g. straight line, quadratic function, cubic function, polynomial function, exponential function, logarithmic function, convex function, concave function, etc.). In this way, the control points are in/on the mapping function y=f(x). In some embodiments, line fitting (e.g. least square, linear regression, robust linear regression, orthogonal regression, Deming regression, major axis regression, segmented regression, polynomial regression, regression dilution) may be applied to obtain/compute the mapping for the compensation. The line fitting may be applied piecewise. The resulting mapping may be monotonic increasing or non-increasing. In this way, there may be one or more control points that are not in/on the mapping.

In some embodiments, there may be two control points 0% point and 100% point such that all MS values between the 0% MS point and the 100% MS point may be mapped to 0% target MS point and 100% target MS point. The line may be a straight line, or quadrate, or cubic, or polynomial, other monotonic line or a regression line. Or, 1%point and 99%point such that essentially all MS values are mapped to target MS values. Outside the range of 1%point and 99%point, mapping may be an identical function (i.e. y=f(x)=x). In some embodiments, there may be three control points which may be {0% point, 50% point (median) and 100% point}, or {1% point, 50% point, 99% point}, or {0% point, 40% point, 100% point} or {1% point, 30% point, 99% point}, etc. In some embodiments, there may be four control points which may be {0% point, 30% point, 70% point, 100% point}, or {1% point, 20% point, 50% point, 99% point}, etc.

In some embodiments, classification (or quantization) may be applied to MS based on the univariate histogram of the MS to classify (or quantize) the MS into N classes (e.g. two classes: "MOTION" and "NO MOTION"). Clustering/ training may be performed on the MS, or the histogram of MS to compute N-1 threshold T_1, T_2, .., T_{N-1} such that any MS<T_1 (MS in region 1) may be classified/quantized as Class 1, T_1<MS<T 2 (MS in region 2) may be Class 2, .., T_{N-1}<MS (MS in region N) may be Class N. And N-1 "control points" may be obtained based the N-1 threshold and the corresponding percentile value. Two more control points may/may not be added: 0%point and 100%-point. Using the method above, the compensation may be the mapping y=f(x) obtained by connecting/fitting/ regressing the control points using some kind of monotonic line.

In some embodiments, a centroid value may be computed for each decision/quantization region (i.e. MS<T_1 being region 1, T_1<MS<T_2 being region 2, .., T_{N-1}<MS being region N). And N "control points" may be obtained based on the N centroid values. Two more control points may/may not be added: 0%point and 100%-point. Using the method above, the compensation may be the mapping y=f(x) obtained by connecting/fitting/regressing the control points using some kind of monotonic line.

In some embodiments, cumulative distribution F1 of MS may be obtained. Cumulative distribution F2 of target MS may also be obtained. The compensation may be the mapping $y=f(x)=F2^{-1}[F1(x)]$ which is the function F1 followed by $F2^{-1}$ which is the inverse function of F2. In some embodiments, the function $F2^{-1}[F1(x)]$ may be implemented using a table look-up. But it may be complicated and may require consider computation. Thus an alternative embodiment is to approximate the function $F2^{-1}[F1(x)]$ by a simpler function, such as a linear function, affine function, piecewise linear function, polynomial, piecewise polynomial, spline, regression function, etc. The simpler function may be used as the mapping for compensation.

In some embodiments, the behavior may be bivariate or multivariate histograms/distributions. Similar to the above, control points may be determined for the bivariate/multivariate distributions. Three or four or more control points may be connected by a plane, a hyperplane, a surface or a manifold. The compensation (or the mapping) may be a collection of the plane/hyperplane/curve/manifolds.

In some embodiments, for multivariate distribution (say, N-variate), let MS vector be an N-tuple $X=[x_1, x_2, .., x_N]$, target MS vector be an N-tuple $T=[T_1, T_2, .., T_N]$ and the mapped/compensated MS vector be $Y=[y_1, y_2, .., y_N]$. The cumulative distribution functions F1 of MS (i.e. MS vector X) and cumulative function F2 of target multivariate distribution (i.e. of target MS vector T) may be obtained, and the compensation may be the mapping $Y=f(X)=F2^{-1}[F1(X)]$, or an approximation of the mapping using planes, hyperplanes, surfaces, or manifolds. In some embodiments, for N=2 (bivariate distribution), $[y_1, y_2]=f(x_1, x_2)=F2^{-1}[F1(x_1, x_2)]$. The $y_1$ may be obtained by $y_1=f1(x_1)=F2'^{-1}[F1'(x_1)]$, where F1' is univariate cumulative distribution of $x_1$ and F2' is univariate cumulative distribution of $T_1$. And $y_2=f2(x_2)=F2''^{-1}[F1''(x_2)]$, where F1" is univariate cumulative distribution of $x_2$ and F2" is univariate cumulative distribution of $T_2$. In other words, the X_ and, X_2 may be treated/processed independently to give Y_1 and Y_2. In some embodiments, $y_1$ may be computed as $y_1=f1(x_1)=F2'^{-1}[F1'(x_1)]$, and then $y_2$ is computed/ obtained by $y_2=f3(x_1, x_2)=F2'''^{-1}[F1(x_1, x_2)]$, wherein F2''' is univariate cumulative distribution of $T_2$ given $T_1=y_1=f1(x_1)=F2'^{-1}[F1'(x_1)]$. In some embodiments, $y_2$ may be computed as $y_2=f2(x_2)=F2''^{-1}[F1''(x_2)]$, and then $y_1$ is computed/ obtained by $y_1=f4(x_1, x_2)=F2''''^{-1}[F1(x_1, x_2)]$, wherein F2"" is univariate cumulative distribution of $T_1$ given $T_2=y_2=f2(x_2)=F2''^{-1}[F1''(x_2)]$. In some embodiments, for a N-variate distribution, a first component $y_{i1}$ may be computed first based on $x_{i1}$, wherein i1 is component index of the first component. Then a second component $y_{i2}$ may be computed based on $x_{i2}$, given $T_{i1}=y_{i1}$. Then a third component $y_{i3}$ may be computed based on $x_{i3}$, given $T_{i1}=y_{i1}$ and $T_{i2}=y_{i2}$. And so on. And the $N^{th}$ component $y_{iN}$ may be computed based on $x_{iN}$, given $T_{i1}=y_{i1}$, $T_{i2}=y_{i2}$, ..., $T_{i(N-1)}=y_{i(N-1)}$.

In some embodiments, the target behavior (say, histogram of MS) may not be full specified by user. Instead, the user may specify that the number of classes (e.g. of MS) is N and the respective representative target values (e.g. target MS values) for the N classes (say, $y_1, y_2, .., y_N$). To deduce the mapping for the compensation (e.g. compensation/mapping of MS), a similar procedure may be performed: (1) perform classification, classify (e.g. MS) into N classes, (2) compute the N-1 threshold: $T_1, T_2, .., T_{N-1}$ and N decision regions (e.g. of MS) (3) compute N centroids (of MS, say $C_1, C_2, .., C_N$), each being centroid of a respective decision region (e.g. of MS), (4) construct N control points based on the N centroids and the N representative target values, with the control point being $(C_1, y_1)$, $(C_2, y_2)$, ..., $(C_N, y_N)$, (5) two more control points may/may not be added: 0%point MS and 100%-point MS, (6) Using the method above, the compensation may be the mapping $y=f(x)$ obtained by connecting the control points using some kind of monotonic line.

In some embodiments, the compensation (or associated mapping/functionality) may be computed (e.g. based on artificial intelligence (AI), machine learning, deep learning, neural network (e.g. CNN, RNN, feed-forward network, attention based network, Transformer network or any combination)). Supervised learning, unsupervised learning, semi-supervised learning may be applied to learn the compensation/ mapping/functionality. Input of the compensation may be any of (or any feature/magnitude/phase/ function/statistics/behavior/histogram of): CI, IV, MI/MS/STI, analytics, TSCI, TSMI, TSA. Output of the compensation may be another (i.e. the "compensated") CI, IV, MI/MS/STI, analytics, TSCI, TSMI, TSA (or corresponding feature/magnitude/phase/function/ statistics/behavior/histogram). The training target (i.e. training reference) may be yet another CI, IV, MI/MS/STI, analytics, TSCI, TSMI, TSA (or corresponding feature/magnitude/phase/function/ statistics/behavior/histogram), which may be obtained in some pre-training/pre-calibration stage/phase. An objective/cost/loss function (e.g. measure/norm/score of: similarity/dissimilarity/distance/difference/discrimination/ratio/ fraction/variance/variation/divergence/spread/deviation/TRRS/correlation/covariance/autocorrelation/cross-correlation/inner product/mean square/absolute error) between the training target (e.g. target behavior) and the output (e.g. behavior of output) may be used to train the compensation/mapping/functionality. The objective/cost/loss function may be optimized/minimized/maximized, under/not under certain constraint(s) to find the best/optimized compensation/mapping/functionality. The objective/cost/loss function may be parameterized and the optimization/maximization/minimization may be with respect to the set of parameters.

In some embodiments, for searching, a compensation may be computed by performing a search (e.g. exhaustive search, fast search, reduced search). A number of candidate mappings may be determined and tentatively applied to get/compute the respective compensated behavior. A distance or similarity/dis-similarity score between the target behavior and the respective compensated behavior may be computed (e.g. "behavior distance", e.g. histogram distance, vector norm, city-block distance, Euclidean distance, intersection distance, Bhattacharyya distance (B-distance), Kullback-Leibler distance (KL-distance)). The candidate mapping that gives best match (e.g. smallest distance or largest similarity score) may be chosen as the compensation. Multi-stage searching may be applied. For example, a number of candidate first mappings (e.g. mapping applied to CI) may be determined. And for each candidate first mapping, a number of candidate second mappings (e.g. mapping applied to MS or analytics) may be determined. Both the candidate first mapping and candidate second mapping may be tentatively applied to get/compute the respective compensated behavior. A distance/similarity score between target behavior and respective compensated behavior may be computed such that the candidate first mapping and the candidate second mapping that together give best match (e.g. smallest distance or largest similarity score) may be chosen as the respective compensation. In some embodiments, deduction and searching may be applied together. For example, a number of candidate first mapping (e.g. to the CI) may be determined and tentatively applied. Then for each candidate first mapping, a second mapping (e.g. to the MS) may be deduced. Both the candidate first mapping and respective deduced second mapping may be tentatively applied to get/compute the respective compensated behavior. A distance/similarity score between target behavior and respective compensated behavior may be computed such that the candidate first mapping and the respective deduced second mapping that together give best match (e.g. smallest distance or largest similarity score) may be chosen as the respective compensation.

In some embodiments, for different compensation for different MS/MI/STI/analytics, more than one MS/MI/STI/analytics may be computed for the same task, or for different tasks (e.g. a first one for motion detection, a second one for breathing detection, a third one for fall-down detection, etc.). Different compensation may be applied for different MS/MI/STI/analytics. In some embodiments, MI may be MS/MI/STI computed based on TSCI (or compensated TSCI).

The following numbered clauses provide examples for compensated wireless monitoring.

Clause C1. A method/device/system/software of a compensated wireless monitoring system, comprising: transmitting a wireless signal from a Type1 heterogeneous wireless device of the wireless sensing system through a wireless multipath channel of a venue, wherein the wireless multipath channel is impacted by a motion of an object in the venue; receiving the wireless signal by a Type2 heterogeneous wireless device of the system through the wireless multipath channel, wherein the received wireless signal differs from the transmitted wireless signal due to the wireless multipath channel of the venue and the motion of the object; obtaining a time series of channel information (CI) of the wireless multipath channel based on the received wireless signal using a processor, a memory and a set of instructions; computing a time series of motion information (MI) based on the time series of CI (TSCI); computing a time series of analytics based on the time series of MI (TSMI); computing a time series of compensated analytics by applying a compensation to the computation of the time series of analytics (TSA), wherein the compensation comprises a monotonic mapping, monitoring the motion based on the time series of compensated analytics.

Clause C2. The method/device/system/software of the compensated wireless monitoring system in clause C1: wherein the monotonic mapping comprises at least one of: a univariate mapping, a bivariate mapping, a multi-variate mapping, a monotonic increasing mapping, a monotonic decreasing mapping, a monotonic non-decreasing mapping, a monotonic non-increasing mapping, a linear mapping, a nonlinear mapping, an affine mapping, a piecewise linear mapping, a concave mapping, a convex mapping, a quadratic mapping, a cubic mapping, a polynomial mapping, an exponential mapping, a logarithmic mapping, a fitted mapping, a regression mapping, a spline mapping, a function, an inverse function, a function of function, a mapping of another mapping, a composite mapping, a parametric mapping, a non-parametric mapping, a time-varying mapping, or a time-invariant mapping.

Clause C3. The method/device/system/software of the compensated wireless monitoring system in clause C1 (or 2), further comprising: wherein the compensation comprises at least one of: a first compensation tailor-made for a pair of a generic Type1 device and a generic Type2 device, a second compensation tailor-made for the Type1 device, a third compensation tailor-made for the Type2 device, and a fourth compensation tailed-made for the pair of the Type1 device and the Type2 device.

Clause C4. The method/device/system/software of the compensated wireless monitoring system in clause C3 (or 1 or 2), further comprising: wherein the monotonic mapping comprises at least one of: a first monotonic mapping tailor-made for a pair of a generic Type1 device and a generic Type2 device, a second monotonic mapping tailor-made for the Type1 device, a third monotonic mapping tailor-made for the Type2 device, and a fourth monotonic mapping tailed-made for the pair of the Type1 device and the Type2 device.

In some embodiments, the monotonic mapping may be applied to CI, intermediate value (IV), MI and/or analytics.

Clause C5. The method/device/system/software of the compensated wireless monitoring system in clause C1 (or 2 or 3 or 4), further comprising: wherein the compensation or the monotonic mapping is applied to at least one of: a CI, a number of CI, a number of CI in a sliding time window, a number of CI in the TSCI, the TSCI, another TSCI obtained based on another wireless signal received by the Type2 device, another TSCI obtained based on yet another wireless signal transmitted by the Type1 device, a feature of a CI, a magnitude of a CI, a phase of a CI, s a component of a CI, a number of components of a CI, a magnitude of a component of a CI, or a phase of a component of a CI; computing a time series of compensated MI based on all compensated CI, compensated features of CI and compensated components of CI; computing the time series of compensated analytics based on the time series of compensated MI.

In some embodiments, the monotonic mapping may be applied to intermediate value (IV) between TSCI and TSMI. IV may be computed based on TSCI; TSMI may be computed based on IV.

Clause C6. The method/device/system/software of the compensated wireless monitoring system in any previous C clause , further comprising: wherein the compensation or the monotonic mapping is applied to at least one of: an intermediate value(IV) computed based on the TSCI, a number of IV, a number of IV in a sliding time window, a feature of a IV, a magnitude of a IV, a phase of a IV, a component of a IV, a number of components of a IV, a magnitude of a component of a IV, or a phase of a component of a IV; computing a time series of compensated MI based on all compensated IV, compensated features of IV and compensated components of IV; computing the time series of compensated analytics based on the time series of compensated MI.

In some embodiments, the monotonic mapping may be applied to intermediate value (IV) between TSMI and TSA. IV may be computed based on TSMI; TSA may be computed based on IV.

Clause C7. The method/device/system/software of the compensated wireless monitoring system in any previous C clause, further comprising: wherein the compensation or the monotonic mapping is applied to at least one of: an intermediate value(IV) computed based on the TSMI, a number of IV, a number of IV in a sliding time window, a feature of a IV, a magnitude of a IV, a phase of a IV, a component of a IV, a number of components of a IV, a magnitude of a component of a IV, or a phase of a component of a IV; computing the time series of compensated analytics based on all compensated IV, compensated features of IV and compensated components of IV.

In some embodiments, the monotonic mapping may be applied to MI.

Clause C8. The method/device/system/software of the compensated wireless monitoring system in any previous C clause , further comprising: wherein the compensation or the monotonic mapping is applied to at least one of: a MI, a number of MI, a number of MI in a sliding time window, a number of MI in the TSMI, the TSMI, a feature of a MI, a magnitude of a MI, a phase of a MI, a component of a MI, a number of components of a MI, a magnitude of a component of a MI, or a phase of a component of a MI; monitoring the motion based on all compensated MI, compensated features of MI, and compensated component of MI.

In some embodiments, the monotonic mapping may be applied to TSA.

Clause C9. The method/device/system/software of the compensated wireless monitoring system in any previous C clause : wherein the compensation or the monotonic mapping is applied to at least one of: an analytics, a number of analytics, a number of analytics in a sliding time window, a number of analytics in the TSA, the TSA, a feature of an analytics, a magnitude of an analytics, a phase of an analytics, a component of an analytics, a number of components of an analytics, a magnitude of a component of an analytics, or a phase of a component of an analytics.

In some embodiments, the monotonic mapping may be changed over time.

Clause C10. The method/device/system/software of the compensated wireless monitoring system in any previous C clause, further comprising: changing the compensation or the monotonic mapping based on at least one of: a change in a target behavior, a change in the wireless signal, a change in a bandwidth of the wireless signal, a change in a band of the wireless signal, a specification, a system setting, a user input, a situation, an event, a time table, a strategy, a plan.

In some embodiments, the compensation may be computed/learned/derived/deduced/ trained in a (past or current or real-time or future) calibration phase.

Clause C11. The method/device/system/software of the compensated wireless monitoring system in clause C1 (to 10), further comprising: in a calibration phase: transmitting a calibration wireless signal from a calibration Type1 heterogeneous wireless device through a calibration wireless multipath channel of a calibration venue, wherein the calibration wireless multipath channel is impacted by a calibration motion of a calibration object in the calibration venue; receiving the calibration wireless signal by a calibration Type2 heterogeneous wireless device through the calibration wireless multipath channel, wherein the received calibration wireless signal differs from the transmitted calibration wireless signal due to the calibration wireless multipath channel of the calibration venue and the calibration motion of the calibration object; obtaining a time series of calibration CI of the calibration wireless multipath channel based on the received calibration wireless signal using a calibration processor, a calibration memory and a set of calibration instructions; determining the compensation based on the time series of calibration CI.

In some embodiments, the monotonic mapping is determined by comparing a "calibration behavior" and a "target behavior". In some embodiments, the goal is to find a compensation or monotonic mapping such that, after the compensation of monotonic mapping is applied, the compensated calibration behavior would be similar to the target behavior.

Clause C12. The method/device/system/software of the compensated wireless monitoring system in clause C11, further comprising: in the calibration phase: computing a time series of calibration MI based on the time series of calibration CI; computing a time series of calibration analytics based on the time series of calibration MI; comparing a behavior associated with the time series of calibration CI, calibration MI or calibration analytics with a target behavior; computing the monotonic mapping based on the comparing of the calibration behavior and the target behavior.

Clause C13. The method/device/system/software of the compensated wireless monitoring system in clause C12: wherein the behavior comprises at least one of: statistical behavior, temporal behavior, frequency behavior, transform domain behavior, projection domain behavior, magnitude behavior, phase behavior, component behavior, histogram, probability distribution, cumulative histogram, characteristic function, moment generating function, cumulative distribution, univariate distribution, univariate histogram, bivariate distribution, bivariate histogram, multi-variate distribution, multi-variate histogram, restricted distribution, restricted histogram, conditional distribution, conditional histogram, histogram of CI, MI or analytics between two time stamps, histogram of observables associated with CI, MI or analytics between two time stamps, histogram of observables associated with CI, MI or analytics restricted to be between the two time stamps, histogram of CI, MI or analytics between two characteristic points of CI, MI or analytics, histogram of observables associated with CI, MI or analytics between two characteristic points of CI, MI or analytics, histogram of observables associated with CI, MI or analytics between two characteristic points of processed CI, MI or analytics, histogram of observables associated with CI, MI or analytics between two characteristic points of the observables, histogram of observables associated with CI, MI or analytics between two characteristic points conditioned on a condition of the two characteristic points, statistics, conditional statistics, trimmed statistics, first order statistics, second order statistics, high order statistics, scatter plots, mean, weighted mean, conditional mean, restricted mean, arithmetic mean, trimmed mean, geometric mean, harmonic mean, mode, median, percentile, maximum, minimum, zero-crossing, range, magnitude, phase, component magnitude, component phase, variance, standard deviation, dispersion, variability, kurtosis, entropy, information, moments, central moments, correlation, correlation coefficient, covariance, auto-correlation function (ACF), auto-covariance function, cross correlation, or cross covariance; wherein any observable associated with an item comprises at least one of: the item, a magnitude of the item, a phase of the item, a feature of the item, a component of the item, a magnitude of the component of the item, a phase of the component of the item, a feature of the component of the item, or a function of any of the above; wherein any characteristic point is one of: a local maximum, a local minimum or a zero crossing.

In some embodiments, possible ways to obtain the monotonic mapping include: (1a) searching, in calibration phase, (1b) deduction, in calibration phase, (2) from user or another device (e.g. server) or system setting/specification.

Clause C14. The method/device/system/software of the compensated wireless monitoring system in clause C12 or C13, further comprising: wherein the monotonic mapping is: (1) computed based on a searching or a deduction in the calibration phase; (2) obtained from a specification, a setting, a user input or another device; (3) computed based on supervised learning, unsupervised learning or semi-supervised learning; or (4) obtained by artificial intelligence, machine learning, deep learning or neural network.

In some embodiments, a possible way to obtain the monotonic mapping includes: (1a) searching, in calibration phase. In some embodiments, trial compensation is performed for each candidate monotonic mapping. After compensation, compute similarity score between target behavior and the resulting calibration behavior after compensation.

Clause C15. The method/device/system/software of the compensated wireless monitoring system in clause C14, further comprising: in the calibration phase: determining and searching a number of candidate monotonic mappings; for each candidate monotonic mapping: applying a respective compensation comprising the candidate monotonic mapping to the computation of the TSA, and computing a respective similarity score between the target behavior and the resulting behavior associated with the time series of calibration CI, calibration MI or calibration analytics after the respective compensation is applied; choosing the monotonic mapping as the candidate monotonic mapping with the largest similarity score.

In some embodiments, a possible way to obtain the monotonic mapping includes: (1b) deduction, in calibration phase.

Clause C16. The method/device/system/software of the compensated wireless monitoring system in clause C14 or C15, further comprising: in the calibration phase: deducing the monotonic mapping based on the comparing of the target behavior and the behavior associated with the time series of calibration CI, calibration MI or calibration analytics.

In some embodiments, N control points may be obtained, to deduce the monotonic mapping based on the N control points.

Clause C17. The method/device/system/software of the compensated wireless monitoring system in clause C16, further comprising: in the calibration phase: determining the behavior of a univariate observable X associated with CI, MI or analytics based on the time series of calibration CI, the time series of calibration MI or the time series of calibration analytics, wherein the behavior comprises N scalar values $\{x\_1, x\_2, ... x\_N\}$ with $x\_1<x\_2< ..<x\_N$, determining the target behavior of a univariate target observable Y, wherein the target behavior comprises N scalar values $\{y\_1, y\_2, ... y\_N\}$, with $y\_1<y\_2< ..<y\_N$, defining N control points of the monotonic mapping by mapping the N scalar values of X to the N scalar values of Y, the control points being $(x\_1, y\_1), (x\_2, y\_2), .., (x\_N, y\_N)$; deducing the monotonic mapping based on the N control points.

In some embodiments, N control points may be connected with a line (continuous).

Clause C18. The method/device/system/software of the compensated wireless monitoring system in clause C17, further comprising: in the calibration phase: deducing the monotonic mapping as a monotonic line connecting the N control points.

In some embodiments, there is a possible line connecting the control points.

Clause C19. The method/device/system/software of the compensated wireless monitoring system in clause C18, further comprising: wherein the monotonic line comprises at least one of: a linear map, a quadratic map, a cubic map, an affine map, a polynomial map, an exponential map, a logarithmic map, a convex map, a concave map, a spline map, a piecewise linear map, a piecewise quadratic map, a piecewise cubic map, a monotonic increasing map, a monotonic non-decreasing map.

In some embodiments, curve fitting may be used instead (such that some control points may not reside on the fitted curve).

Clause C20. The method/device/system/software of the compensated wireless monitoring system in clause C17-C19, further comprising: deducing the monotonic mapping as a curve that fits the N control points in accordance with a fitting criterion.

In some embodiments, curve fitting may be least square fitting.

Clause C21. The method/device/system/software of the compensated wireless monitoring system in clause C20, further comprising: wherein the curve fits the N control points in accordance with a least-square fitting criterion.

In some embodiments, curve fitting may be obtained by regression.

Clause C22. The method/device/system/software of the compensated wireless monitoring system in clause C17-C21, further comprising: wherein the monotonic mapping is deduced by applying a regression, linear regression, robust linear regression, orthogonal regression, Deming regression, major axis regression, segmented regression, polynomial regression, or regression dilution to the N control points.

In some embodiments, there are possible choices of control points.

Clause C23. The method/device/system/software of the compensated wireless monitoring system in clause C17-C22, further comprising: wherein the N control points comprise at least one of: (a mean of X, a mean of Y), (a weighted mean of X, a weighted mean of Y), (a trimmed mean of X, a trimmed mean of Y), (an arithmetic mean of X, a arithmetic mean of Y), (a geometric mean of X, a geometric mean of Y), (a harmonic mean of X, a harmonic mean of Y), (a median of X, a median of Y), (a mode of X, a mode of Y), (a percentile of X, a percentile of Y), (a 0-percentile of X, a 0-percentile of Y), (a 1-percentile of X, a 1-percentile of Y), (a 5-percentile of X, a 5-percentile of Y), (a 10-percentile of X, a 10-percentile of Y), (a 20-percentile of X, a 20-percentile of Y), (a 30-percentile of X, a 30-percentile of Y), (a 40-percentile of X, a 40-percentile of Y), (a 50-percentile of X, a 50-percentile of Y), (a 60-percentile of X, a 60-percentile of Y), (a 70-percentile of X, a 70-percentile of Y), (a SO-percentile of X, a 80-percentile of Y), (a 90-percentile of X, a 90-percentile of Y), (a 95-percentile of X, a 95-percentile of Y), (a 99-percentile of X, a 99-percentile of Y), or (a 100-percentile of X, a 100-percentile of Y).

In some embodiments, the monotonic mapping may be computed directly based on cumulative distribution function $F\_X$ and $F\_Y$.

Clause C24. The method/device/system/software of the compensated wireless monitoring system in clause C16-C23, further comprising: in the calibration phase: determining the behavior of a univariate observable X associated with CI, MI or analytics based on the time series of calibration CI, the time series of calibration MI or the time series of calibration analytics, wherein the behavior comprises a cumulative univariate distribution $F\_X$ of X, determining the target behavior of a target observable Y, wherein the target behavior comprises a cumulative univariate target distribution $F\_Y$ of Y, deducing the monotonic mapping based on the cumulative univariate distribution $F\_X$ and the cumulative univariate target distribution $F\_Y$.

In some embodiments, the monotonic mapping may be computed directly based on a formula of cumulative distribution function $F\_X$ and $F\_Y$.

Clause C25. The method/device/system/software of the compensated wireless monitoring system in clause C24, further comprising: deducing the monotonic mapping as $F\_Y^{-1}[F\_X(X)]$, wherein $F\_Y^{-1}$ is the inverse of function $F\_Y$.

In some embodiments, as the formula may be complex, an approximation may be used instead.

Clause C26. The method/device/system/software of the compensated wireless monitoring system in clause C24 or C25, further comprising: deducing the monotonic mapping as an approximation of $F\_Y^{-1}(F\_X(X))$, wherein $F\_Y^{-1}$ is the inverse of function $F\_Y$, wherein the approximation comprises at least one of: a linear map, a quadratic map, a cubic map, an affine map, a polynomial map, an exponential map, a logarithmic map, a convex map, a concave map, a spline map, a piecewise linear map, a piecewise quadratic map, or a piecewise cubic map.

In some embodiments, for multi-variate observable X, each X (and Y) is a multi-variate tuple. N control points may be obtained.

Clause C27. The method/device/system/software of the compensated wireless monitoring system in clause C16-C26, further comprising: in the calibration phase: determining the behavior of a multi-variate observable X associated with CI, MI or analytics based on the time series of calibration CI, the time series of calibration MI or the time series of calibration analytics, wherein the behavior comprises N multi-variate tuple $\{x\_1, x\_2, ... x\_N\}$, determining the target behavior of a multi-variate target observable Y, wherein the target behavior comprises N multi-variate tuple $\{y\_1, y\_2, ... y\_N\}$, defining N control points of the monotonic mapping by mapping the N multi-variate tuple of X to the N multi-variate tuple of Y, the control points being $(x\_1, y\_1), (x\_2, y\_2), .., (x\_N, y\_N)$; deducing the monotonic mapping based on the N control points.

In some embodiments, hyperplane or manifold may be used to connect some control points (in a piecewise manner).

Clause C2S. The method/device/system/software of the compensated wireless monitoring system in clause C27, further comprising: in the calibration phase: deducing the monotonic mapping as a combination of multiple piecewise hyperplanes each connecting some of the N control points.

In some embodiments, hyperplane or manifold may be used to fit/approximate the control points (e.g. in a piecewise manner).

Clause C29. The method/device/system/software of the compensated wireless monitoring system in clause C27 or C28, further comprising: in the calibration phase: deducing the monotonic mapping as a manifold or a hyperplane that fits the N control points in accordance with a fitting criterion.

In some embodiments, monotonic mapping may be deduced based on formula.

Clause C30. The method/device/system/software of the compensated wireless monitoring system in clause C27-C29, further comprising: in the calibration phase: deducing the monotonic mapping based on a cumulative distribution function of X and a cumulative distribution function of Y.

In some embodiments, for multi-stage search, searching may be combined with deduction to find the best combination.

Clause C31. The method/device/system/software of the compensated wireless monitoring system in clause C14-C30, further comprising: in the calibration phase: determining the monotonic mapping comprises a first monotonic mapping and a second monotonic mapping; determining and searching a number of candidate first monotonic mappings; for each candidate first monotonic mapping: deducing the respective second monotonic mapping, applying a respective compensation comprising the candidate first monotonic mapping and the respective deduced second monotonic mapping to the computation of the TSA, and computing a respective similarity score between the target behavior and the resulting behavior associated with the time series of calibration CI, calibration MI or calibration analytics after the respective compensation is applied; choosing the monotonic mapping as the candidate first monotonic mapping and the respective deduced second monotonic mapping with the largest similarity score.

Clause C32. The method/device/system/software of the compensated wireless monitoring system in clause C12-C31, further comprising: obtaining the target behavior from a specification, a system setting, a user input, a server, a cloud server, a remote server, a local server, a sensing server, or another device.

Clause C33. The method/device/system/software of the compensated wireless monitoring system in clause C12-C32, further comprising: changing the target behavior based on a specification, a system setting, a user input, a situation, an event, a time table, a strategy, a plan.

Clause C34. The method/device/system/software of the compensated wireless monitoring system in clause C11-C33: wherein a first band of the calibration wireless signal comprises a second band of the wireless signal; wherein a first bandwidth of the calibration wireless signal comprises a second bandwidth of the wireless signal.

Clause C35. The method/device/system/software of the compensated wireless monitoring system in clause C11-C34: wherein the calibration object resembles the object in a first way; wherein the calibration motion of the calibration object resembles the motion of the object in a second way.

Clause C36. The method/device/system/software of the compensated wireless monitoring system in clause C11-C35, further comprising: in a pre-calibration phase before the calibration phase: transmitting a reference wireless signal from a reference Type1 heterogeneous wireless device through a reference wireless multipath channel of a reference venue, wherein the reference wireless multipath channel is impacted by a reference motion of a reference object in the reference venue; receiving the reference wireless signal by a reference Type2 heterogeneous wireless device through the reference wireless multipath channel, wherein the received reference wireless signal differs from the transmitted reference wireless signal due to the reference wireless multipath channel of the reference venue and the reference motion of the reference object; obtaining a time series of reference CI of the reference wireless multipath channel based on the received reference wireless signal using a reference processor, a reference memory and a set of reference instructions; computing a time series of reference MI based on the time series of reference CI; computing a time series of reference analytics based on the time series of reference MI; computing the target behavior as a behavior of at least one of: the time series of reference CI, the time series of reference MI or the time series of reference analytics.

Clause C37. The method/device/system/software of the compensated wireless monitoring system in clause C36, further comprising: wherein the target behavior comprises a set of fundamental behaviors that can appear in the compensated wireless monitoring system.

Clause C38. The method/device/system/software of the compensated wireless monitoring system in clause C11-C37, further comprising: in a re-calibration phase after the calibration phase: computing a time series of re-calibration MI based on a re-calibration TSCI, comparing a behavior of the time series of re-calibration MI with a re-calibration target behavior, and computing a renewed monotonic mapping based on the comparing of the behavior of time series of re-calibration MI and the re-calibration target behavior; replacing the monotonic mapping by the renewed monotonic mapping in the compensation to the computation of the TSA.

In some embodiments, the monotonic mapping is determined by comparing a "calibration behavior" and a "target behavior".

Clause C39. The method/device/system/software of the compensated wireless monitoring system in clause C11-C38: wherein the calibration Type1 device is a reference Type1 device; wherein the calibration Type2 device is the Type2 device; wherein the compensation and the monotonic mapping are tailor-made for the Type2 device for compensating the computation of any respective TSA based on any respective TSCI obtained from any respective wireless signal received by the Type2 device for the monitoring of the motion.

Clause C40. The method/device/system/software of the compensated wireless monitoring system in clause C11-C39: wherein the calibration Type1 device is the Type1 device; wherein the calibration Type2 device is a reference Type2 device; wherein the compensation and the monotonic mapping are tailor-made for the Type1 device for compensating the computation of any respective TSA based on any respective TSCI obtained from any respective wireless signal transmitted by the Type1 device for the monitoring of the motion.

Clause C41. The method/device/system/software of the compensated wireless monitoring system in clause C11-C40: wherein the calibration Type1 device is the Type1 device; wherein the calibration Type2 device is the Type2 device; wherein the compensation and the monotonic mapping are tailor-made for the pair of the Type1 device and the Type2 device for compensating the computation of the TSA based on the TSCI obtained from the wireless signal transmitted between the pair of devices for the monitoring of the motion.

Clause C42. The method/device/system/software of the compensated wireless monitoring system in clause C11-C41: wherein the calibration Type1 device is a representative Type1 device; wherein the calibration Type2 device is a representative Type2 device; wherein the compensation and the monotonic mapping are tailor-made for any pair of a generic Type1 device represented by the representative Type1 device and a generic Type2 device represented by the representative Type2 device for compensating the computation of any respective TSA based on any respective TSCI obtained from any respective wireless signal transmitted between any pair of devices for the monitoring of the motion; wherein the Type1 device is a generic Type1 device and the Type2 device is a generic Type2 device.

In some embodiments, the monotonic mapping may be different when Type1 device and Type2 device and wireless signal are same, but a different analytics is used.

Clause C43. The method/device/system/software of the compensated wireless monitoring system in any previous C clause, comprising: computing a time series of second analytics based on the time series of MI; computing a time series of compensated second analytics by applying a second compensation to the computation of the time series of second analytics, wherein the second compensation comprises a second monotonic mapping, monitoring the motion based on the time series of compensated second analytics.

In some embodiments, the monotonic mapping may be same when Type1 device, Type2 device and wireless signal are same, but a different analytics is used. (e.g. compensation applied to CI or MI).

Clause C44. The method/device/system/software of the compensated wireless monitoring system in clause C43, comprising: wherein the second compensation and the compensation are the same; wherein the second monotonic mapping and the first monotonic mapping are the same.

In some embodiments, the monotonic mapping may be different when Type1 device and Type2 device and wireless signal are same, but a different MI is used.

Clause C45. The method/device/system/software of the compensated wireless monitoring system in any previous C clause, comprising: computing a time series of second MI based on the TSCI; computing a time series of second analytics based on the time series of second MI; computing a time series of compensated second analytics by applying a second compensation to the computation of the time series of second analytics, wherein the second compensation comprises a second monotonic mapping, monitoring the motion based on the time series of compensated second analytics.

In some embodiments, the monotonic mapping may be same when Type1 device, Type2 device and wireless signal are same, but a different MI is used. (e.g. compensation applied to CI).

Clause C46. The method/device/system/software of the compensated wireless monitoring system in clause C45, comprising: wherein the second compensation and the compensation are the same; wherein the second monotonic mapping and the first monotonic mapping are the same.

In some embodiments, the monotonic mapping may be different when Type1 device is different but Type2 device is same.

Clause C47. The method/device/system/software of the compensated wireless monitoring system in any previous C clause, comprising: transmitting a second wireless signal from a second Type1 heterogeneous wireless device of the wireless sensing system through the wireless multipath channel of the venue; receiving the second wireless signal by the Type2 heterogeneous wireless device of the system through the wireless multipath channel, wherein the received second wireless signal differs from the transmitted second wireless signal due to the wireless multipath channel of the venue and the motion of the object; obtaining a second TSCI of the wireless multipath channel based on the received second wireless signal using the processor, the memory and the set of instructions; computing a time series of second MI based on the second TSCI; computing a time series of second analytics based on the time series of second MI; computing a time series of compensated second analytics by applying a second compensation to the computation of the time series of second analytics, wherein the second compensation comprises a second monotonic mapping, monitoring the motion based on the time series of compensated second analytics.

In some embodiments, the monotonic mapping may be same when Type1 device is different but Type2 device is same.

Clause C4S. The method/device/system/software of the compensated wireless monitoring system in clause C47, comprising: wherein the second compensation and the compensation are the same; wherein the second monotonic mapping and the first monotonic mapping are the same.

In some embodiments, the monotonic mapping may be different when Type2 device is different but Type1 device is same.

Clause C49. The method/device/system/software of the compensated wireless monitoring system in any previous C clause, comprising: transmitting a second wireless signal from the Type1 heterogeneous wireless device of the wireless sensing system through the wireless multipath channel of the venue; receiving the second wireless signal by a second Type2 heterogeneous wireless device of the system through the wireless multipath channel, wherein the received second wireless signal differs from the transmitted second wireless signal due to the wireless multipath channel of the venue and the motion of the object; obtaining a second TSCI of the wireless multipath channel based on the received second wireless signal using a second processor, a second memory and a second set of instructions; computing a time series of second MI based on the second TSCI; computing a time series of second analytics based on the time series of second MI; computing a time series of compensated second analytics by applying a second compensation to the computation of the time series of second analytics, wherein the second compensation comprises a second monotonic mapping, monitoring the motion based on the time series of compensated second analytics.

In some embodiments, the monotonic mapping may be same when Type2 device is different but Type1 device is same.

Clause C50. The method/device/system/software of the compensated wireless monitoring system in clause C49, comprising: wherein the second compensation and the compensation are the same; wherein the second monotonic mapping and the first monotonic mapping are the same.

In some embodiments, the monotonic mapping may be different when Type1 device and Type2 device are same, but wireless signal is different (e.g. different band/bandwidth).

Clause C51. The method/device/system/software of the compensated wireless monitoring system in any previous C clause, comprising: transmitting a second wireless signal from the Type1 heterogeneous wireless device of the wireless sensing system through the wireless multipath channel of the venue; receiving the second wireless signal by the Type2 heterogeneous wireless device of the system through the wireless multipath channel, wherein the received second wireless signal differs from the transmitted second wireless signal due to the wireless multipath channel of the venue and the motion of the object; obtaining a second TSCI of the wireless multipath channel based on the received second wireless signal using the processor, the memory and the set of instructions; computing a time series of second MI based on the second TSCI; computing a time series of second analytics based on the time series of second MI; computing a time series of compensated second analytics by applying a second compensation to the computation of the time series of second analytics, wherein the second compensation comprises a second monotonic mapping, monitoring the motion based on the time series of compensated second analytics.

In some embodiments, the monotonic mapping may be same when Type1 device and Type2 device are same but wireless signal is different.

Clause C52. The method/device/system/software of the compensated wireless monitoring system in clause C51, comprising: wherein the second compensation and the compensation are the same; wherein the second monotonic mapping and the first monotonic mapping are the same.

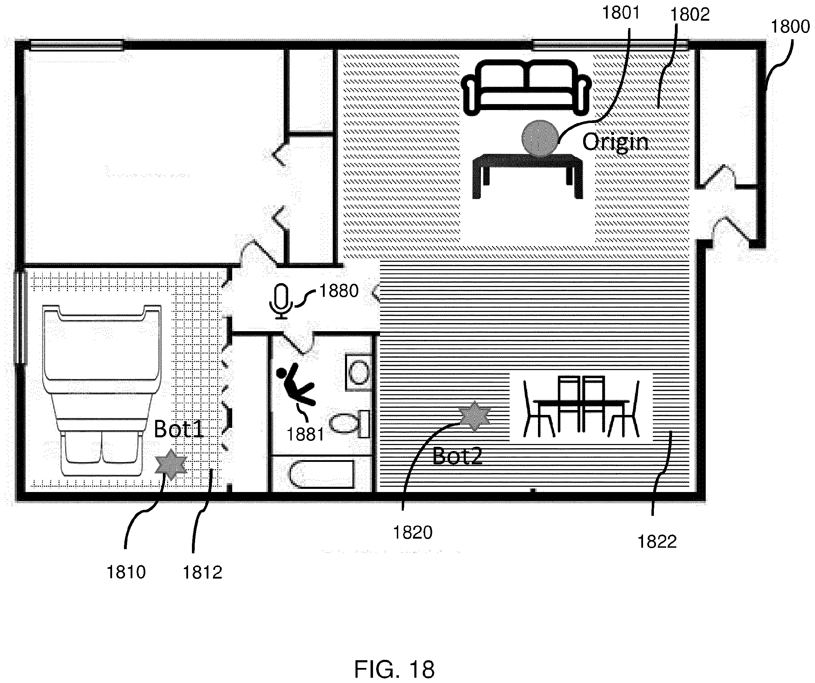

FIG. 18 illustrates an exemplary scenario where a motion/event/activity (e.g. fall/abrupt/impulsive/transient/impactful motion, motion that generates sound/vibration/pressure change, or target motion) is detected (or monitored) based on wireless signal or auxiliary signal or both in a venue, according to one embodiment of the present teaching. For example, as shown in FIG. 18, in a 2-bedroom apartment 1800, Origin 1801 (Type2 device) may be placed in the living-room area 1802, Bot 1 1810 (e.g. Type1 device) may be placed in a bedroom1-area 1812, and Bot 2 1820 (e.g. Type1 device) may be placed in the dining-room area 1822. A sensor 1880 (e.g. microphone/camera/light sensor/pressure sensor) may also be placed somewhere in the apartment 1800 to capture auxiliary sensing signals (e.g. sound/image/video/light/ pressure/vibration). During the motion/event/activity (e.g. a person 1881 falls down making sound that is picked up by microphone sensor 1880), each of Bot 1 1810 and Bot 2 1820 can transmit a wireless (sounding/probe) signal to the Origin 1801, which can obtain channel information of a wireless multipath channel based on the wireless signal. A motion information (MI) or radio-based MI (RMI) can be computed based on the channel information. During the motion/event/activity, the sensor 1880 can capture an associated auxiliary signal. An aux-based motion information (AMI) can be computed based on the auxiliary signal. The motion/event/activity can be detected/monitored individually based on the RMI alone (radio-based), individually based on the AMI alone (aux-based), or jointly based on both the RMI and AMI (radio-aux-based, e.g. sequentially radio-based followed by aux-based, or sequentially aux-based followed by radio-based, or simultaneously/contemporaneously by both radio-based and aux-based).

If object motion/activity is detected based on wireless signals transmitted by both Bot 1 1810 and Bot 2 1820, localization may be performed such that a location of the activity/motion/event or the object (e.g. person/user) may be determined the living-room area 1802. If detection is based only on wireless signals transmitted by Bot 1 1810, the location may be determined to be in the bedroom-1 area 1812. If detection is based only on wireless signals transmitted by Bot 2 1820, the location may be determined in the dining-room area 1822. If target motion/event/activity cannot be detected based on wireless signals transmitted by either Bot 1 1810 or Bot 2 1820, then it may be determined that nobody and no object is in the apartment 1800. The corresponding area where the activity/motion/event/person/user is detected may be marked with a predetermined pattern.

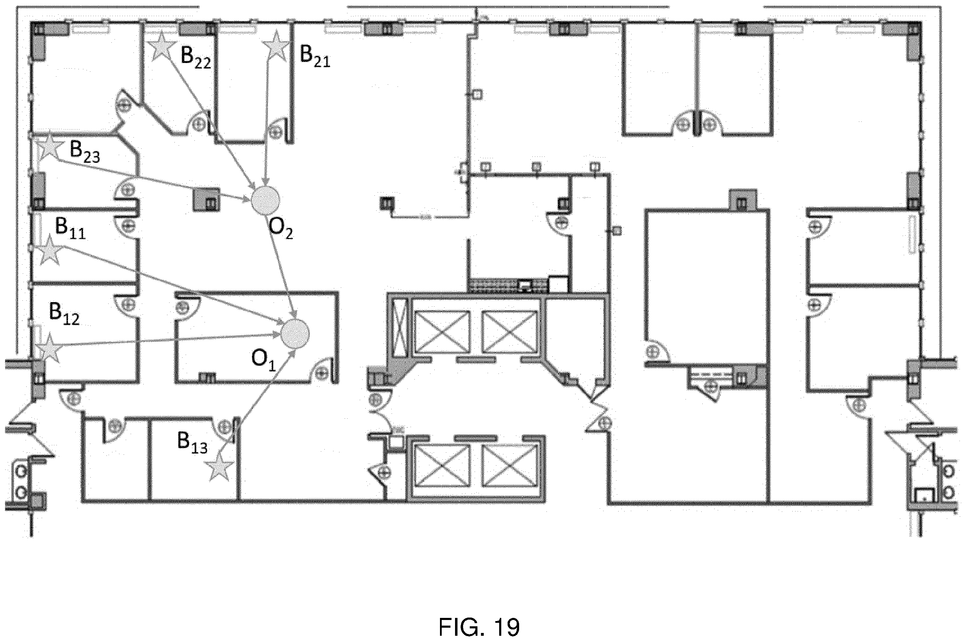

FIG. 19 illustrates an exemplary floor plan and placement of wireless devices for wireless monitoring with motion/event/activity detection and localization, according to some embodiments of the present disclosure. In the example shown in FIG. 19, there are two origins O1 and O2. Each origin is associated with three bots. For example, O1 is associated with bots B11, B12, and B13 (together constitute a first origin group); and O2 is associated with bots B21, B22, and B23 (together constitute a second origin group). Each device of the bots and origins is fixed at a different location. In one embodiment, the origin O1 is called a master origin; and the origin O2 is called a child origin, where the child origin can transmit statistics and information to the master origin for combination.

In some embodiments, the floor plan and placement of wireless devices in FIG. 19 can be used to perform multi-person or multi-object motion localization based on motion statistics. When there are two or more origins, motion statistics (or RMI) measured from different links of different origins are combined to contribute together to the motion localization. For each activated bot, it is determined which origin the bot is associated with, e.g. based on a smoothed motion statistics in a time window. For example, when the smoothed motion statistics of Bot k with respect to Origin j is larger than a threshold, Bot k is determined to be associated with Origin j. The same threshold can be used for other origins. Thus, for each origin, a set of activated bots associated with the origin can be determined. A likelihood is calculated for each activated bot k to detect a motion, and is smoothed over a time window. For each origin group including an origin and its associated bots (e.g. O1, B11, B12, B13), an average motion statistics is computed for the origin group across all the associated bots. Each calculation or computation above can be performed at the origin or the bot.

When there is any motion detected in the environment, an origin group with the highest average motion statistics is chosen. When the average motion statistics of the chosen origin group is larger than a threshold, the motion is determined to be around the origin of the group. Otherwise, when the average motion statistics of the chosen origin group is not larger than a threshold, the motion is determined to be around the bot with the highest likelihood within the origin group.

In some embodiments, the present teaching discloses systems and methods for fall-down detection. In some embodiments, systems and methods are disclosed for fall-down detection based on radio signal plus auxiliary signal (e.g. speech/audio/sound/ light/image/video/vibration/pressure). Fall (or abrupt/impulsive/transient motion, or motion with an impact, or motion that generates sound/vibration/pressure change, or "target motion") would affect radio channel (e.g. WiFi) in a certain way that can be captured using wireless transceivers (e.g. WiFi chips), and thus radio-based fall detection (or radio-based target motion detection) is possible. But fall-related radio "signature"/profile/pattern may resemble that of other (non-target) events leading to confusion. Fall (or abrupt/impulsive/transient motion, or motion with an impact, or motion that generates sound/vibration/pressure change, or target motion) would usually generate an auxiliary signal (e.g. sound/light/pressure/ vibration) that can be captured/sensed using a sensor (e.g. microphones to capture sound, camera/light sensor to capture light, pressure sensor for pressure), and thus aux-based fall detection is possible. But fall-related auxiliary signal (e.g. sound) may be similar to/confusable with that of other (non-target) events leading to confusion/false alarm.

One goal of the present teaching is to use radio signal plus auxiliary (aux) signal to do better hybrid target motion (e.g. fall) detection. For example, the system can combine radio and aux to detect target motion (e.g. fall) jointly. The system may use radio and aux, one-at-a time/sequentially.

Option (a): the system may use radio-based target-motion detection first (tentatively/preliminary/initial) and then use aux-based target-motion detection to confirm/reject. Only when radio-based detection reports a potential target motion (e.g. fall) based on some radio-based criterion, the aux-based target-motion detection starts to check some aux-based criterion (e.g. if auxiliary signal is significant, e.g. sound is loud and brief). If yes, then target motion (e.g. fall) is detected/reported; otherwise, the tentative/preliminary/initial detection a false alarm.

Option (b): the system may use aux-based fall detection first and use radio-based fall detection to confirm/reject.

Instead of one-at-a-time, in option (c): the system may also use both auxiliary and radio together/two-at-a-time to detect target motion.

Radio-based target motion detection: Obtain time series of channel information (TSCI). Features (e.g. magnitude) may be computed. A time series of spatial-temporal information (TSSTI) may be computed based on features of TSCI. Each STI may be speed or acceleration (derivative of speed). Recognition of target radio behavior of TSSTI associated with target motion may be performed. For example, target motion may be fall (fall down). For free fall of an object, the acceleration may abruptly increase from 0 to 1G (i.e. acceleration due to gravity, approx. 9.8 m/s) until it lands on floor abruptly (when acceleration decreases from 1G to 0G abruptly). Sometimes a falling person may try to hold onto/grab something (e.g. furniture/table/chair) slowing him/her down, leading to acceleration abruptly increasing from 0 to less-than-1G. Sometimes a fall person may free fall before holding onto/grabbing/hitting something, such that (s.t.) acceleration increases from 0 to 1G (free fall) and then to less-than-1G. A fall person may fall onto something soft (e.g. sofa, mattress, carpet) and have a soft landing, with acceleration decreasing from 1G to 0G gradually (instead of abruptly).

Aux-based target motion detection: Obtain time series of auxiliary samples (TSAS). Features (e.g. magnitude, magnitude square) may be computed for each AS to get a time series of auxiliary feature (TSAF). Recognition of target auxiliary behavior of TSAF may be performed. If recognized, target motion may be detected. Auxiliary signal may be sound s.t. target auxiliary behavior may be a loud/brief/impulsive sound (target auxiliary behavior). To detect loud sound, AF should be larger than some threshold, or larger than some threshold for a majority of time within a short window (e.g. 0.1 second). Or, AF may be smoothed and the local max should be greater than some threshold to detect the loud sound.

In some embodiments, auxiliary signal is NOT radio signal. It may be audible/acoustic/sound/speech/vocal/audio signal, which may be sensed/captured by acoustic sensor (e.g. microphone). Auxiliary signal may be light/image/video, which may be sensed by camera/IR/PIR/light sensor. Auxiliary signal may be pressure/vibration signal, which may be sensed/captured by pressure/vibration sensor. Auxiliary signal may not be a radio signal. Auxiliary signal may not be data communication signal. Motion may be fall down motion, abrupt motion, transient motion, or motion with an impact. Microphone may be mono (one channel), stereo (two channels), or multiple channels.

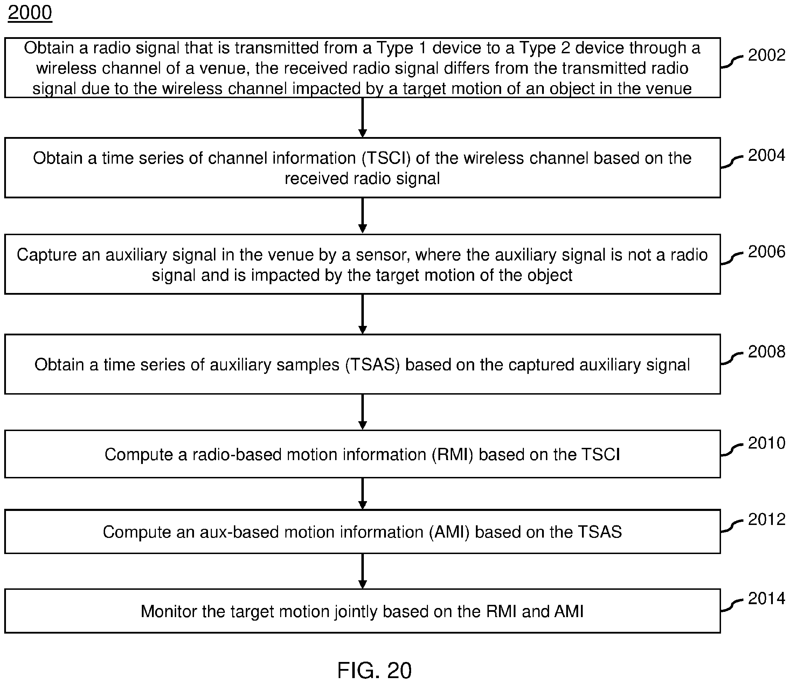

FIG. 20 illustrates a flow chart of an exemplary method 2000 for hybrid radio-plus-aux fall-down detection based on wireless sensing, according to some embodiments of the present disclosure. In various embodiments, the method 2000 can be performed by the systems disclosed above. At operation 2002, a radio signal is obtained, where the radio signal is transmitted from a Type 1 device to a Type 2 device through a wireless channel of a venue, the received radio signal differs from the transmitted radio signal due to the wireless channel impacted by a target motion of an object in the venue. At operation 2004, a time series of channel information (TSCI) of the wireless channel is obtained based on the received radio signal. At operation 2006, an auxiliary signal is captured in the venue by a sensor, where the auxiliary signal is not a radio signal and is impacted by the target motion of the object.

At operation 2008, a time series of auxiliary samples (TSAS) is obtained based on the captured auxiliary signal. At operation 2010, a radio-based motion information (RMI) is computed based on the TSCI. At operation 2012, an aux-based motion information (AMI) is computed based on the TSAS. At operation 2014, the target motion is monitored jointly based on the RMI and AMI. The order of the operations in FIG. 20 may be changed according to various embodiments of the present teaching.

In some embodiments, the system first performs radio-based detection based on RMI and/or radio-based criterion, then performs aux-based detection based on AMI and/or aux-based criterion, sequentially. In some embodiments, the system first performs aux-based detection based on AMI and/or aux-based criterion, then performs radio-based detection based on RMI and/or radio-based criterion, sequentially. In some embodiments, the system simultaneous and contemporaneously performs an aux-radio-based detection based on both RMI and AMI (radio-aux-criterion).

The following numbered clauses provide examples for wireless sensing using multiple groups of wireless devices.

Clause D1. A system for wireless sensing, comprising: a set of heterogeneous wireless devices in a venue, wherein the set of heterogeneous wireless devices comprise: a first device, a second device, and a particular device, the particular device comprises a first radio and a second radio, the particular device is configured to communicate with the first device through a first wireless channel based on a first protocol using the first radio, the particular device is configured to communicate with the second device through a second wireless channel based on a second protocol using the second radio; and a processor configured for: obtaining a time series of channel information (TSCI) of the second wireless channel based on a wireless signal that is communicated between the particular device and the second device through the second wireless channel using the second radio of the particular device, wherein each channel information (CI) comprises at least one of: channel state information (CSI), channel impulse response (CIR) or channel frequency response (CFR), computing a pairwise sensing analytics based on the TSCI, and computing a combined sensing analytics based on the pairwise sensing analytics, wherein the particular device is configured to transmit the combined sensing analytics to the first device through the first wireless channel using the first radio of the particular device, wherein the set of heterogeneous wireless devices is configured to perform a wireless sensing task based on the combined sensing analytics.

Clause D2. The system of clause D1, wherein: a first subset of the set of heterogeneous wireless devices is configured to perform the wireless sensing task using a first wireless network in the venue; the first subset comprises the particular device and the first device; a second subset of the set of heterogeneous wireless devices is configured to further perform the wireless sensing task using a second wireless network in the venue; the second subset comprises the particular device and the second device; the first radio, the first wireless channel, and the first protocol are associated with the first wireless network; the second radio, the second wireless channel, and the second protocol are associated with the second wireless network; and one of the first protocol or the second protocol comprises at least one of: a WiFi standard, a UWB standard, a WiMax standard, an IEEE standard, an IEEE 802 standard, an IEEE 802.11 standard, an IEEE 802.11bf standard, an 802.15 standard, an 802.15.4 standard, or an 802.16 standard.

Clause D3. The system of clause D2, wherein: the particular device and the first device are authenticated and associated in the first wireless network; and the particular device and the second device are authenticated and associated in the second wireless network.

Clause D4. The system of clause D3, wherein: the wireless signal is transmitted from the second device to the particular device based on the second protocol; and the TSCI is extracted from the wireless signal by the particular device.

Clause D5. The system of clause D4, wherein: the wireless signal is transmitted based on a trigger signal received by the second device from an access point (AP) of the second wireless network, based on the second wireless protocol.

Clause D6. The system of clause D4 or D5, wherein: the wireless signal is received based on a trigger signal received by the particular device from an access point (AP) of the second wireless network, based on the second wireless protocol.

Clause D7. The system of clause D3-D6, wherein: the wireless signal is transmitted from the particular device to the second device based on the second protocol; and the TSCI is extracted from the wireless signal by the second device.

Clause D8. The system of clause D3-D7, wherein: the second subset of the set of heterogeneous wireless devices comprises: a third device and a fourth device that are associated with the second wireless network; the fourth device is configured to obtain a second TSCI of the second wireless channel based on a second wireless signal that is transmitted from the third device to the fourth device through the second wireless channel in the second wireless network; the particular device is configured to obtain a second pairwise sensing analytics computed based on the second TSCI; and the combined sensing analytics is computed based on the pairwise sensing analytics and the second pairwise sensing analytics.

Clause D9. The system of clause D8, wherein the fourth device is configured to: compute the second pairwise sensing analytics based on the second TSCI; and transmit the second pairwise sensing analytics to the particular device.

Clause D10. The system of clause D8 or D9, wherein: the fourth device is configured to transmit the second TSCI to the particular device; and the particular device is configured to compute the second pairwise sensing analytics based on the second TSCI.

Clause D11. The system of clause D8-D10, wherein the particular device is configured to: obtain a second combined sensing analytics associated with a third wireless network, wherein the combined sensing analytics is computed based on the second combined sensing analytics.

Clause D12. The system of clause D11, wherein: a third subset of the set of heterogeneous wireless devices is configured to further perform the wireless sensing task using the third wireless network in the venue; the third subset comprises: a fifth device and a sixth device that are associated with the third wireless network, and a second particular device; the sixth device is configured to obtain a third TSCI of a third wireless channel based on a third wireless signal that is transmitted from the fifth device to the sixth device through the third wireless channel in the third wireless network; the second particular device is configured to obtain a third pairwise sensing analytics computed based on the third TSCI, and compute the second combined sensing analytics based on the third pairwise sensing analytics; and the particular device is configured to obtain the second combined sensing analytics from the second particular device.

Clause D13. The system of clause D12, wherein the particular device is configured to: obtain a third combined sensing analytics associated with a fourth wireless network, wherein the combined sensing analytics is computed further based on the third combined sensing analytics.

Clause D14. The system of clause D13, wherein: a fourth subset of the set of heterogeneous wireless devices is configured to further perform the wireless sensing task using the fourth wireless network in the venue; the fourth subset comprises: a seventh device and an eighth device that are associated with the fourth wireless network, and a third particular device; the eighth device is configured to obtain a fourth TSCI of a fourth wireless channel based on a fourth wireless signal that is transmitted from the seventh device to the eighth device through the fourth wireless channel in the fourth wireless network; the third particular device is configured to obtain a fourth pairwise sensing analytics computed based on the fourth TSCI, and compute the third combined sensing analytics based on the fourth pairwise sensing analytics; and the particular device is configured to obtain the third combined sensing analytics from the third particular device.

Clause D15. The system of clause D14, wherein: a fifth subset of the set of heterogeneous wireless devices is configured to further perform the wireless sensing task using a fifth wireless network in the venue; the fifth subset comprises: a ninth device and a tenth device that are associated with the fifth wireless network, and a fourth particular device; the tenth device is configured to obtain a fifth TSCI of a fifth wireless channel based on a fifth wireless signal that is transmitted from the ninth device to the tenth device through the fifth wireless channel in the fifth wireless network; the fourth particular device is configured to obtain a fifth pairwise sensing analytics computed based on the fifth TSCI, and compute a fourth combined sensing analytics based on the fifth pairwise sensing analytics; and the first device is configured to obtain the fourth combined sensing analytics from the fourth particular device, wherein the wireless sensing task is performed based on the fourth combined sensing analytics.

Clause D16. The system of clause D15, wherein: the wireless sensing task is performed by the set of heterogeneous wireless devices using multiple wireless networks in the venue, with at least two of the set of heterogeneous wireless devices assigned to each of the multiple wireless networks; the multiple wireless networks have a multi-tier structure comprising at least two tiers; the first wireless network is a Tier-1 network of the multiple wireless networks and comprises at least: the particular device and the first device; the second wireless network is a Tier-2 network of the multiple wireless networks and comprises at least: the particular device, the second device, the third device and the fourth device; the particular device serves as a gateway device between the first wireless network and the second wireless network, such that sensing results of the wireless sensing task are transmitted from the particular device via the first wireless network to the first device; heterogeneous wireless devices in a Tier-K network of the multiple wireless networks are configured to report sensing results obtained in the Tier-K network to a heterogeneous wireless device in a Tier-(K-1) network of the multiple wireless networks via a gateway device between the Tier-K network and the Tier-(K-1) network, wherein K is an integer greater than one, wherein the reported sensing results comprise at least one of: a combined sensing analytics, a pairwise sensing analytics or TSCI.

Clause D17. The system of clause D16, wherein: the first wireless network further comprises: the second particular device and the third particular device; the third wireless network is a Tier-3 network of the multiple wireless networks and comprises at least: the second particular device that is a gateway device, the fifth device, and the sixth device, the second particular device is a gateway device, such that sensing results obtained in the third wireless network are transmitted from the second particular device via the first wireless network to the particular device; the fourth wireless network is a Tier-3 network of the multiple wireless networks and comprises at least: the third particular device that is a gateway device, the seventh device, and the eighth device; and the third particular device is a gateway device, such that sensing results obtained in the fourth wireless network are transmitted from the third particular device via the first wireless network to the particular device.

Clause D18. The system of clause D17, wherein: the first wireless network further comprises the fourth particular device; the fourth wireless network is a Tier-2 network of the multiple wireless networks and comprises at least: the fourth particular device, the ninth device, and the tenth device; and the fourth particular device is a gateway device, such that sensing results are transmitted from the fourth particular device via the first wireless network to the first device.

Clause D19. The system of clause D18, wherein: the first wireless network is associated with a zone of the venue; the second wireless network is associated with a first sub-zone of the zone; the third wireless network is associated with a first sub-sub-zone of the first sub-zone; and the fourth wireless network is associated with a second sub-sub-zone of the first sub-zone.

Clause D20. The system of clause D19, wherein the set of heterogeneous wireless devices is configured to: perform the wireless sensing task for the zone based on at least one of: any pairwise sensing analytics associated with the first wireless network, the combined sensing analytics associated with the second wireless network, or the fourth combined sensing analytics associated with the fifth wireless network; perform the wireless sensing task for the first sub-zone based on at least one of: any pairwise sensing analytics associated with the second wireless network, the combined sensing analytics associated with the second wireless network, the second combined sensing analytics associated with the third wireless network, or the third combined sensing analytics associated with the fourth wireless network; associate the third wireless network with the first sub-zone of the zone; perform a wireless sensing subtask associated with the first sub-zone based on the third wireless network and another combined sensing analytics; and associate the fourth wireless network with a second sub-zone of the zone.

Clause D21. A method performed by a set of heterogeneous wireless devices in a venue for wireless sensing, comprising: communicatively coupling a particular device of the set with a first device of the set through a first wireless channel based on a first protocol using a first radio of the particular device; communicatively coupling the particular device with a second device of the set through a second wireless channel based on a second protocol using a second radio of the particular device; performing a pairwise sub-task by the particular device and the second device based on a wireless signal communicated between the particular device and the second device through the second wireless channel using the second radio of the particular device; obtaining by the particular device a pairwise sensing analytics computed based on a time series of channel information (TSCI) of the second wireless channel extracted from the wireless signal, wherein each channel information (CI) comprises at least one of: channel state information (CSI), channel impulse response (CIR) or channel frequency response (CFR); computing a combined sensing analytics by the particular device based on the pairwise sensing analytics; transmitting the combined sensing analytics by the particular device to the first device through the first wireless channel using the first radio of the particular device; and performing a wireless sensing task based on the combined sensing analytics.

The following numbered clauses provide examples for a hybrid radio-plus-aux fall-down detection.

Clause E1. A method/device/system/software of a hybrid radio-plus-aux monitoring system for monitoring a target motion in a venue, comprising: receiving a radio signal by a Type 2 heterogeneous wireless device in a venue, wherein the radio signal is transmitted to the Type 2 device by a Type 1 heterogeneous wireless device through a wireless multipath channel of the venue, wherein the received radio signal differs from the transmitted radio signal due to the wireless multipath channel impacted by the target motion of an object in the venue; obtaining a time series of channel information (TSCI) of the wireless multipath channel based on the received radio signal based on a processor, a memory and a set of instructions; capturing an auxiliary signal in the venue by a sensor, wherein the auxiliary signal is not a radio signal and comprises at least one of: an audible signal less than 100kHz, a perceptual signal, an acoustic signal, a sound signal, a speech signal, a vocal signal, an audio signal, a visual signal, a light signal, an image, a video, a mechanical signal, a vibration signal, or a pressure signal, wherein the auxiliary signal is impacted by the target motion of the object; obtaining a time series of auxiliary samples (TSAS) based on the captured auxiliary signal; and monitoring the target motion jointly based on the TSCI and TSAS.

In some embodiments, RMI may be speed/acceleration. AMI may be sound volume/energy score (for detecting "loud" sound). E.g. AMI may be sum of magnitude (or magnitude square) of all AA in a time window.

Clause E2. The method/device/system/software of the hybrid radio-plus-aux monitoring system of clause E1, comprising: computing a radio-based motion information (RMI) based on the TSCI; computing an aux-based motion information (AMI) based on the TSAS; monitoring the target motion jointly based on the RMI and AMI.

In some embodiments, detection can be performed using radio/aux either one-at-a-time (radio-based + aux-based), or two-at-a-time (radio-aux-based).

Clause E3. The method/device/system/software of the hybrid radio-plus-aux monitoring system of clause E2, comprising: computing at least one of: a radio-based detection of the target motion of the object based on the RMI or the TSCI, a aux-based detection of the target motion of the object based on the AMI or the TSAS, or a radio-aux-based detection of the target motion of the object based on the RMI and the AMI, or the TSCI and the TSAS; detecting the target motion of the object jointly based on the at least one of: the radio-based detection, the aux-based detection, or the radio-aux-based detection.

In some embodiments, one-at-a-time (option a or b), in which radio-based and aux-based are performed sequentially.

Clause E4. The method/device/system/software of the hybrid radio-plus-aux monitoring system of clause E3, comprising: computing the radio-based detection and aux-based detection sequentially; detecting the target motion of the object jointly based on the radio-based detection and the aux-based detection.

Clause E5. The method/device/system/software of the hybrid radio-plus-aux monitoring system of clause E4, comprising: wherein the target motion is detected in the radio-based detection when a radio-based criterion is satisfied, and wherein the target motion is detected in the aux-based detection when an aux-based criterion is satisfied.

In some embodiments, one-at-a-time, (option a): radio-based based on RMI first. Then use aux-based based on AMI to confirm.

Clause E6. The method/device/system/software of the hybrid radio-plus-aux monitoring system of clause E5, comprising: computing a tentative detection of the target motion of the object based on the radio-based detection; when the target motion is detected in the tentative detection: computing the AMI based on the TSAS, computing a final detection of the target motion of the object based on the aux-based detection.

In some embodiments, one-at-a-time, (option b): aux-based first. Then use radio-based to confirm.

Clause E7. The method/device/system/software of the hybrid radio-plus-aux monitoring system of clause E5 or E6, comprising: computing a tentative detection of the target motion of the object based on the aux-based detection; when the target motion is detected in the tentative detection: computing the RMI based on the TSCI, computing a final detection of the target motion of the object based on the radio-based detection.

In some embodiments, two-at-a-time (option c), in which radio-aux-based detection based on both RMI and AMI is performed.

Clause E8. The method/device/system/software of the hybrid radio-plus-aux monitoring system of clause E3-E7, comprising: computing the radio-aux-based detection; detecting the target motion of the object jointly based on the radio-aux-based detection.

Clause E9. The method/device/system/software of the hybrid radio-plus-aux monitoring system of clause E8, comprising: wherein the target motion is detected in the radio-aux-based detection when a radio-aux-based criterion is satisfied.

In some embodiments, details of RMI and the radio-based criterion, features of CI, and STI can be computed based on the CI features, and RMI based on STI.

Clause E10. The method/device/system/software of the hybrid radio-plus-aux monitoring system of clause E5-E9, comprising: computing a feature for each channel information (CI) of the TSCI, wherein the feature comprises at least one of: transform, magnitude, amplitude, energy, power, intensity, strength, a monotonic function of any of the above, or a sliding aggregate of any of the above, wherein any aggregate comprises at least one of: sum, weighted sum, average, weighted average, trimmed mean, arithmetic mean, geometric mean, harmonic mean, percentile, median, mode, another monotonic function of any of the above, or another aggregate of any of the above. Computing a time series of spatial-temporal information (TSSTI) of the target motion of the object based on the feature of CI of the TSCI, wherein each spatial-temporal information (STI) comprises at least one of: a location, a zone, a direction, a speed, or an acceleration; computing the RMI based on the TSSTI.

Clause E11. The method/device/system/software of the hybrid radio-plus-aux monitoring system of clause E10, comprising: recognizing a target radio behavior of the TSSTI associated with the target motion of the object; wherein the radio-based criterion is satisfied when the target radio behavior of the TSSTI is recognized.

In some embodiments, RMI is acceleration, or derivative of speed.

Clause E12. The method/device/system/software of the hybrid radio-plus-aux monitoring system of clause E11, comprising: wherein the target motion is a fall-down motion; wherein the STI is one of: a speed, or an acceleration, of the motion of the object; wherein the speed is non-negative; wherein RMI is the acceleration which is a first-order derivative of speed; wherein the target behavior of TSSTI comprises the following sequence of events: acceleration remaining at near-zero for a first duration; then increasing from near-zero to near-1G within a second duration, then remaining at near-1G for a third duration, then decreasing from near-1G to near-zero within a fourth duration, and then remaining at near-zero for a fifth duration; wherein near-zero is a range of acceleration less than a sixth threshold, comprising zero G, wherein G is acceleration due to gravity; wherein near-1G is a range of acceleration greater than a seventh threshold, comprising one G.

Clause E13. The method/device/system/software of the hybrid radio-plus-aux monitoring system of clause E12, comprising: wherein the first duration is greater than a first threshold; wherein the second duration is less than a second threshold; wherein the third duration is less than a third threshold; wherein the fourth duration is less than a fourth threshold; wherein the fifth duration is greater than a fifth threshold;

Clause E14. The method/device/system/software of the hybrid radio-plus-aux monitoring system of clause E10-E13, comprising: wherein each STI is computed based on a similarity score between a pair of temporally adjacent CI of the TSCI.

Clause E15. The method/device/system/software of the hybrid radio-plus-aux monitoring system of clause E14, comprising: wherein each STI is computed based on a function in a time window associated with the STI; wherein the function comprises at least one of: transform, frequency transform, inverse frequency transform, Fourier transform, inverse Fourier transform, convolution, auto correlation function, cross correlation function, auto covariance function, or cross covariance function, wherein the function is computed based on the TSCI or the features of the TSCI in the time window.

Clause E16. The method/device/system/software of the hybrid radio-plus-aux monitoring system of clause E15, comprising: wherein the STI is computed based on a characteristic point of the function; wherein the characteristic point comprises at least one of: a global maximum, a global minimum, a local maximum, a local minimum, a constrained local maximum, a constrained local minimum, a first local maximum, a first local minimum, a k-th local maximum, a k-th local minimum, a pair of adjacent local maximum and a local minimum, a zero-crossing, an aggregate, an average, a weighted average, a percentile, a median, a mode, a mean, or a trimmed mean.

In some embodiments, details of AMI and the aux-based criterion (detection of loud sound associated with fall). TSAS may be preprocessed. Aux feature may be extracted.

Clause E17. The method/device/system/software of the hybrid radio-plus-aux monitoring system of clause ES-E16, comprising: preprocessing the TSAS, wherein the preprocessing comprises at least one of: denoising, smoothing, lowpass filtering, bandpass filtering, highpass filtering, or conditioning; computing a feature for each preprocessed auxiliary sample (AS) in a time window of the preprocessed TSAS, wherein the feature comprises at least one of: transform, magnitude, amplitude, energy, power, intensity, strength, a monotonic function of any of the above, or a sliding aggregate of any of the above, wherein any aggregate comprises at least one of: sum, weighted sum, average, weighted average, trimmed mean, arithmetic mean, geometric mean, harmonic mean, percentile, auxiliary, mode, another monotonic function of any of the above, or another aggregate of any of the above.

Clause E18. The method/device/system/software of the hybrid radio-plus-aux monitoring system of clause E17, comprising: recognizing a target auxiliary behavior of the TSAS associated with the target motion of the object; wherein the aux-based criterion is satisfied when the target auxiliary behavior of the TSAS is recognized.

In some embodiments, possibility 1: AMI is aggregate of AS magnitude in a time window and is greater than a threshold.

Clause E19. The method/device/system/software of the hybrid radio-plus-aux monitoring system of clause E18, comprising: computing the AMI as a second aggregate of the features of the AS in the time window of the TSAS, wherein the target auxiliary behavior of the TSAS is recognized when the AMI is greater than a threshold.

In some embodiments, possibility 2: AMI is all AS magnitude in time window. Each AS magnitude may be compared with a first threshold. The amount (or percentage) of AS magnitude greater than first threshold may be counted/computed. Criterion may be satisfied if the amount (or percentage) is greater than second threshold.

Clause E20. The method/device/system/software of the hybrid radio-plus-aux monitoring system of clause E18 or E19, comprising: wherein the AMI comprises all the features of the AS in the time window; wherein the target auxiliary behavior of the TSAS is recognized when the amount of AMI in the time window exceeding a first threshold is greater than a second threshold.

In some embodiments, details of RMI/AMI and the radio-aux-based criterion. CI may have multiple components (e.g. subcarriers). AS may have multiple components (e.g. stereo sound has left/right components/channels, 5.1 surround sound may have 6 components/channels). Feature (e.g. magnitude, magnitude square) of each component may be computed. STI may be computed based on CI component features. AF may be computed by aggregating component features of each AS.

Clause E21. The method/device/system/software of the hybrid radio-plus-aux monitoring system of clause E9-E20, comprising: wherein each CI and each AS comprise at least one component, computing a first component feature for each component of each channel information (CI) of the TSCI; computing a time series of spatial-temporal information (TSSTI) of the target motion of the object based on the first component features of the components of the CI of the TSCI; computing a second component feature for each component of each auxiliary sample (AS) of the TSAS; computing a time series of auxiliary features (TSAF) based on the second component features of the components of the AS of the TSAS, each auxiliary feature being an aggregate of the second component features of a respective AS, wherein any aggregate comprises at least one of: sum, weighted sum, average, weighted average, trimmed mean, arithmetic mean, geometric mean, harmonic mean, percentile, median, mode, another monotonic function of any of the above, or another aggregate of any of the above.

In some embodiments, RMI/AMI may be recognition score of target radio/auxiliary behavior. Target motion may be recognized if RMI>T1 and AMI>T2, or if aggregate of RMI and AMI >T3.

Clause E22. The method/device/system/software of the hybrid radio-plus-aux monitoring system of clause E21, comprising: recognizing a target radio behavior of the TSSTI associated with the target motion of the object; computing the RMI as a recognition score of the recognition of the target radio behavior of the TSSTI; recognizing a target auxiliary behavior of the TSAF associated with the target motion of the object; computing the AMI as a recognition score of the recognition of the target auxiliary behavior of the TSAF; computing a combined motion information (CMI) as an aggregate of the RMI and the AMI; wherein the radio-aux-based criterion is satisfied when the CMI exceeds a first threshold; wherein the radio-aux-based criterion is also satisfied when RMI exceeds a second threshold and AMI exceeds a third threshold.

In some embodiments, the present teaching discloses systems and methods for wireless sensing based on a network comprising local groups of wireless devices.

In some embodiments, a combination of local subsystems with a main system is disclosed. One aspect of the present teaching is about performing wireless sensing in a venue with multiple groups of devices, establishing (a) a local wireless sensing subsystem based on each group of devices, and (b) a main wireless sensing system in the venue linking/connecting all the local wireless sensing subsystems.

Grouping of devices. For a venue, a user may define a number of groups and assign each device in the venue to a group. The user may use a user device (e.g. smart phone, computer, tablet or other smart device) to connect/interact with each device to assign the device to the groups. For example, each group of devices may be associated with a certain area/zone (e.g. bedroom, kitchen, living room) in the venue (e.g. a house). There may be multi-tier or hierarchical grouping. In a high level, there may be no subdivision such that all devices are in the same group (e.g. all devices in a house considered as one group). In the next level, the group may be subdivided into two or more subgroups (e.g. a "first-floor" subgroup comprising devices in first floor of the house, a "second-floor" subgroup comprising devices in second floor). In the next level, a subgroup may be subdivided into two or more sub-sub-groups (e.g. second-floor devices subdivided into sub-subgroups such as "bedroom 1", "bedroom 2", etc.; first-floor devices subdivided into sub-subgroups such as "kitchen", "living room", "dining room", etc.) and so on.

Network of networks configuration. The main wireless sensing system comprises a network of devices in a star configuration with a device in the center of the star configuration (called "main center device") and a number of devices at the vertices of the star configuration (called "main terminal devices") linked/connected radially to the main center device. Similarly, each subsystem comprises a network (or sub-network, called "local sub-network") of devices in a star configuration with a corresponding local device in the center of the star configuration (called "local center device") and a number of devices at the vertices of the star configuration (called "local terminal devices") linked/connected radially to the local center devices.

In some embodiments, the user may designate each device to the roles of main center device, main terminal devices, local center devices and local terminal devices. A device may be designated to multiple roles (e.g. both a main terminal device and a local terminal device). If the user does not differentiate the devices in a particular into local center device and local terminal device, a certain algorithm may be applied to automatically choose one of a group of devices to be local center device. For example, a criterion for the choice of local center device may be the ability to be an access point (AP), or the ability to multicast/broadcast. The device with recent multi-cast may be chosen.

Wireless sensing in local subsystems/networks. Wireless sensing measurements take place in the subsystems (local sub-networks). The devices may be configured to perform wireless sensing in each link (or branch) of each subsystem (local sub-network). In other words, pairwise wireless sensing is performed between the local center device and each local terminal device. In each link, a first device (either the local center device or the local terminal device) functions as a Type1 device to transmit a wireless signal (e.g. a train of sounding signals, null-data-packet, NDP, NDP announcement, NDPA, NDPA sounding, trigger-based (TB) sensing, non-TB sensing, trigger frame (TF) sounding, NDPA sounding, initiator-to responder (I2R) sounding, responder-to-initiator (R2I) sounding) to the second device. The second device (either the local terminal device or the local center device) obtains information of the wireless channel (channel information or CI, such as CSI, CIR, CFR, etc.) based on the received wireless signal. It may perform some WiFi sensing computational tasks based on the CI to obtain raw sensing data. It may transmit/report the raw sensing data to the first device. It may transmit/report the CI to the first device which would perform some WiFi sensing computational tasks based on the CI to obtain raw sensing data.

In some embodiments, if the local center device is the first device in multiple links in the local sub-network, it may broadcast, multi-cast and/or unicast the wireless signal(s) to the local terminal devices in the multiple links. In some embodiments, if the local center device is the second device in multiple links, the local terminal devices in the multiple links may transmit the respective wireless signals simultaneously and/or sequentially. Such transmission may be in response to some signal (e.g. trigger signal, trigger frame, polling signal) from the local center device.

In some embodiments, the main center device may establish a local subsystem (local sub-network) with itself being the local center device of the star configuration of the local subsystem and may take part in wireless sensing accordingly.

Details of sensing setup and signaling. More than one wireless signals may be simultaneously transmitted using different frequency bands, different frequency subbands, different frequency carriers, different antennas, and/or different beamforming/beamformer. The configuration of the devices, configuration (e.g. sensing session setup, sensing measurement setup, sensing-by-proxy (SBP) setup) of each pair of devices performing pairwise wireless sensing, transmission of the wireless signal, and/or the obtaining of channel information may be based on a standard (e.g. 802.11, 802.11bf, 4G, 5G, 6G, 7G, 8G, etc.). The transmission of the wireless signal may be in response or correlated to another wireless signal or wireless handshake/procedure (e.g. trigger signal, request, SBP request, sensing session set up, sensing measurement set up). The transmission of the wireless signal may be associated with an identification (ID, e.g. session ID, measurement ID, session setup ID, measurement setup ID, measurement instance ID, time code, etc.).

Main network and local sub-network linkage. In some embodiments of the present teaching, a local sub-network may be linked to the main network in 3 possible methods: (a) via the local center device of the local sub-network, (b) via the local terminal devices of the local sub-network, or (c) via both the local center device and at least one terminal device.

In Method (a), the local center device of the local sub-network is linked/connected to the main center device in the main network. In other words, the local center device is also a main terminal device. The local center device may function as the second device such that the wireless signals may be transmitted from the local terminal devices to the local center device. TB sensing with TF sounding may be used. Non-TB sensing with I2R sounding may be used. Alternatively, the local center device may function as the first device such that the wireless signal(s) may be broadcasted/multi-casted/unicasted/ transmitted from the local center device to the local terminal devices. TB sounding may be used. NDPA sounding may be used. The CI obtained by the local terminal device, or raw sensing data computed based on the CI by the local terminal device, may be transmitted/reported to the local center device.

In Method (b), each local terminal device is linked/connected to the main center device. In other words, each local terminal device is also a main terminal device. The local center device may function as the first device such that the wireless signal(s) may be broadcasted, multi-casted or unicasted from the local center device to the local terminal devices. Alternatively, the local center device may function as the second device such that the wireless signals are transmitted from the local terminal devices to the local center device. The CI obtained by the local center device, or raw sensing data computed based on the CI by the local center device, may be transmitted/reported to the local terminal device.

In Method (c), both the local center device and at least one local terminal device are linked/connected to the main center device. Method (c) is a hybrid between (a) and (b). The local center device may function as the first device such that the wireless signal(s) may be broadcasted, multi-casted or unicasted from the local center device to the local terminal devices. The local center device may function as the second device in some links such that the wireless signals are transmitted from the local terminal devices to the local center device.

Center devices being AP. Note that each center device may be an access point of the corresponding network. For example, a local center device may be the access point (AP) of the corresponding local sub-network, while the main center device may be AP of the main network. The center device may have broadband/internet access.

Local terminal device network usage. For Method (a), (b) or (c) in which a local device in a local sub-network (either local center device or local terminal device) may be simultaneously a main terminal device, the local device may be associated with the local sub-network using one channel or one band (e.g. a 2.4GHz channel, a 5GHz channel, a 60GHz channel, a millimeter wave channel, a UWB channel) and a second network of the main center device using a second channel or a second band (e.g. a 2.4GHz channel, a 5GHz channel, a 60GHz channel, a millimeter wave channel, a UWB channel) simultaneously. The local device may be a dual-band, tri-band, quad-band or higher-band device. It may use the first network to perform wireless sensing (transmit or receive wireless signal and obtain raw sensing result/data computed based on CI obtained from the wireless signal) and use the second network to transmit the raw sensing data to the main center device.

Main center device network usage. The main center device may simultaneous be a local center device associated with its own or "personal" local sub-network. In addition to be AP for the main network, it may simultaneously be the AP of its personal local sub-network. It may use a first channel or a first band for the main network and use a second channel or a second band for the personal local sub-network. The first channel and the second channel may be the same or different. The personal local sub-network and the main network may be the same network.

The main center device may use the main network to receive raw sensing data (or result) from the local sub-networks and to send configuration or software update information to the local terminal devices. Configuration or software update information for the local center device may be sent by the main center device to the local center device directly using the main network. The configuration or software update information for the local center device may also be sent by the main center device to a local terminal device using the main network enroute to the local center device via the local sub-network.

In some embodiments, the main center device may use its personal local sub-network to perform wireless sensing. The resulting raw sensing data obtained in the wireless sensing may be transmitted/reported to the main center device via the personal local sub-networks or the main network.

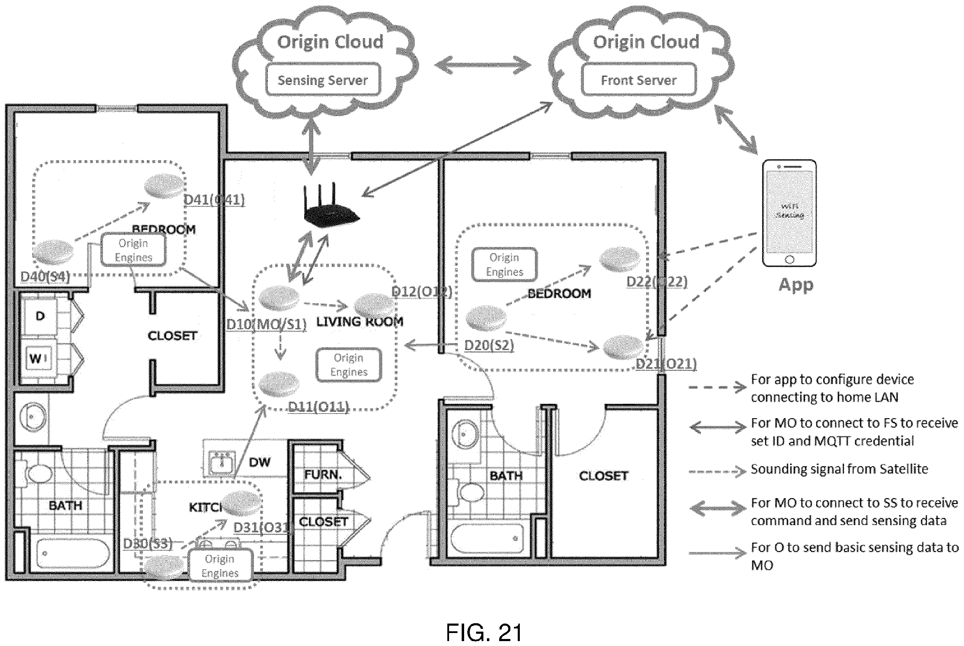

An example of performing wireless sensing in a venue with multiple groups of devices based on the disclosed method is illustrated in FIG. 21. The venue is a house with four zones, including two bedroom areas, a living room area and a kitchen area. The house has a WiFi router that has broadband connection to two cloud servers: a sensing server and a front server. The WiFi router establishes a home WiFi network with SSID MYHOME. The wireless sensing system comprises four subsystems formed by ten WiFi devices in the house. Three of the devices D10, D11, and D12 together form the first subgroup of devices that constitute a first subsystem or local sub-network to monitor a first zone (the living room area). Another three devices D20, D21, and D22 together form the second subgroup of devices that constitute a second subsystem or local sub-network to monitor a second zone (a bedroom area). Two devices D30 and D31 together form the third subgroup of devices that constitute a third subsystem to monitor a third zone (the kitchen area). Two devices D40 and D41 together form the fourth subgroup of devices that constitute a fourth subsystem (local sub-network) to monitor a fourth zone (another bedroom area).

In some embodiments, among the devices, D10 is the designated main center device, called master origin (MO), of the main network and the designated local center device of the first local sub-network. D11 and D12 are the designated local terminal devices of the first local sub-network. D10 has a dual band WiFi router chip with a 2.4 GHz band radio and a 5 GHz band radio. D10 is configured to be an AP to establish the first local sub-network with an SSID of "SENSE-SUBNET-01" using the 5 GHz radio. Each of D11 and D12 is a WiFi-enabled IoT device and is configured to associate with D10 and join the SENSE-SUBNET-01 network using the 5 GHz radio. Wireless sensing is performed in the first local sub-network with D10 broadcasting/multi-casting/unicasting a series of sounding signals to D11 and D12, each of which extracts CSI from the received sounding signals and performs at least one sensing computation task based on the CSI (e.g. motion detection, breathing detection, fall-down detection, etc.) to obtain raw sensing results (e.g. labels such as "motion detected", "motion not detected", "breathing detected", "breathing not detected", "fall-down detected", "fall-down not detected", motion statistics, breathing rate, etc.). In FIG. 21, a device marked "(O##)" is an Origin (or Type2 device); a device marked "(S#)" (e.g. "S1" or "S2" or "S3" or "S4") is a Satellite. In some embodiments, a Satellite is basically Tx or transmitter or Type1 device; while an Origin is basically Rx or receiver or Type2 device.

In some embodiments, all the devices (D10, D11, D12, D20, D21, D22, D30, D31, D40, D41) are configured to associate with the MYHOME home WiFi network using the 2.4 GHz radio. The subsystems report/transmit raw sensing results to the main center device (D10) or cloud server using MYHOME home WiFi network.

In a similar manner, each of the other local sub-networks has a designated local center device (e.g. D20 for the second local sub-network, D30 for the third and D40 for the fourth) and a number of designated local terminal devices. The designated local center device is configured to be an AP to establish the corresponding local sub-network with a corresponding SSID ("SENSE-SUBNET-02" for the second local sub-network, "SENSE-SUBNET-03" for the third, "SENSE-SUBNET-04" for the fourth) using the 5 GHz radio. Each designated local terminal device is configured to associate with the corresponding local center device and join the corresponding local sub-network. To perform the wireless sensing, the local center device transmits/broadcasts/multi-casts/unicasts a series of sounding signals to the local terminal device(s). Each local terminal device obtains CSI from the received sounding signals, performs at least one sensing computation task based on the CSI to obtain raw sensing results. The raw sensing results may be reported/transmitted to the main center device (D10) or cloud servers using the MYHOME home WiFi network. In some embodiments, the main center device (D10) may be configured to combine/process/fuse the raw sensing results and/or to report/transmit the results to the cloud servers.

In some embodiments, a plurality of wireless devices (a set of heterogeneous wireless devices) in a venue (e.g. home) may be grouped into N+1 groups/networks (N>2), comprising a "main group" (Tier-1 network) and N "subgroups" (Tier-2/3/../K networks). The groups/networks may be overlapping, e.g. two groups/networks may share a common device (i.e. a device may be in both the main group and a subgroup). A subgroup (Tier-2/Tier-3/../Tier-K network) may/may not be a subset of the main group. Each subgroup (Tier-K network for some K>1) may comprise at least one device (called "particular wireless devices") being also in the main group (Tier-1 network).

In some embodiments, one of the N+1 groups may be a "main" group (first subset) of wireless devices, and may constitute a "main wireless sensing system", with one device being "main center device" (first device) and the others in the group being "main terminal device(s)" (Tier-1 devices). All devices in the main group may be communicatively coupled in a "main" wireless network/Tier-1 network (e.g. home WiFi network "MyHome") via a "main" wireless channel (first wireless channel) based on a "main" protocol (first protocol, e.g. 2.4GHz, WiFi, 802.11 standard, 20MHz channel). All devices in the main group may be associated with a "main" access-point (AP, e.g. home AP) and the "main" wireless network may be a wireless network associated with the main AP (e.g. home WiFi network).

In some embodiments, each of N remaining groups may be a "subgroup" (or Tier-2 group) of wireless devices, and may constitute a "sub-system" or a local system, with one device being "local center device" and the others in the subgroup being "local terminal device(s)". All devices in a respective subgroup may be communicatively coupled in a respective wireless network or a respective local sub-network via a respective "local" wireless channel based on a respective "local" protocol (e.g. one subgroup may use SGHz, WiFi, 802.11 standard, 20 MHz channel; another subgroup may use SGHz, WiFi, 80MHz channel). The respective local center device may function as a respective AP and the respective wireless network or respective local sub-network may be the associated wireless network.

In a subgroup, the local center device may be either the main center device, or a main terminal device. None, one, more than one, or all, of local terminal device(s) of the subgroup may be either the main center device, or a main terminal device. Wireless channels used by the main group (e.g. 2.4GHz WiFi) may be different from those used by the subgroups (e.g. 5GHz WiFi).

Except for the main group, each group (Tier-K network, K>1) may be used to perform wireless sensing measurement (sensing measurement setup, transmission of wireless sounding signals, extraction of channel information (TSCI), polling/reporting/termination, computation of pairwise sensing analytics, aggregation of pairwise sensing analytics to compute combined sensing analytics). A gateway device in each group/Tier-K network may be used to report sensing results obtained/computed in the Tier-K network to a device in a Tier-(K-1) network.

The N+1 networks may be configured to form a network of networks, with a multi-tier structure. A Tier-K network reports sensing results to a Tier-(K-1) network, for any K>1. Tier-1 network may be the main network. The N groups may form N sub-networks (performing sensing measurements), and one group may be in a main network (enabling communication of sensing results/analytics/TSCI).

A gateway device may comprise multiple radios (e.g. 2.4GHz radio and 5GHz radio), and may use them to join more than one networks. For example, a Tier-3 gateway device (e.g. third particular wireless device) may use SGHz/6GHz radio to join a Tier-3 network for performing/obtaining sensing measurements (e.g. TSCI, pairwise sensing analytics) and may use 2.4GHz radio to join Tier-1 network (e.g. first network, home network)) and use the Tier-1 network to send sensing results computed in the Tier-3 network from the Tier-3 network to a gateway device of a Tier-2 network (e.g. second particular wireless device) via the Tier-1 network. Similarly, the Tier-2 network may use its own 2.4GHz radio to join the Tier-1 network and to use its SGHz/6GHz radio to perform/obtain sensing measurements.

In some embodiments, a gateway device links/connects two networks: a Tier-(K-1) network and a Tier-K network (e.g. Tier-1 network and Tier-2 network). Sensing results obtained in the Tier-K network may be transmitted from the Tier-K network to the Tier-(K-1) network (via the Tier-1 network).

In some embodiments, first combined analytics is associated with first wireless network (Tier-1). First device may compute first combined analytics by aggregating combined analytics from Tier-2 networks. Second combined analytics/particular device are associated with second wireless network (Tier-2). Third combined analytics/particular device are associated with third wireless network (Tier-3). Fourth combined analytics/particular device are associated with fourth wireless network (Tier-3). Fifth combined analytics/particular device are associated with fifth wireless network (Tier-2).

In some embodiments, pairwise sensing analytics may comprise a characteristics/spatial-temporal information(STI)/motion information(MI) of an object or a motion of the object. Pairwise sensing analytics may be based on a similarity score between a pair of temporally adjacent CI of a TSCI. The combined sensing analytics may be simply the pairwise sensing analytics (e.g. in the case of only 2 sensing devices in second network (Tier-K network).

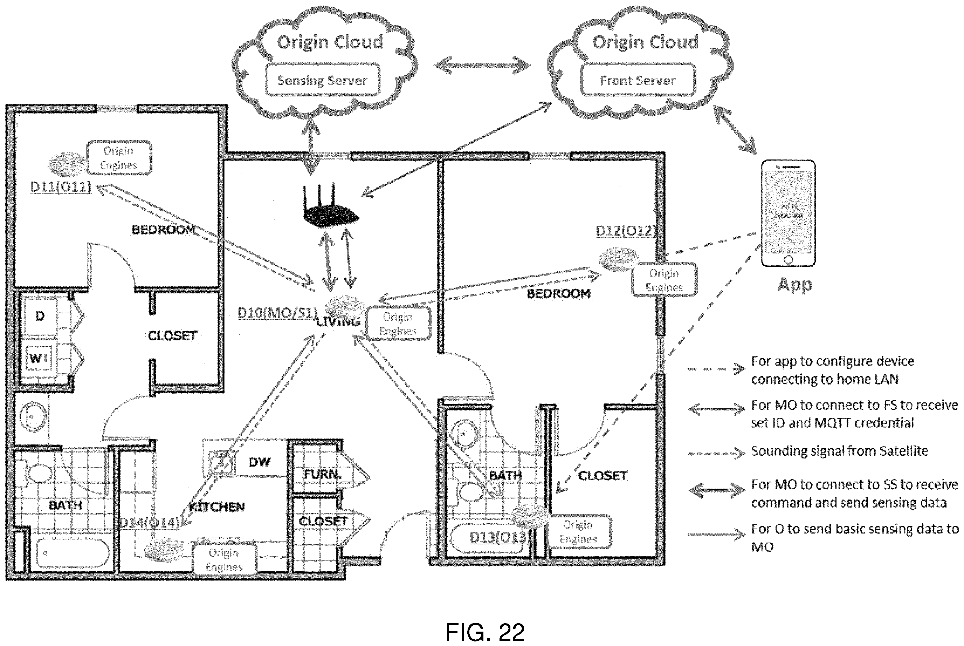

While FIG. 21 illustrates a per-room deployment for wireless sensing, FIG. 22 illustrates a whole-home deployment for wireless sensing. The per-room deployment may be suitable to sense fall detection, automation, and/or activity of daily living (ADL) on a per-room basis. The whole-home deployment may be suitable to sense home security and/or ADL on a whole-home basis. In some embodiments, a same system can support both deployment manners.

In some embodiments, all devices for wireless sensing are provisioned to a front server (FS) with UN/PW (password). One can extend FS or create an AS to support this development. All devices may be in softAP mode with known SSID/PW. As shown in FIG. 21, the set of devices at home includes: {D10, D11, D12, D20, D21, D22, D30, D31, D40, D41}.

During an onboarding process, an app can be used to scan QR codes and register devices to FS under a user account. FS then generates a set ID for the user and creates a sounder.conf file that contains the set ID. When a set is created in FS, FS can generate a default sounder.conf. It is similar to ABC or DEF with AS, where AS generates a set ID and creates dummy sounder.conf with the set ID. The file may be sent to a master Origin (MO) when it connects to AS.

In some embodiments, the app obtains the device credentials from FS. The app connects to the devices (using the device SSID/PW and credentials), provides them the home router SSID and PW for them to connect to the home router as clients. The app can select one device to be MO, e.g. D10. The app may connect to the device to: assign its role to MO, give MO the FS URL, and give MO credentials of other devices in MO network. MO may need credentials to connect to other devices. In some embodiments, the app connects to MO through home LAN.

In some embodiments, using its credential, MO connects to FS to receive: the sounder.conf file with the generated set ID (a.k.a. MO device ID); and sensing server (SS) MQTT credential and URL. A set ID generated by FS may allow changing/replacing MO device without losing sensing settings.

MO can connect to SS using the set ID and MQTT credential. The app may select devices for groups as follows {{D10, D11, D12}; {D20, D21, D22}; {D30, D31}; {D40, D41}} based on room setting. In some embodiments, the grouping can also apply to whole home setting, which includes a single group as shown in FIG. 22.

In some embodiments, the app sends to MO through SS the List-3 in groups, ex {MO ID, {D10, D11, D12}; {D20, D21, D22}; {D30, D31}; {D40, D41} }. For the scenario shown in FIG. 21, cloud and user do not know which one is Satellite or Origins in each group. They only know if these devices belong to which group. For the scenario shown in FIG. 22, the Satellite is in a specific location (e.g. middle of the house) to ensure motion localization performance. So the API should allow app to specify Satellite as well. Specifying Satellite devices could be an option.

In some embodiments, List-3 API will be modified to accommodate grouping. In some embodiments, format is in array and can be updated.

If Satellite is not specified, MO can compare the multicast list. The most recent multicast may be taken as the Satellite for that group. The other ones are the Origins in the group regardless if it is on or not. In this way, there is a device providing sounding.

MO may then build a sounder.conf and save it to FS. The system can modify FS or create an AS for this development. If an MO device is changed, the new MO device can go to FS to get the sounder.conf to restore the services. MO can return to SS the MO network. As such, the app can start the service.

In some embodiments, when receiving a start service command, MO already knows a device in MO network should be Satellite or Origin based on the onboarding. A Satellite can be chosen by MO or by user/app specification. Other devices are Origins.

For a device to be Origin, MO uses the device credential to connect to the Origin. MO provides the Origin the following: the ID and credential of the Satellite associated with the Origin; MO address for Origin to send basic engine output back; and the basic engine types for Origin to run. Origin will use the Satellite credential to connect to the Satellite.

Origin can look up in its multicast table to find the Satellite and its MAC address. Origin may use the Satellite credential to connect to the Satellite. Origin then sends the Satellite the start sounding command.

In some embodiments, Satellite starts the sounding signal if it has not started yet. After that, Origin runs the basic engines that MO requested. Origins send back to MO the basic engine outputs. MO runs fusion engine. MO then sends the fusion engine output to SS.

In some embodiments, when a user stops a service, the app sends MO the stop command. MO sends Origin a stop engine command. Origin stops its associated basic engines. If Origin has no other basic engine to run, Origin then sends the Satellite the stop sounding command.

For example, home security and sleep monitoring are running. Therefore, Origin needs to run motion and breathing. Then a user stops sleep monitoring. MO sends Origin stop breathing engine command. Because Origin still runs motion engine, Origin should not stop Satellite from sounding. In some embodiments, Satellite stops sounding if no other Origins need its sounding signal.

In some embodiments, during operation, MO may be offline or powered down. When resumed online, MO connects to FS to get: Sounder.conf file; SS MQTT credential and URL. MO may then connect to SS to receive service restoration information. If the service was running before MO was offline, MO starts the service. If the service was not running, MO waits to receive start service command.

In some embodiments, during operation, the user may add a device to the sensing network (assuming the device is not MO). Before proceeding adding/removing devices, the app should stop engines on MO. Adding a device is similar to onboarding a device. The device first needs to be provisioned to FS. As a neutral device, the device is in softAP mode with known SSID/PW. Before proceeding, app stops engines on MO. App scans QR codes and registers the device to FS under a user account. App obtains the device credential from FS. App connects to the device (using the device SSID/PW and credential), provides it the home router SSID and PW for it to connect to the home router as a client. App sends MO the new List-3 that includes the new device. MO then builds a sounder.conf and saves it to FS. MO returns to SS the MO network. App then can restart the service.

In some embodiments, during operation, the user may remove a device from the sensing network (assuming the device is not MO). Before proceeding adding/removing devices, app should stop the engines on MO. App stops the engines. App sends MO the new List-3 that excludes the removed device. MO then builds a sounder.conf and saves it to FS. MO returns to SS the MO network. App then can restart the service. After removal, the removed device is still in client mode to home router. Factory reset will neutralize the device. After factory reset, the device is in softAP mode with known SSID/PW, waiting to be configured to connect to home router.

In some embodiments, during operation, the user may replace MO. MO device may not be functional, or user wants to assign another device as MO. App attempts to stop engines on MO. If successful, MO then stops all engines on Origins, and Origins stop Satellite from sending sounding signal. App can deregister MO from user account in FS. Then MO is no longer able to connect to FS unless it gets registered again. FS revolts MQTT credential that MO uses. MO is no longer able to connect to SS. App selects another device to be MO. If the new MO is a neutral device, app connects to the device and makes it connect to home router.

App then connects to the device to: assign its role to MO; give MO the FS URL; and give MO credentials of other devices in MO network. MO may need credentials to connect to other devices. App connects to MO through home LAN. Using its credential, MO connects to FS to receive: the sounder.conf file with the existing set ID (a.k.a MO device ID); and SS MQTT credential and URL. A set ID generated by FS allows changing/replacing MO device without losing sensing settings. MO connects to SS using the set ID and MQTT credential. MO receives restoration information from SS to resume the service. The old MO can be neutralized through factory reset.

In some embodiments, any device (MO, Origin, or Satellite) can be neutralized through factory reset. When neutralized, the device is in softAP mode with known SSID/PW, waiting to be configured to connect to home router.

In some embodiments, any device in the WiFi sensing network can be Satellite. A Satellite device can be chosen by MO or by user/app/cloud. MO can select a device to be Satellite by comparing the multicast list. MO takes the most recent multicast in a group as the Satellite for that group. Satellite keeps track of the start request from Origins. When an Origin requests sounding, if it is not in Satellite list, Satellite will add the Origin into its list. If an Origin is already in Satellite list requests sounding again, Satellite does not add the Origin into the list. In other words, Satellite list does not include duplicated Origins.

Periodically, Origin can tell Satellite to keep sending sounding signal if the Origin still runs its basic engines. If Satellite does not receive the keep going from an Origin, Satellite will remove the Origin from its list. This is to prevent Satellite from keeping sounding when engine is already stopped but Origin could not have a change to stop Satellite from sounding. An example is when an Origin is dead.

When an Origin stops all of its basic engines, the Origin will send the stop sounding to the Satellite. Satellite then removes the Origin from its list. When the list is empty, Satellite will stop sending out sounding signal. In some embodiments, Satellite does not maintain the list of Origins in persistent storage. When power-cycled, the list of Origin is empty, and thus Satellite does not send out sounding signal. If an Origin is running on the CSI from the Satellite, no CSI is received. Origin then sends the Satellite the request for start sounding. When Satellite is powered on and receives the start sounding, it resumes sending out the sounding signal.

In some embodiments, Origin is a device selected by MO. It is to provide basic engine data to MO to run fusion engines. MO can run multiple fusion engines.

MO should keep track which engine an Origin should run and tell the Origin to stop when no fusion engine requests the basic engine data. For example, soft security and ADL are running, where both requires basic motion engine. When stop Soft Security, MO should not send stop motion engine command to Origin because the ADL fusion engine still needs motion data from the Origin. In some embodiments, Origin does not keep track of the number of fusion engine consuming its output as in Linux system.

Periodically, MO sends Origin the start command for the needed basic engine. If Origin does not receive the start command from MO for a basic engine, Origin will stop the basic engine and thus possibly stops Satellite from sending sounding signal. This is to prevent Origin from running basic engines and Satellite from sending sounding signal forever when MO is dead.

In some embodiments, sample code for wireless sensing may include codes to: announce engine capabilities; provide connection among different devices in the sensing network; act in different roles (MO, Satellite, Origin) when assigned; provide connection to cloud; generate sounding signal and capture and parse CSI; send and receive control, command, and sensing data/detection; run engines in library, etc. In some embodiments, the library for wireless sensing may include core algorithms in basic engines and fusion engines, and produce sensing data and detection.

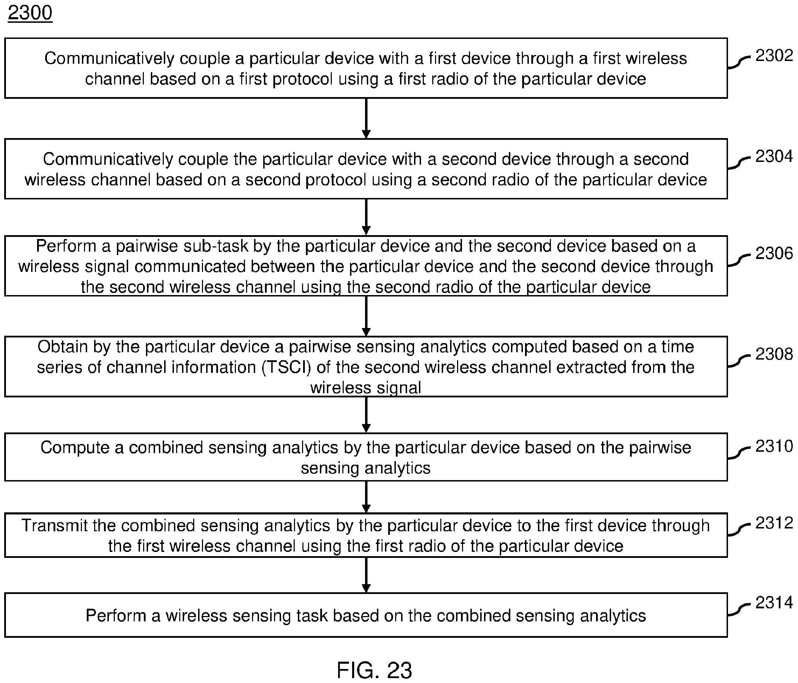

FIG. 23 illustrates a flow chart of an exemplary method 2300 for performing wireless sensing in a venue with multiple groups of devices, according to some embodiments of the present disclosure. In various embodiments, the method 2300 can be performed by the systems disclosed above. At operation 2302, a particular device is communicatively coupled with a first device through a first wireless channel based on a first protocol using a first radio of the particular device. At operation 2304, the particular device is communicatively coupled with a second device through a second wireless channel based on a second protocol using a second radio of the particular device. In some embodiments, the first device, the second device, and the particular device are in a same wireless sensing system in a venue.

At operation 2306, a pairwise sub-task is performed by the particular device and the second device based on a wireless signal communicated between the particular device and the second device through the second wireless channel using the second radio of the particular device. At operation 2308, the particular device obtains a pairwise sensing analytics computed based on a time series of channel information (TSCI) of the second wireless channel extracted from the wireless signal. Each channel information (CI) may comprise at least one of: channel state information (CSI), channel impulse response (CIR) or channel frequency response (CFR). At operation 2310, a combined sensing analytics is computed by the particular device based on the pairwise sensing analytics. At operation 2312, the combined sensing analytics is transmitted by the particular device to the first device through the first wireless channel using the first radio of the particular device. At operation 2314, a wireless sensing task is performed based on the combined sensing analytics. The order of the operations in FIG. 23 may be changed according to various embodiments of the present teaching.

The following numbered clauses provide examples for wireless sensing with multiple groups of wireless devices.

Clause F1. A method/device/system/software of a wireless sensing system, comprising: performing a wireless sensing task by a set of heterogeneous wireless devices in a venue, wherein a second particular heterogeneous wireless device comprises a first radio and a second radio; coupling communicatively the second particular device with a first heterogeneous wireless device of the set through a first wireless channel based on a first protocol using the first radio of the second particular device; coupling communicatively the second particular device with a second heterogeneous wireless device of the set through a second wireless channel based on a second protocol using the second radio of the second particular device; performing a pairwise subtask of the wireless sensing task by the second particular device and the second device based on a wireless signal communicated between the second particular device and the second device through the second channel using the second radio of the second particular device; obtaining by the second particular device a pairwise sensing analytics computed based on a time series of channel information (TSCI) of the second wireless channel extracted from the received wireless signal, wherein each channel information (CI) comprises at least one of: channel state information (CSI), channel impulse response (CIR) or channel frequency response (CFR); computing a second combined sensing analytics by the second particular device based on the pairwise sensing analytics; transmitting the second combined sensing analytics by the second particular device to the first device through the first wireless channel using the first radio of the second particular device; performing the wireless sensing task based on the second combined sensing analytics.

In some embodiments, first radio and second radio may be same/different. First protocol and second protocol may be same/different. They may have same/different carrier frequency (e.g. 2.4GHz/5GHz/6GHz/28GHz/60GHz), frequency channel/band (e.g. band 7 Vs band 23), bandwidth (e.g. 20/40/80/160/320 MHz), protocol (e.g. WiFi, IEEE 802.11n/ac/ax/ay/az/be/bf, 802.15.3/4UWB, Bluetooth, BLE, Zigbee, WiMax, 4G/LTE/5G/6G/7G/8G), protocol settings/parameters, modulation (ASK, PSK, QAM 16/64/256/1024/4096), signaling, etc.

Clause F2. The method/device/system/software of the wireless sensing system of clause F1, comprising: wherein one of the first radio, the first wireless channel, and the first protocol differs from a corresponding one of the second radio, the second wireless channel, and the second protocol in at least one of: carrier frequencies, frequency channel, frequency band, bandwidths, modulation, beamforming, standard protocol, protocol setting, or signaling scheme.

In some embodiments, carrier frequency of one may be higher than the other one, even if they both have the same protocol (e.g. both are WiFi with one being 2.4GHz and the other SGHz, or 2.4GHz/6GHz, or SGHz/6GHz; e.g. the second particular device may have dual-band, tri-band or quad-band WiFi). First radio may be 2.4GHz WiFi. Second radio may be 5GHz or 6MHz WiFi. Bandwidth of second radio (e.g. 20/40/80/160/320MHz) may/may not be higher than bandwidth of first radio (e.g. 20MHz).

Clause F3. The method/device/system/software of the wireless sensing system of clause F2, comprising: wherein a carrier frequency of the second radio is higher than that the first radio.

Clause F4. The method/device/system/software of the wireless sensing system of clause F3, comprising: wherein a carrier frequency of the second radio is higher than SGHz; wherein a carrier frequency of the first radio is less than 4GHz.

In some embodiments, the set of devices may form a network of networks. The network of networks may have two tiers. Some device may be in a Tier-1 network, a top-level network (e.g. both second particular device and first device are in Tier-1 network). Some may be in a Tier-2 network, a level lower than top level (e.g. second particular device and second device are in a Tier-2 network). A device may be in both the Tier-1 network and the Tier-2 network (e.g. second particular device). (A device may be in more than one Tier-2 networks.) Each network (Tier-1 or Tier-2, or Tier-K) may be used to perform wireless sensing (e.g. all or some devices in the current network may collaborate as a group to do pairwise (wireless sensing) subtask; each pair of devices may transmit/receive wireless signal between themselves; TSCI may be obtained from each received wireless signal; pairwise analytics may be computed based on each TSCI; pairwise analytics may be sent to a selected device (in current network) that perform fusion to compute a combined analytics based on all pairwise analytics and any combined analytics received from lower level networks linked to the current network; combined analytics may be sent to upper level network linked to current network). Each Tier-2 network may form a local wireless sensing subsystem. The Tier-1 network may form a main wireless sensing system.

A Tier-2 network may be linked/connected to a Tier-1 network by having one or more devices in the Tier-2 network to function as gateway devices between the two networks. Each gateway device (e.g. the second particular device) may have at least two radios and may use them both to connect/associate with the two networks (e.g. the second particular device may uses first radio to join the Tier-1 network and second radio to join the Tier-2 network). More than one Tier-2 networks may be linked/connected to the same Tier-1 network. The Tier-1 network (e.g. its AP) may have access to internet, some external network or some cloud server. The Tier-2 networks may access the internet, the external network or the cloud server via the Tier-1 network (and the corresponding gateway devices). A network-of-networks may have more than 2 tiers. A (or more than one) Tier-K network may be linked/connected to a Tier-(K-1) network (e.g. recursively) and may be communicatively coupled with upper level networks (i.e. Tier-(K-2) network, Tier-(K-3) networks, .. , Tier-2 networks, Tier-1 network), internet, the external network and the cloud server via the Tier-(K-1) network. Recursively, a Tier-(K-1) network may link/connect one or more Tier-K networks. A Tier-(K-1) network may be associated with a zone/region/area (e.g. Tier-1 network may be associated with the whole venue), for any K>1. Each Tier-K network may be associated with a sub-zone/sub-region/sub-area (e.g. Tier-2 networks may be associated with living room, dining room, family room, kitchen, entrance, exit, garage, basement, bedroom1, bedroom 2, first floor, second floor, or some combination/grouping) of the zone/region/area associated with the Tier-(K-1) network. Two sub-zones/sub-regions/sub-areas may/may not overlap. The zone/region/area may comprise a union of more than sub-zones/sub-regions/sub-areas. A portion of the zone/region/area may not belong to any sub-zone/sub-region/sub-area.

For example, Tier-1 may be associated with the whole venue which may be a 2-floor house. A first Tier-2 network may be associated with the whole first floor (a first sub-zone of venue) and a second Tier-2 network may be associated with the whole second floor (a second sub-zone of venue). A first Tier-3 network, under the first Tier-2 network, may be associated with the kitchen (a first sub-sub-zone of first sub-zone) in first floor. A second Tier-3 network, under the first Tier-2 network, may be associated with the living room (a second sub-sub-zone of first sub-zone) in first floor. A third Tier-3 network (under first Tier-2 network) may be associated with the dining room (a third sub-sub-zone of first sub-zone) in the first floor. A fourth Tier-3 network (under second Tier-2 network) may be associated with a first bedroom (a first sub-sub-zone of second sub-zone) in the second floor. A fifth Tier-3 network (under second Tier-2 network) may be associated with a second bedroom (a second sub-sub-zone of second sub-zone) in the second floor. The entrance/foyer area of first floor may be a portion of first sub-zone that is not in any sub-sub-zone of first sub-zone.

Devices in a Tier-K network (called "Tier-K devices") may be used in pairs to perform pairwise wireless sensing in the associated sub-zone/sub-region/sub-area, and corresponding TSCI in Tier-K (called "Tier-K TSCI") may be obtained. Pairwise sensing analytics for Tier-K (called "Tier-K pairwise analytics") may be computed based on the corresponding Tier-K TSCI. A Tier-K combined analytics may be computed based on the Tier-K pairwise analytics, the Tier-K TSCI and any Tier-(K+1) combined analytics obtained from any Tier-(K+1) networks linked/connected to the Tier-K network. The Tier-K combined analytics may be transmitted to the linked/connected Tier-(K-1) network via respective gateway device(s) between the Tier-K and the Tier-(K-1) networks. And this may be recursively performed for all K. In some examples, two or more devices in kitchen network (Tier-3 network, first sub-sub-zone of first sub-zone) in first floor (first sub-zone) may be used to perform pairwise wireless sensing to generate Tier-3 TSCI in the kitchen. Two or more devices in living-room network (Tier-3 network, second sub-sub-zone of first sub-zone) in first floor may be used to perform pairwise wireless sensing in the living room to generate Tier-3 TSCI in living room. Two or more devices in dining-room network (Tier-3 network, third sub-sub-zone of first sub-zone) in first floor may be used to perform pairwise wireless sensing in the dining room to generate Tier-3 TSCI in living room.

For each of the three sub-sub-zones of first sub-zone (say, the kitchen area), one or more Tier-3 pairwise analytics of/for/corresponding to the sub-sub-zone (e.g. kitchen) may be computed based on respective Tier-3 TSCI of the sub-sub-zone (e.g. kitchen or kitchen network). A Tier-3 combined analytics for the sub-sub-zone (e.g. kitchen) may be computed based on all the Tier-3 pairwise analytics and Tier-3 TSCI of the sub-sub-zone (e.g. kitchen area) (e.g. by a respective fusion algorithm). The three Tier-3 combined analytics of the three sub-sub-zones of the first sub-zone may be transmitted from the respective Tier-3 network (kitchen network, living-room network or dining-room network) to a first-floor network (Tier-2 network, first sub-zone) via the respective gateway device. In the first sub-zone (first floor), two or more devices in first-floor network (e.g. in entrance/foyer area, kitchen area, living room area, dining room area of first floor) may be used to perform pairwise wireless sensing to generate Tier-2 TSCI in the first floor. One or more Tier-2 pairwise analytics of the first floor may be computed based on respective Tier-2 TSCI of the first floor. A Tier-2 combined analytics for the first floor may be computed based on all the Tier-2 pairwise analytics and Tier-2 TSCI of the first floor and all the Tier-3 combined analytics from the three Tier-3 networks (e.g. by some respective fusion algorithm).

Different Tier-K network may have different radio, wireless channels, or protocols. E.g. One Tier-K network may be a 5GHz WiFi network; a second Tier-K may be a 6GHz WiFi. For example, a first Tier-2 network may be a first WiFi (e.g. 5GHz), a secondTier-2 network may be a second WiFi (e.g. 6Hz), a third Tier-2 network may be UWB, a fourth Tier-2 network may be Bluetooth, a fifth Tier-2 network may use millimeter wave (mmWave, e.g. 28GHz, 60GHz, 70+GHz). The first WiFi may use 11az for sensing while second WiFi may use 11bf for sensing. The first/second WiFi may have same/different carrier frequency, frequency channel/band, bandwidth, protocol, etc.

Tier-K network may have one or more access point (AP). The second particular device and/or second device may be AP. The wireless signal may be communicated in a trigger-based (TB) manner or non-TB manner. In TB sensing, a train (time series) of sounding signals (e.g. null-data-packet (NDP) may be sent from a first Tier-K device (e.g. AP, non-AP, and/or Type1 heterogeneous wireless device) to other Tier-K device(s) (e.g. non-AP, or another AP in mesh network) in the Tier-K network using uni-cast, multi-cast, and/or broadcast. A non-AP (or another AP) Tier-K device may transmit or receive a sounding signal in response to a trigger signal from a Tier-K AP device (e.g. transmit in response to a trigger frame (TF) in TF sounding, or receive in response to a NDP announcement frame (NDPA) in NDPA sounding). In peer-to-peer sounding, a non-AP Tier-K device may transmit a sounding signal to another non-AP Tier-K device. In non-TB sensing, a train of sounding signals (e.g. NDP) may be sent from a Tier-K AP device to a Tier-K non-AP (or another Tier-K AP) device, each sounding signal accompanied by another sounding signal sent in reverse direction, i.e.

from non-AP to AP. In some embodiments, the first radio/wireless channel/protocol may be for Tier-(K-1) network (called "first wireless network"), for any K>1, such as 2, 3, 4, ... The second radio/wireless channel/protocol may be for Tier-K network (called "second wireless network").

Clause F5. The method/device/system/software of the wireless sensing system of any previous F clause, comprising: performing the wireless sensing task by a first subset of the set of heterogeneous wireless devices using a first wireless network in the venue, wherein the first subset comprises the second particular device and the first device; performing the wireless sensing task by a second subset of the set of heterogeneous wireless devices using a second wireless network in the venue, wherein the second subset comprises the second particular device and the second device; wherein the first radio, the first wireless channel, and the first protocol are associated with the first wireless network; wherein the second radio, the second wireless channel, and the second protocol are associated with the second wireless network; wherein one of the first protocol or the second protocol comprises at least one of: a WiFi standard, a UWB standard, a WiMax standard, an IEEE standard, an IEEE 802 standard, an IEEE 802.11 standard, an IEEE 802.11bf standard, an 802.15 standard, an 802.15.4 standard, and an 802.16 standard.

Clause F6. The method/device/system/software of the wireless sensing system of clause F5, comprising: wherein the second particular device and first device are authenticated and associated in the first wireless network; wherein the second particular device and second device are authenticated and associated in the second wireless network.

In Case 1: wireless signal received by second particular device. Pairwise analytics computed by second particular device. In Case 1a: Non-TB sensing (TB="trigger based"). No trigger signal may be needed.

Clause F7. The method/device/system/software of the wireless sensing system of clause F5 or F6, comprising: transmitting the wireless signal from the second device to the second particular device based on the second protocol; extracting the TSCI from the received wireless signal by the second particular device; computing the pairwise sensing analytics based on the TSCI by the second particular device.

In Case 1: Trigger based sensing. AP may sent trigger signal to non-AP before sounding signal (e.g. NDP) being received by non-AP (e.g. triggered by NDPA), or transmitted by non-AP (e.g. triggered by TF). In Case 1b: TB sensing with TF sounding. AP may be second particular device. Trigger signal may be TF. Alternatively, AP may be a third device. Both second particular device and second device may be peer devices. This may be peer-to-peer sensing. Transmission of wireless signal may be triggered.

Clause F8. The method/device/system/software of the wireless sensing system of clause F7, comprising: transmitting the wireless signal based on a trigger signal received by the second device from an access point device (AP) of the second wireless network, based on the second protocol.

Clause F9. The method/device/system/software of the wireless sensing system of clause F8, comprising: wherein the second particular device is the AP of the second wireless network.

In Case 1c: TB sensing with NDPA sounding. AP may be second device. Trigger signal may be NDPA. Alternatively, AP may be a third device. Both second particular device and second device may be peer devices. This may be peer-to-peer sensing. Reception of wireless signal may be triggered.

Clause F10. The method/device/system/software of the wireless sensing system of clause F7-F9, comprising: receiving the wireless signal by the second particular device based on a trigger signal received by the second particular device from an access point device (AP) of the second wireless network, based on the second protocol.

Clause F11. The method/device/system/software of the wireless sensing system of clause F10, comprising: wherein the second device is the AP of the second wireless network.

In Case 2: Wireless signal received by second device. TSCI extracted in second device. Similar to Case 1, this may be non-TB sensing, TB-sensing with NDPA sounding or TB-sensing with TF sounding. In Case 2a: Pairwise analytics computed by second device and reported to second particular device.

Clause F12. The method/device/system/software of the wireless sensing system of clause F5-F11, comprising: transmitting the wireless signal from the second particular device to the second device based on the second protocol; extracting the TSCI from the received wireless signal by the second device; computing the pairwise sensing analytics based on the TSCI by the second device; transmitting the pairwise sensing analytics from the second device to the second particular device.

In Case 2b: TSCI reported to second particular device. Pairwise analytics computed by second particular device.

Clause F13. The method/device/system/software of the wireless sensing system of clause F5-F12, comprising: transmitting the wireless signal from the second particular device to the second device based on the second protocol; extracting the TSCI from the received wireless signal by the second device; transmitting the TSCI from the second device to the second particular device; computing the pairwise sensing analytics based on the TSCI by the second particular device.

In some embodiments, there may be other devices in Tier-K network. Two devices in the Tier-K network may perform a similar pairwise sensing sub-task, by communicating another wireless signal using the Tier-K network/second protocol/second wireless channel, extracting another TSCI from the received another wireless signal, obtaining another pairwise analytics (computed based on the another TSCI) by the second particular device, and computing the combined analytics based on both the pairwise analytics and the another pairwise analytics. Tier-K combined analytics may be computed based further on the TSCI and another TSCI. The Tier-K combined analytics may comprise the pairwise analytics, the another pairwise analytics, the TSCI and/or the another TSCI. The third device may/may not be the second particular device or the second device. The fourth device may/may not be the second particular device or the second device. Combined sensing analytics may be computed in/for the second network based on an aggregation of the pairwise sensing analytics and the second pairwise sensing analytics (and any additional pairwise analytics generated in the second network).

Clause F14. The method/device/system/software of the wireless sensing system of clause F5-F13, comprising: performing a second pairwise sub-task of the wireless sensing task by a third heterogeneous wireless device in the second subset and a fourth heterogeneous wireless device in the second subset based on a second wireless signal transmitted from the third device to the fourth device through the second wireless channel in the second wireless network, wherein the two devices of the set of heterogeneous wireless devices are associated with the second wireless network; obtaining a second TSCI of the second wireless channel by the fourth device based on the received second wireless signal; obtaining by the second particular device a second pairwise sensing analytics computed based on the second TSCI; computing the second combined sensing analytics by the second particular device further based on the second pairwise sensing analytics.

In some embodiments, second pairwise sensing analytics computed by fourth device, then sent to second particular device (e.g. using the second wireless network/Tier-K network, or using the first wireless network/Tier-(K-1) network).

Clause F15. The method/device/system/software of the wireless sensing system of clause F14, comprising: computing the second pairwise sensing analytics by the fourth device based on the second TSCI; transmitting the second pairwise sensing analytics by the fourth device to the second particular device.

In some embodiments, second pairwise sensing analytics computed by second particular device based on second TSCI transmitted from fourth device to second particular device (e.g. using the second wireless network/Tier-K network, or using the first wireless network/Tier-(K-1) network).

Clause F16. The method/device/system/software of the wireless sensing system of clause F14 or F15, comprising: transmitting the second TSCI by the fourth device to the second particular device; computing the second pairwise sensing analytics by the second particular device based on the second TSCI.

In some embodiments, Tier-K combined sensing analytics may be computed based on combined analytics from Tier-(K+1) network, called "third wireless network".

Clause F17. The method/device/system/software of the wireless sensing system of clause F14-F16, comprising: obtaining a third combined sensing analytics associated with a third wireless network by the second particular device; computing the second combined sensing analytics by the second particular device further based on the third combined sensing analytics.

Clause F18. The method/device/system/software of the wireless sensing system of clause F17, comprising: performing the wireless sensing task further by a third subset of the set of heterogeneous wireless devices using the third wireless network in the venue; performing a third pairwise sub-task of the wireless sensing task by a fifth heterogeneous wireless device in the third subset and a sixth heterogeneous wireless device in the third subset based on a third wireless signal transmitted from the fifth device to the sixth device through a third wireless channel in the third wireless network, wherein the two devices of the third subset are associated with the third wireless network; obtaining a third TSCI of the third wireless channel by the sixth device based on the received third wireless signal; obtaining by a third particular heterogeneous wireless device in the third subset a third pairwise sensing analytics computed based on the third TSCI; computing the third combined sensing analytics by the third particular device based on the third pairwise sensing analytics; obtaining by the particular device the third combined sensing analytics from the third particular device.

Clause F19. The method/device/system/software of the wireless sensing system of clause F18, comprising: obtaining a fourth combined sensing analytics associated with a fourth wireless network by the particular device; computing the second combined sensing analytics by the particular device further based on the fourth combined sensing analytics.

Clause F20. The method/device/system/software of the wireless sensing system of clause F19, comprising: performing the wireless sensing task further by a fourth subset of the set of heterogeneous wireless devices using the fourth wireless network in the venue; performing a fourth pairwise sub-task of the wireless sensing task by a seventh heterogeneous wireless device in the fourth subset and a eighth heterogeneous wireless device in the fourth subset based on a fourth wireless signal transmitted from the seventh device to the eighth device through a fourth wireless channel in the fourth wireless network, wherein the two devices of the fourth subset are associated with the fourth wireless network; obtaining a fourth TSCI of the fourth wireless channel by the eighth device based on the received fourth wireless signal; obtaining by a fourth particular heterogeneous wireless device in the fourth subset a fourth pairwise sensing analytics computed based on the fourth TSCI; computing the fourth combined sensing analytics by the fourth particular device based on the fourth pairwise sensing analytics; obtaining by the particular device the fourth combined sensing analytics from the fourth particular device.

In some embodiments, there may be another Tier-K network, in parallel to the Tier-K network (second wireless network) described in clause F1/5, doing similar things. It may be associated with Tier-(K-1) network (first wireless network and may perform wireless sensing (sending wireless signals, obtaining TSCI, computing pairwise analytics, then computing combined analytics) and send its combined analytics to first device in Tier-(K-1) network.

Clause F21. The method/device/system/software of the wireless sensing system of clause F20, comprising: performing the wireless sensing task further by a fifth subset of the set of heterogeneous wireless devices using a fifth wireless network in the venue; performing a fifth pairwise sub-task of the wireless sensing task by a ninth heterogeneous wireless device in the fifth subset and a tenth heterogeneous wireless device in the fifth subset based on a fifth wireless signal transmitted from the ninth device to the tenth device through a fifth wireless channel in the fifth wireless network, wherein the two devices in the fifth subset are associated with the fifth wireless network; obtaining a fifth TSCI of the fifth wireless channel by the tenth device based on the received fifth wireless signal; obtaining by a fifth particular heterogeneous wireless device in the fifth subset a fifth pairwise sensing analytics computed based on the fifth TSCI; computing a fifth combined sensing analytics by the fifth particular device based on the fifth pairwise sensing analytics; obtaining the fifth combined sensing analytics by the first device from the fifth particular device; performing the wireless sensing task based on the fifth combined sensing analytics.

In some embodiments, Fifth subset=first subset. Fifth particular device=first device. First device may be in two (or more) networks: the first wireless network (e.g. 2.4GHz) and the fifth wireless network (e.g. SGHz). The fifth wireless network may be for performing sensing measurement (sensing measurement setup, polling, sending wireless sounding signal/trigger signal, reporting raw sensing measurements/TSCI, sensing measurement termination). The first network may be for communicating any of: user/system wireless sensing parameters/setup/control, computed results based on raw measurements, pairwise analytics/combined analytics, etc.

Clause F22. The method/device/system/software of the wireless sensing system of clause F21, comprising: wherein the fifth subset is the first subset; wherein the fifth particular device is the first device.

Clause F23. The method/device/system/software of the wireless sensing system of clause F22, comprising: performing the wireless sensing task by the set of heterogeneous wireless devices using more than one wireless networks in the venue, with at least two of the heterogeneous wireless devices in each of the wireless networks in the venue; configuring the more than one wireless networks to have a multi-tier structure, comprising at least two tiers, wherein the first wireless network is a Tier-1 network comprising at least the second particular device, and the first device, wherein the second wireless network is a Tier-2 network comprising at least the second particular device, the second device, the third device and the fourth device, wherein the second particular device being in both the first and the second networks serves as a gateway device between the two networks such that sensing results can be transmitted from the second particular device via the first wireless network to the first device; configuring the heterogeneous wireless devices in a Tier-K network to report sensing results obtained in the Tier-K network to a heterogeneous wireless device in a Tier-(K-1) network via a gateway device between the two networks, wherein K is an integer greater than one, wherein sensing results comprise at least one of: combined sensing analytics, pairwise sensing analytics and TSCI.

Clause F24. The method/device/system/software of the wireless sensing system of clause F23, comprising: wherein the first wireless network further comprises the third particular device and the fourth particular device; wherein the third wireless network is a Tier-3 network, comprising at least the third particular device, the fifth device, and the sixth device, wherein the third particular device is a gateway device; wherein the fourth wireless network is a Tier-3 network, comprising at least the fourth particular device, the seventh device, and the eighth device, wherein the fourth particular device is a gateway device; wherein sensing results obtained in the third wireless network are transmitted from the third particular device via the first wireless network to the second particular device; wherein sensing results obtained in the fourth wireless network are transmitted from the fourth particular device via the first wireless network to the second particular device.

Clause F25. The method/device/system/software of the wireless sensing system of clause F24, comprising: wherein the first wireless network further comprises the fifth particular device; wherein the fifth wireless network is a Tier-2 network comprising the fifth particular device, the ninth device, and the tenth device, wherein the fifth particular device is a gateway device; wherein sensing results are transmitted from the fifth particular device via the first wireless network to the first device.

Clause F26. The method/device/system/software of the wireless sensing system of clause F25, comprising: associating the first wireless network with a zone of the venue; associating the second wireless network with a first sub-zone of the zone; associating the third wireless network with a first sub-sub-zone of the first sub-zone; associating the fourth wireless network with a second sub-sub-zone of the first sub-zone.

Clause F27. The method/device/system/software of the wireless sensing system of clause F26, comprising: performing the wireless sensing task for the zone based on at least one of: any pairwise sensing analytics associated with the first wireless network; the combined sensing analytics associated with the second wireless network, or a fourth combined sensing analytics associated with a fifth network in the venue; performing the wireless sensing task for the first sub-zone based on at least one of: the combined sensing analytics associated with the second wireless network, any pair-wise sensing analytics associated with the second wireless network, the second combined sensing analytics associated with the third wireless network, or the third combined sensing analytics associated with the fourth wireless network; associating the third wireless network with a first subzone of the zone; performing a wireless sensing subtask associated with the first subzone based on the third wireless network and the another combined sensing analytics, associating the fourth wireless network with a second subzone of the zone.

In some embodiments, the present teaching discloses systems and methods for a display of WLAN sensing motion statistics(MS)/motion information(MI)/analytics/spatial-temporal-information (STI) associated with more than one "regions" or "zones" in a venue.

In some embodiments, the regions or zones may be established and named by a user. For example, the venue may be a room; a zone or region may be "living room", "dining room", "kitchen", "bedroom 1", "bedroom 2", ... , "rest room 1", "rest room 2", ... , "first floor", "second floor", "basement", "garage", "office 1", "office 2", etc. A region may be large, special or important s.t. more than one "sub-regions" of the region may be established and named by the user. For example, a living room may be a large region of a house s.t. the user established/named sub-regions such as "part of living room near kitchen", "part of living room near front door", "part of living room near back door", "part of living room facing the street", "part of living room near dining room", "part of living room around the TV", part of living room around the piano", etc.

There may be multiple Type1 (Tx) devices and Type2 (Rx) devices in the venue such that TSCI may be obtained for each region/zone and/or sub-region. For each region or sub-region, one or more MS/MI/STI/analytics may be computed, and a magnitude/characteristics of such may be displayed graphically using graphical user-interface (GUI).

In some embodiments, some related MS may be grouped together in the GUI display. Some MS associated with same or neighboring region/sub-region may be grouped together in GUI. Some MS of same kind may be grouped together. For example, some or all breathing statistics (or fall-down statistics, or presence statistics or sleep analytics) may be grouped together.

In some embodiments, some grouped MS may be displayed in a number of ways. They may be displayed in a consecutive/connected/linked/chained manner, forming a line/ring, or spanning/scanning (e.g. zigzag spanning, or raster scanning) of a connected area of the GUI. MS that are grouped together may be displayed in a physically close proximity manner (e.g. forming a cluster).

The MS may be displayed in a number of ways. A MS may be displayed by itself. For example, it may be displayed as a figure/ sliding figure/ graph/ plot/ chart/ bar/pie/ circle/ shape/ histogram/ histogram-like graph/animation. A magnitude of the MS and/or a function of the magnitude may be displayed/ represented/coded/encoded as a number/ numeral, a x-coordinate /y-coordinate of a figure/graph/ plot, a coordinate/ height/ length (e.g. of a bar or bar chart), an area (e.g. of a circle, rectangle, or shape), a volume (e.g. of a 3D animation), a size (e.g. of a circle, rectangle, bar, shape, graph, figure, chart), an animation, animation characteristics, duration, timing, flashing, pattern, shading, effect, color, and/or light/color intensity. The function of the magnitude may be different for different MS.

Some MS may be displayed together (e.g. overlaid, stacked, staggered, and/or concentric). Each MS may be displayed as a figure and the multiple figures may be overlaid, stacked and/or staggered. Each MS may be displayed as a concentric "ring" (e.g. circular or rectangular) around a center point. The rings may /may not overlap. Two MS may be displayed as a two-dimensional shape (e.g. ellipse/rectangle).

The magnitude of MS may be a magnitude/phase of a complex number, value/absolute value/sign of a real number, a norm of a vector (e.g. L_1/L_2/ ..., L_k/, .., L_infinity norm), a statistics, a mean/variance/correlation/covariance, and/or a thresholding/hard/soft thresholding. The function may be univariate, monotonic increasing, monotonic non-decreasing, piecewise linear, and/or an exponential / logarithmic / polynomial / trigonometric / hyperbola function, and/or a function of another function. The function may be a univariate function (e.g. as described) of a multivariate function (e.g. filtering, transform, moving average, weighted average, median/mean/mode, arithmetic/geometric/ hyperbolic mean, maximum/minimum, statistics, ordered statistics, variance, percentile, histogram, probability function, cumulative distribution function, count) of a sliding window of MS. The function may be a multivariate function of univariate function(s) of the sliding window of MS. The function may also be univariate function of multivariate function of univariate function(s) of the sliding window of MS.

Different MS measuring different characteristics of motion may have different functions. For example, a function of breathing MS may be different from a function of fall- down MS. A function of an MS may be determined based on online/offline learning, or training/ update using training / past data, and/or analysis of characteristics of motion.

Different MS measuring same characteristics of motion may have different functions. For example, breathing MS may have different functions for different regions/sub-regions. Alternatively, MS for some regions/sub-regions (e.g. some grouped MS) may be same, at least for a period of time. The function of a MS may be adaptively determined based on the MS (e.g. past MS or training MS from training data) and/or associated TSCI.

At least one statistics is wireless-sensing MS computed based on TSCI. Some statistics may be non-wireless-sensing statistics obtained in some ways. The statistics may be presented in some presentation device (e.g. displayed visually on a screen/monitor/ smart phone/tablet, generate visual signal/animation using lamp/light bulb/panel, play sound on a speaker, generate vibration/motion/action).

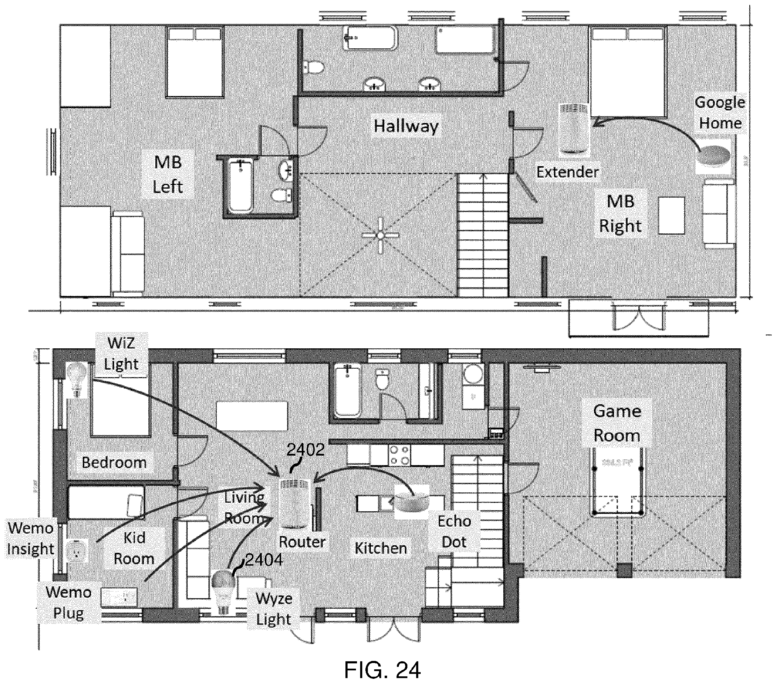

FIG. 24 illustrates an exemplary floor plan for wireless sensing to display sensing motion statistics and analytics, according to some embodiments of the present disclosure. In some embodiments, a location name is assigned to each wireless device, e.g. a router, an extender, etc., based on its location, e.g. bedroom, living room, kitchen, etc. Then, devices with a same location name can be grouped together. For example, a router 2402 and a Wyze light 2404 in FIG. 24 may be grouped together because they have the same location name: Living Room.

In some embodiments, the grouping can enable a system to have multiple IoT devices in the same room to cover big rooms. There may be overlapping sensing zones. In some embodiments, the system may use the wireless sensing and detection to gate a display of motion statistics, e.g. in form of live-view bubbles in a graphical user interface. The system may display sensing motion statistics and analytics that are not the same as the original output of the detection. For example, the system can apply an exponential curve to amplify detections with strong motion statistics and subpress detections with weak motion statistics.

The following numbered clauses provide examples for a wireless sensing presentation system.

Clause G1. A method/device/system/software of a wireless sensing presentation system, comprising: obtaining a plurality of time series of channel information (TSCI) of respective wireless multipath channels in a venue, wherein each TSCI is obtained based on a respective wireless signal transmitted from a respective Type1 heterogeneous wireless device to a respective Type2 heterogeneous wireless device through a respective wireless multipath channel in the venue, wherein the wireless multipath channels are impacted by a motion of an object in the venue; computing a plurality of time series of motion statistics (TSMS) associated with the motion of the object based on a processor, a memory and a set of instructions, each TSMS computed based on at least one respective TSCI; computing a mapping function for each TSMS based on a respective characteristics of the TSMS; processing each TSMS with the respective mapping function; presenting the plurality of processed TSMS on a presentation device of a user.

Clause G2. The method/device/system/software of the wireless sensing presentation system of Clause G1: wherein any mapping function comprises at least one of: a linear function, a piecewise-linear function, a non-linear function, a monotonic function, a piecewise monotonic function, a monotonic increasing function, a monotonic decreasing function, a monotonic non-increasing function, a monotonic non-decreasing function, a piecewise monotonic increasing function, a piecewise monotonic decreasing function, a polynomial function, an exponential function, a logarithmic function, a trigonometric function, a hyperbolic function, a sigmoid function, a thresholding function, an indicator function, an inverse function, a look-up table, a composite function, or a function of function.

The mapping function may be a univariate function of a MS, or a univariate function of a scalar feature/magnitude/phase of the MS. It may be a multivariate function of more than one MS (e.g. a sliding window of the TSMS), or a multivariate function of a scalar feature/magnitude/phase of each of more than one MS. The mapping function may be a combination of univariate or multivariate functions (e.g. composite function or nested function).

Clause G3. The method/device/system/software of the wireless sensing presentation system of Clause G2: wherein each mapping function comprises at least one of: a univariate function, a multivariate function, a composite function comprising a univariate function of a multivariate function, a composite function comprising a multivariate function of univariate functions, a composite function comprising a univariate function of a multivariate function of other univariate functions, a composite function comprising a multivariate function of univariate functions of other multivariate functions, or a vectored function with each vector component being one of the above.

The mapping function may be piecewise concave and piecewise convex.

Clause G4. The method/device/system/software of the wireless sensing presentation system of Clause G3: wherein a mapping function is at least one of: globally concave, globally convex, locally concave, locally convex, piecewise concave, piecewise convex, or partly concave and partly concave.

The mapping function may be applied to MS, feature/magnitude of MS, sliding window of MS, weighted average of the sliding window of MS (e.g. filtering or transformation). TSMS may be filtered or transformed or processed before mapping function is applied. The filtering/transformation/processing may be applied to MS, or feature/magnitude of MS.

Clause G5. The method/device/system/software of the wireless sensing presentation system of Clause G4: applying the respective mapping function to at least one of: a MS of the respective TSMS, a feature or magnitude of the MS, a sliding window of the TSMS, a weighted average of the sliding window of the TSMS, a filtered MS of a filtered TSMS obtained by applying a filter on the TSMS, a feature or magnitude of the filtered MS, a sliding window of the filtered TSMS, a weighted average of the sliding window of the filtered TSMS, another filtered MS of another filtered TSMS obtained by applying a filter on a feature or magnitude of the TSMS, a feature or magnitude of the another filtered MS, a sliding window of the another filtered TSMS, or a weighted average of the sliding window of the another filtered TSMS.

The mapping function for a TSMS may be computed (e.g. using histogram equalization) based on probability density function (pdf) or histogram of MS. The histogram may be the historical histogram based on the TSMS, or the past MS in the TSMS, or a past window in the TSMS, or another TSMS. The another TSMS may be training TSMS obtained using another wireless signal transmitted from another (e.g. same, similar or different) Type1 device to another (e.g. same, similar or different) Type2 device in another (e.g. same, similar or different) venue when the wireless multipath channel of the another venue is impacted by another (e.g. same, similar or different) motion of another (e.g. same, similar or different) object.

Clause G6. The method/device/system/software of the wireless sensing presentation system of Clause G5, further comprising: computing the mapping function for a TSMS based on at least one of: a probability distribution (or histogram) of the MS (or feature/magnitude of the MS) based on the TSMS, a probability distribution (or histogram) of the MS (or its feature/magnitude) based on another TSMS, a modification of a probability distribution, a statistic characteristics of the MS (or its feature/magnitude), a histogram equalization applied to the histogram of the MS (or its feature/magnitude) or a modification of the histogram, an emphasis of a first domain and a de-emphasis of a second domain of the MS (or feature/magnitude), or a selection or preference of the user.

More details of "emphasis of first domain" and "de-emphasis of second domain".

Clause G7. The method/device/system/software of the wireless sensing presentation system of Clause G6: wherein the first domain is mapped to a first range, and the second domain is mapped to a second range through the mapping function; wherein a first fraction of a first length of the first range over a second length of the first domain is larger than a second fraction of the first length of the second range over the second length of the second domain.

More details of "emphasis of first domain" and "de-emphasis of second domain".

Clause G8. The method/device/system/software of the wireless sensing presentation system of Clause G7: wherein the emphasis of the first domain causes the first fraction to be greater than a first threshold; wherein the de-emphasis of the second domain causes the second fraction to be smaller than a second threshold.

Clause G9. The method/device/system/software of the wireless sensing presentation system of Clause G8: wherein the first threshold is not less than one.

Clause G10. The method/device/system/software of the wireless sensing presentation system of Clause G8 or G9: wherein the second threshold is not greater than one.

More details of "emphasis of first domain" and "de-emphasis of second domain".

Clause G11. The method/device/system/software of the wireless sensing presentation system of Clause G8-G10: wherein the mapping function has a first local slope in the first domain greater than a second local slope in the second domain.

Clause G12. The method/device/system/software of the wireless sensing presentation system of Clause G11: wherein the mapping function has a local slope greater than one in the first domain and a local slope less than one in the second domain.

Some description of a generalization of histogram equalization. In histogram equalization, the first and second reference function should both be uniform distribution (which has a constant value over its support). The support of first and second pdf should be the same as that of the MS. The support of first and second pdf should be from the minimum support of the MS to the maximum support of MS.

Clause G13. The method/device/system/software of the wireless sensing presentation system of Clause G6-G12: computing a first difference between the probability distribution of the MS (or feature/magnitude of the MS, or the filtered MS, or the another filtered MS) and a first reference function; computing the first domain as the domain where the first difference is positive; computing a second difference between the probability distribution of the MS and a second reference function; computing the second domain as the domain where the second difference is negative.

Clause G14. The method/device/system/software of the wireless sensing presentation system of Clause G13: wherein at least one of the following is true: the first reference function is a constant function, the first reference function is non-negative, the first reference function is a probability distribution, the first reference function is a uniform distribution, the first reference function has the same support as the probability distribution of the MS (or feature/magnitude of the MS), the second reference function is a constant function, the second reference function is non-negative, the second reference function is a probability distribution, the second reference function is a uniform distribution, the second reference function has the same support as the probability distribution of the MS (or feature/magnitude of the MS), or the first reference function and the second reference function are the same.

Grouping of MS. More than one MS may be computed for a region/sub-region, such as presence, motion intensity, speed, breathing, activity, fall-down, sleep, etc. MS of the region/sub-region may be grouped together. MS of different sub-regions of a region may be grouped together. MS of nearby or related regions/sub-region may be grouped together (e.g. all rooms in second floor, or all rooms in first floor, or all toilets, or all bedrooms, or "Peter's activity region", or "Peter's activity region on weekdays", or "Peter's activity region on weekends"). Grouping may be based on some criteria or characteristics (e.g. proximity, location, functionality, timing, user behavior, naming). For example, the user may enter a name or classification for each region/sub-region. The grouping may be based on the naming or classification. Or, the grouping may be chosen/selected/performed by the user. There may be more than one way to do the groupings (e.g. a toilet in first floor may be grouped into both "all rooms in first floor", and "all toilets").

Clause G15. The method/device/system/software of the wireless sensing presentation system of any previous G Clause (preferably Clause G1 or Clause G6), further comprising: grouping a number of subsets of the plurality of TSMS into the number of groups based on respective grouping criteria, each subset being grouped into a respective group based on a respective grouping criterion; coordinating a presentation of the number of groups of TSMS on the presentation device of the user.

Clause G16. The method/device/system/software of the wireless sensing presentation system of Clause G15, further comprising: partitioning the venue into more than one regions; associating each region with at least one respective TSMS; grouping at least one TSMS comprising those associated with a particular region into a particular group, wherein the respective grouping criteria is based on at least one of: the association with the particular region, an event or a timing.

Clause G17. The method/device/system/software of the wireless sensing presentation system of Clause G16, further comprising: grouping at least one TSMS comprising those associated with the particular region or another region into another particular group, wherein the respective grouping criteria is based on at least one of: the association with at least one of the particular region or the another region, a distance between the particular region and the another region, a proximity between the particular region and the another region, a relationship between the particular region and the another region, an event, or a timing.

Clause G18. The method/device/system/software of the wireless sensing presentation system of Clause G16 or G17, further comprising: partitioning a particular region into more than one sub- regions; associating each sub-region with at least one respective TSMS; grouping at least one TSMS comprising those associated with a particular sub-region into another particular group, wherein the respective grouping criteria is based on at least one of: the association with the particular sub-region, an association with the particular region, an event, or a timing.

Clause G19. The method/device/system/software of the wireless sensing presentation system of Clause G18 or Clause G17, further comprising: grouping at least one TSMS comprising those associated with the particular sub-region or another sub-region into the group, wherein the grouping criteria is based on at least one of: the association with at least one of: the particular sub-region, the another sub-region, the particular region, or another region encompassing the another sub-region, a distance between the particular sub-region and the another sub-region, a proximity between the particular sub-region and the another sub-region, a distance between the particular region and the another region, a proximity between the particular region and the another region, an event, or a timing.

Clause G20. The method/device/system/software of the wireless sensing presentation system of Clause G15-G19, further comprising: coordinating the presentation of the number of groups of TSMS by at least one of the following: presenting a group of TSMS in a spatially neighboring manner, presenting a group of TSMS in a spatial neighborhood, presenting a group of TSMS in a spatially connected manner, presenting a group of TSMS in a chained manner, presenting a group of TSMS in neighboring locations in a matrix arrangement, presenting a group of TSMS in neighboring locations in a lattice arrangement, or presenting a group of TSMS in neighboring locations in a patterned arrangement.

Clause G21. The method/device/system/software of the wireless sensing presentation system of Clause G15-G20, further comprising: coordinating the presentation of the number of groups of TSMS by at least one of the following: presenting a group of TSMS each with a respective set of presentation attributes, presenting a group of TSMS each with a respective coordinated set of presentation attributes, presenting a group of TSMS with a common set of presentation attributes (e.g. color scheme, texture, patterns, intensity, shading, animation, shape size to represent magnitude, animation frequency to represent magnitude, etc.), presenting a group of TSMS with a common presentation attribute, presenting a group of TSMS with a same set of presentation attributes, presenting a group of TSMS with a same presentation attribute, presenting a group of TSMS with a first set of presentation attributes for a first type of TSMS in the group (e.g. breathing statistics) and a second set of presentation attributes for a second type of TSMS in the group (e.g. fall-down statistics), presenting a group of TSMS with a first presentation attribute for a first type of TSMS in the group (e.g. breathing statistics) and a second presentation attribute for a second type of TSMS in the group (e.g. fall-down statistics), presenting a group of TSMS each with a respective mapping function (in Clause G1), presenting a group of TSMS each with a respective coordinated set of mapping function, presenting a group of TSMS with a common mapping function, presenting a group of TSMS with a same mapping function, presenting a group of TSMS with a first mapping function for a first type of TSMS in the group (e.g. breathing statistics) and a second mapping function for a second type of TSMS in the group (e.g. fall-down statistics), or presenting a group of TSMS with a same set of mapping function.

In some embodiments, the present teaching discloses systems and methods for wireless sensing using two-way sensing in which sounding signals are transmitted between two wireless devices in both ways: from a first device to a second device and from the second device to the first device such that sensing measurement results are obtained/generated in both devices. In one-way sensing, sounding signals are transmitted in one way only such that sensing results are generated in one device only. The sensing results may be used locally in the device or optionally reported to another device.

One disadvantage of one-way sensing is that, when sensing results are generated in a first device but is needed in a second device, undesirable reporting frames may be used by the first device to transmit/report the sensing results to the wirelessly second device. The use of reporting frames is undesirable because they tend to be very large/bulky and may take a long time and a lot of data bandwidth to transmit, and a lot of memory to store, especially when there are many antenna pairs between a TX (wireless transmitter) and a RX (wireless transmitter), many TX/RX pairs, many TX/RX pairs, and wide analog BW used to obtain sensing results (e.g. channel information/CI, such as CSI, CIR, CFR, etc.). An advantage of two-way sensing over one-way sensing is that, with sounding signals transmitted both ways, sensing results are generated in both the first device and the second device, and there may be no need to use the undesirable reporting frames to report the sensing results.

Wireless sensing may be performed in a wireless network based on a standard (e.g. IEEE 802.11, 802.11bf, 4G/5G/6G/7G/8G, 802.15, 802.16, UWB, Bluetooth, etc.), a specification and/or a protocol. In the wireless network, there may be one or more access point (AP) station (STA) (e.g. WiFi AP, mesh network AP, 4G/5G/6G/7G base station, cellular base station, repeater, etc.), and there may be one or more non-AP STA. WLAN or WiFi sensing may be performed based on WLAN/WiFi signal (e.g. compliant to IEEE 802.11, 802.11bf standard, WiFi Alliance).

Non-data packet (NDP) may be used as a sounding signal. A Sensing NDP Announcement (NDPA) frame may be defined that allows a STA to indicate the transmission of NDP frame(s) used to obtain sensing measurements. A Trigger frame variant may be defined that allows an AP STA to solicit NDP transmission(s) from STA(s) to obtain sensing measurements.

Non-TB Wireless Sensing. A non-AP STA may be the sensing initiator and an AP STA may be the sensing responder, and together they may perform non-triggered-based (non-TB or NTB) wireless sensing. The non-AP STA may send an NDPA frame to AP followed by two NDPs: an I2R NDP (initiator-to-responder NDP frame, for uplink sounding) from non-AP STA to AP and an R2I NDP (responder-to-initiator NDP frame, for downlink sounding) from AP to non-AP STA. For the I2R NDP, the non-AP STA is the Type1 device (TX) and the AP is the Type2 device (RX). For the R2I NDP, the AP is the Type1 device (TX) and the non-AP STA is the Type2 device (RX).

One-way non-TB sensing may be performed in which only one of the two NDPs may be used for generating sensing measurement results (e.g. CI, CSI, CIR, CFR, etc.) while the other NDP may not. For example, I2R NDP may be used for uplink sounding to generate sensing measurement results at the AP while the R2I NDP may not lead to sensing measurement results at the non-AP STA. The sensing responder (AP) may optionally report the sensing results to sensing initiator (non-AP STA). When the sensing results are generated, the MAC layer management entity (MLME) of AP may send a first signal/message/primitive to the Station management entity (SME) of AP to indicate the availability of the sensing measurement results. The AP may optionally use sensing measurement report frame to report the sensing results to the non-AP STA (initiator). To do so, the SME may send a second signal/message/primitive to the MLME to request the transmission of the sensing measurement report frame.

In another example, R2I NDP may be used for downlink sounding to generate sensing results at the non-AP STA (initiator). No sensing measurement report frame may be needed because the initiator already has the results. When the sensing results are generated, the MLME of non-AP STA may send the first signal/ message/primitive to the SME of non-AP STA to indicate the availability of the sensing measurement results. The SME may not send the second signal/message/primitive to the MLME to request the transmission of the sensing measurement report frame.

Two-way non-TB sensing may be performed in which both NDP are used for generating sensing results on both side (i.e. at sensing initiator and also at sensing responder).

For example, the I2R NDP may be transmitted from non-AP STA to the AP to generate sensing results at AP (responder) while the R2I NDP may be transmitted from AP to non-AP STA to generate sensing results at non-AP STA (initiator). No sensing measurement report frame may be needed/used by sensing responder to report any sensing results to sensing initiator, as both AP and non-AP already have a version of the sensing measurement results.

Upon the generation of a first (AP-side) sensing results at AP, the MLME of AP may send the first signal/message/ primitive to the SME of AP to indicate the availability of the first sensing results. And upon the generation of a second (non-AP side) sensing results at non-AP STA, the MLME of non-AP STA may send the first signal/message/ primitive to the SME of non-AP STA to indicate the availability of the second sensing measurement results. As no sensing measurement report frames may be used, none of the SME (SME of AP and SME of non-AT STA) may send the second signal/message/primitive to the respective MLME to request the transmission of sensing measurement report frames.

In an alternative example, the AP (responder) may optionally report its sensing results to the non-AP STA (initiator) using sensing measurement report frames. If so, the SME of AP may send the second signal/message/ primitive to MLME of AP to request the transmission of sensing measurement report frames to the non-AP STA (initiator).

TB Wireless Sensing. An AP STA may be the sensing initiator and a number (one or more) of STA(s) (e.g. other AP STA or non-AP STA) may be the sensing responders. They may jointly perform trigger-based (TB) sensing. There may be a polling phase in which the AP transmits a polling frame to check the availability of the number of STA(s) for TB sensing. If a STA is available, it may respond with a CTS-to-self. After the polling phase, there may be a one-way downlink sounding phase (e.g. NDPA sound phase), a one-way uplink sounding phase (e.g. trigger frame sounding phase), or a two-way uplink-downlink sounding phase.

One-way TB sensing. In the one-way downlink sounding phase, for each STA that is available, the AP may send an NDPA frame followed by an NDP frame as downlink sounding signal to the STA to generate sensing measurement results at the STA. A separate NDPA frame may be sent to each available STA, or a common/shared NDPA frame may be sent to multiple (e.g. some or all) available STA(s). Each STA (responder) may optionally use sensing measurement report frame to report its sensing results to the AP (initiator). When the sensing results are available at an STA, the MLME of the STA may send the first signal/message/primitive to the SME of the STA. If sensing results of the STA is optionally reported to the AP, the SME may send the second signal/message/primitive to the MLME of the STA to request the transmission of sensing measurement report frame to the AP (initiator).

In the one-way uplink sounding phase, for each STA that is available, the AP (initiator) may send a Trigger frame (TF) to the STA (responder), followed by the STA sending an NDP as uplink sounding signal to the AP to generate sensing measurement results at the AP (initiator). A separate Trigger frame may be sent to each available STA, or a common/shared Trigger frame may be sent to multiple (e.g. some or all) available STA(s). When sensing results are available at a STA, the MLME of the STA may send the first signal/message/primitive to the SME of the STA to indicate availability of the sensing results. No sensing measurement report frame may needed/used to transmit sensing results to the initiator, because the sensing results are already generated at the initiator. As such, the SME of the STA may not send the second signal/message/primitive to request transmission of sensing measurement report frame.

Two-way TB sensing. In the two-way uplink-downlink sounding phase, for each STA that is available, the AP may send a special "NDPA-Trigger" frame to the STA followed by two NDPs: an I2R NDP (initiator-to-responder NDP frame) transmitted from AP to the STA as downlink sounding signal to generate sensing results at the STA, and an R2I NDP (responder-to- initiator NDP frame) transmitted from the STA to AP as uplink sounding signal to generate sensing results at AP. The I2R NDP may precede the R2I NDP, or the R2I NDP may precede the I2R NDP. The special NDPA-Trigger frame may be the NDPA frame, the Trigger frame or another frame. A separate NDPA-Trigger frame may be sent to each available STA, or a common/shared NDPA-Trigger frame may be sent to multiple (e.g. some or all) available STA(s). No sensing measurement report frame may be needed/used by sensing responder to report any sensing results to sensing initiator, as both AP and STA already have a version of the sensing measurement results.

Upon the generation of a first (AP-side) sensing results at AP, the MLME of AP may send the first signal/message/ primitive to the SME of AP to indicate the availability of the first sensing results. And upon the generation of a second (non-AP side) sensing results at the STA, the MLME of the STA may send the first signal/message/primitive to the SME of the STA to indicate the availability of the second sensing measurement results. As no sensing measurement report frames may be used, none of the SME (SME of AP and SME of non-AT STA) may send the second signal/message/primitive to the respective MLME to request the transmission of sensing measurement report frames to the initiator.

In an alternative two-way uplink-downlink sounding phase, the STA (responder) may optionally report its sensing results to AP (initiator) using sensing measurement report frames. If so, the SME of the STA may send the second signal/message/ primitive to MLME of STA to request the transmission of sensing measurement report frames to the AP (initiator).

Peer-to-peer Wireless Sensing (P2P sensing). In peer-to-peer wireless sensing, one or more pairs of non-AP STA may be determined in a wireless network associated with an AP. With each pair, NDP(s) may be transmitted between a first non-AP STA and a second non-AP STA as sounding signal(s) to generate sensing results in non-AP STA(s). The NDP(s) transmission may/may not be transmitted with a help/signaling/trigger from an associated AP.

One-way P2P sensing. In one example of one-way P2P sensing, the AP may be the sensing initiator and both the first and second non-AP STA may be sensing responders. In another example, a non-AP STA may be a sensing-by-proxy (SBP) initiator that requests the AP (SBP responder) to perform one-way P2P sensing in which the AP may be the sensing initiator and both the first and second non-AP STAs may be sensing responders.

In both examples, the AP may configure/negotiate/ arrange individually with the two non-AP STAs such that the two non-AP STAs can identify each other (each with at least one corresponding ID, e.g. identifiable network address, identifiable wireless network address/ID, AP assigned ID, initiator assigned ID, user defined ID, MAC address) and the first non-AP STA would send NDP as sounding signal to the second non-AP STA so that sensing measurement results may be obtained/generated in the second non-AP STA. The AP may send a first P2P- sensing-triggering frame to the pair of non-AP STAs. The first P2P-sensing-triggering frame may be a NDPA frame, a Trigger Frame, the special NDPA-Trigger frame (mentioned above), or another frame. A separate first P2P-sensing-triggering frame may be sent to each pair of non-AP STAs, or a common/shared first P2P-sensing-triggering frame may be sent to multiple (e.g. some or all) available STA(s). The first non-AP STA would then send the NDP to the second non-AP STA to generate sensing measurement results at the second non-AP STA. The sensing measurement results may be used/needed in the second non-AP STA, or the sensing results can optionally be transmitted from the second non-AP STA (sensing responder) to the AP (sensing initiator). In the SBP example, the AP (SBP responder) may further report the sensing results to the SBP initiator.

Two-way P2P sensing. In one example of two-way P2P sensing, the AP may be the sensing initiator and both the first and second non-AP STA may be sensing responders. In another example, a non-AP STA may be a sensing-by-proxy (SBP) initiator that requests the AP (SBP responder) to perform two-way P2P sensing in which the AP may be the sensing initiator and both the first and second non-AP STAs may be sensing responders.

In both examples, the AP may configure/negotiate/ arrange individually with the two non-AP STAs such that the two non-AP STAs can identify each other (each with at least one corresponding ID, e.g. identifiable network address, identifiable wireless network address/ID, AP assigned ID, initiator assigned ID, user defined ID, MAC address) and the two non-AP STAs would send NDP as sounding signal to each other so that sensing measurement results may be obtained/generated in both non-AP STA. The AP may send a second P2P-sensing-triggering frame to the pair of non-AP STAs. The second P2P-sensing-triggering frame may be a NDPA frame, a Trigger Frame, the special NDPA-Trigger frame (mentioned above), the first P2P- sensing-triggering frame, or another frame. A separate first P2P-sensing-triggering frame may be sent to each pair of non-AP STAs, or a common/shared first P2P-sensing-triggering frame may be sent to multiple (e.g. some or all) available STA(s). Then the first non-AP STA would send NDP to the second non-AP STA to generate sensing measurement results at the second non-AP STA and the second non-AP STA would send NDP to the first non-AP STA to generate sensing measurement results at the first non-AP STA. The sensing measurement results may be used/needed in the second non-AP STA, or the sensing results can optionally be transmitted from the second non-AP STA (sensing responder) to the AP (sensing initiator). In the SBP example, the AP (SBP responder) may further report the sensing results to the SBP initiator.

In another example, the first and second non-AP STA may perform the one-way P2P sensing or the two-way P2P sensing without signaling from AP. The two non-AP STA may be able to identify each other and configure/negotiate/arrange with each other. In one-way P2P sensing, NDP may be transmitted one-way from a first non-AP STA to a second non-AP STA to generate sensing results in the second non-AP STA. The second non-AP STA may optionally transmit its sensing results to the first non-AP STA. In two-way P2P sensing, NDPs may be transmitted both ways between the two non-AP STA, without signaling from AP.

Generalized Daisy-chain sensing. An AP may, or a non-AP STA may request/cause the AP to, configure/negotiate/arrange/set up a number of STA(s) (e.g. the AP, another AP, or non- AP STAs) to establish a scanning order (e.g. a daisy chain, or a fully-connected configuration) for sensing. For example, the AP may send a daisy-chain sensing polling frame to check availability of the number of STA(s). If a STA is available, it may respond with a response frame accordingly (e.g. with a CTS-to-self). NDP may be transmitted among adjacent STAs in the daisy chain to generate sensing measurement results.

One-way daisy-chain sensing. One-way daisy-chain sensing may be performed by NDP being transmitted in one way (from upstream to downstream along the daisy chain).

The AP may send a first daisy-chain trigger frame (e.g. NDPA frame, NDPA frame variant, Trigger frame, Trigger frame variant, "NDPA-Trigger" frame, first P2P sensing-trigger frame, second P2P sensing-trigger frame, another frame) to the number of STA(s). A first STA may transmit a first NDP to a second STA. The second STA may (a) receive the first NDP and generate first sensing measurement results (e.g. CSI computed based on the received first NDP), and (b) transmit a second NDP (downstream) to a third STA (which may be the first STA or second STA). The second NDP may be transmitted after a first time delay (e.g. SIF) after reception of the first NDP. The third STA may receive the second NDP and generate sensing measurement results, and to transmit a third NDP to a fourth STA (perhaps after a second time delay), and so on. The first, second, third, fourth, ... STA may form a daisy, each receiving an NDP from "upstream" or previous STA, and transmit another NDP to "downstream" or next STA (perhaps with some time delay). The daisy chain may form a close loop (i.e. the last STA in the daisy chain may be the first STA or another STA in the daisy chain).

For any of the number of STA(s), the sensing measurement results may be used locally by the STA for sensing, or may optionally be reported to the AP. In the case of SBP, the reported sensing results may be reported to the SBP-requesting non-AP STA.

Two-way daisy-chain sensing may be performed by NDP being transmitted in both manners (from upstream to downstream, and also from downstream to upstream along the daisy chain).

The AP may send a second daisy-chain trigger frame (e.g. NDPA frame, NDPA frame variant, Trigger frame, Trigger frame variant, "NDPA-Trigger" frame, first P2P sensing-trigger frame, second P2P sensing-trigger frame, the first daisy-chain trigger frame, another frame) to the number of STA(s). A first STA may transmit a first NDP to a second STA. The second STA may (a) receive the first NDP and generate first sensing measurement results (e.g. CSI computed based on the received first NDP), (b) transmit a second NDP (downstream) to a third STA, and (c) transmit another NDP (upstream) back to the first STA to generate sensing measurement results at the first STA. The second NDP may precede the another NDP, or vice versa. There may be a first time delay (e.g. SIF) after reception of the first NDP before transmission of the second NDP and the another NDP. The third STA may receive the second NDP and generate sensing measurement results, transmit a third NDP to a fourth STA and transmit yet another NDP back to second STA, and so on. The first, second, third, fourth, ... STA may form a scanning order or a daisy chain, each receiving an NDP from upstream/previous STA, transmit an NDP to downstream/next STA (perhaps with some time delay) and transmit an NDP (upstream) back to upstream/previous STA. The scanning order/daisy chain may form a close loop (i.e. the last STA in the daisy chain may be the STA device or another STA in the daisy chain).

Termination/Pause of a session setup/measurement setup associated with a sensing responder. An AP may determine, or AP may receive a determination from SBP initiator in the case of sensing-by-proxy (SBP), that the sensing measurement results (e.g. CSI, CIR, CFR, RSSI) associated with a particular sensing responder may be useless, not useful, and/or least useful for a task (e.g. too noisy, too unstable, too chaotic, too much interference, unreliable, faulty, or a user "pause" or "stop" the sensing associated with the particular responder, or a user "pause" or "stop" the sensing associated with all sensing responders, etc.). The determination may be based on (i) a test on the sensing measurement results (e.g. based on a test/measure for noise, stability, variability, randomness/chaos, interference, reliability, fault, error and/or mistakes) and/or (ii) a state/condition/test of system (e.g. the sensing measurement results transmission/storage/associated processing/sensing computation may consume too much bandwidth/memory/processing power/time, or generate too much power, or another task of higher priority needs resources currently allocated to the sensing measurement results) . There may be a determination that sensing measurement results associated with another sensing responder may be useful, not useless and/or more useful for the task.

As a result, the AP may, or may receive a request from SBP initiator in the case of SBP to, terminate the sensing session setup associated with the particular sensing responder. The AP may, or may receive a request from SBP initiator in the case of SBP to, wait for a period of time (e.g. wait until some interfering/noisy/9unstable/unreliable/ adverse condition is finished, or wait until a user "un-pause" or "un-stop" the sensing) and then start another sensing session (by performing sensing session setup) with the particular sensing responder, using identical or similar or modified sensing session setup settings as the terminated sensing session setup. The determination of the period time may be based on some criteria.

Alternatively, instead of terminating the sensing session setup, the AP may, or may receive a request from SBP initiator in the case of SBP to, terminate a particular sensing measurement setup associated with the particular sensing responder. The AP may, or may receive a request from SBP initiator in the case of SBP to, wait for the period of time and then start another sensing measurement setup with the particular sensing responder, with identical or similar setting as the particular terminated sensing measurement setup.

Alternatively, the AP may, or may receive a request from SBP initiator in the case of SBP to, pause the sensing session (i.e. sensing session setup) with the particular sensing responder for the period of time, and resume the sensing session after the period of time.

Alternatively, the AP may, or may receive a request from SBP initiator in the case of SBP to, pause a particular sensing measurement session with the particular sensing responder for the period of time, and resume the particular sensing measurement session after the period of time.

Multicast or Broadcast of sounding signals from AP to more than one sensing responders in SBP. An AP may be a sensing initiator and also a sensing transmitter (e.g. in sensing session, or in SBP). It may send a sounding signal (e.g. NDP) to each of a number of sensing responders separately (i.e. point-to-point sounding). Alternatively, it may send sounding signal (e.g. NDP) to more than one sensing responders using multicast or broadcast such that sensing measurement results may be generated in the more than one sensing responders simultaneously or contemporaneously. The sensing measurement results may optionally (i.e. may/may not) be reported to the AP. In the case of SBP, the AP may optionally (i.e. may/may not) report the sensing measurement results to the SBP initiator.

Selective SBP. A proxy-initiator (e.g. SBP-initiator) may send a request to a wireless access point (AP) which is a proxy-responder (e.g. SBP-responder), such that non-selective wireless sensing (e.g. SBP) is performed between the AP (acting as sensing initiator, on behalf of the proxy-initiator) with any available sensing responders (e.g. non-AP STAs/devices, another AP, mesh AP) in the AP's wireless network. Each of the available sensing responders may be assigned/associated with an identity (ID, e.g. MAC address). The proxy-initiator (e.g. SBP- initiator) may send another request to the AP to perform selective wireless sensing (e.g. selective SBP) with a group of selected sensing responders in the AP's wireless network. Each selected sensing responders may be identified by the respective ID. Same or different sensing settings may be used for different sensing responders. For a sensing responder, same or different sensing settings may be used for different target tasks (for the case of more than one target tasks) or different proxy-initiators (for the case of more than one proxy-initiators).

The proxy-initiator may request the AP to provide a list of sensing-capable devices in the AP's network that support/is capable of wireless sensing (e.g. 802.11bf compatible), with associated device information (e.g. device name, host name, vendor class ID, device product name). The proxy-initiator may select the selected sensing responders based on the list and the associated device information.

The proxy-initiator may use a two-stage approach to do selective wireless sensing for a target task. In stage 1, the proxy-initiator may request/perform/use non-selective wireless sensing (i.e. sensing with all available sensing responders) to perform a trial/testing/training task with all the sensing responders and select the selected sensing responders based on the sensing results and some criteria. The trial/testing/training task may be a motion detection task. A location (or a mapping to some target physical device) of each sensing responder in a venue may be estimated in the trial/testing/training task and the selection may be based on the estimated locations (or the mapping) of the sensing responders. The proxy-selector may also select some device from the list of sensing-capable devices that did not participate in stage 1.

Then in stage 2, the proxy-initiator may request/perform selective wireless sensing for the target task with the selected sensing responders. The trial/testing/training task may be related to the target task in a certain way. The trial/testing/training task may have a low sensing requirement such that all sensing-capable wireless responders can satisfy the requirement and are capable of taking part in the non-selective wireless sensing. The trial/testing/ training task may have sensing results useful for the selection of the selected sensing responders.

The proxy-initiator may use the two-stage approach to do selective wireless sensing for two target tasks. For each target task, a respective stage 1 followed by a respective stage 2 may be performed. Alternatively, a common stage 1 may be performed in which a first group of selected sensing responders may be selected for the first target task and a second group selected for the second target task. The first group may or may not overlap with the second group. Then separate stage 2 may be performed for the two target tasks (e.g. sequentially, simultaneously or contemporaneously) based on the respective group of selected sensing responders. If the first group and the second group overlap with at least one common sensing responder appearing in both groups, sensing results associated with the common sensing responder may be shared by both target tasks.

Two different proxy-initiators may use the two-stage approach to do selective wireless sensing for their respective target tasks. For each target task of each proxy-initiator, a respective stage 1 followed by a respective stage 2 may be performed. Alternatively, a first common stage 1 may be performed for the first proxy-initiator (to select a group of selected sensing responders for each of its target tasks) followed by separate stage 2 (to perform selective wireless sensing for each of its target tasks). Similarly, a second common stage 1 may be performed for the second proxy-initiator followed by separate stage 2 for each of its target tasks. Alternatively, a third common stage 1 may be performed for both proxy-initiators followed by separate stage 2 for each target tasks. If a common sensing responder is selected for more than one target tasks, sensing results associated with the common sensing responder may be shared by the more than one target tasks.

The proxy-initiator may be an "authorized" or "trusted" device that the AP allows/authorizes/ authenticates to initiate one of the non-selective SBP, or non-selective SBP, or both. A first qualification test/setup/procedure may be performed in order for the SBP-initiator to be authorized by the AP to initiate non-selective SBP (first authorization). A second qualification test/setup/procedure may be performed in order for the SBP-initiator to be authorized by the AP to initiate selective SBP (second authorization). The SBP-initiator may have one of the first authorization and the second authorization, or both. One of the first authorization and the second authorization may imply the other.

The proxy-initiator may be connected to the AP via a wireless connection (e.g. the AP's wireless network, WiFi, WiMax, 4G/5G/6G/7G/8G, Bluetooth, UWB, mmWave, etc.) or via a wired connection (e.g. Ethernet, USB, fiber optics, etc.).

A sensing responder may/may not support non-selective proxy sensing (e.g. SBP), or selective proxy sensing or both. When sending sensing results to the AP for onward transmission to the proxy-initiator, a sensing responder may encrypt/process the sensing results so that the sensing results may not be decrypted/interpreted/consumed/made sense by AP (which does not have decryption key) but may be by the proxy-initiator (which has the decryption key).

Two devices each sending respective wireless signal (e.g. NDP) in respective ways (i.e. directions) in session based on a protocol/standard. First device transmits first wireless signal to second device which generates sensing measurement results (e.g. channel info/CI, TSCI, CSI, CIR, CFR, RSSI, etc.) from received first wireless signal. Second device transmits second wireless signal to third device (e.g. first device) which generates CI from received second wireless signal. The sensing measurement generations are in succession also.

Important special case: third device is first device, s.t. both first and second devices have their own TSCI and can perform wireless sensing computing. And there is no reporting of sensing measurement results. Protocol may be a default protocol, an industry standard, a national standard, an international standard, WLAN standard, WiFi, IEEE 802.11, 802.11bf, Bluetooth, UWB, 802.15, 802.16, cellular communication standard, 4G/5G/6G/7G/8G, WiMax, etc.

The following numbered clauses provide examples for two-way sensing.

Clause H1. A method/device/system/software of a wireless two-way sensing system, comprising: transmitting two wireless (sounding) signals in succession by two devices through a wireless multipath channel of a venue based on a protocol, a first wireless signal transmitted from a first heterogeneous wireless device to a second heterogeneous wireless device and a second wireless signal transmitted from the second heterogeneous wireless device to a third heterogeneous wireless device, wherein the wireless multipath channel is impacted by a motion of an object in the venue; receiving the two wireless signals in two ways in succession by two devices, the first wireless signal by the second device in the first way and the second wireless signal by the third device in the second way, wherein the transmitted first wireless signal differs from the received first wireless signal due to the multipath channel and the motion of the object, wherein the transmitted second wireless signal differs from the received second wireless signal due to the multipath channel and the motion of the object; obtaining two time series of channel information (TSCI) of the wireless multipath channel in the venue in succession by two devices based on the two received wireless signals, a first TSCI obtained by the second device based on the received first wireless signal and a second TSCI obtained by the third device based on the received second wireless signal; making the two TSCI available in two devices, the first TSCI available in the second device and the second TSCI available in the third device for applications.

In second and third devices: respective MLME sends a respective internal (electronic) signal/message to respective SME to indicate availability of respective TSCI in the respective device.

Clause H2. The method/device/system/software of the wireless two-way sensing system in clause H1, further comprising: sending two electronic signals in succession to indicate the availability of the two TSCI in the two devices based on the protocol; sending a second electronic signal within the second device from a second MAC layer management entity (MLME) of the second device to a second station management entity (SME) of the second device to indicate the availability of the first TSCI; sending a third electronic signal within the third device from a third MLME of the third device to a third SME of the third device to indicate the availability of the second TSCI.

Special case 1: First device=third device. First device is sensing initiator.

Clause H3. The method/device/system/software of the wireless two-way sensing system in clause H1 or 2, further comprising: initiating a sensing session by the first device based on the protocol, wherein the third device is the first device, wherein the first device is a sensing initiator, wherein the second device is a sensing responder, wherein the first wireless signal is an initiator- to responder (I2R) sounding signal, wherein the second wireless signal is a responder-to-initiator (R2I) sounding signal.

No report frame may be used by sensing responder to report its sensing measurement results to sensing initiator.

Clause H4. The method/device/system/software of the wireless two-way sensing system in clause H3, further comprising: wherein no wireless report frames are used by the sensing responder to transmit its TSCI (i.e. first TSCI) to the sensing initiator.

Alternatively, report frame may be optionally used by sensing responder to its report sensing measurement results to sensing initiator.

Clause H5. The method/device/system/software of the wireless two-way sensing system in clause H3 or H4, further comprising: wherein wireless report frames are optionally used by the second device (sensing responder) to transmit the first TSCI (i.e. sensing measurement generated in second device) to the first device (sensing initiator) such that both the first TSCI and second TSCI are available to the first device.

Trigger signal may be used to trigger the transmission of the two wireless signals in succession by the two devices. Trigger signal may be an NDPA frame, a Trigger Frame (TF), or another frame for triggering.

Clause H6. The method/device/system/software of the wireless two-way sensing system in any previous H clause, further comprising: transmitting a trigger signal by the sensing initiator to the sensing responder based on the protocol to trigger the transmission of the two wireless signals in succession by the two devices.

Special case 1a: Non-TB sensing, with a non-AP STA initiating sensing session. AP may be AP in WiFi/WLAN or base station in cellular communication.

Clause H7. The method/device/system/software of the wireless two-way sensing system in clause H3-H6, further comprising: wherein the first device is a non-access point device (non-AP station or non-AP STA).

Special case 1b: TB sensing with an AP initiating sensing session. AP may be AP in WiFi/WLAN or base station in cellular communication.

Clause H8. The method/device/system/software of the wireless two-way sensing system in clause H3-H7, further comprising: wherein the first device is an access point device (AP).

TB sensing may have a polling phase for sensing initiator (first device) to check "availability" of a number of devices (including second device). Each available device may reply to indicate that it is available.

Clause H9. The method/device/system/software of the wireless two-way sensing system in clause H8, further comprising: checking wirelessly for availability of a number of wireless heterogeneous devices (i.e. wireless station/STA) by the AP based on the protocol, wherein the second device is one of the number of wireless heterogeneous devices and is available.

The AP may send at least one polling frame to check for availability of the devices.

Clause H10. The method/device/system/software of the wireless two-way sensing system in clause H9, further comprising: transmitting a polling frame by the AP to the number of wireless heterogeneous devices based on the protocol to check wirelessly for their availability.

The available devices may send some wireless reply signal (e.g. "availability signal") as a reply to indicate they are available.

Clause H11. The method/device/system/software of the wireless two-way sensing system in clause H9 or H10, further comprising: transmitting a wireless availability signal by any available wireless heterogeneous device to the AP based on the protocol to indicate it is available.

The wireless reply signal in previous clause H may be a "reply frame".

Clause H12. The method/device/system/software of the wireless two-way sensing system in clause H11, further comprising: transmitting a reply frame by any available wireless heterogeneous device to the AP based on the protocol to indicate it is available.

All the devices being polled by AP may send some wireless reply signal (e.g. "availability signal") as a reply to indicate its "availability".

Clause H13. The method/device/system/software of the wireless two-way sensing system in clause H11or H12, further comprising: transmitting a wireless availability signal by any wireless heterogeneous device to the AP based on the protocol to indicate its availability.

The wireless reply signal in previous clause H may be a "reply frame".

Clause H14. The method/device/system/software of the wireless two-way sensing system in clause H13, further comprising: transmitting a reply frame by any wireless heterogeneous device to the AP based on the protocol to indicate its availability.

(SBP case) In SBP, a non-AP STA requests the AP to initiate sensing session.

Clause H15. The method/device/system/software of the wireless two-way sensing system in clause H8-H14, further comprising: requesting the AP to initiate the sensing session by a non-AP heterogeneous wireless device.

(SBP case) In SBP, the non-AP STA initiates an SBP session by making SBP-request to AP.

Clause H16. The method/device/system/software of the wireless two-way sensing system in clause H15, further comprising: initiating a sensing-by-proxy (SBP) session by the non-AP device based on the protocol; making a SBP request by the non-AP device to the AP based on the protocol.

(SBP case) In SBP, the non-AP STA configures the SBP session.

Clause H17. The method/device/system/software of the wireless two-way sensing system in clause H16, further comprising: configuring the SBP session by the non-AP device.

(SBP case) In SBP, the non-AP STA configures the SBP session.

Clause H18. The method/device/system/software of the wireless two-way sensing system in clause H17, further comprising: configuring the sensing session indirectly by the non- AP device, by configuring the SBP session.

(SBP case) In SBP, the non-AP STA configures the SBP session.

Clause H19. The method/device/system/software of the wireless two-way sensing system in clause H18, further comprising: configuring the SBP session such that the AP does not report any TSCI to the non-AP device.

(SBP case) In SBP, the non-AP STA configures the SBP session.

Clause H20. The method/device/system/software of the wireless two-way sensing system in clause H19, further comprising: configuring the sensing session indirectly such that sensing responders do not use wireless report frames to report their TSCI to the AP.

Clause H21. The method/device/system/software of the wireless two-way sensing system in clause H8-H20, further comprising: configuring the second device by the AP regarding the transmission of the second wireless signal and the reception of the first wireless signal in succession, the generation of the first TSCI based on the received first wireless signal, and the making available of the first TSCI, based on the protocol.

Clause H22. The method/device/system/software of the wireless two-way sensing system in clause H21, further comprising: configuring the second device and another device jointly by the AP based on at least one of: a combined set-up, a combined negotiation, or a combined configuration.

Clause H23. The method/device/system/software of the wireless two-way sensing system in clause H21 or H22, further comprising: configuring the second device and another device separately by the AP based on at least one of: respective individual set-ups, respective individual negotiations, or respective individual configurations.

A generalized daisy chain sensing with a scanning order of a plurality of devices (at least two devices). For example, a daisy chain comprises a simple scanning order in which each device appears once and only once in the scanning order except that the last device in the scanning order may be the first device in the scanning ordering.

The following numbered clauses provide examples for daisy-chain sensing.

Clause I1. A method/device/system/software of the wireless two-way sensing system, comprising: monitoring a motion of an object in a venue based on a plurality of heterogeneous wireless devices in the venue; determining a scanning order of the plurality of devices, wherein each device is scanned at least once in the scanning order; configuring the plurality of devices to perform sequential and iterative wireless sensing in succession according to the scanning order by obtaining wireless sensing measurement result between each pair of consecutively "scanned" devices based on the scanning order; transmitting sequentially and iteratively a series of wireless signals in succession through a wireless multipath channel of the venue based on a protocol among the plurality of devices according to the scanning order, each respective wireless signal transmitted between a respective pair of consecutively scanned devices; receiving the series of wireless signals sequentially and iteratively among the plurality of devices in succession according to the scanning order, each respective received wireless signal differs from the respective transmitted wireless signal due to the multipath channel and the motion of the object in the venue; obtaining a series of the wireless sensing measurement results sequentially and iteratively in succession based respectively on the series of wireless signals received sequentially and iteratively, making the series of wireless sensing measurement results available.

Clause I2. The method/device/system/software of the wireless two-way sensing system of Clause I1, further comprising: initializing the iterative wireless sensing by transmitting a wireless signal from a first scanned device to a second scanned device; receiving the wireless signal by the second scanned device; obtaining a second wireless sensing measurement result by the second scanned device based on the received wireless signal; making the second wireless sensing measurement result available by the second scanned device; transmitting another wireless signal from the second scanned device to a third scanned device; iteratively and sequentially receiving by a current scanned device a current wireless signal transmitted from a previous scanned device to the current scanned device; iteratively and sequentially obtaining a current wireless sensing measurement result by the current scanned device based on the received current wireless signal; iteratively and sequentially making the current wireless sensing measurement result available by the current scanned device; iteratively and sequentially transmitting a next wireless signal from the current scanned device to a next scanned device.

Clause I3. The method/device/system/software of the wireless two-way sensing system of Clause I2, further comprising: transmitting yet another wireless signal from the second scanned device to the first scanned device; iteratively and sequentially transmitting a current backward wireless signal from the current scanned device to the previous scanned device.

Clause I4. The method/device/system/software of the wireless two-way sensing system of Clause I3, further comprising: transmitting the yet another wireless signal before the another wireless signal; iteratively and sequentially transmitting the current backward wireless signal before the next wireless signal.

Clause I5. The method/device/system/software of the wireless two-way sensing system of any previous I Clause , comprising: initiating a sensing session by an access-point device (AP); transmitting a polling signal by the AP to poll the plurality of devices for their availability; transmitting a reply signal by each of the plurality of devices to the AP to indicate that it is available; configuring the plurality of devices by the AP. A non-AP STA (SBP initiator) may request the AP to initiate the sensing session.

Clause I6. The method/device/system/software of the wireless two-way sensing system of Clause IS, comprising: requesting by a non-AP heterogeneous wireless device the initiation of the sensing session by the AP.

Clause I7. The method/device/system/software of the wireless two-way sensing system of Clause I5 or I6, comprising: configuring the plurality of devices separately by the AP.

Clause I8. The method/device/system/software of the wireless two-way sensing system of Clause I5-I7, comprising: configuring the plurality of devices jointly by the AP.

Coordinate the transmission/reception of wireless signals and TSCI generation.

Clause I9. The method/device/system/software of the wireless two-way sensing system of Clause I5-I8, comprising: coordinating by the AP the sequential and iterative transmission and reception of the series of wireless signals and generation of the series of wireless sensing measurement results based on the wireless signals in succession by the plurality of devices according to the scanning order.

Clause I10. The method/device/system/software of the wireless two-way sensing system of Clause I5-I9, comprising: triggering the plurality of devices to transmit the series of wireless signals sequentially and iteratively in succession.

Clause 111. The method/device/system/software of the wireless two-way sensing system of Clause I10, comprising: triggering by the AP the transmission of the wireless signal by the first scanned device; iteratively triggering the transmission of the next wireless signal by the reception of the current wireless signal.

Clause I12. The method/device/system/software of the wireless two-way sensing system of Clause I10 or I11, comprising: triggering by the AP all the transmission of the wireless signals by the respective scanned device.

Clause I13. The method/device/system/software of the wireless two-way sensing system of any previous I Clause, comprising: wherein the scanning order comprises every possible pairing among the plurality of devices, similar to a fully-connected network.

Clause I14. The method/device/system/software of the wireless two-way sensing system of any previous I Clause, comprising: wherein the last scanned device is the first scanned device in the scanning order.

Clause I15. The method/device/system/software of the wireless two-way sensing system of any previous I Clause, comprising: wherein the scanning order establishes a daisy chain of devices.

In regular SBP, any client devices of the network can be considered by AP (sensing initiator) as sensing responder. But in "selective SBP," only some client devices can be considered. SBP is "selective" because the number N (or amount) and the N particular client devices (identified by MAC) are selected by SBP-initiator and ultimately by SBP-responder, and are specified/communicated/ negotiated between SBP-initiator and SBP-responder.

The following numbered clauses provide examples for selective SBP.

Clause J1. A method/device/system/software of a wireless sensing system, comprising: configuring an access-point (AP) device of a wireless data communication network based on a wireless protocol by a first particular client device of the wireless network to perform a sensing-by-proxy (SBP) procedure selectively, wherein the wireless protocol comprises one of: a wireless local area network (WLAN) protocol, WiFi, Zigbee, Bluetooth, IEEE 802, IEEE 802.11, IEEE 802.11bf, IEEE 802.15, IEEE 802.16, 4G/5G/6G/7G/8G, wherein the first particular client device functions as a SBP-initiator device and the AP device functions as a SBP-responder device in the SBP procedure; performing the SBP procedure selectively by the AP device based on the wireless protocol by configuring, on behalf of the first particular client device, a number of second particular client devices of the wireless network to perform a wireless sensing procedure, where the AP device functions as a sensing-initiator device and all the second particular client devices function as sensing-responder devices in the wireless sensing procedure.

Clause J2. The method/device/system/software of a wireless sensing system of clause J1, comprising: configuring the AP device by the first particular client device using at least one configuration field of at least one configuration frame exchanged between the two devices during a setup procedure of the SBP procedure based on the wireless protocol.

Clause J3. The method/device/system/software of a wireless sensing system of clause J2, comprising: communicating at least one selected item between the AP device and the first particular client device based on the wireless protocol using the at least one configuration field of the at least one configuration frame.

In one embodiment, SBP-initiator may want N sensing responders, and may select/specify k particular ones. The other (N-k) may be selected by SBP-responder.

Clause J4. The method/device/system/software of a wireless sensing system of clause J3, comprising: selecting at least one of the second particular client devices by the first particular client device and ultimately by the AP device.

Clause J5. The method/device/system/software of a wireless sensing system of clause J4: wherein at least one of the second particular client devices is not selected by the first particular client device, but is selected by the AP device.

In another embodiment, SBP-initiator may want N sensing responders, and select/specify N particular ones.

Clause J6. The method/device/system/software of a wireless sensing system of clause J4 or J5, comprising: selecting all of the second particular client devices by the first particular client device and ultimately by the AP device.

Clause J7. The method/device/system/software of a wireless sensing system of clause J6, comprising: selecting by the first particular client device to include itself in the number of the selected second particular client devices.

Clause J8. The method/device/system/software of a wireless sensing system of clause J6 or J7, comprising: selecting by the first particular client device not to include itself in the number of the selected second particular client devices.

Clause J9. The method/device/system/software of a wireless sensing system of clause J6-J8, comprising: selecting an amount of the second particular client devices by the first particular client device and ultimately by the AP device.

Clause J10. The method/device/system/software of a wireless sensing system of clause J4-J9, comprising: selecting an amount of the at least one of the second particular client devices by the first particular client device and ultimately by the AP device.

In yet another embodiment, SBP-initiator may select/provide a wish-list of N+k candidate sensing responders. SBP-responder may choose N out of the N+k candidates.

Clause J11. The method/device/system/software of a wireless sensing system of clause J4-J10, comprising: providing by the first particular client device to the AP device based on the wireless protocol a list of selected client devices comprising the number of second particular client devices.

Clause J12. The method/device/system/software of a wireless sensing system of clause J11, comprising: wherein the list comprise the first particular client device, as selected by the first particular client device.

Clause J13. The method/device/system/software of a wireless sensing system of clause J6-J12, comprising: wherein the list do not comprise the first particular client device, as selected by the first particular client device.

SBP-initiator may identify each sensing responder/candidate sensing responder with associated MAC address. SBP-responder may reply with associated MAC address and ID.

Clause J14. The method/device/system/software of a wireless sensing system of clause J6-J13, comprising: identifying each selected second particular client device by at least one of: its respective MAC address, or a identifier (ID) in the at least one configuration field of the at least one configuration frame.

Clause J15. The method/device/system/software of a wireless sensing system of clause J14, comprising: selecting a set of sensing measurement setup parameters for the wireless sensing procedure by the first particular client device and ultimately by the AP device.

Sensing transmitter may be Type1 device. Sensing receiver may be Type2 device.

Clause J16. The method/device/system/software of a wireless sensing system of clause J15, comprising: for each of the number of second particular client devices: determining the AP device and the respective second particular client device to function as a pair of sensing-transmitter device and a sensing-receiver device based on the set of sensing measurement setup parameters, transmitting a time series of wireless sounding signals (WSS) based on the wireless protocol by the sensing-transmitter device; receiving the time series of WSS (TSWSS) based on the wireless protocol by the sensing-receiver device through a wireless multipath channel; obtaining a time series of channel information (TSCI) of the wireless multipath channel by the sensing-receiver device based on the received TSWSS based on the wireless protocol, wherein each CI comprises at least one of: a channel state information (CSI), a channel impulse response (CIR), or a channel frequency response (CFR), wherein each channel information (CI) of the TSCI is obtained based on a respective received WSS; making the TSCI available either at the sensing receiver or the first particular client device, wherein the TSCI is labeled with the respective ID associated with the respective second particular client device when it is made available at the first particular client device.

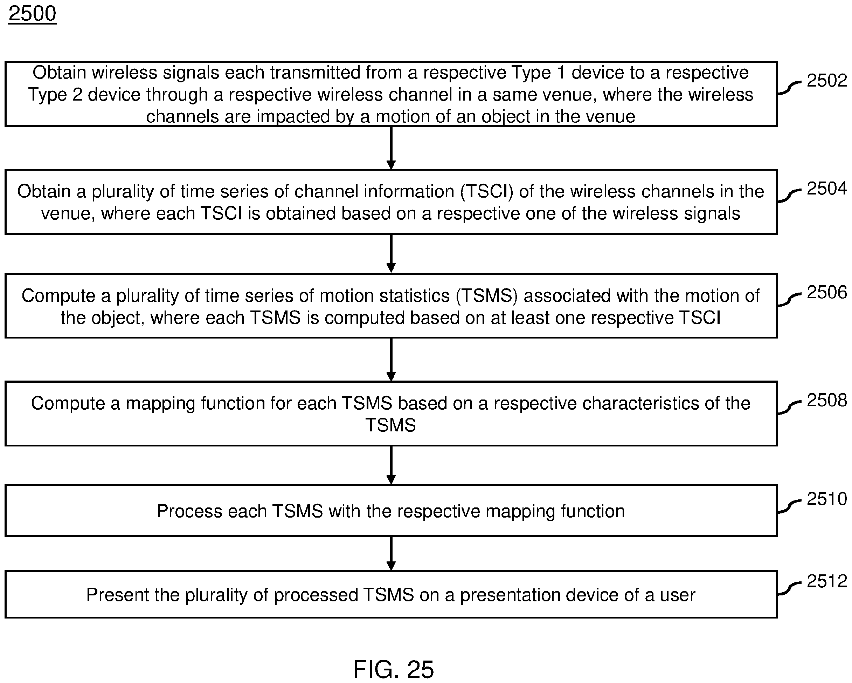

FIG. 25 illustrates a flow chart of an exemplary method 2500 of a wireless sensing presentation system, according to some embodiments of the present disclosure. In various embodiments, the method 2500 can be performed by the systems disclosed above. At operation 2502, the wireless sensing presentation system obtains wireless signals each transmitted from a respective Type 1 device to a respective Type 2 device through a respective wireless channel in a same venue, where the wireless channels are impacted by a motion of an object in the venue. At operation 2504, the system obtains a plurality of time series of channel information (TSCI) of the wireless channels in the venue, where each TSCI is obtained based on a respective one of the wireless signals.

At operation 2506, the system computes a plurality of time series of motion statistics (TSMS) associated with the motion of the object, where each TSMS is computed based on at least one respective TSCI. At operation 2508, a mapping function is computed for each TSMS based on a respective characteristic of the TSMS. At operation 2510, each TSMS is processed with the respective mapping function. At operation 2512, the plurality of processed TSMS are presented on a presentation device of a user. The order of the operations in FIG. 25 may be changed according to various embodiments of the present teaching.

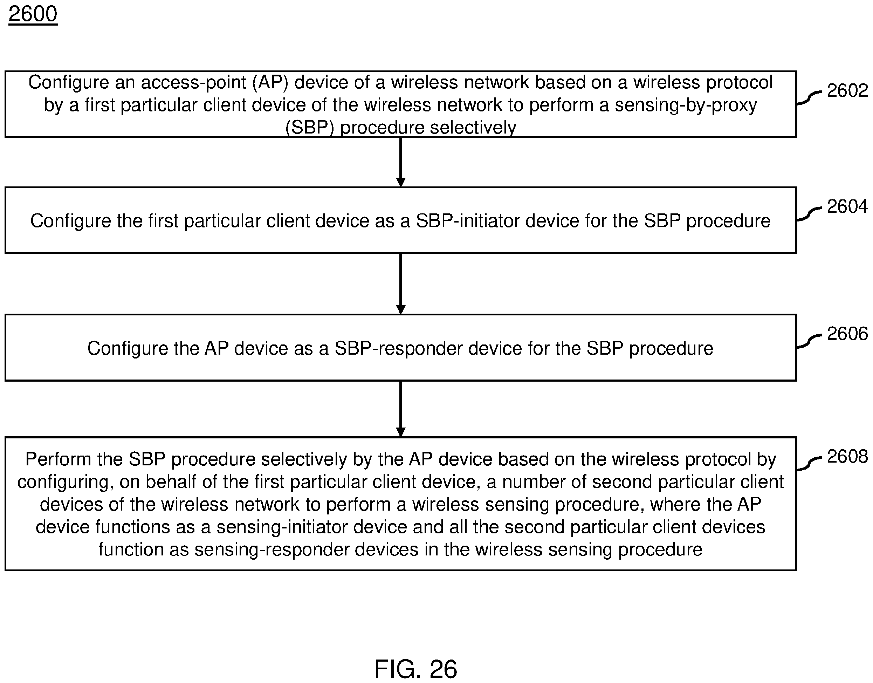

FIG. 26 illustrates a flow chart of an exemplary method 2600 for performing a selective sensing-by-proxy procedure, according to some embodiments of the present disclosure. In various embodiments, the method 2600 can be performed by the systems disclosed above. At operation 2602, an access-point (AP) device of a wireless network is configured based on a wireless protocol by a first particular client device of the wireless network to perform a sensing-by-proxy (SBP) procedure selectively. At operation 2604, the first particular client device is configured as a SBP-initiator device for the SBP procedure. At operation 2606, the AP device is configured as a SBP-responder device for the SBP procedure. At operation 2608, the SBP procedure is performed selectively by the AP device based on the wireless protocol based on configuring, on behalf of the first particular client device, a number of second particular client devices of the wireless network to perform a wireless sensing procedure, where the AP device functions as a sensing-initiator device and all the second particular client devices function as sensing-responder devices in the wireless sensing procedure. The order of the operations in FIG. 26 may be changed according to various embodiments of the present teaching.

Achieving indoor localization enables several intelligent and smart home applications, such as monitoring overall activities of daily living (ADL), smart lighting/temperature control, and triggering location-specific IoT devices. In addition, ADL information further facilitates physical and mental health monitoring and extracting valuable activity insights. WiFi-based solutions to this problem are widely appreciated due to their ubiquity and privacy protection. Current WiFi-based localization approaches either focus on fine-grained target localization demanding high calibration efforts or cannot localize multiple people at the coarser level, making them unfit for robust ADL applications. The present teaching discloses a robust WiFi-based room/zone-level localization solution that is calibration-free, device-free (passive), and built with commercial WiFi chipsets. A disclosed system can extract features, e.g. multi-modal information from WiFi Channel State Information (CSI), indicative of the motion and breathing patterns, thus detecting and localizing a person, pet or object even when there is only subtle physical movement, overcoming the drawbacks of several existing solutions such as the PIR.

In some embodiments, the extracted features from the CSI are compared from multiple receivers to pinpoint the receiver closest to the movement. Understanding the location of the WiFi receivers fixed in a venue can help to localize the movement. A disclosed system can also incorporate the challenging cases of localizing multiple people simultaneously at multiple locations. Furthermore, a disclosed system may use a correlation between the movement patterns, e.g. estimating the number of people in the environment using the correlation between movement patterns, to break ambiguous location scenarios. The disclosed systems can achieve a high average detection rate including different activity levels, and a high localization accuracy based on experiments performed across different environments.

One goal of the present teaching is to build a robust system to extract and log the daily living activities and to focus on coarse positioning, i.e., room-level/"zone-level". In some embodiments, a disclosed system uses a passive design to constantly localize the indoor movement of humans, and can localize and monitor the daily human activities based on multimodal information from WiFi with zero training efforts.

First, a robust presence detection approach is needed to localize a person. Presence detection has been studied in the past by capturing the temporal variations caused by the moving object. For this, time-domain features such as the CSI amplitude, the variance distribution of CSI amplitude, the variance of amplitude variance over subcarriers, eigenvalues of CSI amplitude and phase features are leveraged. In addition, periodic changes in the CSI induced by the human breathing patterns are also used to detect a stationary human. A disclosed system can identify human movements by extracting the first tap of the auto-correlation function (ACF) of the CSI, and further enhance the weak motion signal using maximum ratio combining (MRC). The latter features are robust against environment dependencies and provide insights into the extent of physical motion and the detection, adding more value to the ADL application.

Achieving robust indoor localization with WiFi poses several challenges/questions that will be addressed, including extracting the dynamic signal when the person has little to no physical movement, localizing multiple people simultaneously at multiple locations, making the system robust to location and environment changes.

Human presence is determined by analyzing the changes in the CSI caused by dynamic human-affected multipath. Higher the intensity of motion and proximity to the transceivers, more multipath are affected, resulting in detection with higher confidence. The issue arises when he/she has little to no physical motion in scenarios such as reading a book or sleeping. To identify small movements, a disclosed system may compute the autocorrelation of CSI and analyze changes up to a time lag of several seconds. The extended search window over the time lag enables the system to extract the accumulated changes over the previous few seconds, otherwise not evident from the current CSI. When there are no bodily movements, human breathing induces periodic changes to the indoor multipath, seen as peaks in the ACF.

Multiple people localization has been a challenging task given the complicated interaction of multipath and the low resolution of WiFi. The multiple receiver setups handle part of the problem. With different people in different rooms having a receiver (Rx), each can see the changes induced to the CSI by respective human motion. Separated motion detection for each Rx can thus determine the locations at which people are moving/present. However, one person could be present in a location that affects much multipath received by multiple receivers. If different Rx performs separate motion detection, this scenario is interpreted as multiple users present at multiple Rx locations. Identifying the number of independent motion sources in the environment thus becomes necessary. When the source of motion is the same, the trend of the motion intensities seen by multiple Rx will be the same, which can be determined by the correlation of the motion features.

In some embodiments, a disclosed system does not rely on any location-specific fingerprints. Instead, the disclosed system can extract the degree of changes induced by human motion in the CSI at each Rx and use the relative strength and patterns to estimate the probability and number of active locations. The system aims to predict the Rx, which is closest to the source of motion, rather than providing the exact location of the user, which makes it robust to location and environment changes.

As explained above, the disclosed system can achieve robust indoor localization without training and calibration. It addresses the major challenges faced by the existing WiFi-based indoor localization works. The disclosed system provides a novel and complete pipeline for a WiFi-based whole-home ADL localization using a multiple receiver setting. Moreover, unlike most existing works, the disclosed system does not require re-calibration when transferred to a new environment and is trainingfree. The disclosed system can estimate the user's location even if he/she has very low (reading a book, watching TV) to no motion (sleeping, meditating). The disclosed system may include a micro-motion enhancer module that amplifies even subtle bodily movements enabling localization. The disclosed system can address the issue of multiple active locations/multiple people localization. To remove location ambiguities from involving multiple people, the disclosed system can estimate the number of people/sources of motion in the environment.

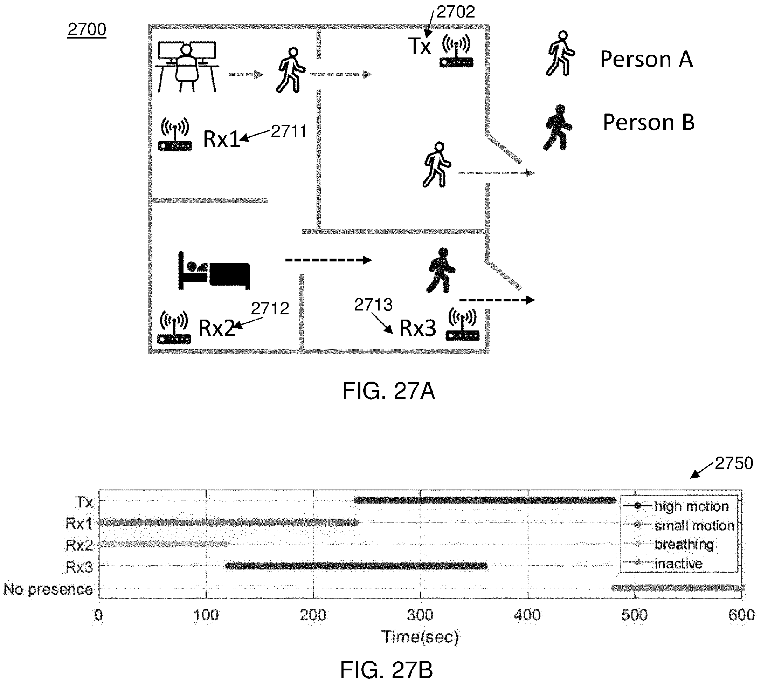

FIG. 27A shows an exemplary environment 2700 for motion monitoring and localization, according to some embodiments of the present disclosure. In the environment 2700, two people, persons A and B, move across rooms with time and perform different activities simultaneously and leave the venue one after other. While a transmitter (Tx) 2702 is placed in one room, three different receivers, Rx1 2711, Rx2 2712, Rx3 2713, are placed in three other rooms respectively. Each of the receivers can receive a wireless signal transmitted by the Tx 2702, via a multipath channel. The wireless signal is impacted by motions of person A and person B, while the persons are in any room of the venue. A disclosed system can compute CSI time series based on the received wireless signals, and extract different features from the CSI time series. The activity logs of the two persons can be extracted by the disclosed system based on the different features extracted from the CSI time series, as shown in chart 2750 in FIG. 27B and Table I below.

Table I

| Person A | Time | 0-2 min | 2-4 min | 4-6 min | 6-8 min | 8-10 min |
|---|---|---|---|---|---|---|
| Person B | **Person A** | Rx1 | Rx1 | Tx | Tx | Outside |
| | **Person B** | Rx2 | Rx3 | Rx3 | Outside | Outside |

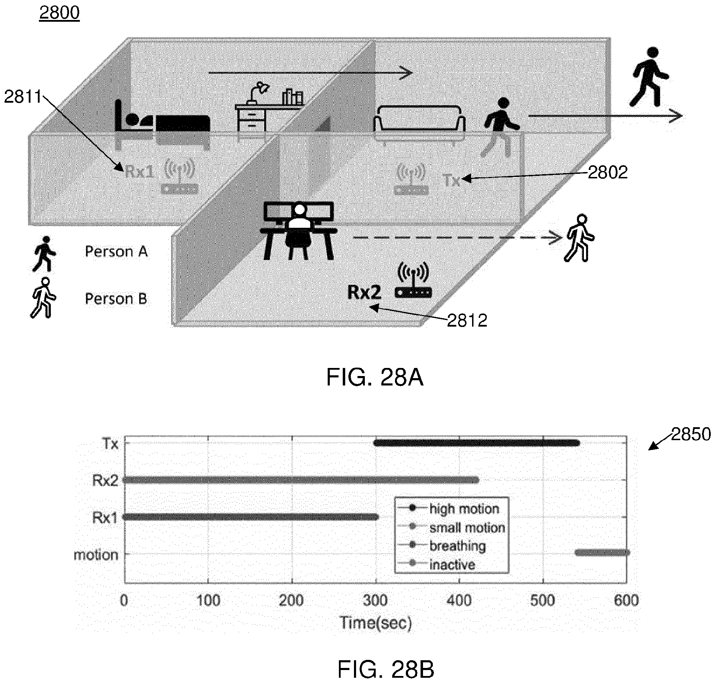

FIG. 28A shows an exemplary another environment 2800 for motion monitoring and localization, according to some embodiments of the present disclosure. In the environment 2800, two people, persons A and B, move across rooms with time and perform different activities simultaneously and leave the venue one after other. While a transmitter Tx 2802 is placed in one room, two different receivers, Rx1 2811, Rx2 2812, are placed in two other rooms respectively. Each of the receivers can receive a wireless signal transmitted by the Tx 2802, via a multipath channel. The wireless signal is impacted by motions of person A and person B, while the persons are in any room of the venue. A disclosed system can compute CSI time series based on the received wireless signals, and extract different features from the CSI time series. The activity logs of the two persons can be extracted by the disclosed system based on the different features extracted from the CSI time series, as shown in chart 2850 in FIG. 28B and Table II below. In various embodiments, the number of transmitters and the number of receivers can be changed to form multiple device pairs, each having a respective transmitter and a respective receiver.

Table II

| Time | Person A | Person B |
|---|---|---|
| 0-5 min | Rx1 | Rx2 |
| 5-7 min | Tx | Rx2 |
| 7-9 min | Tx | Outside |
| 9-10 min | Outside | Outside |

In some embodiments, a disclosed system utilizes the CSI time series from the commercial WiFi chipsets for localization. CSI characterizes the WiFi signal propagation from the Tx to the Rx and profiles a specific state of the wireless multipath environment. The system can leverage the statistical characteristics of the CSI and perform localization by continuously capturing and monitoring the CSI from commercial WiFi chipsets.

Based on the superposition properties of the electromagnetic fields, the received CSI over frequency f at time t can be decomposed into a static part and a dynamic part, as

$$H(t, f) = H_s(t, f) + \sum_{i \in \Omega_d(t)} H_i(t, f). \tag{1}$$

The static part is contributed by the reflections by the stationary objects while the dynamic part is contributed by the set of moving scatterers $\Omega_d(t)$. Since raw CSI suffers from synchronization errors and phase distortions, the system may only exploit the power response of CSI measurement, defined as

$$G(t, f) \triangleq |H(t, f)|^2 = \xi(t, f) + n(t, f), \tag{2}$$

where $\xi(t,f)$ denotes the power of the transmitted signals and $n(t, f)$ is the additive white noise. $\xi(t,f)$ and $n(t, f)$ are independent of each other as they are contributed by independent sources.

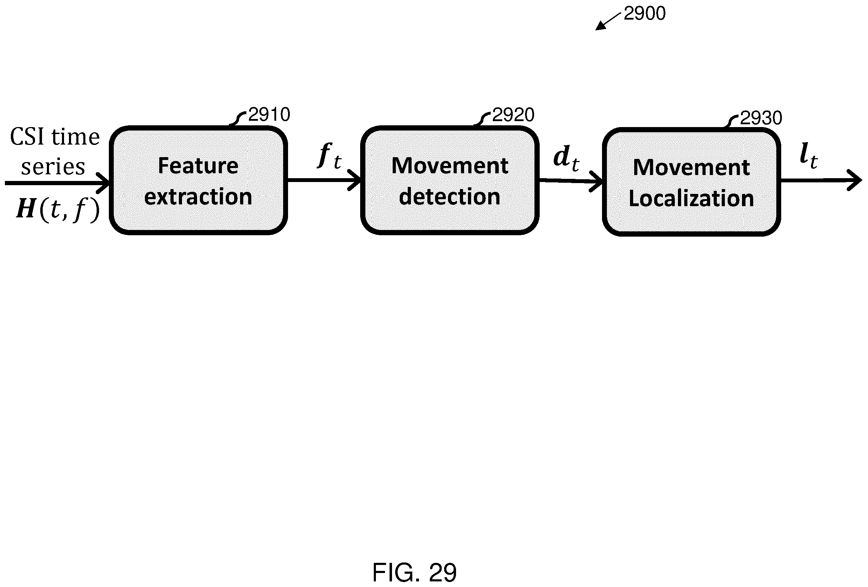

From the power response $G(t,f)$, the system may extract different features that can detect and localize movements in an indoor environment. In some embodiments, the disclosed system comprises three modules: feature extraction, movement detection, and movement localization. FIG. 29 shows an exemplary diagram of a wireless system 2900 for motion monitoring and localization, according to some embodiments of the present disclosure. As shown in FIG. 29, the wireless system 2900 includes a feature extraction module 2910 configured to extract features f_t based on CSI time series obtained from wireless signals; a movement detection module 2920 configured to detect motions, e.g. by generating motion information d_t based on the extracted features; and a movement localization module 2930 configured to determine locations for detected motions, e.g. by generating locations information 1_t based on some correlation scores. Each of these modules will be discussed in detail below.

Feature extraction

Human indoor activities can be either dynamic or quasi-static. Dynamic activities include large body level motion such as walking, sitting down and exercising. Quasi-static movements include activities such as reading and sleeping, during which the intensity of movement is light, thereby creating much lesser disturbance to the wireless multipath propagation. For subtle motion, although the instantaneous movement is negligible, the changes to the multipath propagation are accumulated over time, thus allowing for movement detection when observing the changes over a time window spanning few seconds. However, when a person is sleeping/meditating, the dynamic signal could be very weak. In such cases, the system can leverage the periodic nature of the chest movement during breathing which can be extracted using signal processing techniques. Based on these observations, one can derive three CSI-based features from Auto-Correlation Function (ACF) of CSI power response to detect the dynamic status and the quasi-static status. The ACF can be derived as:

$$\rho_G(\tau, f) = \frac{\sigma_\xi^2(f)}{\sigma_\xi^2(f) + \sigma_n^2(f)} \rho_\xi(\tau, f) + \frac{\sigma_n^2(f)}{\sigma_\xi^2(f) + \sigma_n^2(f)} \delta(\tau), \tag{3}$$

where $\tau$ is the time lag of the ACF, $\sigma_\xi^2(f)$ and $\sigma_n^2(f)$ are the variances of $\xi(t,f)$ and $n(t, f)$, respectively, $\rho_\xi(\tau, f)$ and Dirac delta function $\delta(\tau)$ are the ACFs of $\xi(t,f)$ and $n(t, f)$.

To detect the high motion, like people's walking, running, the system can use motion statistics defined as $\lim_{\tau\to0} \rho_G(\tau,f)$. More specifically, in Equation (3), as $\tau \to 0$, one can have $\delta(\tau) = 0$ due to the whiteness of the additive noise. If motion is present in the propagation coverage of the WiFi devices, the multipath propagation is disturbed and the signal variance $\sigma_\xi^2(f) > 0$. Since $\lim_{\tau\to0} \rho_\xi(\tau,f) = 1$ due to the continuity of motion, the limit of $\rho_G(\tau,f)$ will be a positive value, i.e., $\lim_{\tau\to0} \rho_G(\tau,f) > 0$. If there is no motion, the environment is static and the variance $\sigma_\xi^2(f) = 0$, which suggests that $\lim_{\tau\to0} \rho_G(\tau,f) = 0$. Therefore, $\lim_{\tau\to0} \rho_G(\tau,f)$ is a good indicator of the presence of motion. In practice, a system can use the first lag of ACF of CSI power response to approximate $\lim_{\tau\to0} \rho_G(\tau,f)$ due to the finite sampling rate and define the high motion feature as

$$\phi^h \triangleq \frac{1}{K}\sum_{k=1}^{K} \rho_G\left(\tau = \frac{1}{F_s}, f_k\right), \tag{4}$$

where $F_s$ is the sampling rate and $f_k$ is the frequency at k-th subcarrier. As Equation (4) illustrates, $\phi^h$ is an average of $\lim_{\tau\to0} \rho_G(\tau,f)$ over the total K subcarriers.

Although $\phi^h$ can capture large movements in the environment, it could miss detecting weak/subtle motions (e.g. people's reading or sleeping) due to farther distance from the transceivers, noise in the environment, or transient motions. Therefore, to amplify the subtle changes in the ACF spectrum caused by small motion, the system can leverage the frequency diversity and utilize the maximum ratio combining (MRC) scheme to maximize the signal-noise-ratio (SNR) of the ACF. Since not all subcarriers are sensitive to the slight motion, the system may select the top N subcarriers with the largest $\phi^h$ for MRC and acquire the boosted ACF $\hat{\rho}_G(\tau,f)$. The small motion feature $\phi^s$ is then defined as the limit of $\hat{\rho}_G(\tau,f)$ with $\tau \to 0$, i.e.,

$$\phi^s \triangleq \frac{1}{N}\sum_{k=1}^{N} \hat{\rho}_G\left(\tau = \frac{1}{F_s}, f_k\right). \tag{5}$$

In some embodiments, the small motion feature $\phi^s$ can be defined as the maximum differential of boosted ACF $\hat{\rho}_G$ over $\tau$ as $\phi^s \triangleq \max_\tau(|\Delta\hat{\rho}_G(\tau)|)$.

When there is not much physical movement in the environment, and when neither $\phi^h$ nor $\phi^s$ is substantial, the system can rely on the human vital signs to detect presence. Chest movement caused by breathing is significant compared to other vital signs such as heart rate, which might be too weak to be detected by the WiFi signal. The breathing motion is periodic, introducing periodic changes to the multipath signal propagation. Such a periodic change is evident from the ACF spectrogram as peaks at the corresponding time lag resulting from the high correlation of the CSI power. The breathing rate i.e., the breathing feature $\phi^b$ can be derived as,

$$\phi^b \triangleq \frac{60}{\hat{\tau}} \tag{6}$$

beats per minute (BPM), where $\hat{\tau}$ is the time lag of the first peak in $\hat{\rho}_G(\tau)$.

Movement detection

Given the features extracted from the CSI time series, the next step is to perform detection by determining a suitable threshold. Knowing that the theoretical threshold for $\phi^h$ is 0, the system can use a threshold of $\gamma_h = 0.1$, allowing for a noise margin. The threshold for small movement detection is determined empirically by observing quiet environments without human presence in several different environments, obtained as $\gamma_s = 0.2$. In some embodiments, the threshold for small movement detection is determined as $\gamma_s = 0.0002$, the breathing rates below 10BPM($\gamma_{1b}$) and above 30 BPM ($\gamma_{hb}$) are discarded and considered out of the human breathing range.

At any given time instance, the system can first check for high motion. When a high movement is not detected at the current instance, the system may check for weak and breathing motion only if a significant motion ($\phi^h > \gamma_{hh} = 0.5$) is detected at least once in the last $W_T$ seconds. A conditional check on the weak movement patterns will help alleviate false detections.

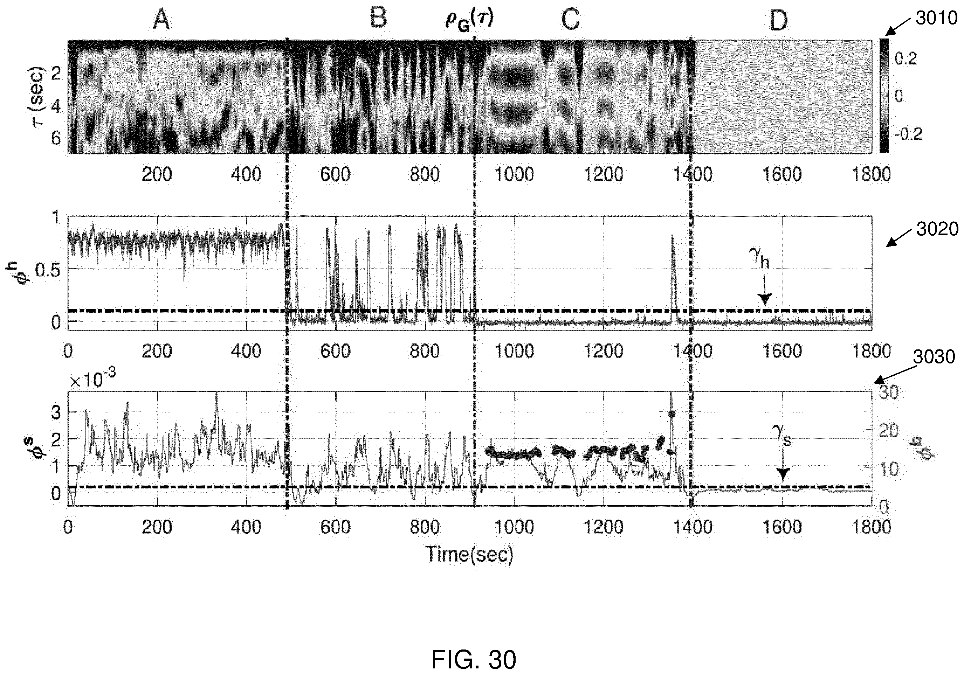

FIG. 30 shows an example of different levels of features extracted from CSI time series. Plot 3010 shows ACF of the CSI power, plot 3020 shows a high motion feature, and plot 3030 shows a small motion feature overlaid with the breathing rate estimates. Dotted horizontal black lines indicate the thresholds for high ($\gamma_h$) and small ($\gamma_s$) motions. In this experiment, a person makes a high-intensity motion for time duration A, small intermittent motions in time duration B, followed by sleeping in the time duration C, and then leave the test area in time duration D.

As shown in FIG. 30, for the time duration A of high-intensity motion, $\phi^h$ is much higher than the threshold $\gamma_h$ and so is $\phi^s > \gamma_s$. For the time duration B of weaker intermittent motion, one can see that using $\phi^s$ can help if high motion is not detected. For the time duration C, the person was sleeping, which was picked up by breathing and $\gamma_s$. In cases of farther distance from the transceiver, $\phi^s$ could be less than $\gamma_s$ while still picking up $\phi^b$. Finally, the user leaves the place for the time duration D, and all the features become insignificant.

### Movement localization

After detecting a movement in the environment, the next step is to assign it to the correct location. The possible locations for movement are at the Rx for which at least one of the movement features is above their respective thresholds. However, when designing a more general system that can include multiple users and various types of device topologies, the system may do more than simple thresholding. Below are a few examples for potential location ambiguities.

In a first example, due to the broad coverage of the WiFi signal, a single person could affect enough multipath to induce high motion statistics $\phi^h$ in more than one Rx. Using a threshold on the motion statistics $\phi^h$ will cause inaccurate location estimates in such scenarios.

In a second example, a person near the Tx can create high $\phi^h$ in all the Rx due to the reciprocity of the WiFi channel. It becomes challenging to differentiate between one person moving near the Tx and multiple people moving at all Rx.

To resolve the above ambiguities, the system can determine if the movement features induced in any given two Rx are from a single body in the environment. Motion statistics $\phi^h$ can indicate the relative motion intensity at a given distance from the transceivers. When two Rx "see" a single motion source, the duration of high and low motion and the transitions between them are "correlated." On the other hand, the pattern of the increase and decrease in the $\phi^h$ is not the same when two Rx have stronger dynamic signals from different motion sources.

In some embodiments, one can observe the correlation of $\phi^h$ over a window ($W_c$) to determine if two Rx are affected/activated by the same motion source. Denoting the high motion features of two Rxi and j by $\Phi_i^h$, and $\Phi_j^h$ respectively, the correlation $C_{ij}$ is calculated as follows:

$$C_{ij}(t) = \frac{\sum_{x=t-W_c+1}^{t} (\phi_{i,x}^h - \bar{\phi}_i^h)(\phi_{j,x}^h - \bar{\phi}_j^h)}{\|\phi_i^h - \bar{\phi}_i^h\| \cdot \|\phi_j^h - \bar{\phi}_j^h\|}, \tag{7}$$

where $\|.\|$ operation denotes the norm of the vector and $\bar{(.)}$ denotes the mean operator.

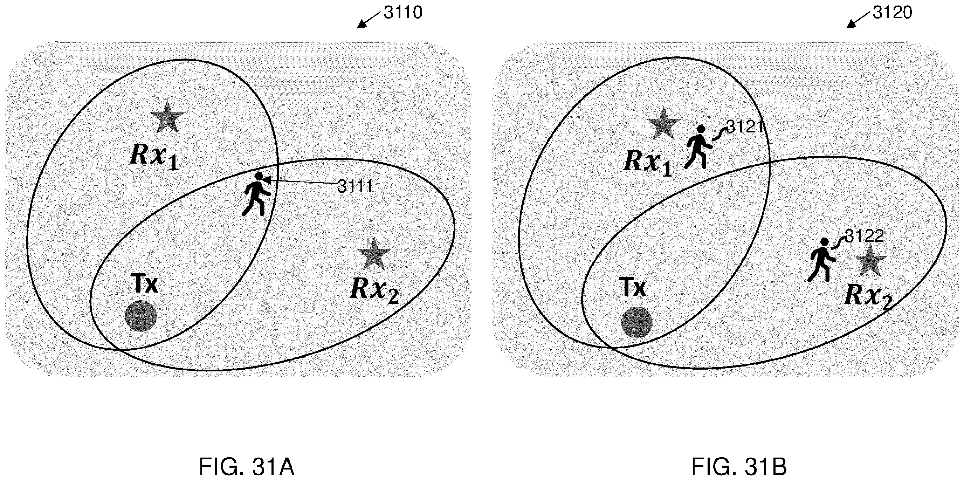

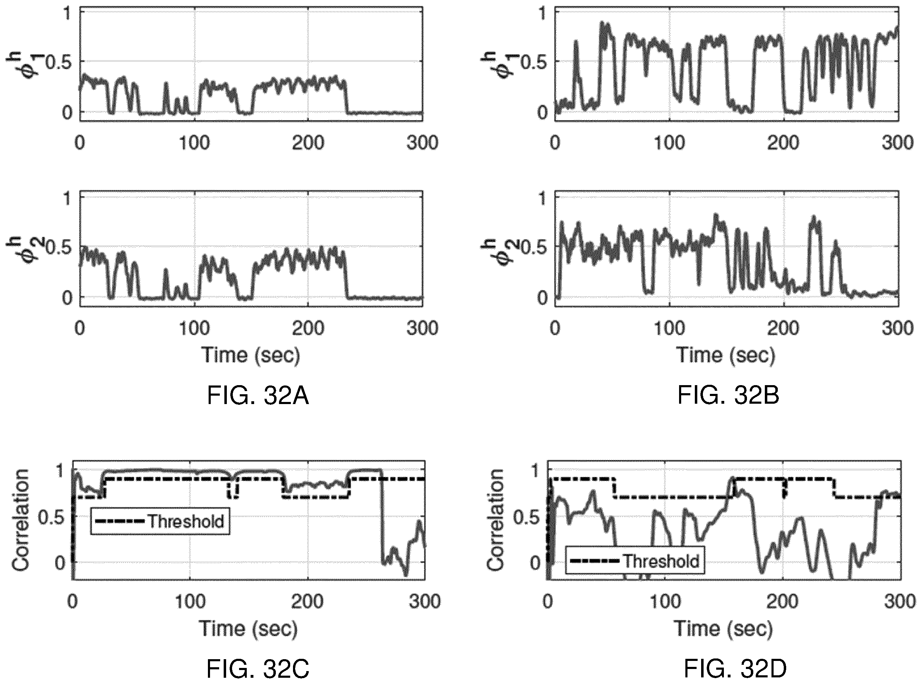

Following is an example of using $C_{ij}$ to solve ambiguous localization scenarios discussed before. Consider the two device setups and movement locations as shown in FIG. 31A and FIG. 31B, respectively. FIG. 32A and FIG. 32B show motion statistics $\phi^h$ from $Rx_1$ and $Rx_2$ in scenario 3110 (1 person) of FIG. 31A and scenario 3120 (2 persons) of FIG. 31B, respectively. FIG. 32C and FIG. 32D show correlation of $\phi^h$ from $Rx_1$ and $Rx_2$ and the correlation threshold in scenario 3110 (1 person) of FIG. 31A and scenario 3120 (2 persons) of FIG. 31B, respectively.

In scenario 3110 shown in FIG. 31A, motion from Person 1 3111 resulted in high $\phi^h$ both in $Rx_1$ and $Rx_2$. However, the trend of the $\phi^h$ is the same in both. On the other hand, $\phi^h$ calculated by $Rx_1$ and $Rx_2$ for persons 3121, 3122, in scenario 3120 shown in FIG. 31B shows a different pattern with time. The amount of similarity is evident from the correlation metric shown in FIG. 32C and FIG. 32D. The dashed black lines in FIG. 32C and FIG. 32D show the correlation threshold used in this example. The correlation between two motion statistics vectors in a window is high when they follow a similar trend of increasing and decreasing patterns. However, when the motion is continuously high, there are no distinct patterns in the motion statistics, and the correlation is not very high. The minor variations in the values could result from a different multipath environment, as seen by the two receivers. Therefore, the system can relax the correlation threshold ($C_{th} = 0.7$) if the $\phi^h$ are higher than $\gamma = 0.1$ for at least 95% of the window time (30 sec). The system can use correlation for motion localization only when a high motion is detected. In some embodiments, the person does not change locations for smaller motion and breathing durations as the walking motion can be captured by the high motion feature during location transitions.

In some embodiments, the steps for movement detection and movement localization are given in Algorithm 1 and Algorithm 2, respectively.

---

**Algorithm 1** Motion detection

1: **if** $\phi^h(t) > \gamma_{hh}$ **then** $d_i^{hh}(t) = 1$
2: **if** $\phi^h(t) > \gamma_h$ **then** $d_i^h(t) = 1$
3: **if** $d_i^h(t) == 0$ & $\text{any}(d_i^{hh}(t - W_T + 1 : t)) = 1$ **then**
4:     **if** $\phi^s(t) > \gamma_s$ **then** $d_i^s(t) = 1$
5:     **if** $\gamma_{bl} < \phi^b(t) < \gamma_{bh}$ **then** $d_i^b(t) = 1$
6: **end if**

---

**Algorithm 2** Motion Localization

$\hat{\phi}_i^h = \frac{1}{W_l} \sum \phi_i^h(t - W_l + 1 : t)$
2: $v^h = \{i/d_i^h(t) = 1\}$
    Sort $v^h$ in decreasing order of $\hat{\phi}_{(}^h.) \to \bar{\alpha}(t)$
4: $\bar{l}(t) = \bar{\alpha}[1], \bar{\beta}(t) = [1], \bar{\alpha}(t) = \bar{\alpha}[2 : end]$
    **for** i in $\bar{\alpha}(t)$ **do**,
6:     **for** j in $\bar{l}(t)$ **do**
        **if** $C_{ij} < \gamma_c$ **then**,
8:         $\bar{l}(t) = [\bar{l}(t), \quad i]$
        **else**
10:         Replace j with i if $\hat{\phi}_j^h < \hat{\phi}_i^h$
        $\bar{\beta}(t)[\bar{l}(t) == j || \bar{l}(t) == i] + = 1$
12:         **end if**
      **end for**
14: **end for**
    $v^s = \{i/d_i^s(t) = 1 || d_i^b(t) = 1\}$
16: Sort $v^h$ in decreasing order of $\hat{\phi}_{(}^h.) \to \bar{\alpha}(t)$
    **if** $\bar{l}(t)$ is empty **then** $\bar{l}(t) = \alpha[1], \bar{\alpha}(t) = (\bar{t})[2 : end]$
18: Repeat steps 5-14 with $\phi^s$ instead of $\phi^h$.
    **if** All bots detect motion/breathing **then**
20:     $\implies$ potential $Tx$ location.
    $\bar{l}(t)[\bar{\beta}(t) == max\{\bar{\beta}(t)\}] = Tx$
22: **end if**
    return $\bar{l}(t)$

---

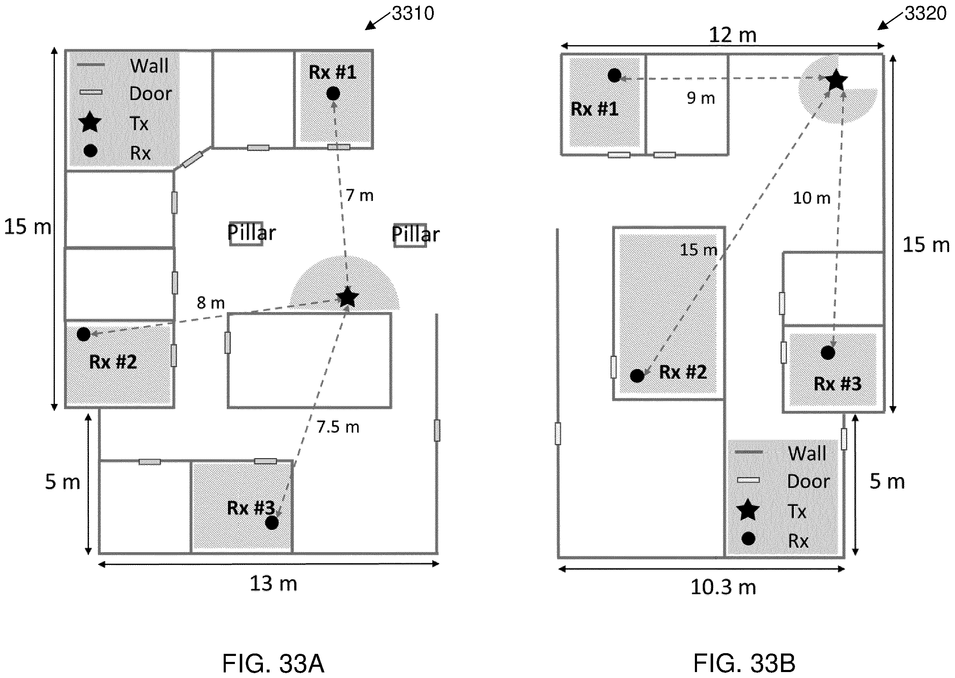

In some examples, the performance of the disclosed system is evaluated in two different device setups and environments 3310, 3320 as shown in FIG. 33A and FIG. 33B respectively. The metrics used for evaluation are the detection rate (DR) for movement detection and localization accuracy. DR for movement detection is the percentage of the time a movement is detected out of the total time of human presence in the monitoring area. Localization accuracy is the percentage of time in which the disclosed system correctly identifies the location of motion out of the total time of movement detection.

In some examples, a prototype is built on commercial WiFi chipsets and uses a sampling rate of 200 Hz for CSI collection. The system operates on 5GHz WiFi with a 40MHz bandwidth and includes a $2 \times 2$ MIMO antenna. In all the experiments, the users maintained a distance of at least 1 m from the respective Tx/Rx. Closer movement is detected even better. In addition, the system does not know about the number of users in the environment beforehand, which is estimated by the system using correlation.

In experiments for one-person scenarios, only one person was in the environment performing different levels of motion at different Rx and at the Tx. The average presence detection rate is 99.72% and 99.62% in setup one and two, respectively. Table III shows a summary of the test cases that include different physical activity levels. In Table III, "high" indicates walking motion, "small" refers to low-intensity movements such as sitting at a desk and working on a laptop, and "sleep" scenario involves taking a nap in a chair. In the localization step, the system can use correlation only when the motion is high using the high motion feature $\phi^h$. When a small motion or breathing is detected, the system can just perform detection using thresholding and continue with the previous location results. In some embodiments, a high motion is triggered when the person transitions between locations. The average localization accuracy when the person is at the Rx is 100%, and the Tx is 92.26%.

Table III: Presence detection in 1-person scenario

| Location | Activity | S1-DR(%) | S2-DR(%) |
|---|---|---|---|
| Rx | High | 100 | 100 |
| | Small | 100 | 98.43 |
| | Sleep | 100 | 100 |
| Tx | High | 100 | 100 |
| | Small | 98.32 | 99.3 |
| | Sleep | 100 | 100 |

In experiments for two-person scenarios, two people performed different levels of physical activities at Tx and Rx, as shown in Table IV. The average presence detection rate is 99.19% and 98.53% in setup one and two, respectively. The average localization accuracy when two people are present at different Rx is 100% and when one person is at the Tx and the other at one of the Rx is 87%. A lower localization accuracy for the latter case is because a high motion at the Tx dominates the motion in all the Tx - Rx links, occasionally masking movements at the Rx. One approach to improve the performance in such cases is to use "multiway-sensing."

Table IV: Presence detection in 2-person scenario

| Loc1 | Loc2 | Activity1 | Activity2 | S1-DR(%) | S2-DR(%) |
|---|---|---|---|---|---|
| $Rx_i$ | $Rx_j$ | High | High | 100 | 100 |
| | | High | Small | 100 | 100 |
| | | High | Sleep | 100 | 100 |
| | | Small | Small | 100 | 99.2 |
| | | Small | Sleep | 97.3 | 100 |
| | | Sleep | Sleep | 100 | 98.2 |
| Tx | $Rx_j$ | High | High | 100 | 94.3 |
| | | High | Small | 100 | 100 |
| | | High | Sleep | 100 | 100 |
| | | Small | High | 100 | 100 |
| | | Small | Small | 99.93 | 99.2 |
| | | Small | Sleep | 99.56 | 91 |
| | | Sleep | High | 100 | 100 |
| | | Sleep | Small | 91.2 | 99.1 |
| | | Sleep | Sleep | 100 | 97 |

Various embodiments of the present teaching disclose a passive indoor localization system built upon commercial WiFi. An improved human presence detection pipeline is used to detect even minute physical human movements. The disclosed micro motion statistics and breathing rate estimation can detect even the weakest physical movements that cannot be detected by the most widely used sensors today. Further, the disclosed correlation-based approach has addressed localization ambiguities that arise in the case of multiple people. As a result, the system can achieve a localization accuracy of 98.5% in single-person scenarios and 93.5% in the case of multiple people over experiments conducted in two different environments and setups. Overall, the present teaching discloses a robust, low-cost, easy-to-use localization solution that can provide reliable human activity logs of daily life. Such information is invaluable to several potential innovative smart home and health care applications.

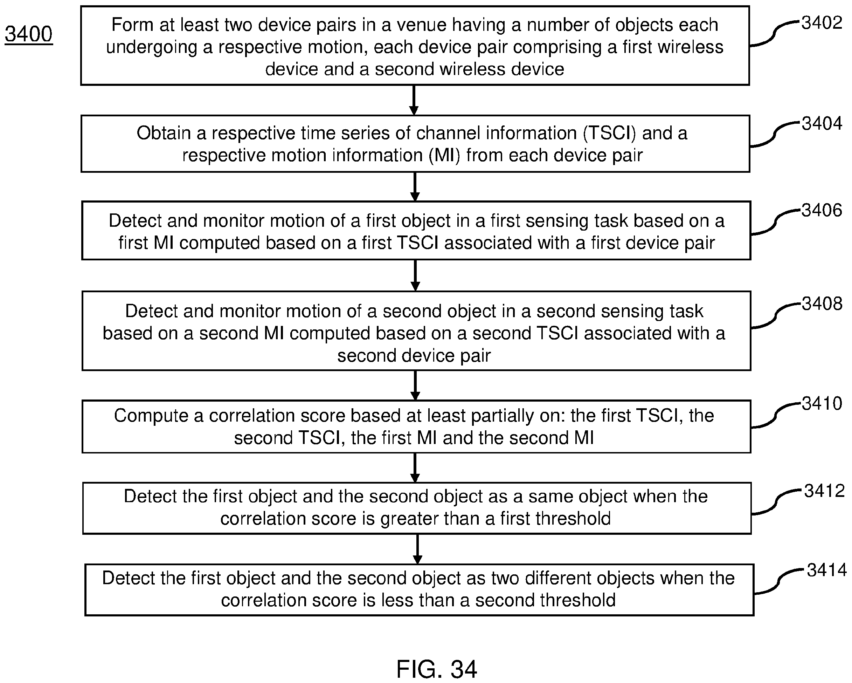

FIG. 34 illustrates a flow chart of an exemplary method 3400 for wireless monitoring and localization, according to some embodiments of the present disclosure. In various embodiments, the method 3400 can be performed by the systems disclosed above. At operation 3402, at least two device pairs are formed in a venue having a number of objects each undergoing a respective motion, each device pair comprising a first wireless device and a second wireless device. At operation 3404, a respective time series of channel information (TSCI) and a respective motion information (MI) are obtained from each device pair. At operation 3406, motion of a first object is detected and monitored in a first sensing task based on a first MI computed based on a first TSCI associated with a first device pair. At operation 3408, motion of a second object is detected and monitored in a second sensing task based on a second MI computed based on a second TSCI associated with a second device pair. At operation 3410, a correlation score is computed based at least partially on: the first TSCI, the second TSCI, the first MI and the second MI. At operation 3412, the first object and the second object are detected as a same object when the correlation score is greater than a first threshold. At operation 3414, the first object and the second object are detected as two different objects when the correlation score is less than a second threshold.

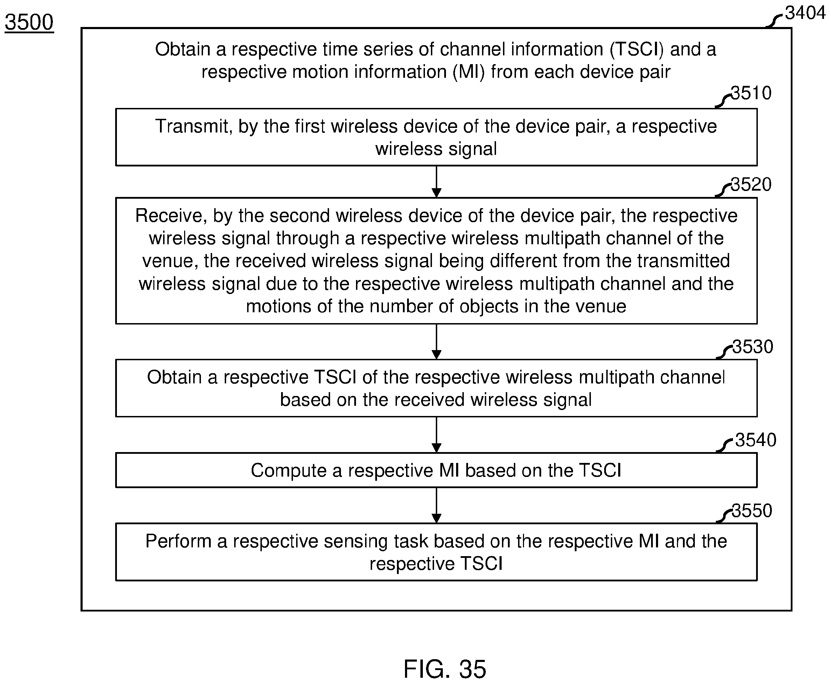

FIG. 35 illustrates a flow chart showing detailed operations 3500 for wireless monitoring and localization, according to some embodiments of the present disclosure. In some embodiments, the operations 3500 may be performed by each device pair as part of the operation 3404 in FIG. 34. At operation 3510, a respective wireless signal is transmitted by the first wireless device of the device pair. At operation 3520, the respective wireless signal is received by the second wireless device of the device pair through a respective wireless multipath channel of the venue, the received wireless signal being different from the transmitted wireless signal due to the respective wireless multipath channel and the motions of the number of objects in the venue. At operation 3530, a respective TSCI of the respective wireless multipath channel is obtained based on the received wireless signal. At operation 3540, a respective MI is computed based on the TSCI. At operation 3550, a respective sensing task is performed based on the respective MI and the respective TSCI.

The order of the operations in any one of the drawings may be changed according to various embodiments of the present teaching.

In some embodiments, there may be at least one wireless network (e.g. WiFi, WLAN, IEEE 802.11/11n/11ac/11ax/ 11be/11bf, 4G/LTE/5G/6G/7G/8G, mesh network, IEEE 802.15/16, Bluetooth, WiMax, Zigbee) in a venue comprising an access point(AP)/router/mesh router/hub/base station for each wireless network and a number of heterogeneous wireless devices each in a respective wireless network. A disclosed system/device/method/software can comprise multiple pairs of Type1 (TX) devices and Type2 (RX) devices in a many-to-many configuration. Any Type1 device and/or Type2 device may be any of the access point(AP)/router/mesh router/hub/base station of the at least one wireless network, or any of various heterogeneous wireless devices in the wireless network. In some embodiments, some or all of the multiple Type1 (TX) devices may be a common device (e.g. the AP/router/mesh router/hub/base station) such that the associated pairs of TX/RX form a one-to-many configuration. In some embodiments, some or all of the multiple Type2 (RX) devices may be a common device (e.g. the AP/router/mesh router/hub/base station) such that the associated TX/RX pairs form in a many-to-one configuration.

In some embodiments, for each pair of Type1 (TX) device and Type2 (RX) device, a respective wireless signal (e.g. sounding signal, NDP, NDPA, TF) is transmitted from the respective TX to the respective RX through a respective wireless multipath channel in the venue. The respective received wireless signal differs from the respective transmitted wireless signal due to the respective wireless multipath channel of the venue and motion of a number (e.g. unknown number) of objects (e.g. people, users, pets, machines) in the venue. A respective time series of channel information (TSCI) of the respective wireless channel is obtained.

In some embodiments, a respective sensing task associated with the respective pair of TX and RX is performed based on the respective TSCI to monitor the motion of the objects. The task may comprise one or more subtasks. The task or subtask may comprise any of: motion detection/estimation, presence detection, breathing detection/estimation, heart beat detection/estimation, fall-down detection, tracking, sleep monitoring, pet monitoring, well-being monitoring, activity of daily living (ADL) monitoring, etc. A respective motion information (MI)/motion statistics (MS)/characteristics/spatial-temporal information (STI) may be computed based on the respective TSCI. The respective sensing task or an associated subtask may be performed based on the respective MI/MS/characteristics/STI. For example a first task or subtask (e.g. sleeping monitoring) may be performed based on a first MI/MS/characteristics/STI computed based on a first TSCI obtained from a first wireless signal transmitted from a first TX to a first RX. A different task or subtask (e.g. activity of daily living or ADL) maybe performed based on a second MI/MS/characteristics/STI computed based on a second TSCI obtained from a second wireless signal transmitted from a second TX to a second RX. There may/may not be a common/same task or subtask (e.g. motion detection) performed in all pairs of TX and RX.

In some embodiments, a common MI/MS/STI/characteristics may be computed based on each of the TSCI such that the common MI/MS/STI/characteristics of the multiple TX/RX pairs may be compared. For each TX/RX pair, the respective common MI/MS/STI/characteristics computed based on the respective TSCI may be used to perform the respective sensing task (e.g. the common/same task or subtask). The amount of the objects may be unknown. There may be zero, one, two, or more objects. The location of the number of objects in the venue may be unknown. In other words, there may be an unknown number of objects at unknown locations undergoing respective motion (e.g. some may be stationary/nonstationary/moving/walking/running/exercising/sleeping/working/talking, moving quickly/slowly/regularly/irregularly/intermittently, etc.).

In some embodiments, the disclosed system/device/method/software may detect/acquire/capture/monitor motion of the objects based on any/some/all of the TSCI and locate/count/track the objects (e.g. one or some or all) based on the detected/monitored motion. For each pair of TX and RX, motion may/may not be detected/acquired/captured/monitored based on the respective TSCI. For example, the motion of an object far away from (or beyond) an effective range/neighborhood of the pair of TX/RX may not be detected/acquired/captured/monitored by the pair of TX/RX and the respective TSCI. As a result, some motion (i.e. some motion of some object(s)) may be detected/acquired/captured/monitored in some pairs of TX/RX while no motion may be detected/acquired/captured/monitored in some other pairs of TX/RX.

In some embodiments, any detected/acquired/captured/monitored motion by a pair of TX/RX (based on the respective TSCI) may be associated with one or more objects (e.g. unknown objects, or known objects). Typically, it is more likely to be associated with one object (instead of more than one objects). In the case of more than one objects whose motions are detected/acquired/captured/monitored by the pair of TX/RX, the more than one objects may be near each other such that they are "located" at the "same location" (i.e. same zone/region/neighborhood in the venue).

The venue may be partitioned into multiple zones (or regions/neighborhoods/partitions/ divisions) in the venue, each zone associated with either a Type1 device (TX), or a Type2 device (RX), or a pair of TX/RX. The zones may/may not overlap. A device may function as TX in a first TX/RX pair, as TX in a second TX/RX pair, as RX in a third TX/RX pair and as RX in a fourth TX/RX. In this case, there may be four zone associated with the device, a first zone for its role as TX in the first TX/RX pair, a second zone for its role as TX in the second TX/RX pair, a third zone for its role as RX in the third TX/RX pair and a fourth zone for its role as RX in the fourth TX/RX pair.

In some embodiments, a first motion (associated with one or more first particular objects) may be detected/acquired/ captured/monitored by a pair of first particular TX and first particular RX in a first sensing task based on an associated first TSCI obtained from a first wireless signal transmitted from the first particular TX to the first particular RX (and the associated first MI/MS/characteristics/STI computed based on the first TSCI), the one or more first particular objects (e.g. known/unknown objects) may be located in/associated with a first zone associated with at least one of: the first particular TX, the first particular RX, or the pair of first particular TX and first particular RX. In some embodiments, a second motion (associated with one or more second particular objects) is detected/ acquired/captured/monitored by a pair of second particular TX and second particular RX in a second sensing task based on an associated second TSCI obtained from a second wireless signal transmitted from the second particular TX to the second particular RX (and the associated second MI/MS/characteristics/STI computed based on the first TSCI), the one or more second particular objects (e.g. known/unknown objects) may be located in/associated with a second zone associated with at least one of: the second particular TX, the second particular RX, or the pair of second particular TX and second particular RX.

While the first sensing task may be different from the second sensing task, the first MI/MS/STI/ characteristics may be mathematically and/or statistically similar to (or the same as) the second MI/MS/characteristics/STI, such that they can be compared. For example, their mathematical formula may be identical if the first TSCI is identical to the second TSCI. For example, the mathematical formula of both may be similar because they are both an average/weighted average (e.g. over a time window/time period/range/band/spatial streams/CI components, etc.), or a variance, or a statistics, or an autocorrelation function (ACF), or a feature of the autocorrelation function, or a transform, or a projection, or a derivative/differential/difference, or an integration/ sum, or a linear/nonlinear function. For example, the mathematical formula of both may be similar because both are a same function of: a magnitude or a magnitude square or a phase of CI, or a second function of the magnitude or the phase.

In some embodiments, each wireless signal may comprise a time series of sounding signals. Each CI may be obtained from a respective received sounding signal. The first wireless signal and second wireless signal may be similar or different, with similar/different configuration, frequency band (e.g. 2.4GHz/SGHz/24GHz/60GHz/70+GHz), bandwidth (e.g. 20MHz/40MHz/80MHz/160MHz/320MHz/ 640MHz), amount of spatial streams (due to amount of TX antennas and RX antennas) (e.g.½2/3/4/ 6/8/9/10/12/etc.), timing of sounding signals, sounding frequency (e.g. 0.01Hz/0.1Hz/1Hz/10Hz/ 100Hz/1kHz/10kHz, or irregular/intermittent), sounding period, etc. As a result, the first TSCI and the second TSCI may be similar/different/asynchronous/ not aligned.

To further locate the objects, the disclosed system/device/method/software resolves/distinguishes/differentiates/decides/detects between two cases/situations/possibilities based on the first TSCI and second TSCI, and the associated first MI/MS/characteristics/STI and second MI/MS/STI/ characteristics computed based on the first and second TSCI. In the first case, the first particular objects are the second particular objects and the first motion is the second motion. In the second case, the first particular objects are not the second particular objects (at least not entirely, i.e. at least one object in the first particular object is not present in the second particular objects) and the first motion is not the second motion. In the first case, the first particular objects and the second particular objects are registered/ grouped/regarded/classified/detected/considered/combined/merged/joined/consolidated/united/ linked/pooled/fused/mixed/associated/connected/attached as the same object(s) at the same location (i.e. the same zone, an intersection zone of the first zone and the second zone). In the second case, the first particular objects and the second particular objects are registered/ grouped/regarded/classified/detected/ considered/separated/split/broken up/set apart/divided/ segregated/differentiated/distinguished/ dissociated/disconnected/detached as different/distinct/ separate/detached/disconnected/independent/ individual object(s) at different locations (i.e. first particular objects at first zone and second particular objects at second zone).

In some embodiments, to differentiate/distinguish between the two cases, the first MI/MS/STI/characteristics may be compared with the second MI/MS/STI/characteristics. Recall that the first MI/MS/STI/characteristics may be mathematically/statistically similar (or the same as) the second MI/MS/STI/characteristics, and thus they can be compared. In the first case, the first MI/MS/STI/characteristics should be highly correlated with the second MI/MS/STI/characteristics because their fluctuations are due to the same motion of the same object(s). In the second case, the first MI/MS/STI/ characteristics should not be highly correlated (e.g. uncorrelated, or close to uncorrelated) with the second MI/MS/STI/characteristics because their fluctuations are due to the different (independent) motion of different object(s).

In some embodiments, a score/measure of correlation between the time series of first MI/MS/STI/characteristics and the time series of second MI/MS/STI/characteristics may be computed. If the measure of correlation is higher than a first threshold (T1), the first case may be detected. If the measure of correlation is lower than a second threshold (T2, e.g. a T2<T1), the second case may be detected. The first threshold may be equal to the second threshold. If the measure of correlation is between the first threshold and second threshold, a second measure of correlation may be computed (e.g. between the first MI/MS/STI/characteristics and the second MI/MS/STI/characteristics, or between a third MI/MS/STI/characteristics computed based on the first TSCI and a fourth MI/MS/STI/characteristics computed based on the second TSCI). If the second measure of correlation is higher than a third threshold (T3), the first case may be detected. If the second measure of correlation is lower than a fourth threshold (T4, e.g. a T4<T3), the second case may be detected. If the second measure of correlation is between the third threshold and the fourth threshold, an additional measure of correlation may be computed (e.g. between the first and second MI/MS/STI/characteristics, between the third and fourth MI/MS/STI/characteristics, or between a fifth MI/MS/STI/characteristics computed based on the first TSCI and a sixth MI/MS/STI/characteristics computed based on the second TSCI). So on and so forth.

In some embodiments, a time series of the first MI/MS/STI/characteristics may be computed based on the first TSCI, each MI/MS/STI/characteristics computed based on a respective sliding window of the TSCI. A time series of the second MI/MS/STI/characteristics may be computed based on the second TSCI. As the first TSCI and second TSCI may be different (e.g. different sounding frequency, sounding timing, bandwidth, spatial stream, etc.) A compensation may be performed on the first MI/MS/STI/characteristics and the second MI/MS/STI/characteristics. A synchronization may be performed on the first TSCI and the second TSCI. The first MI/MS/STI/characteristics and the second MI/MS/STI/characteristics may be time synchronized.

The following numbered clauses provide examples for correlation-based wireless monitoring and localization.

Clause K1. A method for correlation-based wireless monitoring, comprising: forming, by a plurality of first wireless devices and a plurality of second wireless devices in a venue, at least two device pairs, each device pair comprising a first wireless device and a second wireless device, wherein the venue includes a number of objects each undergoing a respective motion; for each device pair: transmitting, by the first wireless device of the device pair, a respective wireless signal, receiving, by the second wireless device of the device pair, the respective wireless signal through a respective wireless multipath channel of the venue, wherein the received wireless signal differs from the transmitted wireless signal due to the respective wireless multipath channel and the motions of the number of objects in the venue, obtaining a respective time series of channel information (TSCI) of the respective wireless multipath channel based on the received wireless signal, computing a respective motion information (MI) based on the TSCI, and performing a respective sensing task based on the respective MI and the respective TSCI; detecting and monitoring motion of a first object in a first sensing task based on a first MI computed based on a first TSCI associated with a first device pair; detecting and monitoring motion of a second object in a second sensing task based on a second MI computed based on a second TSCI associated with a second device pair; computing a correlation score based at least partially on: the first TSCI, the second TSCI, the first MI and the second MI; detecting the first object and the second object as a same object when the correlation score is greater than a first threshold; and detecting the first object and the second object as two different objects when the correlation score is less than a second threshold.

Clause K2. The method of clause K1, further comprising: aligning the first MI and the second MI in the time domain; and computing the correlation score between the aligned first MI and the aligned second MI.

Clause K3. The method of clause K1 or K2, further comprising: computing a time series of first MI based on the first TSCI, wherein each first MI is computed based on a respective first sliding window of the first TSCI; computing a time series of second MI based on the second TSCI, wherein each second MI is computed based on a respective second sliding window of the second TSCI; and computing the correlation score between the time series of first MI and the time series of second MI.

Clause K4. The method of any previous K clause, further comprising: computing a time series of first MI based on the first TSCI, wherein each first MI is computed based on a respective first sliding window of the first TSCI; computing a time series of second MI based on the second TSCI, wherein each second MI is computed based on a respective second sliding window of the second TSCI; and computing the correlation score between a time window of the time series of first MI and the same time window of the time series of second MI.

Clause K5. The method of clause K4, wherein: determining that the time window covers N1 first MI in the time series of first MI and N2 second MI in the time series of second MI; resampling the time series of first MI to generate N3 aligned first MI in the time window; resampling the time series of second MI to generate N3 aligned second MI in the time window, wherein each of the N3 aligned second MI is time aligned with a respective one of the N3 aligned first MI; and computing the correlation score based on the N3 aligned first MI and the N3 aligned second MI in the time window.

Clause K6. The method of any previous K clause, wherein a MI computed based on a TSCI comprises at least one of: an average within a sliding time window; a magnitude of each CI in the sliding time window; a magnitude square of each component of each CI in the sliding time window; a correlation between two temporally adjacent CI of the TSCI; a similarity score between two temporally adjacent CI of the TSCI; an inner product of two vectors of temporally adjacent CI of the TSCI; an average of component-wise correlation between components of two temporally adjacent CI of the TSCI; or a weighted average of a number of largest component-wise correlation.

Clause K7. The method of any previous K clause, wherein the correlation score is computed based on at least one of: correlation, correlation coefficient, absolute value of correlation, or a monotonic function of the absolute value of correlation, between the first MI and the second MI.

Clause K8. The method of any previous K clause, wherein the first sensing task and the second sensing task are different.

Clause K9. The method of any previous K clause, wherein the first MI and the second MI are the same mathematically or statistically.

Clause K10. The method of any previous K clause, wherein: the first sensing task and the second sensing task comprise a common subtask; and both the first MI and the second MI are used to perform the common subtask.

Clause K11. The method of clause K10, wherein: the common subtask comprises motion detection.

Clause K12. The method of any previous K clause, further comprising: determining that the correlation score is greater than the first threshold; detecting the first object and the second object as a first common object because the correlation score is greater than the first threshold; computing a first representative MI associated with the first common object representing the first object and the second object based on the first MI and the second MI; detecting and monitoring motion of a third object in a third sensing task based on a third MI computed based on a third TSCI associated with a third device pair; computing a second correlation score between the third MI and the first representative MI based on: the first TSCI, the second TSCI, the third TSCI, the first representative MI and the third MI; detecting the third object and the first common object as a same object when the second correlation score is greater than the first threshold; and detecting the third object and the first common object as two different objects when the second correlation score is less than the second threshold.

Clause K13. The method of clause K12, further comprising: determining that the second correlation score is greater than the first threshold; detecting the first object, the second object and the third object as the first common object because the second correlation score is greater than the first threshold; computing a second representative MI associated with the first common object representing the first object, the second object and the third object based on the first MI, the second MI and the third MI; detecting and monitoring motion of a fourth object in a fourth sensing task based on a fourth MI computed based on a fourth TSCI associated with a fourth device pair; computing a third correlation score between the fourth MI and the second representative MI based on the first TSCI, the second TSCI, the third TSCI, the fourth TSCI, the second representative MI and the fourth MI; detecting the fourth object and the first common object as a same object when the third correlation score is greater than the first threshold; and detecting the fourth object and the first common object as two different objects when the third correlation score is less than the second threshold.

Clause K14. The method of clause K13, wherein a representative MI associated with a common object representing multiple objects comprises one of: one of multiple MI associated with the multiple objects, a sum or a weighted sum or a product or a weighted product of the multiple MI, an arithmetic or geometric or harmonic mean of the multiple MI, a weighted arithmetic or geometric or harmonic mean of the multiple MI, a trimmed mean of the multiple MI, a median or weighted median or a percentile of the multiple MI, a maximum or minimum of the multiple MI, one of the multiple MI having a maximum magnitude, or one of the multiple MI having a maximum average magnitude.

Clause K15. The method of any previous K clause, further comprising: locating the first object and the second object by associating the first object with a first zone in the venue and associating the second object with a second zone in the venue, when the correlation score is less than the second threshold, wherein the first zone is a neighborhood around: a location of the first wireless device of the first device pair, a location of the second wireless device of the first device pair, or both, the second zone is a neighborhood around: a location of the first wireless device of the second device pair, a location of the second wireless device of the second device pair, or both; and locating the first object and the second object by associating the same object representing the first object and the second object with a first derived zone related to the first zone and the second zone in the venue, when the correlation score is greater than the first threshold, wherein the first derived zone comprises at least one of: an intersection of the first zone and the second zone, or a union of the first zone and the second zone.

Clause K16. The method of clause K12-K15, further comprising: associating the third object with a third zone in the venue when the second correlation score is less than the second threshold, wherein the third zone is a neighborhood around: a location of the first wireless device of the third device pair, a location of the second wireless device of the third device pair, or both; associating the same object representing the first object, the second object and the third object with a second derived zone related to the first zone, the second zone and the third zone in the venue, when the second correlation score is greater than the first threshold, wherein the second derived zone comprises at least one of: an intersection of the first zone and the second zone, an intersection of the first zone and the third zone, an intersection of the second zone and the third zone, an intersection of the first zone, the second zone, and the third zone, or a union of at least two of: the first zone, the second zone and the third zone; wherein the first zone is a neighborhood around: a location of the first wireless device of the first device pair, a location of the second wireless device of the first device pair, or both; wherein the second zone is a neighborhood around: a location of the first wireless device of the second device pair, a location of the second wireless device of the second device pair, or both.

Clause K17. The method of clause K16, further comprising computing a quantity of the number of objects based on: determining a number of device pairs having motion detected and monitored in the venue; associating a respective tentative object with each of the number of device pairs; iteratively merging any two tentative objects when a respective correlation score indicates the two tentative objects are a same object, to identify at least one final distinct object; computing the quantity of the number of objects as a total quantity of the at least one final distinct object; and locating each of the at least one final distinct object by associating it with a zone in the venue, wherein the zone is associated with a location of the final distinct object or with locations of any tentative object merged to obtain the final distinct object.

Clause K18. The method of clause K17, wherein: each correlation score is between two MI associated with two device pairs respectively, and is computed only if motion is detected in both of the two device pairs based on the two MI respectively.

Clause K19. The method of any previous K clause, wherein each CI comprises at least one of: channel state information (CSI), channel impulse response (CIR) or channel frequency response (CFR).

Clause K20. A system for correlation-based wireless monitoring, comprising: at least two device pairs in a venue, each device pair comprising a first wireless device and a second wireless device, wherein the venue includes a number of objects each undergoing a respective motion, wherein for each device pair: the first wireless device of the device pair is configured to transmit a respective wireless signal, and the second wireless device of the device pair is configured to: receive the respective wireless signal through a respective wireless multipath channel of the venue, wherein the received wireless signal differs from the transmitted wireless signal due to the respective wireless multipath channel and motions of a number of objects in the venue, obtain a respective time series of channel information (TSCI) of the respective wireless multipath channel based on the received wireless signal, compute a respective motion information (MI) based on the TSCI, and perform a respective sensing task based on the respective MI and the respective TSCI; and a processor configured for: computing a correlation score based at least partially on: a first TSCI, a second TSCI, a first MI and a second MI, wherein motion of a first object is detected and monitored in a first sensing task based on the first MI computed based on the first TSCI associated with a first device pair, wherein motion of a second object is detected and monitored in a second sensing task based on the second MI computed based on the second TSCI associated with a second device pair, detecting the first object and the second object as a same object when the correlation score is greater than a first threshold, and detecting the first object and the second object as two different objects when the correlation score is less than a second threshold.

Clause K21. An apparatus for correlation-based wireless monitoring, comprising: a memory having a set of instructions stored therein; and a processor communicatively coupled with the memory and configured for: computing a correlation score based at least partially on: a first time series of channel information (TSCI), a second TSCI, a first motion information (MI) and a second MI, wherein motion of a first object is detected and monitored in a first sensing task associated with a first device pair based on the first MI computed based on the first TSCI obtained from a first wireless signal communicated between the first device pair in a venue, wherein the venue includes a number of objects each undergoing a respective motion, wherein motion of a second object is detected and monitored in a second sensing task associated with a second device pair based on the second MI computed based on the second TSCI obtained from a second wireless signal communicated between the second device pair in the venue, wherein each device pair comprises a first wireless device and a second wireless device, wherein for each device pair: the first wireless device of the device pair is configured to transmit a respective wireless signal, and the second wireless device of the device pair is configured to: receive the respective wireless signal through a respective wireless multipath channel of the venue, wherein the received wireless signal differs from the transmitted wireless signal due to the respective wireless multipath channel and the motions of the number of objects in the venue, obtain a respective TSCI of the respective wireless multipath channel based on the received wireless signal, compute a respective MI based on the TSCI, and perform a respective sensing task based on the respective MI and the respective TSCI; and detecting the first object and the second object as a same object when the correlation score is greater than a first threshold, and detecting the first object and the second object as two different objects when the correlation score is less than a second threshold.

The following numbered clauses provide examples for correlation-based wireless monitoring and localization.

Clause L1. A method/device/system/software of a correlation-based wireless monitoring system, comprising: determining a plurality of Type1 or Type2 heterogeneous wireless devices in a venue forming at least two Type1-Type2 device pairs, each Type1-Type2 device pair comprising a Type1 heterogeneous wireless device (TX) and a Type2 heterogeneous wireless device (RX); determining a number of objects each undergoing a respective motion in the venue; for each Type1-Type2 device pair: transmitting a respective wireless signal by the Type1 device of the pair, receiving the respective wireless signal by the Type2 device of the pair through a respective wireless multipath channel of the venue, wherein the received wireless signal differs from the transmitted wireless signal due to the wireless multipath channel and the motion of the number of objects in the venue, obtaining a respective time series of channel information (TSCI) based on the received wireless signal, computing a respective motion information (MI) based on the TSCI, and performing a respective sensing task based on the respective MI and the respective TSCI; detecting and monitoring motion of a first object in a first sensing task based on a first MI computed based on a first TSCI associated with a first Type1-Type2 device pair; detecting and monitoring motion of a second object in a second sensing task based on a second MI computed based on a second TSCI associated with a second Type1-Type2 device pair; computing a correlation score between the first MI and the second MI based on the first TSCI, the second TSCI, the first MI and the second MI; detecting the first object and the second object as a same object when the correlation score is greater than a first threshold; detecting the first object and the second object as two separate objects when the correlation score is less than a second threshold.

Clause L2. The method/device/system/software of the correlation-based wireless monitoring system of clause L1, comprising: aligning the first MI and the second MI in the time domain; computing the correlation score between the aligned first MI and the aligned second MI.

Clause L3. The method/device/system/software of the correlation-based wireless monitoring system of clause L2, comprising: computing a time series of first MI based on the first TSCI, each first MI computed based on a respective first sliding window of the first TSCI; computing a time series of second MI based on the second TSCI, each second MI computed based on a respective second sliding window of the second TSCI; computing the correlation score between the time series of first MI and the time series of second MI.

Clause L4. The method/device/system/software of the correlation-based wireless monitoring system of clause L3, comprising: computing the correlation score between a time window of the time series of first MI and the same time window of the time series of second MI.

Clause L5. The method/device/system/software of the correlation-based wireless monitoring system of clause L4, comprising: determining that there are N1 of the first MI in the time window of the time series of first MI and N2 of the second MI in the time window of the time series of second MI; resampling the time series of first MI to generate N3 aligned first MI in the time window; resampling the time series of second MI to generate N3 aligned second MI in the time window, each of the N3 aligned second MI is time aligned with a respective one of the N3 aligned MI; computing the correlation score based on the N3 aligned first MI and the N3 aligned second MI in the time window.

Clause L6. The method/device/system/software of the correlation-based wireless monitoring system of any previous L clause, comprising: wherein a MI computed based on a TSCI comprises at least one of: an average within a sliding time window, a magnitude of each CI in the sliding time window, a magnitude square of each component of each CI in the sliding time window, a correlation between two temporally adjacent CI of the TSCI, a similarity score between two temporally adjacent CI of the TSCI, an inner product of two vectors of temporally adjacent CI of the TSCI, an average of component-wise correlation between components of two temporally adjacent CI of the TSCI, a weighted average of a number of largest component-wise correlation.

Clause L7. The method/device/system/software of the correlation-based wireless monitoring system of any previous L clause, comprising: wherein the correlation score between the first MI and the second MI comprises at least one of: correlation, correlation coefficient, absolute value of correlation, a monotonic function of the correlation, between the two MI.

Clause L8. The method/device/system/software of the correlation-based wireless monitoring system of any previous L clause, comprising: wherein the first sensing task and the second sensing task are different.

Clause L9. The method/device/system/software of the correlation-based wireless monitoring system of clause L8, comprising: wherein both the first MI and the second MI are similar mathematically or statistically.

Clause L10. The method/device/system/software of the correlation-based wireless monitoring system of clause L9, comprising: wherein the first sensing task and the second sensing task comprise a common subtask; wherein both the first MI and the second MI are similar mathematically or statistically and are used to perform the common subtask.

Clause L11. The method/device/system/software of the correlation-based wireless monitoring system of clause L10, comprising: wherein the common subtask comprises motion detection.

Clause L12. The method/device/system/software of the correlation-based wireless monitoring system of any previous L clause, comprising: detecting the first object and the second object as a first common object because the correlation score is greater than the first threshold; computing a first representative MI associated with the first common object representing the first object and the second object based on the first MI and the second MI; detecting and monitoring motion of a third object in a third sensing task based on a third MI computed based on a third TSCI associated with a third Type1-Type2 device pair; computing a second correlation score between the third MI and the first representative MI based on the first TSCI, the second TSCI, the third TSCI, the first representative MI and the third MI; detecting the third object and the first common object as a same object when the second correlation score is greater than the first threshold; detecting the third object and the first common object as two separate objects when the second correlation score is less than the second threshold.

Clause L13. The method/device/system/software of the correlation-based wireless monitoring system of clause L12, comprising: detecting the first object, the second object and the third object as the first common object because the second correlation score is greater than the first threshold; computing a second representative MI associated with the first common object representing the first object, the second object and the third object based on the first MI, the second MI and the third MI; detecting and monitoring motion of a fourth object in a fourth sensing task based on a fourth MI computed based on a fourth TSCI associated with a fourth Type1-Type2 device pair; computing a third correlation score between the fourth MI and the second representative MI based on the first TSCI, the second TSCI, the third TSCI, the fourth TSCI, the second representative MI and the fourth MI; detecting the fourth object and the first common object as a same object when the third correlation score is greater than the first threshold; detecting the fourth object and the first common object as two separate objects when the third correlation score is less than the second threshold.

Clause L14. The method/device/system/software of the correlation-based wireless monitoring system of clause L13, comprising: wherein any representative MI associated with a common object representing multiple objects comprises one of: one of the multiple MI associated with the multiple objects, a sum or a weighted sum or a product or a weighted product of the multiple MI, an arithmetic or geometric or harmonic mean of the multiple MI, a weighted arithmetic or geometric or harmonic mean of the multiple MI, a trimmed mean of the multiple MI, a median or weighted median or a percentile of the multiple MI, a maximum or minimum of the multiple MI, one of the multiple MI associated with the multiple objects with maximum magnitude, one of the multiple MI associated with the multiple objects with maximum average magnitude.

Clause L15. The method/device/system/software of the correlation-based wireless monitoring system of any previous L clause, comprising: locating the first and second object by associating the first object with a first zone in the venue and the second object with a second zone in the venue when the correlation score is less than the second threshold, wherein the first zone is a neighborhood around: a location of the Type1 device or a location of the Type2 device of the first Type1-Type2 device pair, or both, wherein the second zone is a neighborhood around: a location of the Type1 device of the second pair, or a location of the Type2 device of the second Type1-Type2 device pair, or both; locating the first and second object by associating the same object of the first object and the second object with a first derived zone related to the first zone and the second zone in the venue when the correlation score is greater than the first threshold, wherein the first derived zone comprises at least one of: an intersection of the first zone and the second zone, or a union of the first zone and the second zone.

Clause L16. The method/device/system/software of the correlation-based wireless monitoring system of clause L12-L15, comprising: associating the third object with a third zone in the venue when the second correlation score is less than the second threshold, wherein the third zone is a neighborhood around: a location of the Type1 device of the third pair, or a location of the Type2 device of the third Type1-Type2 device pair, or both; associating the same object of the first object, the second object and the third object with a second derived zone related to the first zone, the second zone and the third zone in the venue when the second correlation score is greater than the first threshold, wherein the second derived zone comprises at least one of: an intersection of the first zone and the second zone, an intersection of the first zone and the third zone, an intersection of the second zone and the third zone, an intersection of the first zone, the second zone, and the third zone, or a union of any of: the first zone, the second zone or the third zone.

Clause L17. The method/device/system/software of the correlation-based wireless monitoring system of clause L13-L16, comprising: computing a count of the number of objects by: determining a count of Type1-Type2 device pairs with motion detected and monitored, associating a respective tentative object with each of the Type1-Type2 device pairs with motion detected and monitored, iteratively merging any two tentative objects when a respective correlation score indicates the two tentative objects are a same object, computing the count of the number of objects as the amount of final distinct tentative objects, locating each object by associating it with a zone in the venue associated with the final distinct tentative object or any intermediate tentative objects being merged to obtain the final distinct tentative object.

Clause L18. The method/device/system/software of the correlation-based wireless monitoring system of any previous L clause, comprising: wherein each CI comprises at least one of: channel state information (CSI), channel impulse response (CIR) or channel frequency response (CFR).

Clause L19. The method/device/system/software of the correlation-based wireless monitoring system of clause L13-L18, comprising: computing the correlation score between any two MI only if motion is detected in both of the two associated Type1-Type2 device pairs based on the two MI respectively.

The features described above may be implemented advantageously in one or more computer programs that are executable on a programmable system including at least one programmable processor coupled to receive data and instructions from, and to transmit data and instructions to, a data storage system, at least one input device, and at least one output device. A computer program is a set of instructions that may be used, directly or indirectly, in a computer to perform a certain activity or bring about a certain result. A computer program may be written in any form of programming language (e.g., C, Java), including compiled or interpreted languages, and it may be deployed in any form, including as a stand-alone program or as a module, component, subroutine, a browser-based web application, or other unit suitable for use in a computing environment.

Suitable processors for the execution of a program of instructions include, e.g., both general and special purpose microprocessors, digital signal processors, and the sole processor or one of multiple processors or cores, of any kind of computer. Generally, a processor will receive instructions and data from a read-only memory or a random access memory or both. The essential elements of a computer are a processor for executing instructions and one or more memories for storing instructions and data. Generally, a computer will also include, or be operatively coupled to communicate with, one or more mass storage devices for storing data files; such devices include magnetic disks, such as internal hard disks and removable disks; magneto-optical disks; and optical disks. Storage devices suitable for tangibly embodying computer program instructions and data include all forms of non-volatile memory, including by way of example semiconductor memory devices, such as EPROM, EEPROM, and flash memory devices; magnetic disks such as internal hard disks and removable disks; magneto-optical disks; and CD-ROM and DVD-ROM disks. The processor and the memory may be supplemented by, or incorporated in, ASICs (application-specific integrated circuits).

While the present teaching contains many specific implementation details, these should not be construed as limitations on the scope of the present teaching or of what may be claimed, but rather as descriptions of features specific to particular embodiments of the present teaching. Certain features that are described in this specification in the context of separate embodiments may also be implemented in combination in a single embodiment. For example, where embodiments have features in common, features of those different embodiments may be combined. Conversely, various features that are described in the context of a single embodiment may also be implemented in multiple embodiments separately or in any suitable sub-combination.

Similarly, while operations are depicted in the drawings in a particular order, this should not be understood as requiring that such operations be performed in the particular order shown or in sequential order, or that all illustrated operations be performed, to achieve desirable results. In certain circumstances, multitasking and parallel processing may be advantageous. Moreover, the separation of various system components in the embodiments described above should not be understood as requiring such separation in all embodiments, and it should be understood that the described program components and systems may generally be integrated together in a single software product or packaged into multiple software products.

Particular embodiments of the subject matter have been described. Any combination of the features and architectures described above is intended to be within the scope of the following claims. Other embodiments are also within the scope of the following claims. In some cases, the actions recited in the claims may be performed in a different order and still achieve desirable results. In addition, the processes depicted in the accompanying figures do not necessarily require the particular order shown, or sequential order, to achieve desirable results. In certain implementations, multitasking and parallel processing may be advantageous.

**Claims**

**1.** A method for wireless monitoring, comprising:

transmitting a wireless signal from a first wireless device through a wireless multipath channel of a venue, wherein the wireless multipath channel is impacted by a motion of an object in the venue;

receiving the wireless signal by a second wireless device through the wireless multipath channel, wherein the received wireless signal differs from the transmitted wireless signal due to the wireless multipath channel and the motion of the object;

obtaining a time series of channel information (TSCI) of the wireless multipath channel based on the received wireless signal using a processor, a memory communicatively coupled with the processor and a set of instructions stored in the memory;

performing a classification of a sliding time window by analyzing channel information (CI) of the TSCI in the sliding time window;

computing a motion information (MI) for the sliding time window based on the TSCI and the classification of the sliding time window, wherein the MI is computed based on at least one of: a similarity score of two temporally adjacent CI of the TSCI, an autocorrelation function (ACF) of the TSCI, or a characteristic point of the ACF, wherein computing the MI comprises at least one of:

computing the MI in a first manner based exclusively on CI of the TSCI in the sliding time window when the sliding time window is classified as a first sliding-window-class based on the classification,

computing the MI in a second manner based on at least one CI of the TSCI outside the sliding time window when the sliding time window is classified as a second sliding-window-class based on the classification, and

computing the MI in a third manner based on a first subset of the CI of the TSCI in the sliding time window without using a second subset of the CI of the TSCI in the sliding time window, when the sliding time window is classified as a third sliding-window-class based on the classification, wherein the first subset and the second subset are disjoint; and

monitoring the motion of the object based on the MI.

2. The method of claim 1, further comprising:

computing a test score (TS) for each CI of the TSCI in the sliding time window based on a number of respective temporally neighboring CI, wherein the TS comprises at least one of: a difference, a magnitude, a vector similarity, a vector dissimilarity, an inner product, or an outer product; and

classifying each CI of the TSCI in the sliding time window based on the respective TS, wherein

each CI of the TSCI in the sliding time window is classified as a first CI-class when the respective TS is less than a first threshold, and

each CI of the TSCI in the sliding time window is classified as a second CI-class when the respective TS is greater than a second threshold.

3. The method of claim 2, further comprising:

computing a link-wise test score (LTS) based on an aggregate of all TS for the CI of the TSCI in the sliding time window; and

performing the classification of the sliding time window based on the LTS, wherein

the sliding time window is classified as a first sliding-window-class or a second sliding-window-class when the LTS is less than a first threshold,

the sliding time window is classified as the first sliding-window-class when all CI of the TSCI in the sliding time window are first-class CI classified as first CI-class,

the sliding time window is classified as the second sliding-window-class , when all CI of the TSCI in the sliding time window are second-class CI classified as second CI-class,

the sliding time window is classified as a third sliding-window-class when the LTS is greater than a second threshold.

4. The method of claim 2 or 3, further comprising:

identifying at least one run of first-class CI and at least one run of second-class CI in the sliding time window, each run comprising a respective runlength of consecutive CI of a respective same CI-class in the sliding time window, wherein any runlength is one of: a number, a quantity or a count, that is greater than zero; and

classifying the sliding time window based on the runs of first-class CI and second-class CI and the respective run-lengths, wherein

the sliding time window is classified as the third sliding-window-class when at least one selected run of first-class CI is chosen, and

the sliding time window is classified as the second sliding-window-class when no selected run of first-class CI is chosen.

5. The method of claim 4, further comprising:

choosing at least one selected run of first-class CI based on the runlength of each run of first-class CI and the TS associated with the run;

computing the MI based on the at least one selected run of first-class CI, wherein the at least one selected run is the at least one run of first-class CI with longest run-lengths among all runs of first-class CI in the sliding time window, wherein

a first selected run is chosen as a beginning run of consecutive first-class CI comprising a CI being the first in the sliding time window when the respective run-length of the beginning run is greater than a first respective threshold,

a second selected run is chosen as a trailing run of consecutive first-class CI comprising a CI being the last in the sliding time window when the respective run-length of the trailing run is greater than a second respective threshold,

any selected run that is not a beginning run or a trailing run is chosen when the respective run-length of the run is greater than a third respective threshold,

the at least one selected run is chosen such that a quantity of the at least one selected run is less than or equal to a predetermined number, and

the at least one selected run is chosen such that, for each selected run, all the associated TS is less than a threshold.

6. The method of claim 4 or 5, further comprising:
when the sliding time window is classified as the third sliding-window-class:

constructing the first subset by including all of the at least one selected run of first-class CI of the TSCI in the sliding time window, and

constructing the second subset by including all second-class CI of the TSCI in the sliding time window; and when the sliding time window is classified as the second sliding-window-class:

computing the MI as an aggregate of at least one neighboring MI,

wherein each neighboring MI is associated with one of: a past neighboring sliding time window of CI of the TSCI, a future neighboring sliding time window of CI of the TSCI, or a neighboring sliding time window of CI of another TSCI,

wherein a neighboring MI is computed based on at least one CI of the TSCI outside the sliding time window.

7. The method of claim 4 or 5 or 6, further comprising:

computing at least one tentative MI for the sliding time window, wherein each tentative MI is computed based on a respective selected run of first-class CI of the TSCI in the sliding time window; and

computing the MI as an aggregate of the at least one tentative MI.

8. The method of claim 7, further comprising performing at least one of the following lists of operations:

a first list of operations comprising:

determining a selected run as a beginning run of CI of the TSCI in the sliding time window,

combining the beginning run of CI with a trailing run of CI of the TSCI in a previous sliding time window to form a combined run of CI of the TSCI, and

computing a first tentative MI based on the combined run of CI;

a second list of operations comprising:

determining a selected run as a trailing run of CI of the TSCI in the sliding time window,

combining the trailing run of CI with a beginning run of CI of the TSCI in a next sliding time window to form a combined run of CI of the TSCI, and

computing a second tentative MI based on the combined run of CI;

a third list of operations comprising:

for each tentative MI computed based on a respective selected run, computing a respective computed weight based on the run-length of the respective selected run, and

computing the MI as a weighted aggregate of the at least one tentative MI, wherein each tentative MI is weighted by the respective computed weight;

a fourth list of operations comprising:

computing the MI as an aggregate of the at least one tentative MI and at least one neighboring MI, wherein each neighboring MI is associated with one of: a past neighboring sliding time window of CI of the TSCI, a future neighboring sliding time window of CI of the TSCI, or a neighboring sliding time window of CI of another TSCI.

9. A wireless device, comprising:

a receiver configured to receive a wireless signal transmitted by another wireless device through a wireless multipath channel of a venue, wherein

the wireless multipath channel is impacted by a motion of an object in the venue,

the received wireless signal differs from the transmitted wireless signal due to the wireless multipath channel and the motion of the object;

a memory having a set of instructions stored therein; and

a processor communicatively coupled with the memory and configured for:

obtaining a time series of channel information (TSCI) of the wireless multipath channel based on the received wireless signal,

performing a classification of a sliding time window by analyzing channel information (CI) of the TSCI in the sliding time window,

computing a motion information (MI) for the sliding time window based on the TSCI and the classification of the sliding time window, wherein the MI is computed based on at least one of: a similarity score of two temporally adjacent CI of the TSCI, an autocorrelation function (ACF) of the TSCI, or a characteristic point of the ACF, wherein computing the MI comprises at least one of:

computing the MI in a first manner based exclusively on CI of the TSCI in the sliding time window when the sliding time window is classified as a first sliding-window-class based on the classification,

computing the MI in a second manner based on at least one CI of the TSCI outside the sliding time window when the sliding time window is classified as a second sliding-window-class based on the classification, and

computing the MI in a third manner based on a first subset of the CI of the TSCI in the sliding time window without using a second subset of the CI of the TSCI in the sliding time window, when the sliding time window is classified as a third sliding-window-class based on the classification, wherein the first subset and the second subset are disjoint, and

monitoring the motion of the object based on the MI.

10. The wireless device of claim 9, wherein the processor is further configured for:

computing a test score (TS) for each CI of the TSCI in the sliding time window based on a number of respective temporally neighboring CI, wherein the TS comprises at least one of: a difference, a magnitude, a vector similarity, a vector dissimilarity, an inner product, or an outer product;

classifying each CI of the TSCI in the sliding time window based on the respective TS, wherein

each CI of the TSCI in the sliding time window is classified as a first CI-class when the respective TS is less than a first threshold, and

each CI of the TSCI in the sliding time window is classified as a second CI-class when the respective TS is greater than a second threshold;

computing a link-wise test score (LTS) based on an aggregate of all TS for the CI of the TSCI in the sliding time window; and
performing the classification of the sliding time window based on the LTS, wherein

the sliding time window is classified as a first sliding-window-class or a second sliding-window-class when the LTS is less than a first threshold,
the sliding time window is classified as the first sliding-window-class when all CI of the TSCI in the sliding time window are first-class CI classified as first CI-class,
the sliding time window is classified as the second sliding-window-class when all CI of the TSCI in the sliding time window are second-class CI classified as second CI-class,
the sliding time window is classified as a third sliding-window-class when the LTS is greater than a second threshold.

**11.** The wireless device of claim 10, wherein the processor is further configured for:

identifying at least one run of first-class CI and at least one run of second-class CI in the sliding time window, each run comprising a respective runlength of consecutive CI of a respective same CI-class in the sliding time window, wherein any runlength is one of: a number, a quantity or a count, that is greater than zero;
classifying the sliding time window based on the runs of first-class CI and second-class CI and the respective run-lengths, wherein

the sliding time window is classified as the third sliding-window-class when at least one selected run of first-class CI is chosen, and
the sliding time window is classified as the second sliding-window-class when no selected run of first-class CI is chosen;

choosing at least one selected run of first-class CI based on the runlength of each run of first-class CI and the TS associated with the run;
computing the MI based on the at least one selected run of first-class CI, wherein the at least one selected run is the at least one run of first-class CI with longest run-lengths among all runs of first-class CI in the sliding time window, wherein

a first selected run is chosen as a beginning run of consecutive first-class CI comprising a CI being the first in the sliding time window when the respective run-length of the beginning run is greater than a first respective threshold,
a second selected run is chosen as a trailing run of consecutive first-class CI comprising a CI being the last in the sliding time window when the respective run-length of the trailing run is greater than a second respective threshold,
any selected run that is not a beginning run or a trailing run is chosen when the respective run-length of the run is greater than a third respective threshold,
the at least one selected run is chosen such that a quantity of the at least one selected run is less than or equal to a predetermined number, and
the at least one selected run is chosen such that, for each selected run, all the associated TS is less than a threshold.

**12.** The wireless device of claim 11, wherein the processor is further configured for:

when the sliding time window is classified as the third sliding-window-class:

constructing the first subset by including all of the at least one selected run of first-class CI of the TSCI in the sliding time window, and
constructing the second subset by including all second-class CI of the TSCI in the sliding time window;

when the sliding time window is classified as the second sliding-window-class:

computing the MI as an aggregate of at least one neighboring MI,
wherein each neighboring MI is associated with one of: a past neighboring sliding time window of CI of the TSCI, a future neighboring sliding time window of CI of the TSCI, or a neighboring sliding time window of CI of another TSCI,
wherein a neighboring MI is computed based on at least one CI of the TSCI outside the sliding time window;

computing at least one tentative MI for the sliding time window, wherein each tentative MI is computed based on a respective selected run of first-class CI of the TSCI in the sliding time window; and
computing the MI as an aggregate of the at least one tentative MI.

**13.** The wireless device of claim 12, wherein the processor is further configured for performing at least one of the following lists of operations:

a first list of operations comprising:

determining a selected run as a beginning run of CI of the TSCI in the sliding time window,
combining the beginning run of CI with a trailing run of CI of the TSCI in a previous sliding time window to form a combined run of CI of the TSCI, and
computing a first tentative MI based on the combined run of CI;

a second list of operations comprising:

determining a selected run as a trailing run of CI of the TSCI in the sliding time window,
combining the trailing run of CI with a beginning run of CI of the TSCI in a next sliding time window to form a combined run of CI of the TSCI, and
computing a second tentative MI based on the combined run of CI;

a third list of operations comprising:

for each tentative MI computed based on a respective selected run, computing a respective computed weight based on the run-length of the respective selected run, and
computing the MI as a weighted aggregate of the at least one tentative MI, wherein each tentative MI is weighted by the respective computed weight;

a fourth list of operations comprising:
computing the MI as an aggregate of the at least one tentative MI and at least one neighboring MI, wherein each neighboring MI is associated with one of: a past neighboring sliding time window of CI of the TSCI, a future neighboring sliding time window of CI of the TSCI, or a neighboring sliding time window of CI of another TSCI.

**14.** A wireless monitoring system, comprising:

a first wireless device configured to transmit a wireless signal through a wireless multipath channel of a venue, wherein the wireless multipath channel is impacted by a motion of an object in the venue;
a second wireless device configured to receive the wireless signal through the wireless multipath channel, wherein the received wireless signal differs from the transmitted wireless signal due to the wireless multipath channel and the motion of the object; and
a processor configured for:.

obtaining a time series of channel information (TSCI) of the wireless multipath channel based on the received wireless signal,
performing a classification of a sliding time window by analyzing channel information (CI) of the TSCI in the sliding time window,
computing a motion information (MI) for the sliding time window based on the TSCI and the classification of the sliding time window, wherein the MI is computed based on at least one of: a similarity score of two temporally adjacent CI of the TSCI, an autocorrelation function (ACF) of the TSCI, or a characteristic point of the ACF, wherein computing the MI comprises at least one of:

computing the MI in a first manner based exclusively on CI of the TSCI in the sliding time window when the sliding time window is classified as a first sliding-window-class based on the classification, computing the MI in a second manner based on at least one CI of the TSCI outside the sliding time window when the sliding time window is classified as a second sliding-window-class based on the classification, and

computing the MI in a third manner based on a first subset of the CI of the TSCI in the sliding time window without using a second subset of the CI of the TSCI in the sliding time window, when the sliding time window is classified as a third sliding-window-class based on the classification, wherein the first subset and the second subset are disjoint, and

monitoring the motion of the object based on the MI.

15. The wireless monitoring system of claim 14, wherein the processor is further configured for:

computing a test score (TS) for each CI of the TSCI in the sliding time window based on a number of respective temporally neighboring CI, wherein the TS comprises at least one of: a difference, a magnitude, a vector similarity, a vector dissimilarity, an inner product, or an outer product;
classifying each CI of the TSCI in the sliding time window based on the respective TS, wherein

each CI of the TSCI in the sliding time window is classified as a first CI-class when the respective TS is less than a first threshold, and
each CI of the TSCI in the sliding time window is classified as a second CI-class when the respective TS is greater than a second threshold;

computing a link-wise test score (LTS) based on an aggregate of all TS for the CI of the TSCI in the sliding time window; and
performing the classification of the sliding time window based on the LTS, wherein

the sliding time window is classified as a first sliding-window-class or a second sliding-window-class when the LTS is less than a first threshold,
the sliding time window is classified as the first sliding-window-class when all CI of the TSCI in the sliding time window are first-class CI classified as first CI-class,
the sliding time window is classified as the second sliding-window-class when all CI of the TSCI in the sliding time window are second-class CI classified as second CI-class,
the sliding time window is classified as a third sliding-window-class when the LTS is greater than a second threshold.

FIG. 1

FIG. 2

EP 4 295 760 A1

300

302 — Transmit a wireless signal from a first wireless device through a wireless multipath channel of a venue, the wireless multipath channel being impacted by a motion of an object in the venue

304 — Receive the wireless signal by a second wireless device through the wireless multipath channel, the received wireless signal being different from the transmitted wireless signal due to the wireless multipath channel and the motion of the object

306 — Obtain a time series of channel information (TSCI) of the wireless multipath channel based on the received wireless signal

308 — Perform a classification of a sliding time window by analyzing channel information (CI) of the TSCI in the sliding time window

310 — Compute a motion information/motion statistics/spatial-temporal information (MI/MS/STI) for the sliding time window based on the TSCI and the classification of the sliding time window

312 — Monitor the motion of the object based on the MI/MS/STI

FIG. 3

## 400

| | |
|---|---|
| Classify current sliding time window into at least 3 classes and compute MI/MS/STI of the current sliding time window in different ways based on the at least 3 classes | 402 |

↓

| | |
|---|---|
| If current sliding time window is Window-Class 1 (e.g. "NORMAL"), compute MI/MS/STI in a first way based exclusively on CI of TSCI in the current sliding time window | 404 |

↓

| | |
|---|---|
| If current sliding time window is Window-Class 2 (e.g. "SEVERELY ABNORMAL"), compute MI/MS/STI in a second way based on at least one CI of the TSCI outside the current sliding time window | 406 |

↓

| | |
|---|---|
| If current sliding time window is Window-Class 3 (e.g. "MODERATELY ABNORMAL"), compute MI/MS/STI in a third way based on a first subset of CI of the TSCI in the current sliding time window without using a second subset of the CI of the TSCI in the sliding time window, wherein the first subset and second subset are disjoint | 408 |

FIG. 4

<u>500</u>

┌─────────────────────────────────────────────────────────────────┐ ⌐510
│ Compute test score (TS) for each CI of TSCI in sliding time window │
│                                                                     │
│  ┌───────────────────────────────────────────────────────────┐ ⌐512 │
│  │ Compute TS for a CS based on temporally neighboring CI      │ │
│  └───────────────────────────────────────────────────────────┘ │
│                                                                     │
│  ┌───────────────────────────────────────────────────────────┐ ⌐514 │
│  │ Compute component test score (CTS) for each component of a CI; │ │
│  │ compute TS for the CI as aggregate of CTS of the CI          │ │
│  └───────────────────────────────────────────────────────────┘ │
└─────────────────────────────────────────────────────────────────┘

┌─────────────────────────────────────────────────────────────────┐ ⌐520
│ Perform item-wise classification of each CI of the TSCI in the sliding time window │
│ based on the respective TS                                        │
└─────────────────────────────────────────────────────────────────┘

FIG. 5

Compute a link-wise test score (LTS) based on the plurality of TS for the CI
of the TSCI in the sliding time window

610

Perform the classification of the sliding time window based on the LTS

620

Classify the sliding time window as Window-Class 1 if LTS is greater
than T1

622

Classify the sliding time window as a class comprising Window-Class 1
and Window-Class 2 if LTS is less than T2

624

FIG. 6

EP 4 295 760 A1

Perform item-wise classification for each CI of TSCI in sliding time window. Classify each CI as CI-Class 1 (e.g. "NORMAL CI") if respective TS is less than T3, and as CI-Class 2 ("ABNORMAL CI") if the TS is greater than T4 ⟵ 710

Classify sliding time window as Window-Class 1 if all CI in the sliding time window are classified as CI-Class 1 (first-class CI) ⟵ 720

Classify sliding time window as Window-Class 2 if all CI in sliding time window are classified as CI-Class 2 (second-class CI) ⟵ 730

If at least one CI is classified as CI-Class 1 and at least one as CI-Class 2, identify at least one run of first-class CI and at least one run of second-class CI in the sliding time window and classify sliding time window based on the runs of first-class CI and second-class CI and the respective runlengths ⟵ 740

Search for and choose selected run of first-class CI based on a runlength of each run of first-class CI and the plurality of TS associated with the run ⟵ 742

Classify sliding time window as Window-Class 3 if at least one selected run of first-class CI is chosen and compute MI/MS/STI based on the at least one selected run of first-class CI ⟵ 744

Classify sliding time window as Window-Class 2 if no selected run of first-class CI is chosen ⟵ 746

700

FIG. 7

EP 4 295 760 A1

FIG. 8

900

```
                                                          ┌─910
┌────────────────────────────────────────────────────────────────┐
│ Transmit a wireless signal from a Type1 heterogeneous device to a Type2│
│ heterogeneous device through a wireless channel impacted by a motion of an│
│                      object in a venue                          │
└────────────────────────────────────────────────────────────────┘
                              │
                              ▼                           ┌─920
┌────────────────────────────────────────────────────────────────┐
│ Obtain a time series of channel information (TSCI) of the wireless channel│
│ based on the received wireless signal using a processor, a memory and a set│
│                      of instructions                            │
└────────────────────────────────────────────────────────────────┘
                              │
                              ▼                           ┌─930
┌────────────────────────────────────────────────────────────────┐
│ Compute a time series of motion information (TSMI) based on TSCI and│
│          compute a time series of analytics (TSA) based on TSMI │
└────────────────────────────────────────────────────────────────┘
                              │
                              ▼                           ┌─940
┌────────────────────────────────────────────────────────────────┐
│ Compute a time series of compensated analytics by applying a compensation│
│ to the computation of TSA, wherein the compensation comprises a monotonic│
│                        mapping                                  │
└────────────────────────────────────────────────────────────────┘
           │                  │
           │                  ▼                           ┌─950
           │   ┌─────────────────────────────────────────────────┐
           │   │ Change the compensation/monotonic mapping  based on a│
           │   │ change in a target behavior,  wireless signal, bandwidth, band,│
           │   │ specification, setting, user input, situation, event, time table,│
           │   │     strategy, plan; applied changed compensation │
           │   └─────────────────────────────────────────────────┘
           │                  │
           ▼                  ▼                           ┌─960
┌────────────────────────────────────────────────────────────────┐
│ Monitor the motion of the object based on the time series of compensated│
│                        analytics                                │
└────────────────────────────────────────────────────────────────┘
```

FIG. 9

┌─────────────────────────────────────────────────────────────────────────┐ ⌐1010
│ Apply a compensation to the computation of TSA, wherein the compensation  │
│ comprises a monotonic mapping                                             │
└─────────────────────────────────────────────────────────────────────────┘
                                      │
                                      ▼
┌─────────────────────────────────────────────────────────────────────────┐ ⌐1022
│ Compute compensated CI based on compensation/monotonic mapping            │
│ applied to CI                                                             │
└─────────────────────────────────────────────────────────────────────────┘
                                      │
                                      ▼
┌─────────────────────────────────────────────────────────────────────────┐ ⌐1024
│ Compute compensated MI based on: CI/IV, compensated CI/IV, compensated    │
│ feature/magnitude/phase/component of CI/IV, or compensation/monotonic     │
│ mapping applied to MI                                                     │
└─────────────────────────────────────────────────────────────────────────┘
                                      │
                                      ▼
┌─────────────────────────────────────────────────────────────────────────┐ ⌐1026
│ Compute compensated analytics based on: CI/IV/MI/MS/STI, compensated      │
│ CI/IV/MI/MS/STI, compensated feature/phase/magnitude/component of         │
│ CI/IV/MI/MS/STI, or compensation/monotonic mapping applied to analytics   │
└─────────────────────────────────────────────────────────────────────────┘

FIG. 10

**1100**

**1110**

In a calibration phase, transmit a calibration wireless signal from a calibration Type1 device to a calibration Type2 device through a calibration wireless channel impacted by a calibration motion of a calibration object in a calibration venue

**1120**

Obtain a time series of calibration CI of the calibration wireless channel based on the received calibration wireless signal using a calibration processor/memory/instructions

**1130**

Compute a time series of calibration MI/MS/STI based on the time series of calibration CI and compute a time series of calibration analytics based on the time series of calibration MI/MS/STI

**1140**

Compare behavior associated with the time series of calibration CI/MI/MS/STI/analytics with a target behavior

**1150**

Compute the monotonic mapping based on the comparing

**1152**

Compute monotonic mapping based on (1) search/deduction, (2) specification/setting/user input/another device, (3) semi-supervised/supervised/unsupervised learning, or (4) AI/deep learning/machine learning/neural network

FIG. 11

## 1200

**1210**
In a calibration phase, determine and search a number of candidate monotonic mappings

**1220**
For each candidate monotonic mapping: (a) apply respective compensation comprising the candidate monotonic mapping to the computation of the TSA, (b) compute a respective similarity score between the target behavior and the resulting behavior associated with the calibration CI/MI/MS/STI/analytics after respective compensation is applied

**1230**
Compute monotonic mapping based on (1) search/deduction, (2) specification/setting/user input/another device, (3) semi-supervised/supervised/unsupervised learning, or (4) AI/deep learning/machine learning/neural network

**1240**
Choose the monotonic mapping as the candidate monotonic mapping with largest similarity score

FIG. 12

EP 4 295 760 A1

```
                                                              ⌐1310
┌─────────────────────────────────────────────────────────────┐
│  In a calibration phase, determine the behavior of a          │
│  univariate observable X associated with                      │
│  CI/MI/MS/STI/analytics based on calibration                  │
│  CI/MI/MS/STI/analytics, wherein behavior comprises N scalar   │
│  values of X, {x_1, x_2, ... x_N} with x_1<x_2< ..<x_N         │
└─────────────────────────────────────────────────────────────┘
```

1300

In a calibration phase, determine the behavior of a univariate observable X associated with CI/MI/MS/STI/analytics based on calibration CI/MI/MS/STI/analytics, wherein behavior comprises N scalar values of X, {x_1, x_2, ... x_N} with x_1<x_2< ..<x_N

1320

Determine the target behavior of a univariate target observable Y, wherein the target behavior comprises N scalar values of Y {y_1, y_2, ... y_N}, with y_1<y_2< ..<y_N

1330

Define N control points of the monotonic mapping by mapping the N scalar values of X to the N scalar values of Y, the control points being (x_1, y_1), (x_2, y_2), .., (x_N, y_N)

1340

Choose the monotonic mapping based on the N control points

1342

Deduce the monotonic mapping as a monotonic line connecting the N control points

1344

Deduce the monotonic mapping as a curve that fits the N control points in accordance with a fitting criterion

FIG. 13

**1400**

1410 — In a calibration phase, determine the behavior of a univariate observable X associated with CI/MI/MS/STI/analytics based on calibration CI/MI/MS/STI/analytics, wherein behavior comprises a cumulative univariate distribution $F\_X$ of X

1420 — Determine the target behavior of a univariate target observable Y, wherein the target behavior comprises a cumulative univariate target distribution $F\_Y$ of Y

1430 — Deduce the monotonic mapping based on the cumulative univariate distributions $F\_X$ and $F\_Y$

1432 — Deduce monotonic mapping as $F\_Y^{-1}[F\_X(X)]$, wherein $F\_Y^{-1}$ is the inverse of function $F\_Y$

1434 — Deduce monotonic mapping as an approximation of $F\_Y^{-1}[F\_X(X)]$, wherein $F\_Y^{-1}$ is the inverse of function $F\_Y$

FIG. 14

## 1500

1510

In a calibration phase, determine the monotonic mapping to comprise a first and a second monotonic mappings

1520

Determine and search a number of candidate first monotonic mapping

1530

For each candidate first monotonic mapping: (a) deduce the respective second monotonic mapping, (b) apply a respective compensation comprising the candidate first monotonic mapping and the respective deduced second monotonic mapping to the computation of the TSA, (c) compute a respective similarity score between the target behavior and the resulting behavior associated with the calibration CI/MI/MS/STI/ analytics after the respective compensation is applied

1540

Choose the monotonic mapping as the candidate first monotonic mapping and the respective deduced second monotonic mapping with the largest similarity score

FIG. 15

<u>1600</u>

In a pre-calibration phase before the calibration phase, transmit a reference wireless signal from a reference Type1 device to a reference Type2 device through a reference wireless channel impacted by a reference motion of a reference object in a reference venue ↗1610

Obtain a time series of reference CI of the reference wireless channel based on the received reference wireless signal using reference processor/memory/instructions ↗1620

Compute a time series of reference MI/MS/STI based on the time series of reference CI; compute a time series of reference analytics based on the time series of calibration MI/MS/STI ↗1630

Compute the target behavior as a behavior of at least one of: the time series of reference CI, the time series of reference MI, or the time series of reference analytics ↗1640

FIG. 16

<u>1700</u>

In a re-calibration phase after the calibration phase, obtain a re-calibration TSCI — 1710

Compute a time series of re-calibration MI/MS/STI based on the time series of re-calibration CI and compute a time series of re-calibration analytics based on the time series of re-calibration MI/MS/STI — 1720

Compare a behavior of the time series of re-calibration CI/MI/MS/STI/analytics with the target behavior — 1730

Compute a renewed monotonic mapping based on the comparing of the comparing — 1740

Replace the monotonic mapping by the renewed monotonic mapping in the compensation to the computation of the TSA — 1750

FIG. 17

FIG. 18

FIG. 19

| | |
|---|---|
| Obtain a radio signal that is transmitted from a Type 1 device to a Type 2 device through a wireless channel of a venue, the received radio signal differs from the transmitted radio signal due to the wireless channel impacted by a target motion of an object in the venue | 2002 |
| Obtain a time series of channel information (TSCI) of the wireless channel based on the received radio signal | 2004 |
| Capture an auxiliary signal in the venue by a sensor, where the auxiliary signal is not a radio signal and is impacted by the target motion of the object | 2006 |
| Obtain a time series of auxiliary samples (TSAS) based on the captured auxiliary signal | 2008 |
| Compute a radio-based motion information (RMI) based on the TSCI | 2010 |
| Compute an aux-based motion information (AMI) based on the TSAS | 2012 |
| Monitor the target motion jointly based on the RMI and AMI | 2014 |

FIG. 20

FIG. 21

Legend:
- For app to configure device connecting to home LAN
- For MO to connect to FS to receive set ID and MQTT credential
- Sounding signal from Satellite
- For MO to connect to SS to receive command and send sensing data
- For O to send basic sensing data to MO

EP 4 295 760 A1

FIG. 22

| | |
|---|---|
| Communicatively couple a particular device with a first device through a first wireless channel based on a first protocol using a first radio of the particular device | 2302 |

↓

| | |
|---|---|
| Communicatively couple the particular device with a second device through a second wireless channel based on a second protocol using a second radio of the particular device | 2304 |

↓

| | |
|---|---|
| Perform a pairwise sub-task by the particular device and the second device based on a wireless signal communicated between the particular device and the second device through the second wireless channel using the second radio of the particular device | 2306 |

↓

| | |
|---|---|
| Obtain by the particular device a pairwise sensing analytics computed based on a time series of channel information (TSCI) of the second wireless channel extracted from the wireless signal | 2308 |

↓

| | |
|---|---|
| Compute a combined sensing analytics by the particular device based on the pairwise sensing analytics | 2310 |

↓

| | |
|---|---|
| Transmit the combined sensing analytics by the particular device to the first device through the first wireless channel using the first radio of the particular device | 2312 |

↓

| | |
|---|---|
| Perform a wireless sensing task based on the combined sensing analytics | 2314 |

FIG. 23

FIG. 24

Obtain wireless signals each transmitted from a respective Type 1 device to a respective Type 2 device through a respective wireless channel in a same venue, where the wireless channels are impacted by a motion of an object in the venue ⌐ 2502

Obtain a plurality of time series of channel information (TSCI) of the wireless channels in the venue, where each TSCI is obtained based on a respective one of the wireless signals ⌐ 2504

Compute a plurality of time series of motion statistics (TSMS) associated with the motion of the object, where each TSMS is computed based on at least one respective TSCI ⌐ 2506

Compute a mapping function for each TSMS based on a respective characteristics of the TSMS ⌐ 2508

Process each TSMS with the respective mapping function ⌐ 2510

Present the plurality of processed TSMS on a presentation device of a user ⌐ 2512

FIG. 25

## 2600

Configure an access-point (AP) device of a wireless network based on a wireless protocol by a first particular client device of the wireless network to perform a sensing-by-proxy (SBP) procedure selectively — 2602

Configure the first particular client device as a SBP-initiator device for the SBP procedure — 2604

Configure the AP device as a SBP-responder device for the SBP procedure — 2606

Perform the SBP procedure selectively by the AP device based on the wireless protocol by configuring, on behalf of the first particular client device, a number of second particular client devices of the wireless network to perform a wireless sensing procedure, where the AP device functions as a sensing-initiator device and all the second particular client devices function as sensing-responder devices in the wireless sensing procedure — 2608

FIG. 26

EP 4 295 760 A1

EP 4 295 760 A1

FIG. 27A

FIG. 27B

FIG. 28A

FIG. 28B

EP 4 295 760 A1

CSI time
series

$H(t,f)$

Feature
extraction

2910

$f_t$

Movement
detection

2920

$d_t$

Movement
Localization

2930

$l_t$

2900

FIG. 29

FIG. 30

EP 4 295 760 A1

FIG. 31A

FIG. 31B

FIG. 32A

FIG. 32B

FIG. 32C

FIG. 32D

EP 4 295 760 A1

FIG. 33A

FIG. 33B

EP 4 295 760 A1

3400

Form at least two device pairs in a venue having a number of objects each undergoing a respective motion, each device pair comprising a first wireless device and a second wireless device — 3402

Obtain a respective time series of channel information (TSCI) and a respective motion information (MI) from each device pair — 3404

Detect and monitor motion of a first object in a first sensing task based on a first MI computed based on a first TSCI associated with a first device pair — 3406

Detect and monitor motion of a second object in a second sensing task based on a second MI computed based on a second TSCI associated with a second device pair — 3408

Compute a correlation score based at least partially on: the first TSCI, the second TSCI, the first MI and the second MI — 3410

Detect the first object and the second object as a same object when the correlation score is greater than a first threshold — 3412

Detect the first object and the second object as two different objects when the correlation score is less than a second threshold — 3414

FIG. 34

EP 4 295 760 A1

Obtain a respective time series of channel information (TSCI) and a respective motion information (MI) from each device pair

3404

3510

Transmit, by the first wireless device of the device pair, a respective wireless signal

3520

Receive, by the second wireless device of the device pair, the respective wireless signal through a respective wireless multipath channel of the venue, the received wireless signal being different from the transmitted wireless signal due to the respective wireless multipath channel and the motions of the number of objects in the venue

3530

Obtain a respective TSCI of the respective wireless multipath channel based on the received wireless signal

3540

Compute a respective MI based on the TSCI

3550

Perform a respective sensing task based on the respective MI and the respective TSCI

FIG. 35

Europäisches
Patentamt

European
Patent Office

Office européen
des brevets

# EUROPEAN SEARCH REPORT

Application Number

EP 23 18 0767

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2019/166030 A1 (CHEN CHEN [US] ET AL) 30 May 2019 (2019-05-30) * paragraphs [0104] – [0111], [0156], [0157], [0326] – [0353], [0450]; claims 1-30; figures 2,3,13,20,25,27 * | 1-15 | INV. A61B5/0507 A61B5/11 A61B5/00 G01S7/41 G01S13/00 |
| A | ZHENG YUE ET AL: "Combating Cross-Technology Interference for Robust Wireless Sensing with COTS WiFi", 2018 27TH INTERNATIONAL CONFERENCE ON COMPUTER COMMUNICATION AND NETWORKS (ICCCN), IEEE, 30 July 2018 (2018-07-30), pages 1-9, XP033416590, DOI: 10.1109/ICCCN.2018.8487431 [retrieved on 2018-10-09] * the whole document * | 1-15 | G01S13/42 G01S13/50 G01S13/66 G01S13/88 ADD. G01S13/46 |
| A | CN 110 013 252 A (UNIV BEIJING POSTS & TELECOMM) 16 July 2019 (2019-07-16) * the whole document * | 1-15 | |
| A | CN 111 046 084 A (UNIV CHONGQING) 21 April 2020 (2020-04-21) * paragraphs [0005] – [0054] * | 1-15 | TECHNICAL FIELDS SEARCHED (IPC) A61B G01S |
| A | ZHANG HAO ET AL: "Que-Fi: A Wi-Fi Deep-Learning-Based Queuing People Counting", IEEE SYSTEMS JOURNAL, IEEE, US, vol. 15, no. 2, 25 May 2020 (2020-05-25), pages 2926-2937, XP011859002, ISSN: 1932-8184, DOI: 10.1109/JSYST.2020.2994062 [retrieved on 2021-06-04] * the whole document * | 1-15 | |

The present search report has been drawn up for all claims

| Place of search | Date of completion of the search | Examiner |
|---|---|---|
| Munich | 19 October 2023 | Reeck, Guido |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

............................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 18 0767

This annex lists the patent family members relating to the patent documents cited in the above-mentioned European search report.
The members are as contained in the European Patent Office EDP file on
The European Patent Office is in no way liable for these particulars which are merely given for the purpose of information.

19-10-2023

| Patent document cited in search report | Publication date | Patent family member(s) | Publication date |
|---|---|---|---|
| US 2019166030 A1 | 30-05-2019 | NONE | |
| CN 110013252 A | 16-07-2019 | NONE | |
| CN 111046084 A | 21-04-2020 | NONE | |

EPO FORM P0459

For more details about this annex : see Official Journal of the European Patent Office, No. 12/82